# EUROPEAN PATENT APPLICATION

(11) **EP 4 600 243 A2**
(43) Date of publication of application: **13.08.2025**
(21) Application number: 23874417.1
(22) Date of filing: 06.10.2023
(51) Int. Cl.: C07D 215/42, A61K 31/496, A61K 31/5377, A61K 31/4709, A61K 31/444, A61K 31/502, A61P 35/00, C07D 471/04, C07D 491/107, C07D 401/04

(54) **NOVEL BICYCLIC HETEROCYCLYL COMPOUNDS AND USE THEREOF**

(30) Priority: 07.10.2022 KR 20220129102
(71) Applicant: Jeil Pharmaceutical Co., Ltd., Seoul 06543 (KR)
(72) Inventor: LEE, Chang-Seok, Yongin-si Gyeonggi-do 17172 (KR); KIM, Myeong-Seop, Yongin-si Gyeonggi-do 17172 (KR); KIM, Jinju, Yongin-si Gyeonggi-do 17172 (KR); KIM, Jiyoon, Yongin-si Gyeonggi-do 17172 (KR); KU, Jinmo, Yongin-si Gyeonggi-do 17172 (KR); KIM, Hyungguk, Yongin-si Gyeonggi-do 17172 (KR); LEE, Yang-Ha, Yongin-si Gyeonggi-do 17172 (KR)
(74) Representative: HGF
(86) International application number: PCT/IB2023/060044
(87) International publication number: WO 2024/075070

(57) **Abstract**

The present invention relates to a compound represented by chemical formula 1 as an SOS1 inhibitor, optical isomer thereof, stereoisomer thereof, solvate thereof, isotopic variant thereof, tautomer thereof, or pharmaceutically acceptable salt thereof; and to a pharmaceutical composition comprising same as an active ingredient. The compound, optical isomer thereof, stereoisomer thereof, solvate thereof, isotopic variant thereof, tautomer thereof, or pharmaceutically acceptable salt thereof, and the pharmaceutical composition comprising same as an active ingredient can be effectively used for preventing, ameliorating, or treating diseases related to SOS1 activity.

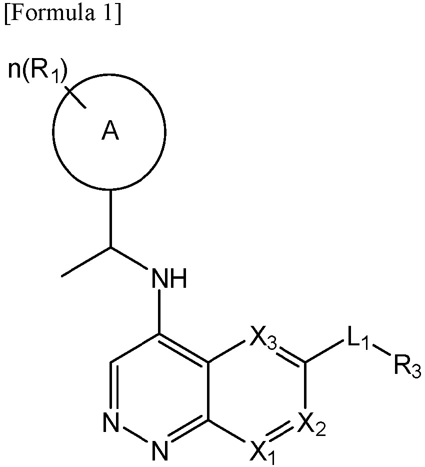

## Description

### Technical Field

The present invention relates to a bicyclic heterocyclyl compound showing SOS1 inhibitory activity, and more specifically, to a novel bicyclic heterocyclyl compound, which shows preventive, ameliorative, or therapeutic activity against various carcinomas due to excellent SOS1 inhibitory activity, optical isomer thereof, stereoisomer thereof, solvate thereof, isotopic variant thereof, tautomer thereof, or pharmaceutically acceptable salt thereof, a use thereof, and a pharmaceutical composition including the same.

### Background

Renin angiotensin system (RAS) family proteins include KRAS, NRAS, and HRAS, and mutations thereof are small GTPases present in cells in a GTP-bound or GDP-bound state. RAS family proteins have weak intrinsic GTPase activity and slow nucleotide exchange rates. Binding of guanine nucleotide exchange factors (GEF) such as Son of Sevenless 1 (SOS1) promotes GDP release from RAS proteins and enables GTP binding. RAS family proteins are active in the GTP-bound state and are associated with effector proteins such as C-RAF and PI3K. These pathways affect a variety of cellular processes, such as proliferation, survival, metabolism, motility, angiogenesis, immunity, and growth. Cancer-associated mutations in RAS family proteins inhibit GAP-induced GTPase activity to increase GTP-bound/active RAS family proteins. It leads to continued activation of effector pathways (for example, MEK/ERK, PI3K/AKT/mTOR, RalGDS pathways) downstream of the RAS family proteins. KRAS mutations (for example, amino acids G12, G13, Q61, A146) are found in a variety of human cancers including lung cancer, colorectal cancer, and pancreatic cancer.

SOS1 is a human homologue of the initially discovered Drosophila protein Son of Sevenless (SOS). SOS1 has two sites binding to RAS family proteins, which are a catalytic site binding to GDP-bound RAS family proteins and an allosteric site binding to GTP-bound RAS family proteins. It was found that a loss of SOS1 reduces the proliferation rate and survival of tumor cells with the KRAS mutations, and has no effect observed on KRAS wild-type cell lines. Additionally, SOS1 modifications are implicated in cancer. The SOS1 mutation is found in embryo rhabdomyosarcoma, Sertoli cell testicular tumor, granular cell tumor of the skin, and lung adenocarcinoma. Meanwhile, overexpression of SOS1 is found in bladder cancer and prostate cancer. In addition to cancer, the genetic SOS1 mutation is associated with the pathogenesis of RAS diseases (RASopathy) such as noonan syndrome (NS), cardio-facio-cutaneous (CFC) syndrome, and hereditary gingival fibromatosis type 1.

With regard to SOS1 inhibition, many efforts have been made so far to discover and optimize a complex which targets an effector binding site of RAS or a catalytic binding site of SOS1 (Lu et al., ChemMedChem. 2016, 11(8):814-21), but have failed to meet expectations.

Meanwhile, a small activating molecule has been recently found, which binds to a lipophilic pocket of SOS1 in proximity to the RAS binding site (Burns et al., Proc. Natl. Acad. Sci. 2014, 111(9):3401-6). However, binding of these molecules appears to increase nucleotide exchange, thus activating RAS instead of inactivating the same.

A number of fragmented molecules have been found in an effort to stabilize a protein-protein interaction of RAS family proteins and SOS1, and to prevent reloading of GTP-bound RAS family proteins (Winter et al., J. Med. Chem. 2015, 58(5):2265-74). However, the reversible binding of fragmented molecules to SOS1 was not interpreted as a measurable effect in nucleotide exchange, but only a weak effect has been identified by small molecules covalently bound to the RAS.

In addition, in recent years, research has been conducted by combining a reasonable design and a screening platform to develop SOS1 small molecule inhibitors (Evelyn et al., Chem. Biol. 2014, 21(12):1618-28; Evelyn et al., J. Biol. Chem. 2015, 290(20):12879-98; Zheng et al., WO 2016/077793). Although compounds with only a slight inhibitory effect on SOS1 have been identified, but they have had only a weak effect on guanine nucleotide exchange and regulation of cell signaling (for example, ERK phosphorylation).

Accordingly, there is a demand for a new SOS1 inhibitor capable of effectively inhibiting SOS1.

### Related Art References

### Patent Documents

(Patent Document 1) International Publication WO 2016/077793

### Non-Patent Documents

(Non-Patent Document 1) Lu et al., ChemMedChem. 2016, 11(8):814-21
(Non-Patent Document 2) Burns et al., Proc. Natl. Acad. Sci. 2014, 111(9):3401-6
(Non-Patent Document 3) Winter et al., J. Med. Chem. 2015, 58(5):2265-74
(Non-Patent Document 4) Evelyn et al., Chem. Biol. 2014, 21(12):1618-28
(Non-Patent Document 5) Evelyn et al., J. Biol. Chem. 2015, 290(20):12879-98

### Detailed Description of the Invention

### Technical Problem

An object of the present invention is to provide a novel compound based on bicyclic heterocyclyl having excellent SOS1 inhibitory activity, optical isomer thereof, stereoisomer thereof, solvate thereof, isotopic variant thereof, tautomer thereof, or pharmaceutically acceptable salt thereof.

In addition, another object of the present invention is to provide a pharmaceutical composition including the compound based on bicyclic heterocyclyl, optical isomer thereof, stereoisomer thereof, solvate thereof, isotopic variant thereof, tautomer thereof, or pharmaceutically acceptable salt thereof as an active ingredient.

Another object of the present invention is to provide a pharmaceutical composition including the compound based on bicyclic heterocyclyl, optical isomer thereof, stereoisomer thereof, solvate thereof, isotopic variant thereof, tautomer thereof, or pharmaceutically acceptable salt thereof as an active ingredient for preventing, ameliorating, or treating various diseases caused by SOS1 activity.

Still another object of the present invention is to provide a use of the compound based on bicyclic heterocyclyl, optical isomer thereof, stereoisomer thereof, solvate thereof, isotopic variant thereof, tautomer thereof, or pharmaceutically acceptable salt thereof for preventing, ameliorating, or treating various diseases caused by SOS1 activity.

Still another object of the present invention is to provide a use of the compound based on bicyclic heterocyclyl, optical isomer thereof, stereoisomer thereof, solvate thereof, isotopic variant thereof, tautomer thereof, or pharmaceutically acceptable salt thereof in the preparation of a medicament for preventing, ameliorating, or treating various diseases caused by SOS1 activity.

Still another object of the present invention is to provide a method for preventing, ameliorating, or treating various diseases caused by SOS1 activity by administering the compound based on bicyclic heterocyclyl, optical isomer thereof, stereoisomer thereof, solvate thereof, isotopic variant thereof, tautomer thereof, or pharmaceutically acceptable salt thereof, or the pharmaceutical composition including the same.

### Technical Solution

Hereinafter, the present invention will be described in more detail. All the combinations of various elements disclosed in the present invention fall within the scope of the present invention. In addition, it cannot be seen that the scope of the present invention is limited to the specific description below.

This research on the present invention was conducted with the support of the Korea Drug Development Fund funded by the Ministry of Science and ICT, the Ministry of Trade, Industry and Energy, and the Ministry of Health and Welfare of the Republic of Korea (Task Identification Number: RS-2023-00217674). The task identification number may be used in combination as "1711198230."

### Compound represented by formula 1

(1) The present invention provides a compound represented by formula 1 below, optical isomer thereof, stereoisomer thereof, solvate thereof, isotopic variant thereof, tautomer thereof, or pharmaceutically acceptable salt thereof. 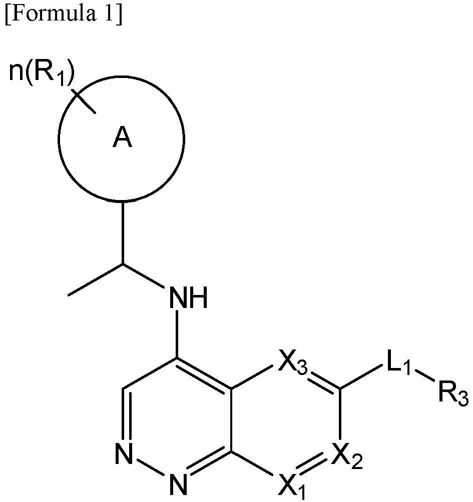 In above formula 1,
   A is C₃₋₈ cycloalkyl, C₃₋₈ cycloalkenyl, 3- to 8-membered heterocycloalkyl including 1 to 3 heteroatoms independently selected from the group consisting of N, O, and S in a ring, 3- to 8-membered heterocycloalkenyl including 1 to 3 heteroatoms independently selected from the group consisting of N, O, and S in a ring, C₆₋₁₂ aryl, 5- to 12-membered heteroaryl including 1 to 3 heteroatoms independently selected from the group consisting of N, O, and S in a ring, 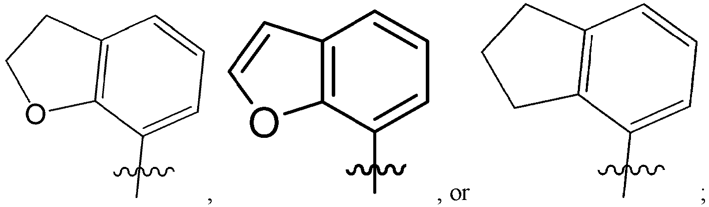
   n is 0, 1, 2, 3, or 4;
   R₁ is C₁₋₆ alkyl, C₆₋₁₂ aryl, -CF₂H, -CF₃, -CN, -OH, -NH₂, or halogen (in which when n is 2 or more, n R₁s are each independent of each other),
   at least one H of C₁₋₆ alkyl, C₆₋₁₂ aryl, -CF₂H, and -OH of the R₁ may be each independently substituted with C₁₋₆ alkyl (in which at least one H of C₁₋₆ alkyl may be each independently substituted with -OH, -O-C₁₋₆ alkyl, or -NRₐR_{b}), or halogen;
   X₁ is CH or N, X₂ is CR₂, and X₃ is CH;
   R₂ is H, C₁₋₆ alkyl, -CF₃, -O-C₁₋₆ alkyl, C₃₋₆ cycloalkyl, -O-C₃₋₆ cycloalkyl, -OH, -OCF₃, - NR_{c}R_{d}, or halogen;
   L₁ is a single bond, -(C=O)-, -(C=O)O-, -O-, -(C=O)NRₑ-, -NRₑ-, -NRₑ(C=O)-, -NRₑSO₂-, or -NRₑ(C=O)NR_{f}-;
   R₃ is H, -OH, C₁₋₆ alkyl, C₃₋₈ cycloalkyl, C₆₋₁₂ aryl, 3- to 12-membered heterocycloalkyl including 1 to 3 heteroatoms independently selected from the group consisting of N, O, and S in a ring, C₃₋₈ cycloalkenyl, 3- to 8-membered heterocycloalkenyl including 1 to 3 heteroatoms independently selected from the group consisting of N, O, and S in a ring, 5- to 12-membered heteroaryl including 1 to 3 heteroatoms independently selected from the group consisting of N, O, and S in a ring, 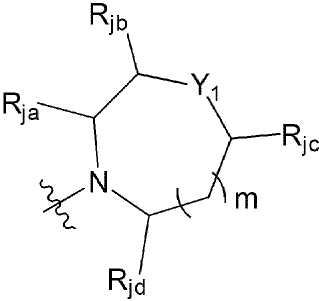 (in which m is 0 or 1, Y₁ is CH₂, NRⱼ, or O, and Rⱼₐ, R_{jb}, R_{jc}, and R_{jd} are each independently H or C₁₋₅ alkyl, provided that two selected from Rⱼₐ, R_{jb}, R_{jc}, and R_{jd} may be linked to form CH₂ or CH₂-CH₂), or 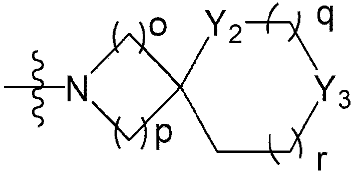 (in which o and p are each independently 1 or 2, q and r are each independently 0, 1, or 2, and Y₂ and Y₃ are each independently CH₂, NRₖ, or O),
   in the R₃, at least one -CH₂- of 3- to 12-membered heterocycloalkyl including 1 to 3 heteroatoms independently selected from the group consisting of N, O, and S in a ring, or 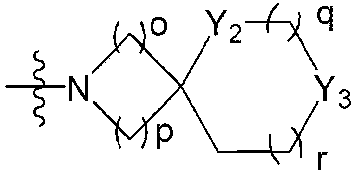 may be substituted with -(C=O)-, -SO-, or -SO₂-,
      at least one -CH₂- of C₃₋₈ cycloalkyl of the R₃ may be substituted with -SO₂-,
      at least one H of the R₃ may be each independently substituted with C₁₋₆ alkyl, -OH, halogen, or -L₂-R₄;
      L₂ is a single bond, C₁₋₆ alkylene, -O-, -(C=O)-, -SO₂-, -(C=O)NR_{g}-, or -NR_{g}(C=O)-;
      R₄ is H, C₁₋₆ alkyl, -O-C₁₋₆ alkyl, C₃₋₈ cycloalkyl, 3- to 8-membered heterocycloalkyl including 1 to 3 heteroatoms independently selected from the group consisting of N, O, and S in a ring, C₆₋₁₂ aryl, 5- to 12-membered heteroaryl including 1 to 3 heteroatoms independently selected from the group consisting of N, O, and S in a ring, -NRₕRᵢ, -CF₃, -CF₂H, -OH, or halogen,
      at least one H of the R₄ may be each independently substituted with C₁₋₆ alkyl, -OH, -O-C₁₋₆ alkyl, -NRₘRₙ, or halogen; and
      Rₐ, R_{b}, R_{c}, R_{d}, Rₑ, R_{f}, R_{g}, Rₕ, Rᵢ, Rⱼ, Rₖ, Rₘ, and Rₙ are each independently H or C₁₋₆ alkyl.

In the present invention, a resonance structure such as the compound represented by formula 1 may be regarded to be identical to a structure represented by formulas 1a and 1b below.

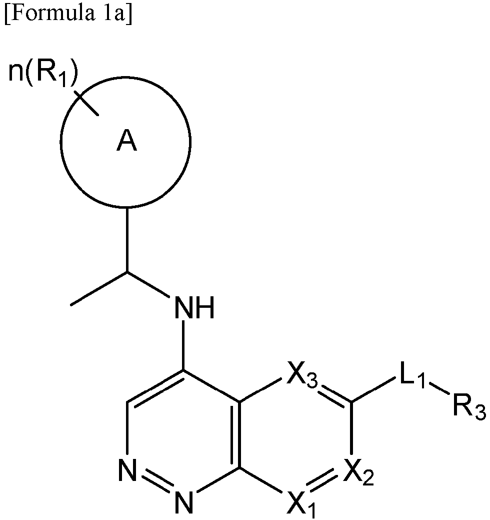

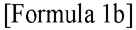

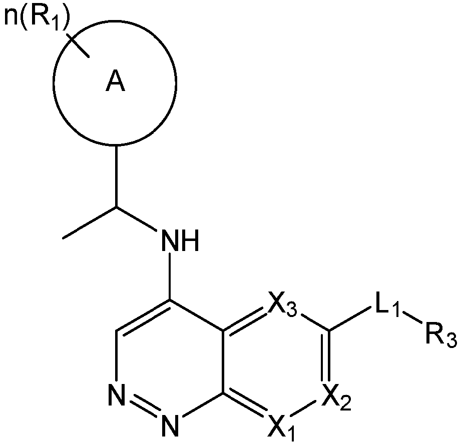

In the above, the resonance structure defined in formula 1 has been exemplarily described, but is not limited thereto.

In one embodiment, in above formula 1, n may be 0, 1, 2, 3, or 4, and preferably 1, 2, or 3.

In one embodiment, when the n is 2 or more, n R₁s may be are each independent of each other. In other words, n R₁s may be the same substituent as each other or different substituents from each other. For example, when n is 2, one R₁ may be one selected from C₁₋₆ alkyl, C₆₋₁₂ aryl, -CF₂H, -CF₃, -CN, -OH, and halogen, and the other R₁ may be one selected from C₁₋₆ alkyl, C₆₋₁₂ aryl, -CF₂H, -CF₃, -CN, -OH, and halogen.

In one embodiment, when the A is aryl, the n may be 2 or more, preferably 2. When the A is heteroaryl, the n may be 1 or more, preferably 1, but is not limited thereto.

In one embodiment, when the L₁ is a linker and L₁ is a single bond, it may refer to a structure in which the R₃ is directly linked to a parent core structure such as 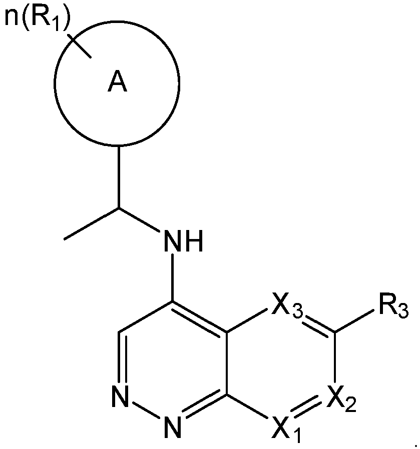 In other words, in the present invention, when L₁ is a single bond, it may mean that -L₁-R₃ is substantially -R₃. In the present specification, when L₁ is a single bond, L₁ may be represented by being absent or null.

In one embodiment, at least one H of the R₃ may be each independently substituted with L₂-R₄, in which when L₂ is a linker and L₂ is a single bond, R₄ may be substituted with at least one H of R₃, thus referring to a structure directly linked to R₃.

In one embodiment, in above formula 1, when A is aryl, n may be 2, and R₃ may be heterocycloalkyl including N or heterocycloalkenyl including N. In this case, the heterocycloalkyl including N or heterocycloalkenyl including N may further include one or more heteroatoms, and may not further include the same.

In one embodiment, in above formula 1, when A is heteroaryl, n may be 1, and R₃ may be heterocycloalkyl including N or heterocycloalkenyl including N. In this case, the heterocycloalkyl including N or heterocycloalkenyl including N may further include one or more heteroatoms, and may not further include the same.

In one embodiment, the 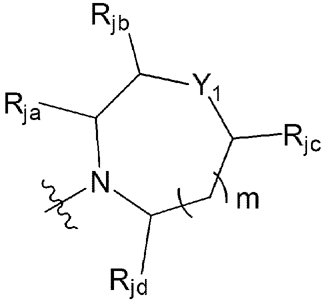 may be, for example, 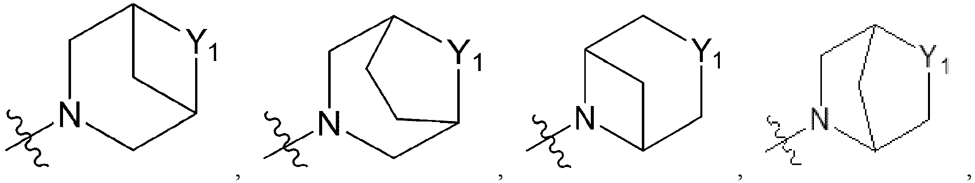 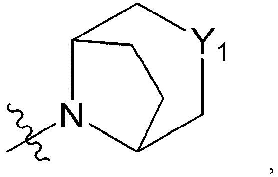 but is not limited thereto. Specifically, the 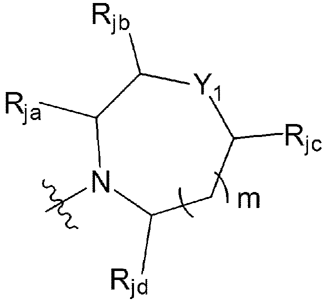 may be 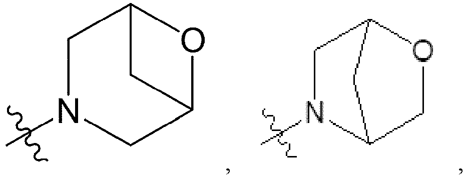 or 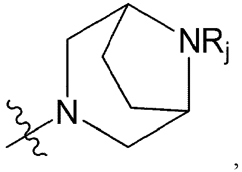 but is not limited thereto. In this case, Rⱼ may be H or C₁₋₆ alkyl.

In one embodiment, the 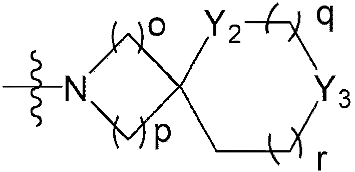 may be, for example, 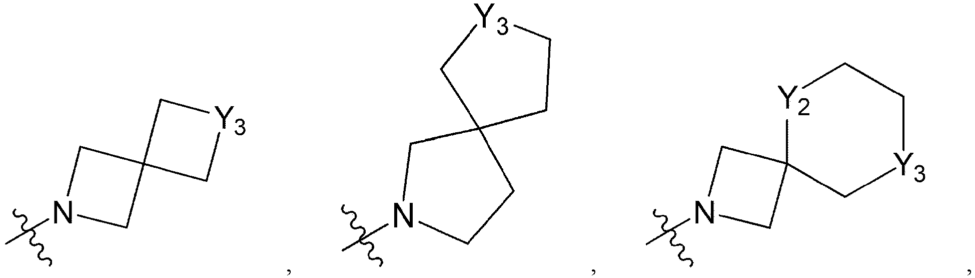 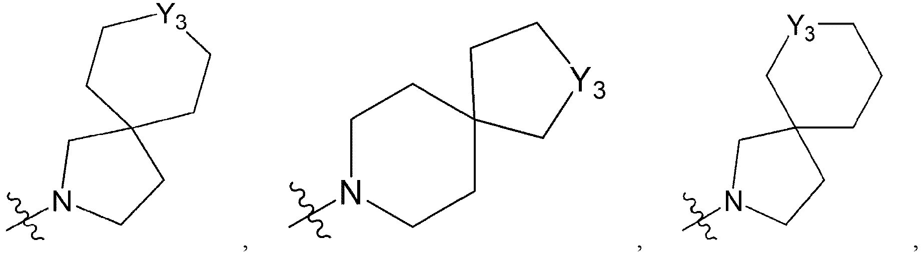 or 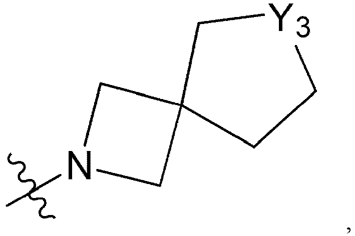 but is not limited thereto. Specifically, the 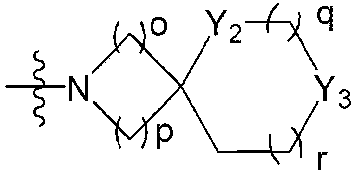 may be 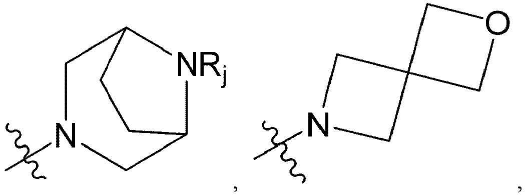 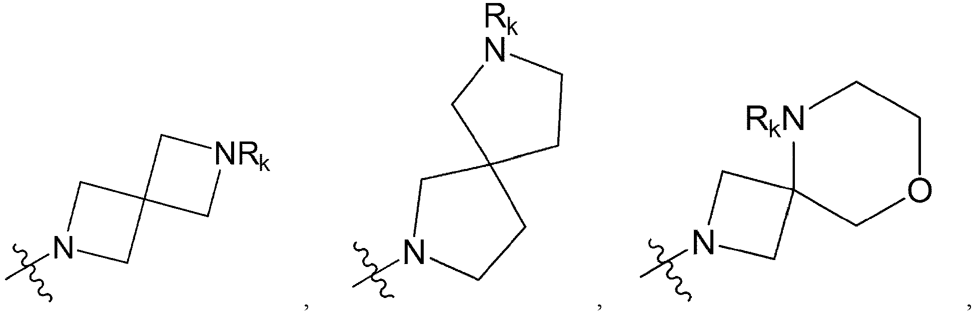 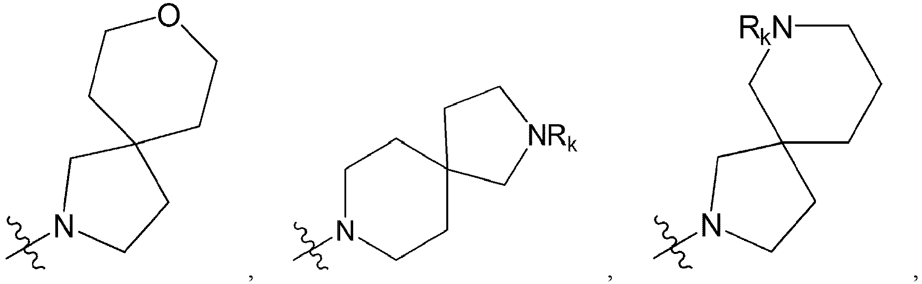 or 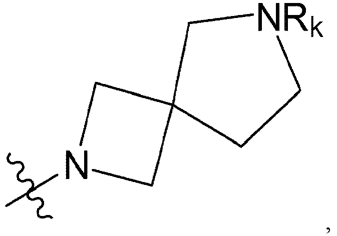 but is not limited thereto. In this case, Rₖ may be H or C₁₋₆ alkyl.

In one embodiment, in the R₃, at least one -CH₂- of 3- to 12-membered heterocycloalkyl including 1 to 3 heteroatoms independently selected from the group consisting of N, O, and S in a ring, or 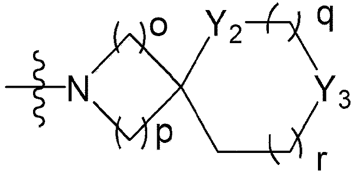 may be substituted with -(C=O)-, -SO-, or -SO₂-, and a structure in which said at least one -CH₂- is substituted with -(C=O)- may be, for example, 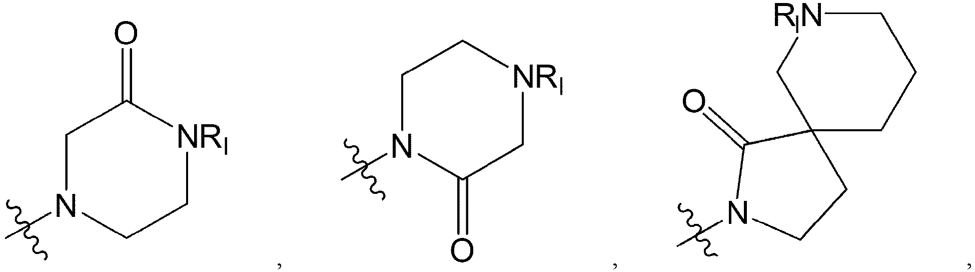 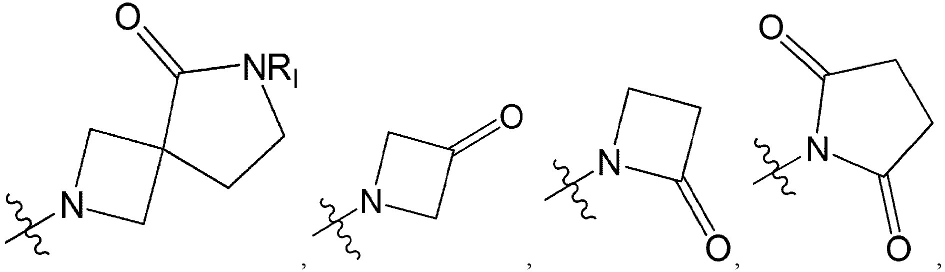 or 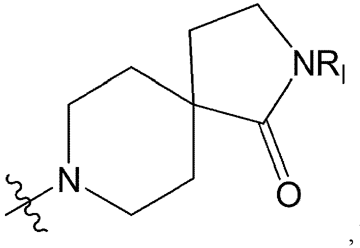 but is not limited thereto. In addition, a structure in which said at least one -CH₂- is substituted with -SO- or -SO₂- may be 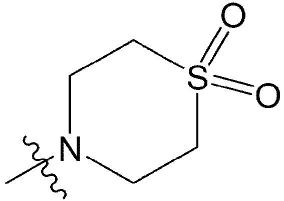 or 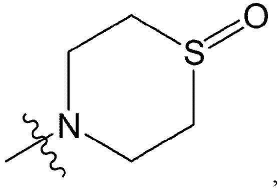 but is not limited thereto.

In one embodiment, at least one -CH₂- of C₃₋₈ cycloalkyl of the R₃ may be substituted with -SO₂-, and at least one -CH₂- of C₃₋₈ cycloalkyl of the R₃ may be substituted with -SO₂- to form a cyclic compound including a heteroatom. For example, a structure in which at least one -CH₂- of C₃₋₈ cycloalkyl of the R₃ is substituted with -SO₂- may be 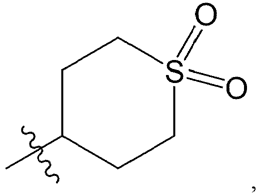 but is not limited thereto.

(2) In one embodiment, in above (1), it may be provided that in above formula 1,
A is C₆₋₁₂ aryl, 5- to 12-membered heteroaryl including 1 to 3 heteroatoms independently selected from the group consisting of N, O, and S in a ring, 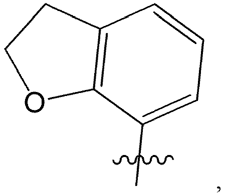 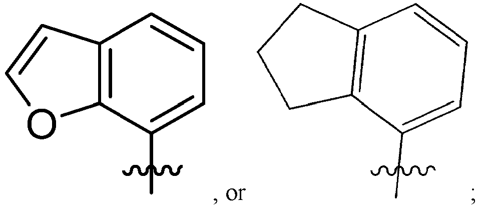
n is 1, 2, or 3;
R₁ is C₁₋₆ alkyl, C₆₋₁₂ aryl, -CF₂H, -CF₃, -CN, or halogen (in which when n is 2 or more, n R₁s are each independent of each other),
at least one H of C₁₋₆ alkyl, C₆₋₁₂ aryl, and -CF₂H of the R₁ may be each independently substituted with C₁₋₆ alkyl (in which at least one H of C₁₋₆ alkyl may be each independently substituted with -OH, -O-C₁₋₆ alkyl, or -NRₐR_{b}), or halogen;
X₁ and X₃ are each CH; and
X₂ is CR₂;
in which R₂, R₃, R₄, L₁, L₂, Y₁, Y₂, Y₃, m, o, p, q, r, Rⱼₐ, R_{jb}, R_{jc}, R_{jd}, Rₐ, R_{b}, R_{c}, R_{d}, Rₑ, R_{f}, R_{g}, Rₕ, Rᵢ, Rⱼ, Rₖ, Rₘ, and Rₙ are each the same as defined in above (1).

(3) In one embodiment, in above (1) or (2), it may be provided that in above formula 1,
A is C₆₋₁₂ aryl;
n is 1 or 2;
R₁ is each independently C₁₋₆ alkyl, -CF₂H, -CF₃, -CN, or halogen;
X₁, X₂, and X₃ are each CH;
L₁ is a single bond or -NRₑ-;
R₃ is 3- to 12-membered heterocycloalkyl including 1 to 3 heteroatoms independently selected from the group consisting of N, O, and S in a ring, C₃₋₈ cycloalkenyl, 3- to 8-membered heterocycloalkenyl including 1 to 3 heteroatoms independently selected from the group consisting of N, O, and S in a ring, or 5- to 12-membered heteroaryl including 1 to 3 heteroatoms independently selected from the group consisting of N, O, and S in a ring, in which said heterocycloalkyl includes 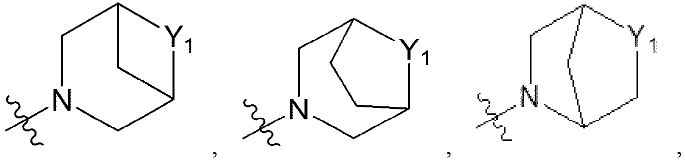 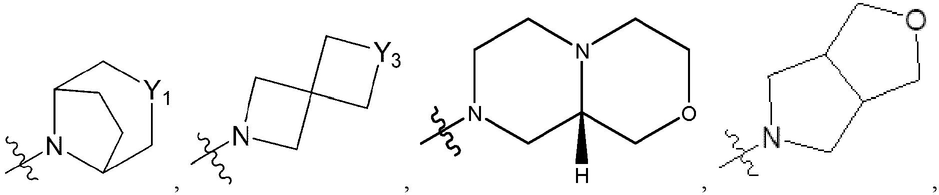 or 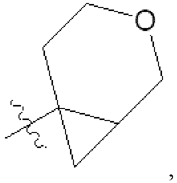 in which Y₁ is O, and Y₃ is each independently CH₂, NRₖ, or O,
   at least one H of said R₃ may be each independently substituted with C₁₋₆ alkyl, -OH, halogen, or -L₂-R₄;
   L₂ is a single bond, C₁₋₆ alkylene, -O-, -(C=O)-, -(C=O)NR_{g}-, or -NR_{g}(C=O)-;
   R₄ is H, -OH, C₁₋₆ alkyl, -O-C₁₋₆ alkyl, C₃₋₈ cycloalkyl, 3- to 8-membered heterocycloalkyl including 1 to 3 heteroatoms independently selected from the group consisting of N, O, and S in a ring, -NRₕRᵢ, or halogen,
   at least one H of the R₄ may be each independently substituted with -OH;
   in which Rₑ, R_{g}, Rₕ, Rᵢ, and Rₖ are each the same as defined in above (1).

(4) In one embodiment, in any one of above (1) to (3), it may be provided that in above formula 1,
A is C₆₋₁₂ aryl;
n is 2;
R₁ is each independently C₁₋₆ alkyl, -CF₂H, -CF₃, -CN, or halogen;
X₁, X₂, and X₃ are each CH;
L₁ is a single bond or -NRₑ-;
R₃ is 3- to 12-membered heterocycloalkyl including 1 to 3 heteroatoms independently selected from the group consisting of N, O, and S in a ring, or 3- to 8-membered heterocycloalkenyl including 1 to 3 heteroatoms independently selected from the group consisting of N, O, and S in a ring, in which the heterocycloalkyl includes 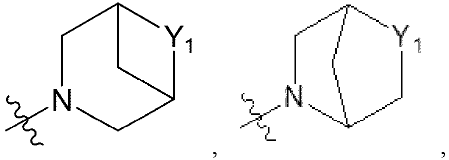 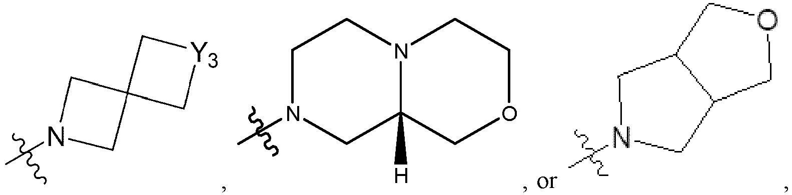 in which Y₁ is O, and Y₃ is each independently CH₂,
   at least one H of the R₃ may be each independently substituted with C₁₋₆ alkyl, -OH, or - L₂-R₄;
   L₂ is a single bond, -(C=O)-, or -NR_{g}(C=O)-;
   R₄ is H, -OH, C₁₋₆ alkyl, C₃₋₈ cycloalkyl, 3- to 8-membered heterocycloalkyl including 1 to 3 heteroatoms independently selected from the group consisting of N, O, and S in a ring, or - NRₕRᵢ,
   at least one H of the R₄ may be each independently substituted with -OH;
   in which Rₑ, R_{g}, Rₕ, and Rᵢ are each the same as defined in above (1).

In one embodiment, in above (1) to (4), the heterocycloalkyl or heterocycloalkenyl of said R₃ may include N. In this case, the heterocycloalkyl or heterocycloalkenyl may be heterocycloalkyl or heterocycloalkenyl in which an atom linked to a parent core structure is N, or may be heterocycloalkyl or heterocycloalkenyl including N in a ring. At this time, the heterocycloalkyl or heterocycloalkenyl may include at least one N in a ring, and may be heterocycloalkyl or heterocycloalkenyl including 0 to 2 heteroatoms (S or O) other than N in the ring, but is not limited thereto.

In the present invention, "Cm-Cn" (in which m and n are each independently an integer of 1 or more) may mean the number of carbons and, for example, "C1-C5 alkyl" may represent alkyl having 1 to 5 carbon atoms.

In the present invention, "alkyl" may mean a linear or branched saturated hydrocarbon group. In the present invention, alkyl may have 1 to 5 carbon atoms. In one embodiment, alkyl may have 1 to 3 carbon atoms. Examples of alkyl may include methyl, ethyl, n-propyl, isopropyl, n-butyl, sec-butyl, tert-butyl, isobutyl, n-pentyl, sec-pentyl, tert-pentyl, isopentyl, sec-isopentyl, neo-pentyl, and the like. In the present invention, alkyl may refer to being unsubstituted, or may mean that at least one H of alkyl is optionally substituted. A substituent in which at least one H of alkyl may be substituted may include, without limitation, a functional group, as long as at least one H of said alkyl is substitutable. For example, said substituent may be one which is defined in the compound represented by formula 1 of the present invention, but is not necessarily limited thereto.

In the present invention, "cycloalkyl" may mean a saturated hydrocarbon ring having 3 or more carbon atoms, and the saturated hydrocarbon ring may include both monocyclic and polycyclic structures. The polycyclic structure may include multi-ring structures such as spiro, bridged, and fused ring structures. Cycloalkyl may be a saturated hydrocarbon ring having 3 to 12 carbon atoms. Examples of cycloalkyl may include at least one selected from cyclobutyl, cyclopentyl, cyclohexyl, cycloheptyl, cyclooctyl, and the like, but are not limited thereto. In the present invention, cycloalkyl may refer to being unsubstituted, or may mean that at least one H of cycloalkyl is optionally substituted. A substituent in which at least one H of cycloalkyl may be substituted may include, without limitation, a functional group, as long as at least one H of said cycloalkyl is substitutable. For example, said substituent may be one which is defined in the compound represented by formula 1 of the present invention, but is not necessarily limited thereto.

In the present invention, "cycloalkenyl" may mean an unsaturated hydrocarbon ring having 3 or more carbon atoms including one or more double bonds, and the hydrocarbon ring may include both monocyclic and polycyclic structures. In other words, in the present invention, cycloalkenyl may mean a ring structure including one or more carbon-carbon double bonds in the cycloalkyl ring. In the present invention, cycloalkenyl may be an unsaturated hydrocarbon ring having 3 to 12 carbon atoms. Examples of cycloalkenyl may include at least one selected from cyclopropenyl, cyclobutenyl, cyclopentenyl, cyclohexenyl, and the like, but are not limited thereto. In the present invention, cycloalkenyl may refer to being unsubstituted, or may mean that at least one H of cycloalkenyl is optionally substituted. A substituent in which at least one H of cycloalkenyl may be substituted may include, without limitation, a functional group, as long as at least one H of said cycloalkenyl is substitutable. For example, said substituent may be one which is defined in the compound represented by formula 1 of the present invention, but is not necessarily limited thereto.

In the present invention, "heterocycloalkyl" may mean a cyclic functional group in which at least one or more carbon atoms constituting the ring are substituted with a heteroatom. Examples of said heteroatom may include nitrogen (N), oxygen (O), or sulfur (S). In this case, the heteroatoms included in the ring of heterocycloalkyl may be one type or two or more types, one or two or more of one type of heteroatom may be included therein, and at least one or more of the two or more types of heteroatoms may be included therein, respectively. In the present invention, in the heterocycloalkyl, an atom linked to a parent-nucleus structure may be a carbon atom and a heteroatom may be included in a ring, or an atom linked to a parent-nucleus structure may be a heteroatom (in which, when there are two or more heteroatoms, a heteroatom may also be included in the ring). Heterocycloalkyl may be a 3- to 12-membered ring. Heterocycloalkyl may include both monocyclic and polycyclic structures. Examples of heterocycloalkyl may include oxiranyl, oxetanyl, morpholinyl, pyrrolidinyl, piperidinyl, piperazinyl, tetrahydrofuranyl, tetrahydrothiophenyl, tetrahydropyranyl, tetrahydrothiopyranyl, azepanyl, and the like, but are not limited thereto. In the present invention, heterocycloalkyl may refer to being unsubstituted, or may mean that at least one H of heterocycloalkyl is optionally substituted. A substituent in which at least one H of heterocycloalkyl may be substituted may include, without limitation, a functional group, as long as at least one H of said heterocycloalkyl is substitutable. For example, said substituent may be one which is defined in the compound represented by formula 1 of the present invention, but is not necessarily limited thereto.

In the present invention, the polycyclic structure may include multi-ring structures such as spiro, bridged, and fused ring structures.

The spiro ring structure may refer to a ring structure in which two rings are composed of one common atom. In the compound of the present invention, a spiro heterocycloalkyl ring structure may be, for example, 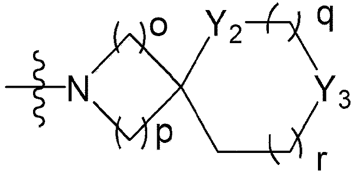 (in which o and p are each independently 1 or 2, q and r are each independently 0, 1, or 2, and Y₂ and Y₃ are each independently CH₂, NRₖ, or O), but is not limited thereto. Specifically, the spiro heterocycloalkyl ring structure may be 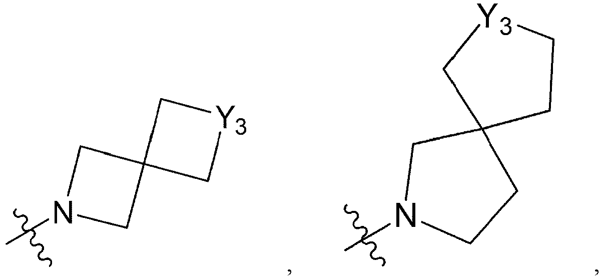 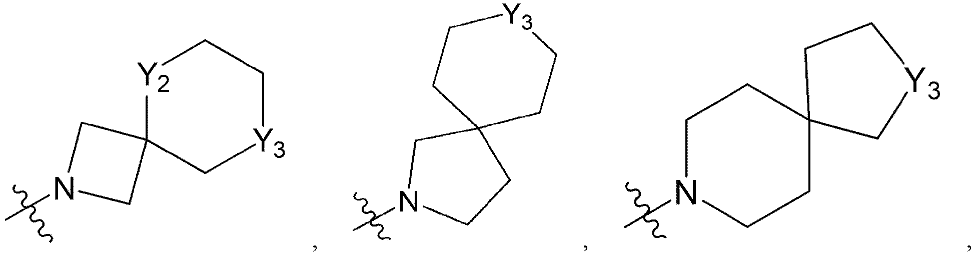 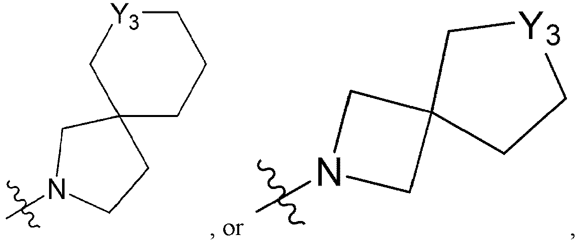 but is not limited thereto.

The bridged ring structure may refer to a hydrocarbon ring structure in which two or more rings share one or more pairs of carbon atoms. In the compound of the present invention, a bridged heterocycloalkyl ring structure may be, for example, 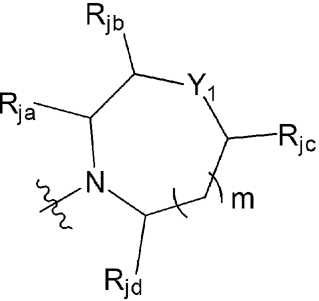 (in which m is 0 or 1, Y₁ is NRⱼ or O, and Rⱼₐ, Rⱼₐ, R_{jc} and R_{jd} are each independently H or C₁₋₅ alkyl, provided that two selected from Rⱼₐ, Rⱼₐ, R_{jc} and R_{jd} may be linked to form CH₂ or CH₂-CH₂), but is not limited thereto. Specifically, in the present invention, the bridged ring structure may be 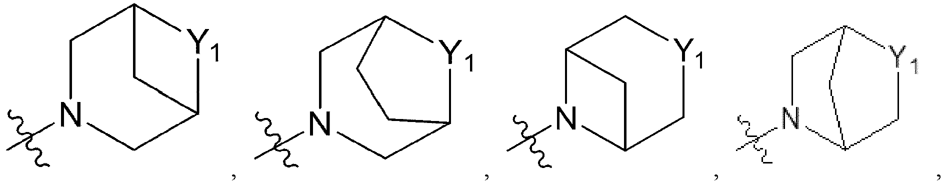 or 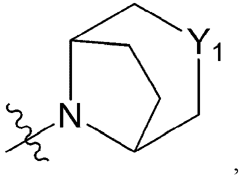 but is not limited thereto.

The fused ring structure may be, for example, a structure such as 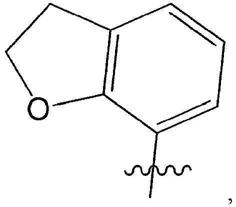 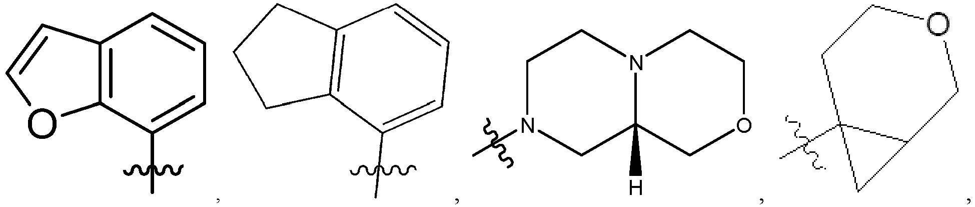 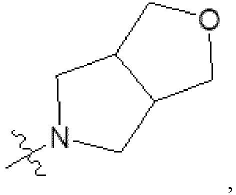 etc., but is not limited thereto.

In the present invention, "heterocycloalkenyl" may mean a cyclic functional group in which at least one or more carbon atoms constituting the cycloalkenyl ring are substituted with a heteroatom. Examples of said heteroatom may include nitrogen (N), oxygen (O), or sulfur (S). In this case, the heteroatoms included in the ring of heterocycloalkenyl may be one type or two or more types, one or two or more of one type of heteroatom may be included therein, and at least one or more of the two or more types of heteroatoms may be included therein, respectively. In the present invention, in the heterocycloalkenyl, an atom linked to a parent-nucleus structure may be a carbon atom and a heteroatom may be included in a ring, or an atom linked to a parent-nucleus structure may be a heteroatom (in which, when there are two or more heteroatoms, a heteroatom may also be included in the ring). Heterocycloalkenyl may be a 3- to 12-membered ring. Examples of heterocycloalkyl may include 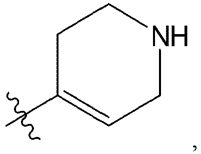 but are not limited thereto. In the present invention, heterocycloalkenyl may refer to being unsubstituted, or may mean that at least one H of heterocycloalkenyl is optionally substituted. A substituent in which at least one H of heterocycloalkenyl may be substituted may include, without limitation, a functional group, as long as at least one H of said heterocycloalkenyl is substitutable. For example, said substituent may be one which is defined in the compound represented by formula 1 of the present invention, but is not necessarily limited thereto.

In the present invention, "aryl" may include a monocyclic aromatic or polycyclic aromatic one, and mean an aromatic hydrocarbon having 6 or more carbon atoms. Aryl may have 6 to 20 carbon atoms. For example, aryl may be phenyl, biphenyl, naphthalenyl, etc. In the present invention, aryl may refer to being unsubstituted, or may mean that at least one H of aryl is optionally substituted. A substituent in which at least one H of aryl may be substituted may include, without limitation, a functional group, as long as at least one H of said aryl is substitutable. For example, said substituent may be one which is defined in the compound represented by formula 1 of the present invention, but is not necessarily limited thereto.

In the present invention, "heteroaryl" may mean a monocyclic or polycyclic hetero ring in which at least one or more carbon atoms are substituted with nitrogen (N), oxygen (O) or sulfur (S), which are heteroatoms, in said aryl. As one example, in the present invention, heteroaryl may be a 5- to 12-membered ring, but is not limited thereto. When heteroaryl includes two or more heteroatoms, the types of heteroatoms may be the same as or different from each other. For example, a case in which heteroaryl includes two or more heteroatoms selected from nitrogen, oxygen, and sulfur may refer to various combinations such as a case of including two nitrogen atoms, a case of including one nitrogen atom and one oxygen atom, a case of including two oxygen atoms and one nitrogen atom, and the like. In addition, in the present invention, in heteroaryl, an atom linked to a parent-nucleus structure may be a carbon atom and a heteroatom may be included in a ring, or an atom linked to a parent-nucleus structure may be a heteroatom (in which, when there are two or more heteroatoms, a heteroatom may also be included in the ring). For example, examples of heteroaryl may include pyridinyl, thiophenyl, triazolyl, tetrazolyl, benzothiazolyl, benzothiophenyl, quinolinyl, indolyl, isoindolyl, benzofuranyl, benzopyrroyl, furanyl, pyrrolyl, thiazolyl, isothiazolyl, imidazolyl, pyrazolyl, oxazolyl, isoxazolyl, pyrazinyl, pyridazinyl, pyrimidinyl, isoquinolinyl, benzoxazolyl, benzoimidazolyl, dihydrobenzothiophenyl, purinyl, indolizinyl, chromenyl, pyrrolopyridinyl, pyrazolopyridinyl, thiadiazolopyridinyl, triazinyl, triazolopyrimidinyl, triazolopyridinyl, triazolopyridazinyl, indazolyl, imidazopyridinyl, imidazopyridazinyl, oxadiazolopyridinyl, benzothiadiazolyl, benzotriazolyl, benzoxadiazole, isomers thereof, and the like. In the present invention, heteroaryl may refer to being unsubstituted, or may mean that at least one H of heteroaryl is optionally substituted. A substituent in which at least one H of heteroaryl may be substituted may include, without limitation, a functional group, as long as at least one H of said heteroaryl is substitutable. For example, said substituent may be one which is defined in the compound represented by formula 1 of the present invention, but is not necessarily limited thereto.

In the present invention, alkyl, aryl, heteroaryl, cycloalkyl, cycloalkenyl, heterocycloalkyl, and heterocycloalkenyl may refer to monovalent substituents or polyvalent substituents of divalent or higher valents having chemical structures according to respective definitions. For example, "alkyl" may include a monovalent alkyl or a divalent alkyl (alkylene), and "aryl" may include a monovalent aryl or a divalent aryl (arylene).

In the present invention, in each definition of alkyl, aryl, heteroaryl, cycloalkyl, cycloalkenyl, heterocycloalkyl, and heterocycloalkenyl, "may be substituted" may mean that each of said alkyl, aryl, heteroaryl, cycloalkyl, cycloalkenyl, heterocycloalkyl, and heterocycloalkenyl may be unsubstituted or substituted. Specifically, when alkyl, aryl, heteroaryl, cycloalkyl, cycloalkenyl, heterocycloalkyl, and heterocycloalkenyl may be substituted, it may mean that alkyl, aryl, heteroaryl, cycloalkyl, cycloalkenyl, heterocycloalkyl, and heterocycloalkenyl may be unsubstituted, or at least one H of alkyl, aryl, heteroaryl, cycloalkyl, cycloalkenyl, heterocycloalkyl, and heterocycloalkenyl may be each independently substituted with a substituent, and said substituent may be available without limitation as long as the substitutent is a functional group capable of substituting at least one H. For example, in each of alkyl, aryl, heteroaryl, cycloalkyl, cycloalkenyl, heterocycloalkyl, and heterocycloalkenyl of the present invention, said substituent may be a substituent defined in the definition of alkyl, aryl, heteroaryl, cycloalkyl, cycloalkenyl, heterocycloalkyl, and heterocycloalkenyl of the compound represented by formula 1 of the present invention, but is not necessarily limited thereto.

In the present invention, "halogen" may be F, Cl, Br or I.

The compound according to the present invention, optical isomer thereof, stereoisomer thereof, solvate thereof, isotopic variant thereof, tautomer thereof, or pharmaceutically acceptable salt thereof may be the compounds shown in table 1 below.

**[Table 1]**

| N o. | Compound Structure | English Name | Korean Name |
|---|---|---|---|
| 1 | 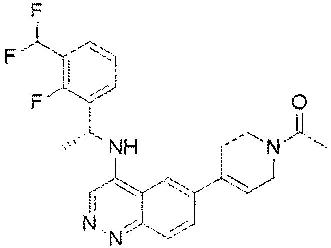 | (R)-1-(4-(4-((1-(3-(difluoromethyl)-2-fluorophenyl)ethyl)amino)cin nolin-6-yl)-5,6-dihydropyridin-1 (2H)-yl)ethanone | (R)-1-(4-(4-((1-(3-(difluoromethyl)-2-fluorophenyl)ethyl)amino)cin nolin-6-yl)-5,6-dihydropyridin-1(2H)-yl)ethanone |
| 2 | 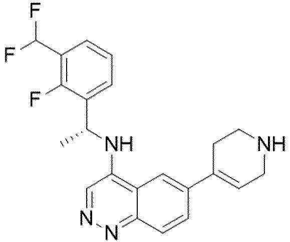 | (R)-N-(1-(3-(difluoromethyl)-2-fluorophenyl)ethyl)-6-(1,2,3,6-tetrahydropyridin-4-yl)cinnolin-4-amine | (R)-N-(1-(3-(difluoromethyl)-2-fluorophenyl)ethyl)-6-(1,2,3,6-tetrahydropyridin-4-yl)cinnolin-4-amine |
| 3 | 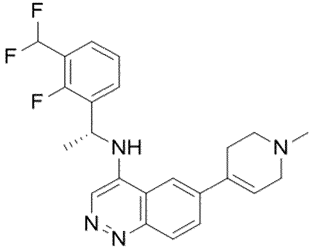 | (R)-N-(1-(3-(difluoromethyl)-2-fluorophenyl)ethyl)-6-(1-methyl-1,2,3,6-tetrahydropyridin-4-yl)cinnolin-4-amine | (R)-N-(1-(3-(difluoromethyl)-2-fluorophenyl)ethyl)-6-(1-methyl-1,2,3,6-tetrahydropyridin-4-yl)cinnolin-4-amine |
| 4 | 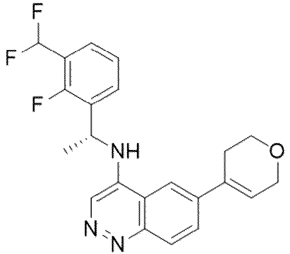 | (R)-N-(1-(3-(difluoromethyl)-2-fluorophenyl)ethyl)-6-(3,6-dihydro-2H-pyran-4-yl)cinnolin-4-amine | (R)-N-(1-(3-(difluoromethyl)-2-fluorophenyl)ethyl)-6-(3,6-dihydro-2H-pyran-4-yl)cinnolin-4-amine |
| 5 | 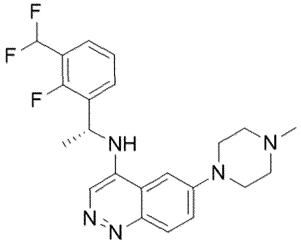 | (R)-N-(1-(3-(difluoromethyl)-2-fluorophenyl)ethyl)-6-(4-methylpiperazin-1-yl)cinnolin-4-amine | (R)-N-(1-(3-(difluoromethyl)-2-fluorophenyl)ethyl)-6-(4-methylpiperazin-1-yl)cinnolin-4-amine |
| 6 | 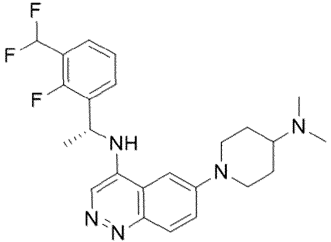 | (R)-N-(1-(3-(difluoromethyl)-2-fluorophenyl)ethyl)-6-(4-(dimethylamino)piperidin-1-yl)cinnolin-4-amine | (R)-N-(1-(3-(difluoromethyl)-2-fluorophenyl)ethyl)-6-(4-(dimethylamino)piperidin-1-yl)cinnolin-4-amine |
| 7 | 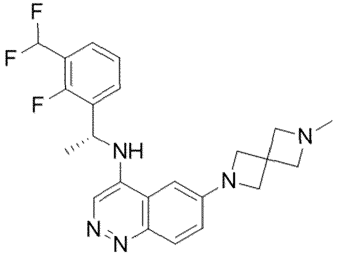 | (R)-N-(1-(3-(difluoromethyl)-2-fluorophenyl)ethyl)-6-(6-methyl-2,6-diazaspiro[3.3]heptan-2-yl)cinnolin-4-amine | (R)-N-(1-(3-(difluoromethyl)-2-fluorophenyl)ethyl)-6-(6-methyl-2,6-diazaspiro[3.3]heptan-2-yl)cinnolin-4-amine |
| 8 | 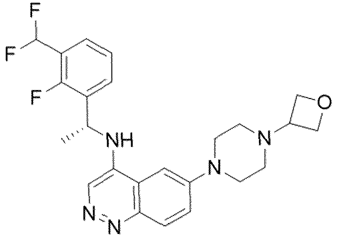 | (R)-N-(1-(3-(difluoromethyl)-2-fluorophenyl)ethyl)-6-(4-(oxetan-3-yl)piperazin-1-yl)cinnolin-4-amine | (R)-N-(1-(3-(difluoromethyl)-2-fluorophenyl)ethyl)-6-(4-(oxetan-3-yl)piperazin-1-yl)cinnolin-4-amine |
| 9 | 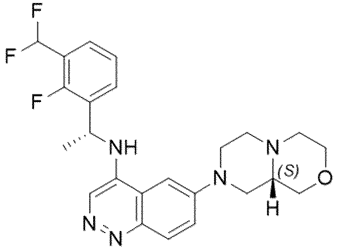 | N-((R)-1-(3-(difluoromethyl)-2-fluorophenyl)ethyl)-6-((S)-hexahydropyrazino[2,1-c][1,4]oxazin-8(1H)-yl)cinnolin-4-amine | N-((R)-1-(3-(difluoromethyl)-2-fluorophenyl)ethyl)-6-((S)-hexahydropyrazino[2,1-c][1,4]oxazin-8(1H)-yl)cinnolin-4-amine |
| 1 0 | 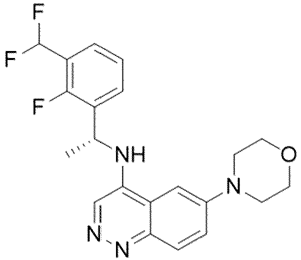 | (R)-N-(1-(3-(difluoromethyl)-2-fluorophenyl)ethyl)-6-morpholinocinnolin-4-amine | (R)-N-(1-(3-(difluoromethyl)-2-fluorophenyl)ethyl)-6-morpholinocinnolin-4-amine |
| 1 1 | 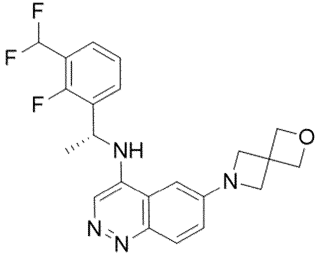 | (R)-N-(1-(3-(difluoromethyl)-2-fluorophenyl)ethyl)-6-(2-oxa-6-azaspiro[3.3]heptan-6-yl)cinnolin-4-amine | (R)-N-(1-(3-(difluoromethyl)-2-fluorophenyl)ethyl)-6-(2-oxa-6-azaspiro[3.3]heptan-6-yl)cinnolin-4-amine |
| 1 2 | 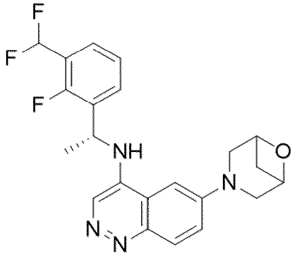 | 6-(6-oxa-3-azabicyclo[3.1.1]heptan-3-yl)-N-((R)-1-(3-(difluoromethyl)-2-fluorophenyl)ethyl)cinnolin-4-amine | 6-(6-oxa-3-azabicyclo[3.1.1]heptan-3-yl)-N-((R)-1-(3-(difluoromethyl)-2-fluorophenyl)ethyl)cinnolin-4-amine |
| 1 3 | 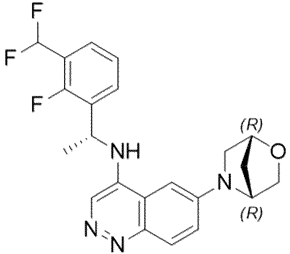 | 6-((1R,4R)-2-oxa-5-azabicyclo[2.2.1]heptan-5-yl)-N-((R)-1-(3-(difluoromethyl)-2-fluorophenyl)ethyl)cinnolin-4-amine | 6-((1R,4R)-2-oxa-5-azabicyclo[2.2.1]heptan-5-yl)-N-((R)-1-(3-(difluoromethyl)-2-fluorophenyl)ethyl)cinnolin-4-amine |
| 1 4 | 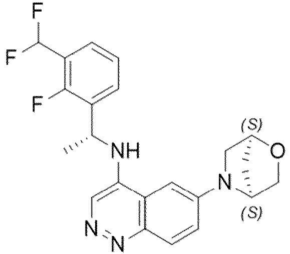 | 6-((1S,4S)-2-oxa-5-azabicyclo[2.2.1]heptan-5-yl)-N-((R)-1-(3-(difluoromethyl)-2-fluorophenyl)ethyl)cinnolin-4-amine | 6-((1S,4S)-2-oxa-5-azabicyclo[2.2.1]heptan-5-yl)-N-((R)-1-(3-(difluoromethyl)-2-fluorophenyl)ethyl)cinnolin-4-amine |
| 1 5 | 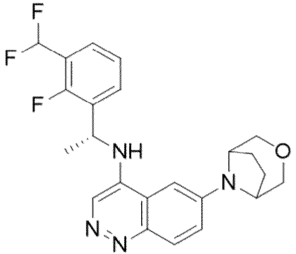 | 6-(3-oxa-8-azabicyclo[3.2.1]octan-8-yl)-N-((R)-1-(3-(difluoromethyl)-2-fluorophenyl)ethyl)cinnolin-4-amine | 6-(3-oxa-8-azabicyclo[3.2.1]octan-8-yl)-N-((R)-1-(3-(difluoromethyl)-2-fluorophenyl)ethyl)cinnolin-4-amine |
| 1 6 | 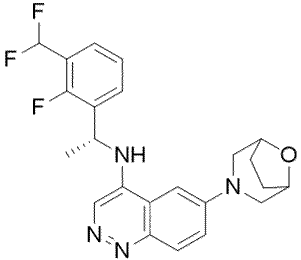 | 6-(8-oxa-3-azabicyclo[3.2.1]octan-3-yl)-N-((R)-1-(3-(difluoromethyl)-2-fluorophenyl)ethyl)cinnolin-4-amine | 6-(8-oxa-3-azabicyclo[3.2.1]octan-3-yl)-N-((R)-1-(3-(difluoromethyl)-2-fluorophenyl)ethyl)cinnolin-4-amine |
| 1 7 | 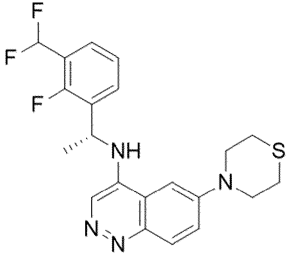 | (R)-N-(1-(3-(difluoromethyl)-2-fluorophenyl)ethyl)-6-thiomorpholinocinnolin-4-amine | (R)-N-(1-(3-(difluoromethyl)-2-fluorophenyl)ethyl)-6-thiomorpholinocinnolin-4-amine |
| 1 8 | 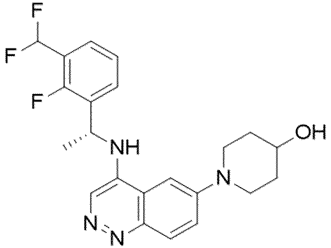 | (R)-1-(4-((1-(3-(difluoromethyl)-2-fluorophenyl)ethyl)amino)cin nolin-6-yl)piperidin-4-ol | (R)-1-(4-((1-(3-(difluoromethyl)-2-fluorophenyl)ethyl)amino)cin nolin-6-yl)piperidin-4-ol |
| 1 9 | 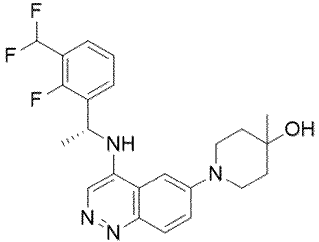 | (R)-1-(4-((1-(3-(difluoromethyl)-2-fluorophenyl)ethyl)amino)cin nolin-6-yl)-4-methylpiperidin-4-ol | (R)-1-(4-((1-(3-(difluoromethyl)-2-fluorophenyl)ethyl)amino)cin nolin-6-yl)-4-methylpiperidin-4-ol |
| 2 0 | 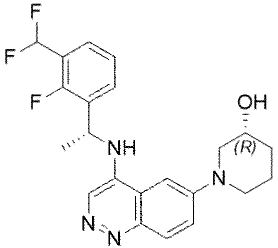 | (R)-1-(4-(((R)-1-(3-(difluoromethyl)-2-fluorophenyl)ethyl)amino)cin nolin-6-yl)piperidin-3-ol | (R)-1-(4-(((R)-1-(3-(difluoromethyl)-2-fluorophenyl)ethyl)amino)cin nolin-6-yl)piperidin-3-ol |
| 2 1 | 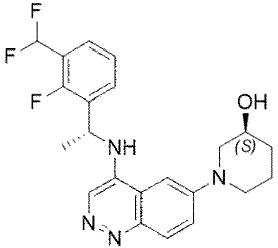 | (S)-1-(4-(((R)-1-(3-(difluoromethyl)-2-fluorophenyl)ethyl)amino)cin nolin-6-yl)piperidin-3-ol | (S)-1-(4-(((R)-1-(3-(difluoromethyl)-2-fluorophenyl)ethyl)amino)cin nolin-6-yl)piperidin-3-ol |
| 2 2 | 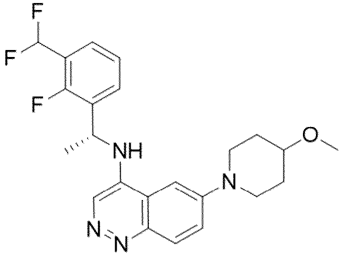 | (R)-N-(1-(3-(difluoromethyl)-2-fluorophenyl)ethyl)-6-(4-methoxypiperidin-1-yl)cinnolin-4-amine | (R)-N-(1-(3-(difluoromethyl)-2-fluorophenyl)ethyl)-6-(4-methoxypiperidin-1-yl)cinnolin-4-amine |
| 2 3 | 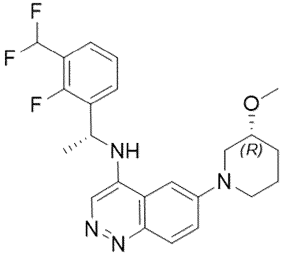 | N-((R)-1-(3-(difluoromethyl)-2-fluorophenyl)ethyl)-6-((R)-3-methoxypiperidin-1-yl)cinnolin-4-amine | N-((R)-1-(3-(difluoromethyl)-2-fluorophenyl)ethyl)-6-((R)-3-methoxypiperidin-1-yl)cinnolin-4-amine |
| 2 4 | 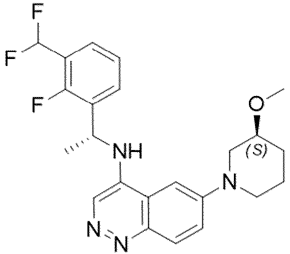 | N-((R)-1-(3-(difluoromethyl)-2-fluorophenyl)ethyl)-6-((S)-3-methoxypiperidin-1-yl)cinnolin-4-amine | N-((R)-1-(3-(difluoromethyl)-2-fluorophenyl)ethyl)-6-((S)-3-methoxypiperidin-1-yl)cinnolin-4-amine |
| 2 5 | 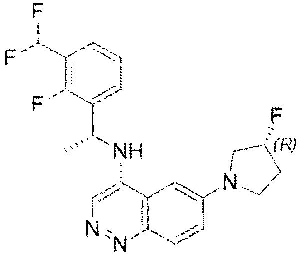 | N-((R)-1-(3-(difluoromethyl)-2-fluorophenyl)ethyl)-6-((R)-3-fluoropyrrolidin-1-yl)cinnolin-4-amine | N-((R)-1-(3-(difluoromethyl)-2-fluorophenyl)ethyl)-6-((R)-3-fluoropyrrolidin-1-yl)cinnolin-4-amine |
| 2 6 | 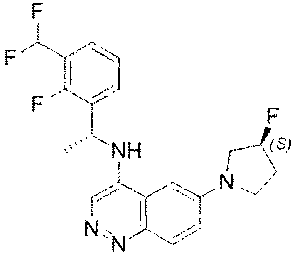 | N-((R)-1-(3-(difluoromethyl)-2-fluorophenyl)ethyl)-6-((S)-3-fluoropyrrolidin-1-yl)cinnolin-4-amine | N-((R)-1-(3-(difluoromethyl)-2-fluorophenyl)ethyl)-6-((S)-3-fluoropyrrolidin-1-yl)cinnolin-4-amine |
| 2 7 | 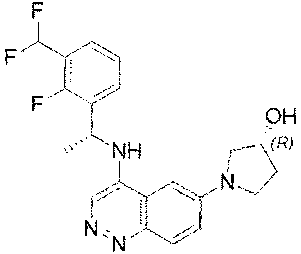 | (R)-1-(4-(((R)-1-(3-(difluoromethyl)-2-fluorophenyl)ethyl)amino)cin nolin-6-yl)pyrrolidin-3-ol | (R)-1-(4-(((R)-1-(3-(difluoromethyl)-2-fluorophenyl)ethyl)amino)cin nolin-6-yl)pyrrolidin-3-ol |
| 2 8 | 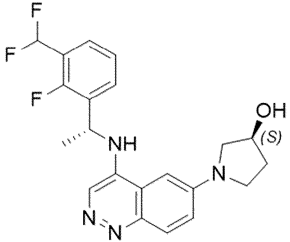 | (S)-1-(4-(((R)-1-(3-(difluoromethyl)-2-fluorophenyl)ethyl)amino)cin nolin-6-yl)pyrrolidin-3-ol | (S)-1-(4-(((R)-1-(3-(difluoromethyl)-2-fluorophenyl)ethyl)amino)cin nolin-6-yl)pyrrolidin-3-ol |
| 2 9 | 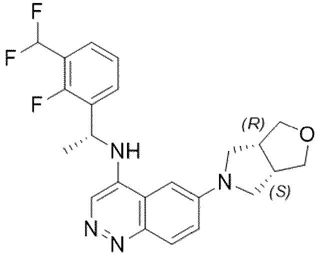 | N-((R)-1-(3-(difluoromethyl)-2-fluorophenyl)ethyl)-6-((3aR,6aS)-tetrahydro-1H-furo[3,4-c]pyrrol-5(3H)-yl)cinnolin-4-amine | N-((R)-1-(3-(difluoromethyl)-2-fluorophenyl)ethyl)-6-((3aR,6aS)-tetrahydro-1H-furo[3,4-c]pyrrol-5(3H)-yl)cinnolin-4-amine |
| 3 0 | 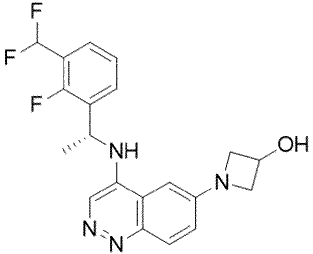 | (R)-1-(4-((1-(3-(difluoromethyl)-2-fluorophenyl)ethyl)amino)cin nolin-6-yl)azetidin-3-ol | (R)-1-(4-((1-(3-(difluoromethyl)-2-fluorophenyl)ethyl)amino)cin nolin-6-yl)azetidin-3-ol |
| 3 1 | 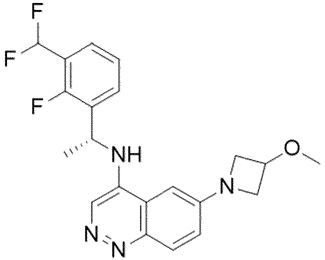 | (R)-N-(1-(3-(difluoromethyl)-2-fluorophenyl)ethyl)-6-(3-methoxyazetidin-1-yl)cinnolin-4-amine | (R)-N-(1-(3-(difluoromethyl)-2-fluorophenyl)ethyl)-6-(3-methoxyazetidin-1-yl)cinnolin-4-amine |
| 3 2 | 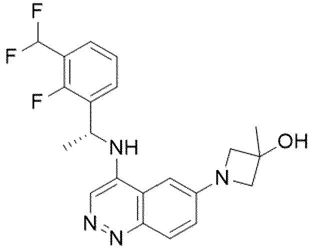 | (R)-1-(4-((1-(3-(difluoromethyl)-2-fluorophenyl)ethyl)amino)cin nolin-6-yl)-3-methylazetidin-3-ol | (R)-1-(4-((1-(3-(difluoromethyl)-2-fluorophenyl)ethyl)amino)cin nolin-6-yl)-3-methylazetidin-3-ol |
| 3 3 | 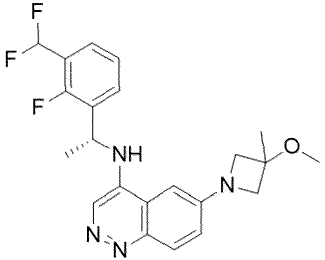 | (R)-N-(1-(3-(difluoromethyl)-2-fluorophenyl)ethyl)-6-(3-methoxy-3-methylazetidin-1-yl)cinnolin-4-amine | (R)-N-(1-(3-(difluoromethyl)-2-fluorophenyl)ethyl)-6-(3-methoxy-3-methylazetidin-1-yl)cinnolin-4-amine |
| 3 4 | 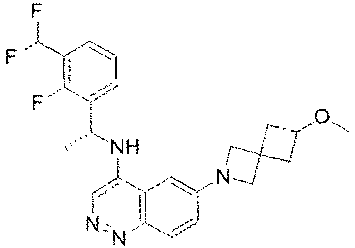 | (R)-N-(1-(3-(difluoromethyl)-2-fluorophenyl)ethyl)-6-(6-methoxy-2-azaspiro[3.3]heptan-2-yl)cinnolin-4-amine | (R)-N-(1-(3-(difluoromethyl)-2-fluorophenyl)ethyl)-6-(6-methoxy-2-azaspiro[3.3]heptan-2-yl)cinnolin-4-amine |
| 3 5 | 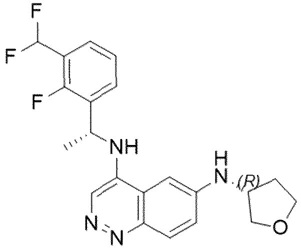 | N4-((R)-1-(3-(difluoromethyl)-2-fluorophenyl)ethyl)-N6-((R)-tetrahydrofuran-3-yl)cinnoline-4,6-diamine | N4-((R)-1-(3-(difluoromethyl)-2-fluorophenyl)ethyl)-N6-((R)-tetrahydrofuran-3-yl)cinnolin-4,6-diamine |
| 3 6 | 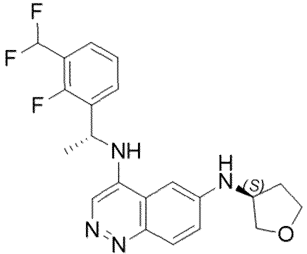 | N4-((R)-1-(3-(difluoromethyl)-2-fluorophenyl)ethyl)-N6-((S)-tetrahydrofuran-3-yl)cinnoline-4,6-diamine | N4-((R)-1-(3-(difluoromethyl)-2-fluorophenyl)ethyl)-N6-((S)-tetrahydrofuran-3-yl)cinnolin-4,6-diamine |
| 3 7 | 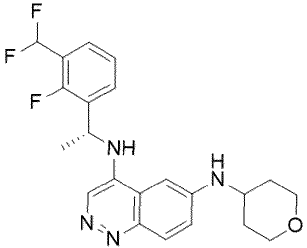 | (R)-N4-(1-(3-(difluoromethyl)-2-fluorophenyl)ethyl)-N6-(tetrahydro-2H-pyran-4-yl)cinnoline-4,6-diamine | (R)-N4-(1-(3-(difluoromethyl)-2-fluorophenyl)ethyl)-N6-(tetrahydro-2H-pyran-4-yl)cinnolin-4,6-diamine |
| 3 8 | 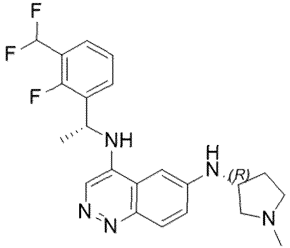 | N4-((R)-1-(3-(difluoromethyl)-2-fluorophenyl)ethyl)-N6-((R)-1-methylpyrrolidin-3-yl)cinnoline-4,6-diamine | N4-((R)-1-(3-(difluoromethyl)-2-fluorophenyl)ethyl)-N6-((R)-1-methylpyrrolidin-3-yl)cinnolin-4,6-diamine |
| 3 9 | 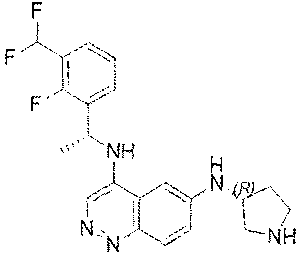 | N4-((R)-1-(3-(difluoromethyl)-2-fluorophenyl)ethyl)-N6-((R)-pyrrolidin-3-yl)cinnoline-4,6-diamine | N4-((R)-1-(3-(difluoromethyl)-2-fluorophenyl)ethyl)-N6-((R)-pyrrolidin-3-yl)cinnolin-4,6-diamine |
| 4 0 | 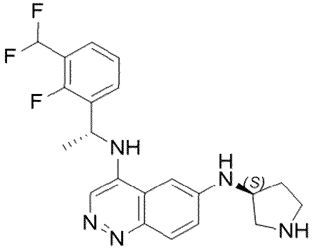 | N4-((R)-1-(3-(difluoromethyl)-2-fluorophenyl)ethyl)-N6-((S)-pyrrolidin-3-yl)cinnoline-4,6-diamine | N4-((R)-1-(3-(difluoromethyl)-2-fluorophenyl)ethyl)-N6-((S)-pyrrolidin-3-yl)cinnolin-4,6-diamine |
| 4 1 | 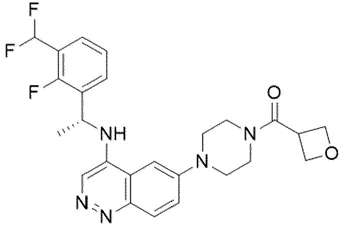 | (R)-(4-(4-((1-(3-(difluoromethyl)-2-fluorophenyl)ethyl)amino)cin nolin-6-yl)piperazin-1-yl)(oxetan-3-yl)methanone | (R)-(4-(4-((1-(3-(difluoromethyl)-2-fluorophenyl)ethyl)amino)cin nolin-6-yl)piperazin-1-yl)(oxetan-3-yl)methanone |
| 4 2 | 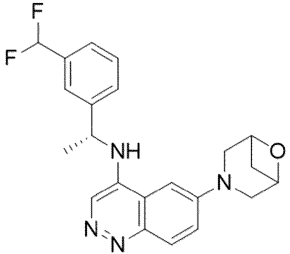 | 6-(6-oxa-3-azabicyclo[3.1.1]heptan-3-yl)-N-((R)-1-(3-(difluoromethyl)phenyl)ethyl )cinnolin-4-amine | 6-(6-oxa-3-azabicyclo[3.1.1]heptan-3-yl)-N-((R)-1-(3-(difluoromethyl)phenyl)ethyl )cinnolin-4-amine |
| 4 3 | 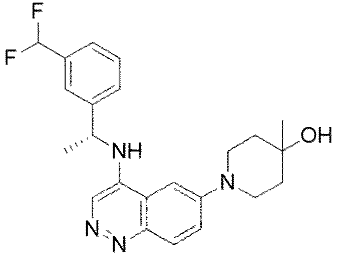 | (R)-1-(4-((1-(3-(difluoromethyl)phenyl)ethyl )amino)cinnolin-6-yl)-4-methylpiperidin-4-ol | (R)-1-(4-((1-(3-(difluoromethyl)phenyl)ethyl )amino)cinnolin-6-yl)-4-methylpiperidin-4-ol |
| 4 4 | 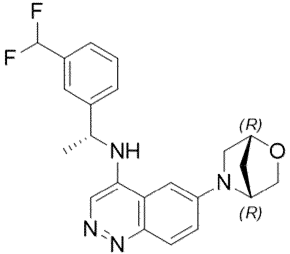 | 6-((1R,4R)-2-oxa-5-azabicyclo[2.2.1]heptan-5-yl)-N-((R)-1-(3-(difluoromethyl)phenyl)ethyl )cinnolin-4-amine | 6-((1R,4R)-2-oxa-5-azabicyclo[2.2.1]heptan-5-yl)-N-((R)-1-(3-(difluoromethyl)phenyl)ethyl )cinnolin-4-amine |
| 4 5 | 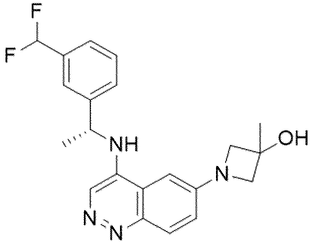 | (R)-1-(4-((1-(3-(difluoromethyl)phenyl)ethyl )amino)cinnolin-6-yl)-3-methylazetidin-3-ol | (R)-1-(4-((1-(3-(difluoromethyl)phenyl)ethyl )amino)cinnolin-6-yl)-3-methylazetidin-3-ol |
| 4 6 | 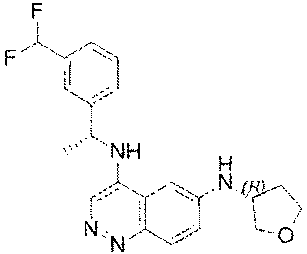 | N4-((R)-1-(3-(difluoromethyl)phenyl)ethyl )-N6-((R)-tetrahydrofuran-3-yl)cinnoline-4,6-diamine | N4-((R)-1-(3-(difluoromethyl)phenyl)ethyl )-N6-((R)-tetrahydrofuran-3-yl)cinnolin-4,6-diamine |
| 4 7 | 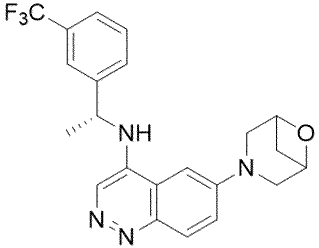 | 6-(6-oxa-3-azabicyclo[3.1.1]heptan-3-yl)-N-((R)-1-(3-(trifluoromethyl)phenyl)ethyl )cinnolin-4-amine | 6-(6-oxa-3-azabicyclo[3.1.1]heptan-3-yl)-N-((R)-1-(3-(trifluoromethyl)phenyl)ethyl )cinnolin-4-amine |
| 4 8 | 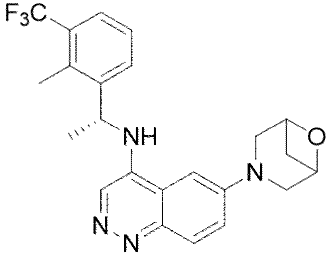 | 6-(6-oxa-3-azabicyclo[3.1.1]heptan-3-yl)-N-((R)-1-(2-methyl-3-(trifluoromethyl)phenyl)ethyl )cinnolin-4-amine | 6-(6-oxa-3-azabicyclo[3.1.1]heptan-3-yl)-N-((R)-1-(2-methyl-3-(trifluoromethyl)phenyl)ethyl )cinnolin-4-amine |
| 4 9 | 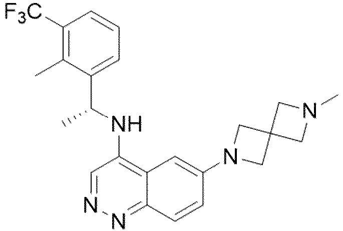 | (R)-N-(1-(2-methyl-3-(trifluoromethyl)phenyl)ethyl )-6-(6-methyl-2,6-diazaspiro[3.3]heptan-2-yl)cinnolin-4-amine | (R)-N-(1-(2-methyl-3-(trifluoromethyl)phenyl)ethyl )-6-(6-methyl-2,6-diazaspiro[3.3]heptan-2-yl)cinnolin-4-amine |
| 5 0 | 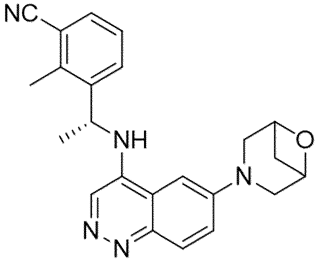 | 3-((1R)-1-((6-(6-oxa-3-azabicyclo[3.1.1]heptan-3-yl)cinnolin-4-yl)amino)ethyl)-2-methylbenzonitrile | 3-((1R)-1-((6-(6-oxa-3-azabicyclo[3.1.1]heptan-3-yl)cinnolin-4-yl)amino)ethyl)-2-methylbenzonitrile |
| 5 1 | 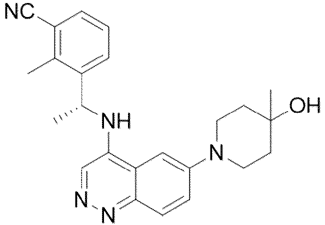 | (R)-3-(1-((6-(4-hydroxy-4-methylpiperidin-1-yl)cinnolin-4-yl)amino)ethyl)-2-methylbenzonitrile | (R)-3-(1-((6-(4-hydroxy-4-methylpiperidin-1-yl)cinnolin-4-yl)amino)ethyl)-2-methylbenzonitrile |
| 5 2 | 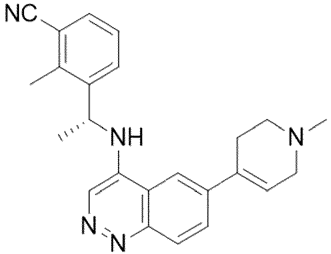 | (R)-2-methyl-3-(1-((6-(1-methyl-1,2,3,6-tetrahydropyridin-4-yl)cinnolin-4-yl)amino)ethyl)benzonitrile | (R)-2-methyl-3-(1-((6-(1-methyl-1,2,3,6-tetrahydropyridin-4-yl)cinnolin-4-yl)amino)ethyl)benzonitrile |
| 5 3 | 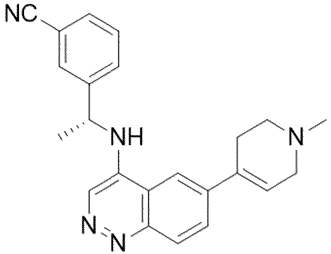 | (R)-3-(1-((6-(1-methyl-1,2,3,6-tetrahydropyridin-4-yl)cinnolin-4-yl)amino)ethyl)benzonitrile | (R)-3-(1-((6-(1-methyl-1,2,3,6-tetrahydropyridin-4-yl)cinnolin-4-yl)amino)ethyl)benzonitrile |
| 5 4 | 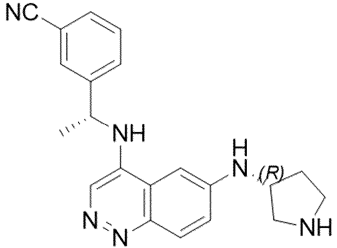 | 3-((R)-1-((6-((R)-pyrrolidin-3-ylamino)cinnolin-4-yl)amino)ethyl)benzonitrile | 3-((R)-1-((6-((R)-pyrrolidin-3-ylamino)cinnolin-4-yl)amino)ethyl)benzonitrile |
| 5 5 | 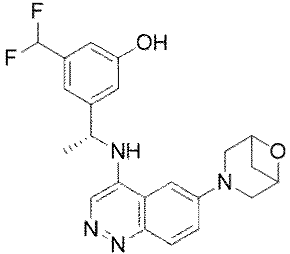 | 3-((1R)-1-((6-(6-oxa-3-azabicyclo[3.1.1]heptan-3-yl)cinnolin-4-yl)amino)ethyl)-5-(difluoromethyl)phenol | 3-((1R)-1-((6-(6-oxa-3-azabicyclo[3.1.1]heptan-3-yl)cinnolin-4-yl)amino)ethyl)-5-(difluoromethyl)phenol |
| 5 6 | 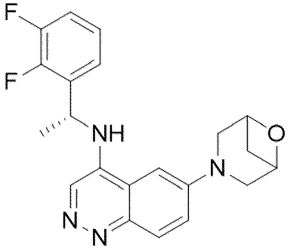 | 6-(6-oxa-3-azabicyclo[3.1.1]heptan-3-yl)-N-((R)-1-(2,3-difluorophenyl)ethyl)cinnolin -4-amine | 6-(6-oxa-3-azabicyclo[3.1.1]heptan-3-yl)-N-((R)-1-(2,3-difluorophenyl)ethyl)cinnolin -4-amine |
| 5 7 | 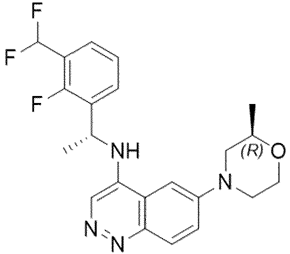 | N-((R)-1-(3-(difluoromethyl)-2-fluorophenyl)ethyl)-6-((R)-2-methylmorpholino)cinnolin-4-amine | N-((R)-1-(3-(difluoromethyl)-2-fluorophenyl)ethyl)-6-((R)-2-methylmorpholino)cinnolin-4-amine |
| 5 8 | 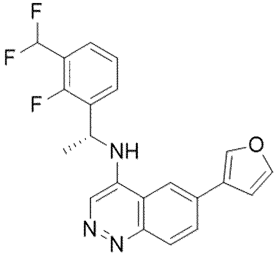 | (R)-N-(1-(3-(difluoromethyl)-2-fluorophenyl)ethyl)-6-(furan-3-yl)cinnolin-4-amine | (R)-N-(1-(3-(difluoromethyl)-2-fluorophenyl)ethyl)-6-(furan-3-yl)cinnolin-4-amine |
| 5 9 | 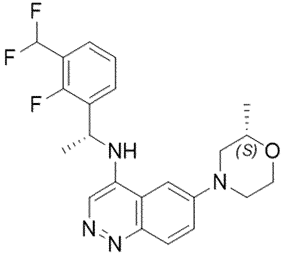 | N-((R)-1-(3-(difluoromethyl)-2-fluorophenyl)ethyl)-6-((S)-2-methylmorpholino)cinnolin-4-amine | N-((R)-1-(3-(difluoromethyl)-2-fluorophenyl)ethyl)-6-((S)-2-methylmorpholino)cinnolin-4-amine |
| 6 0 | 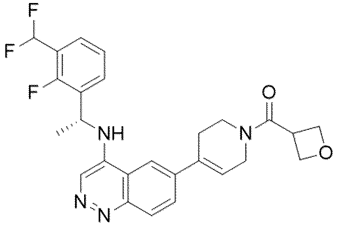 | (R)-(4-(4-((1-(3-(difluoromethyl)-2-fluorophenyl)ethyl)amino)cin nolin-6-yl)-5,6-dihydropyridin-1(2H)-yl)(oxetan-3-yl)methanone | (R)-(4-(4-((1-(3-(difluoromethyl)-2-fluorophenyl)ethyl)amino)cin nolin-6-yl)-5,6-dihydropyridin-1(2H)-yl)(oxetan-3-yl)methanone |
| 6 1 | 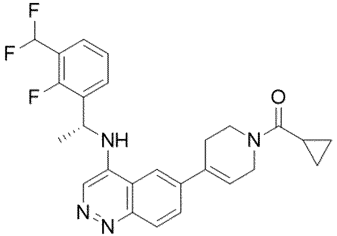 | (R)-cyclopropyl(4-(4-((1-(3-(difluoromethyl)-2-fluorophenyl)ethyl)amino)cin nolin-6-yl)-5,6-dihydropyridin-1(2H)-yl)methanone | (R)-cyclopropyl(4-(4-((1-(3-(difluoromethyl)-2-fluorophenyl)ethyl)amino)cin nolin-6-yl)-5,6-dihydropyridin-1(2H)-yl)methanone |
| 6 2 | 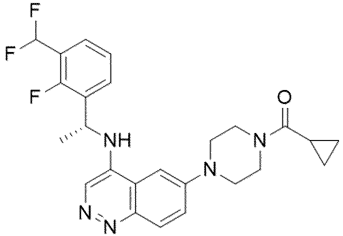 | (R)-cyclopropyl(4-(4-((1-(3-(difluoromethyl)-2-fluorophenyl)ethyl)amino)cin nolin-6-yl)piperazin-1-yl)methanone | (R)-cyclopropyl(4-(4-((1-(3-(difluoromethyl)-2-fluorophenyl)ethyl)amino)cin nolin-6-yl)piperazin-1-yl)methanone |
| 6 3 | 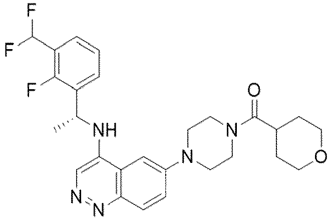 | (R)-(4-(4-((1-(3-(difluoromethyl)-2-fluorophenyl)ethyl)amino)cin nolin-6-yl)piperazin-1-yl)(tetrahydro-2H-pyran-4-yl)methanone | (R)-(4-(4-((1-(3-(difluoromethyl)-2-fluorophenyl)ethyl)amino)cin nolin-6-yl)piperazin-1-yl)(tetrahydro-2H-pyran-4-yl)methanone |
| 6 4 | 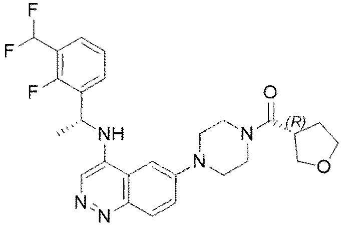 | (4-(4-(((R)-1-(3-(difluoromethyl)-2-fluorophenyl)ethyl)amino)cin nolin-6-yl)piperazin-1-yl)((R)-tetrahydrofuran-3-yl)methanone | (4-(4-(((R)-1-(3-(difluoromethyl)-2-fluorophenyl)ethyl)amino)cin nolin-6-yl)piperazin-1-yl)((R)-tetrahydrofuran-3-yl)methanone |
| 6 5 | 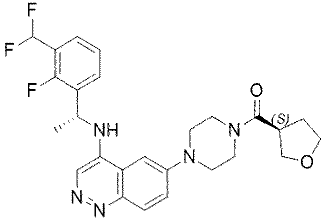 | (4-(4-(((R)-1-(3-(difluoromethyl)-2-fluorophenyl)ethyl)amino)cin nolin-6-yl)piperazin-1-yl)((S)-tetrahydrofuran-3-yl)methanone | (4-(4-(((R)-1-(3-(difluoromethyl)-2-fluorophenyl)ethyl)amino)cin nolin-6-yl)piperazin-1-yl)((S)-tetrahydrofuran-3-yl)methanone |
| 6 6 | 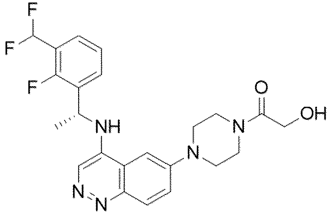 | (R)-1-(4-(4-((1-(3-(difluoromethyl)-2-fluorophenyl)ethyl)amino)cin nolin-6-yl)piperazin-1-yl)-2-hydroxyethanone | (R)-1-(4-(4-((1-(3-(difluoromethyl)-2-fluorophenyl)ethyl)amino)cin nolin-6-yl)piperazin-1-yl)-2-hydroxyethanone |
| 6 7 | 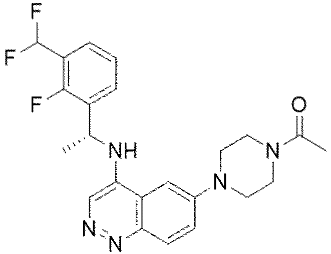 | (R)-1-(4-(4-((1-(3-(difluoromethyl)-2-fluorophenyl)ethyl)amino)cin nolin-6-yl)piperazin-1-yl)ethanone | (R)-1-(4-(4-((1-(3-(difluoromethyl)-2-fluorophenyl)ethyl)amino)cin nolin-6-yl)piperazin-1-yl)ethanone |
| 6 8 | 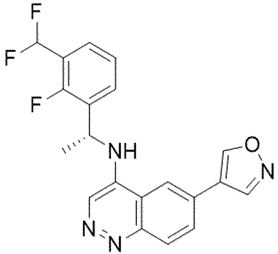 | (R)-N-(1-(3-(difluoromethyl)-2-fluorophenyl)ethyl)-6-(isoxazol-4-yl)cinnolin-4-amine | (R)-N-(1-(3-(difluoromethyl)-2-fluorophenyl)ethyl)-6-(isoxazol-4-yl)cinnolin-4-amine |
| 6 9 | 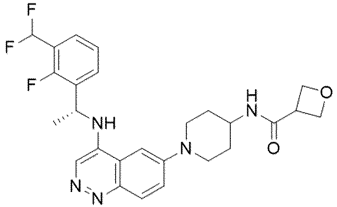 | (R)-N-(1-(4-((1-(3-(difluoromethyl)-2-fluorophenyl)ethyl)amino)cin nolin-6-yl)piperidin-4-yl)oxetane-3-carboxamide | (R)-N-(1-(4-((1-(3-(difluoromethyl)-2-fluorophenyl)ethyl)amino)cin nolin-6-yl)piperidin-4-yl)oxetan-3-carboxamide |
| 7 0 | 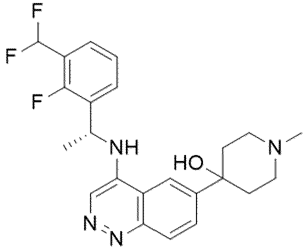 | (R)-4-(4-((1-(3-(difluoromethyl)-2-fluorophenyl)ethyl)amino)cin nolin-6-yl)-1-methylpiperidin-4-ol | (R)-4-(4-((1-(3-(difluoromethyl)-2-fluorophenyl)ethyl)amino)cin nolin-6-yl)-1-methylpiperidin-4-ol |
| 7 1 | 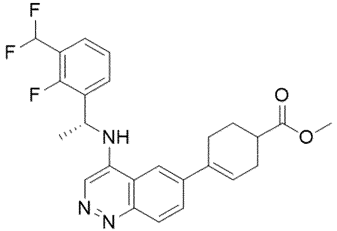 | methyl 4-(4-(((R)-1-(3-(difluoromethyl)-2-fluorophenyl)ethyl)amino)cin nolin-6-yl)cyclohex-3-enecarboxylate | Methyl 4-(4-(((R)-1-(3-(difluoromethyl)-2-fluorophenyl)ethyl)amino)cin nolin-6-yl)cyclohex-3-enecarboxylate |
| 7 2 | 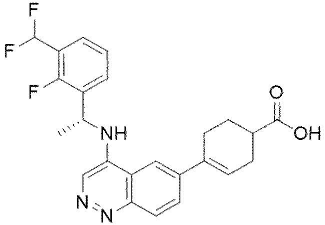 | 4-(4-(((R)-1-(3-(difluoromethyl)-2-fluorophenyl)ethyl)amino)cin nolin-6-yl)cyclohex-3-enecarboxylic acid | 4-(4-(((R)-1-(3-(difluoromethyl)-2-fluorophenyl)ethyl)amino)cin nolin-6-yl)cyclohex-3-enecarboxylic acid |
| 7 3 | 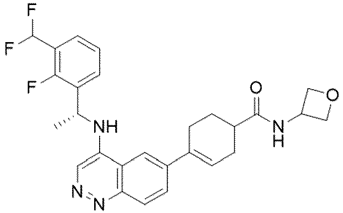 | 4-(4-(((R)-1-(3-(difluoromethyl)-2-fluorophenyl)ethyl)amino)cin nolin-6-yl)-N-(oxetan-3-yl)cyclohex-3-enecarboxamide | 4-(4-(((R)-1-(3-(difluoromethyl)-2-fluorophenyl)ethyl)amino)cin nolin-6-yl)-N-(oxetan-3-yl)cyclohex-3-enecarboxamide |
| 7 4 | 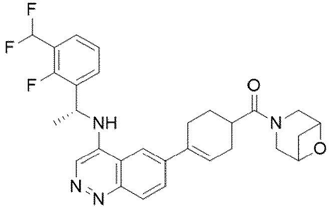 | 6-oxa-3-azabicyclo[3.1.1]heptan-3-yl(4-(4-(((R)-1-(3-(difluoromethyl)-2-fluorophenyl)ethyl)amino)cin nolin-6-yl)cyclohex-3-en-1-yl)methanone | 6-oxa-3-azabicyclo[3.1.1]heptan-3-yl(4-(4-(((R)-1-(3-(difluoromethyl)-2-fluorophenyl)ethyl)amino)cin nolin-6-yl)cyclohex-3-en-1-yl)methanone |
| 7 5 | 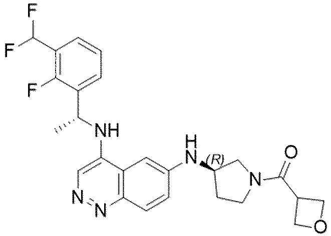 | ((R)-3-((4-(((R)-1-(3-(difluoromethyl)-2-fluorophenyl)ethyl)amino)cin nolin-6-yl)amino)pyrrolidin-1-yl)(oxetan-3-yl)methanone | ((R)-3-((4-(((R)-1-(3-(difluoromethyl)-2-fluorophenyl)ethyl)amino)cin nolin-6-yl)amino)pyrrolidin-1-yl)(oxetan-3-yl)methanone |
| 7 6 | 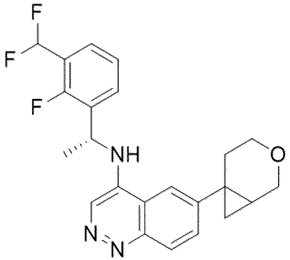 | 6-(3-oxabicyclo[4.1.0]heptan-6-yl)-N-((R)-1-(3-(difluoromethyl)-2-fluorophenyl)ethyl)cinnolin-4-amine | 6-(3-oxabicyclo[4.1.0]heptan-6-yl)-N-((R)-1-(3-(difluoromethyl)-2-fluorophenyl)ethyl)cinnolin-4-amine |

In addition, the compound according to the present invention, stereoisomer thereof, or pharmaceutically acceptable salt thereof may be a compound shown in Table 2 or 3 below.

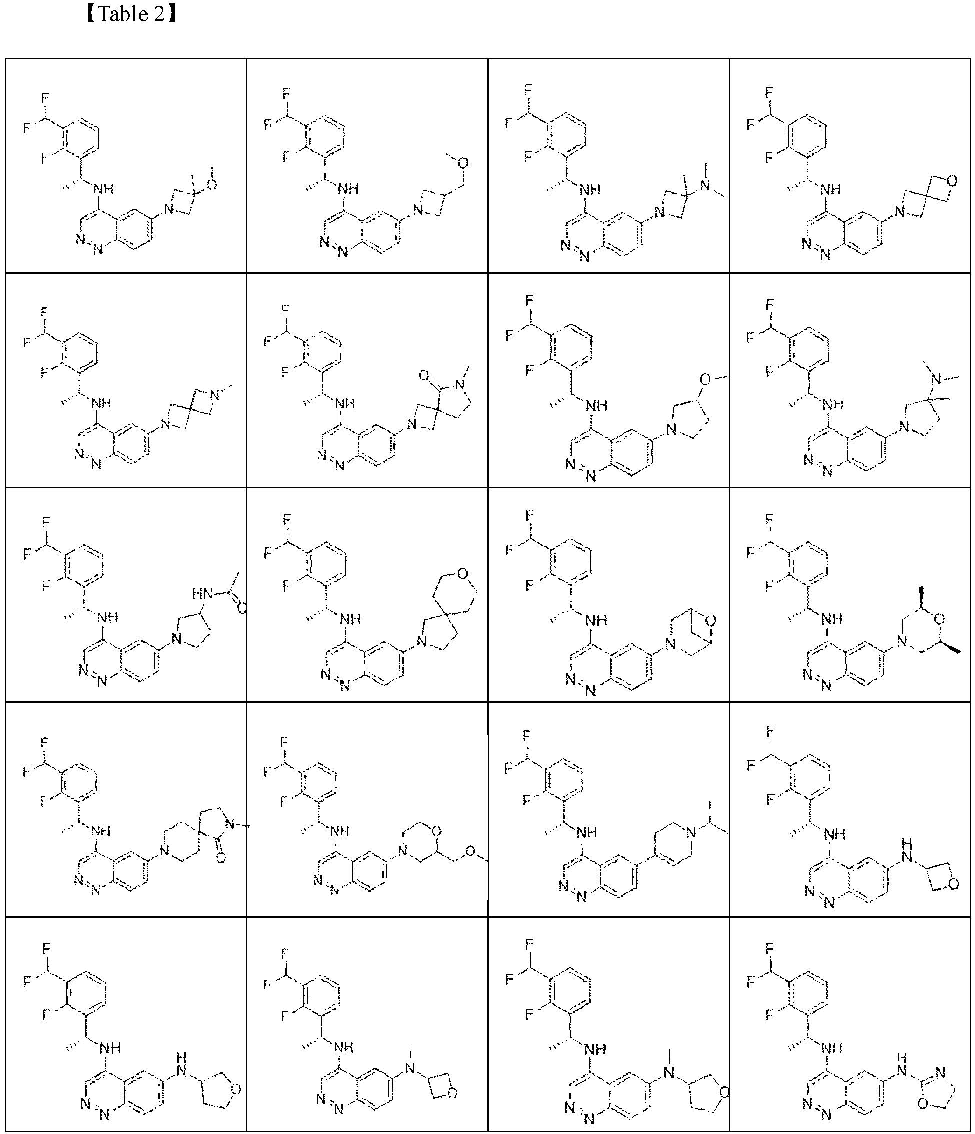

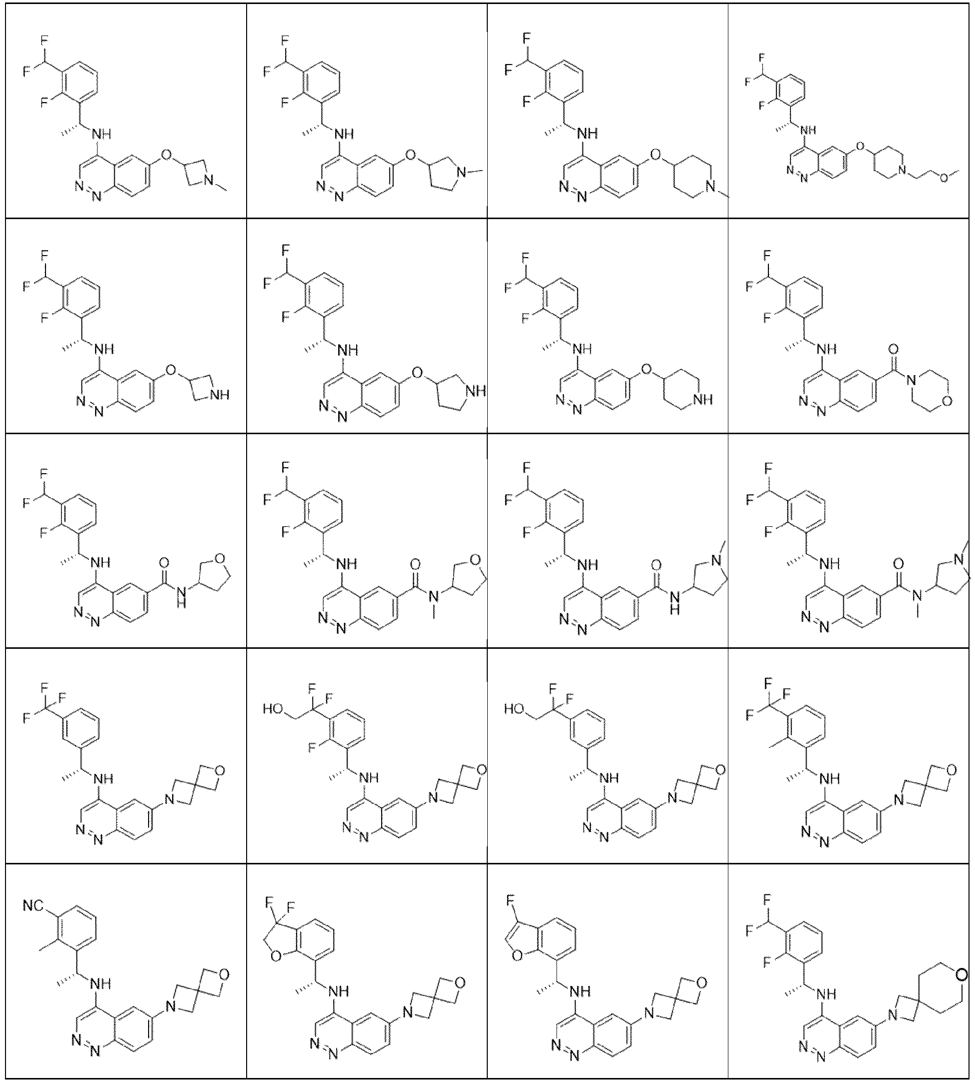

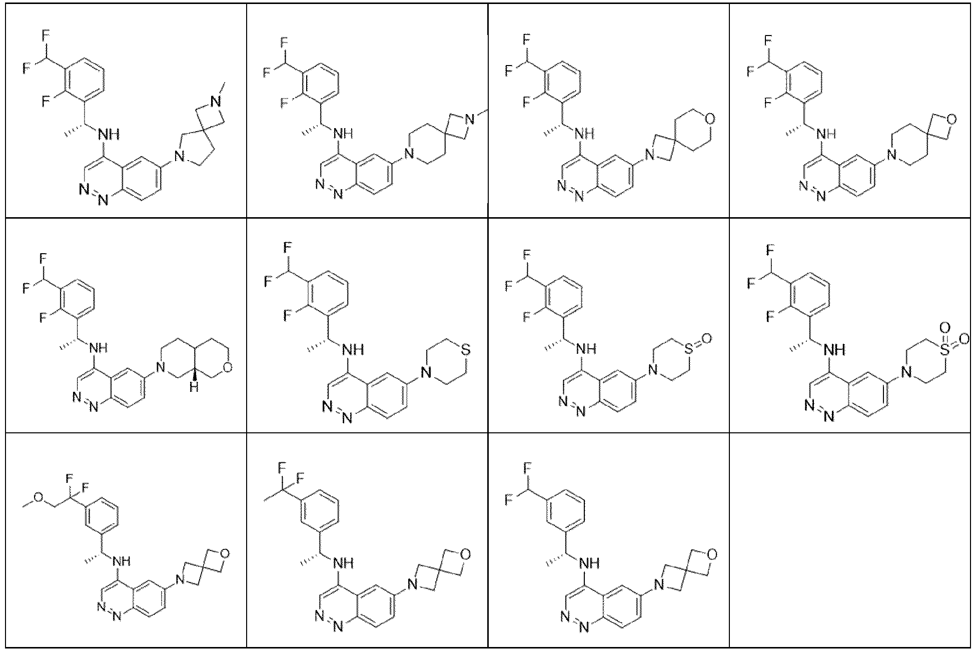

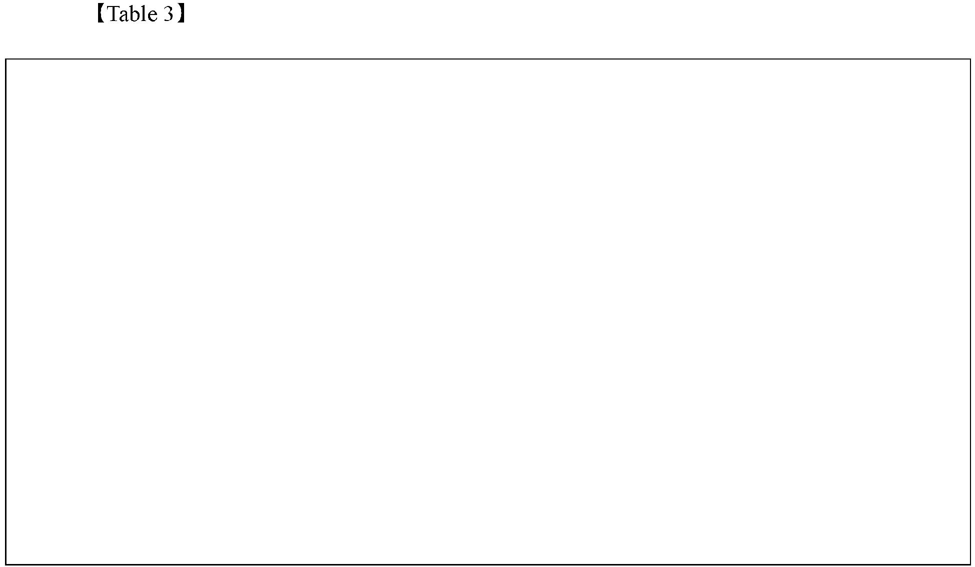

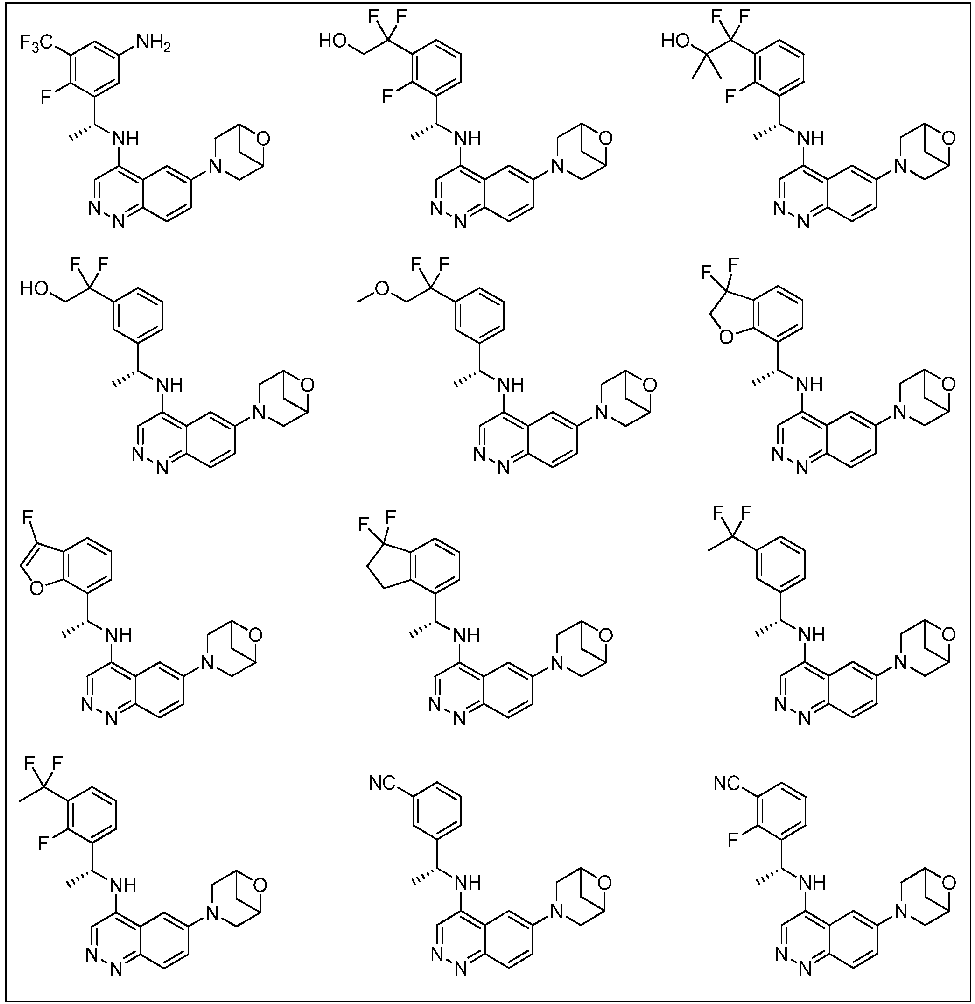

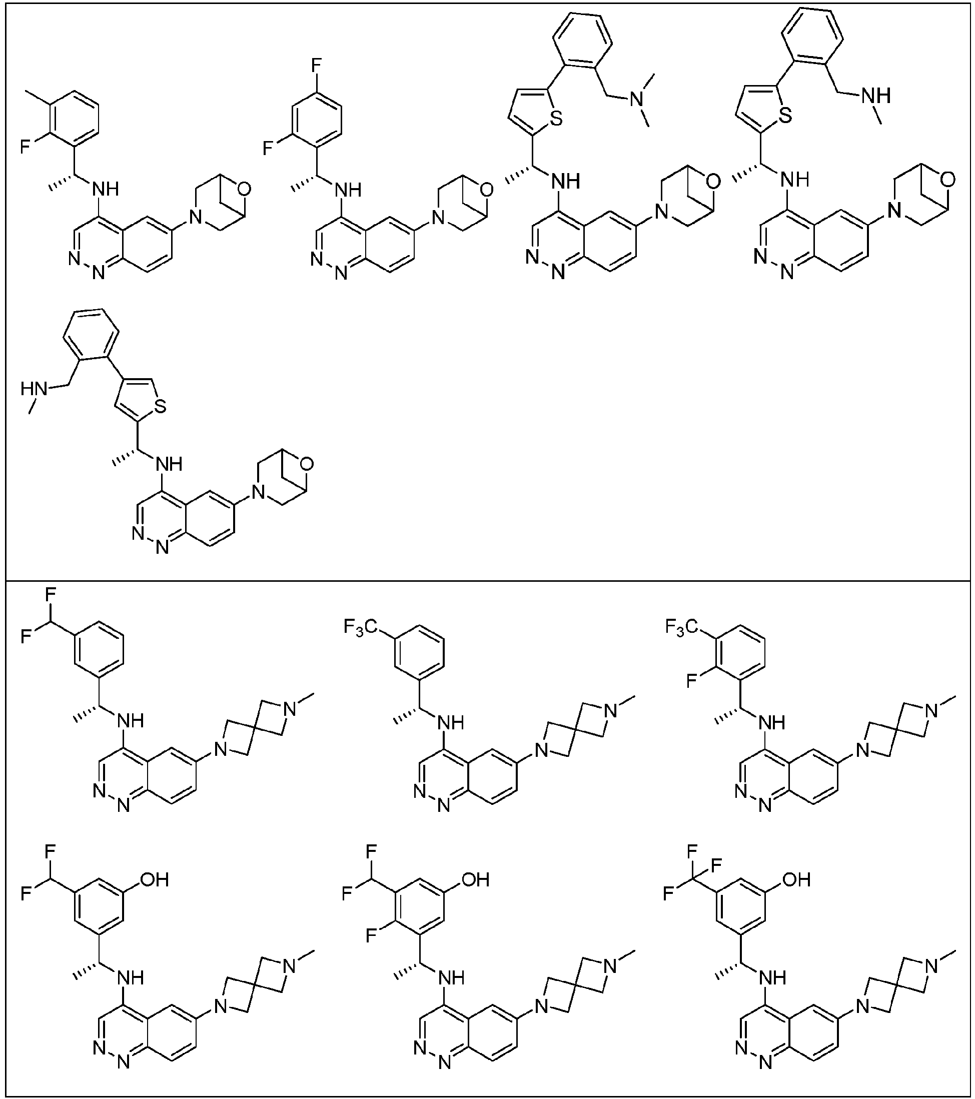

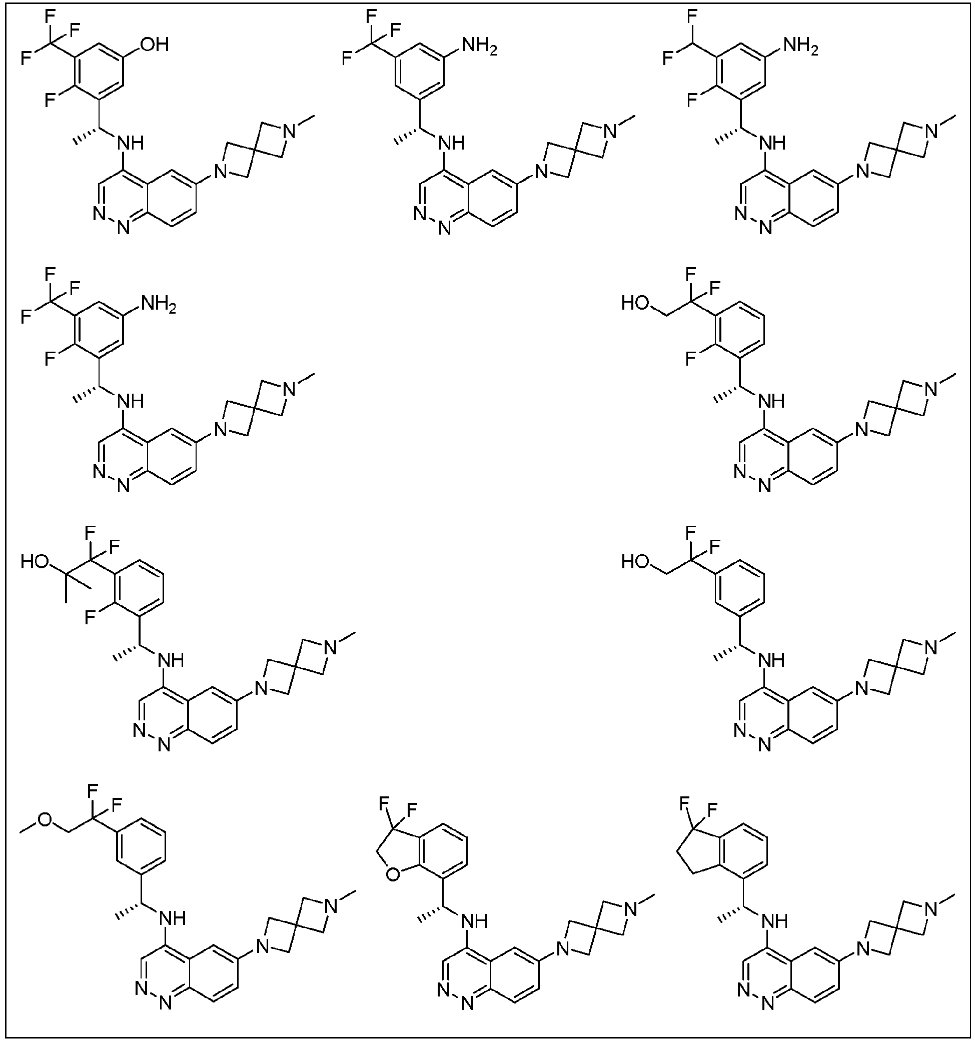

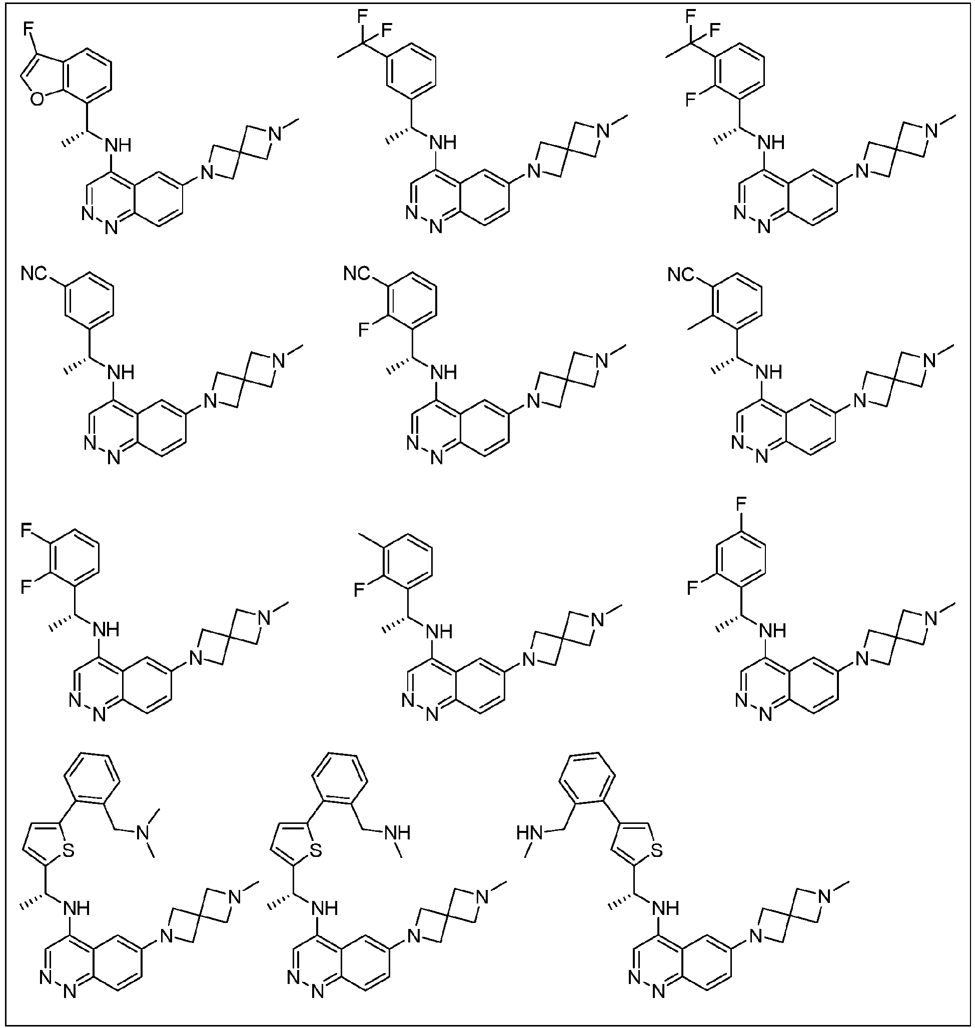

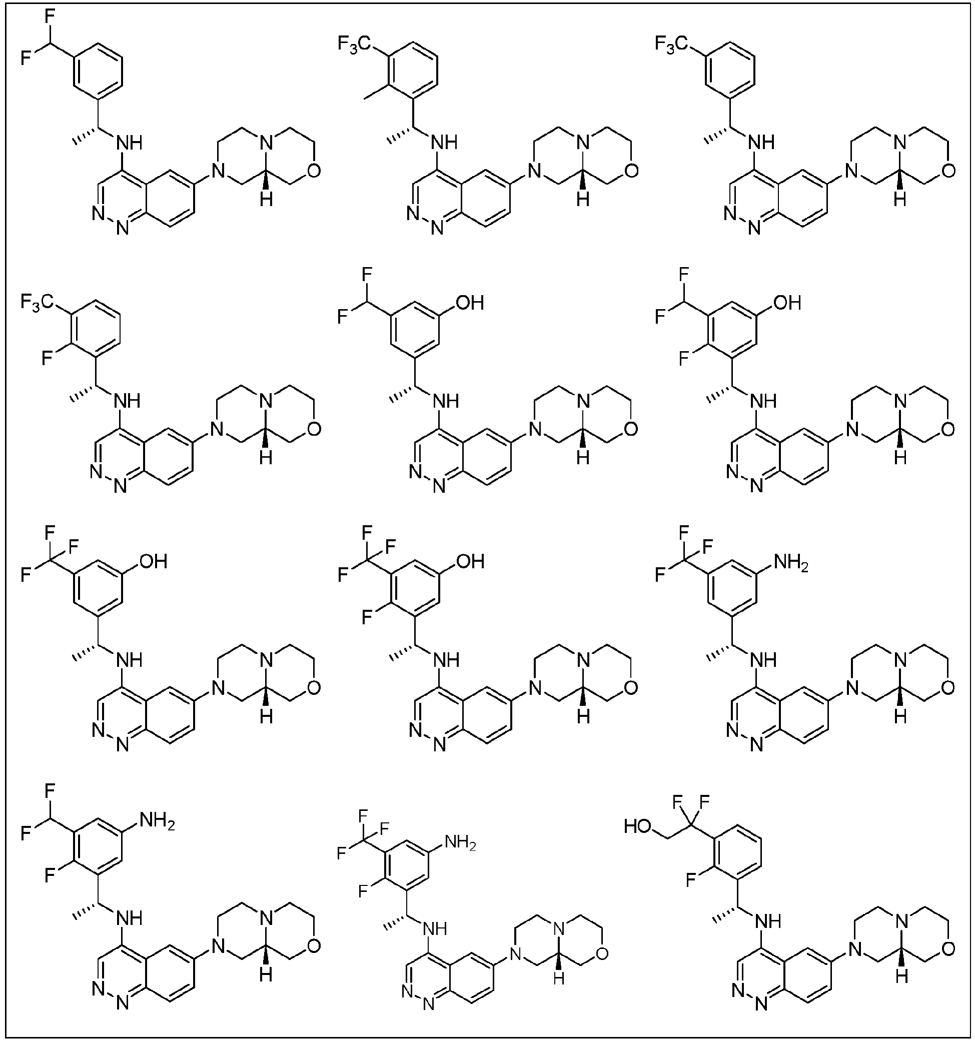

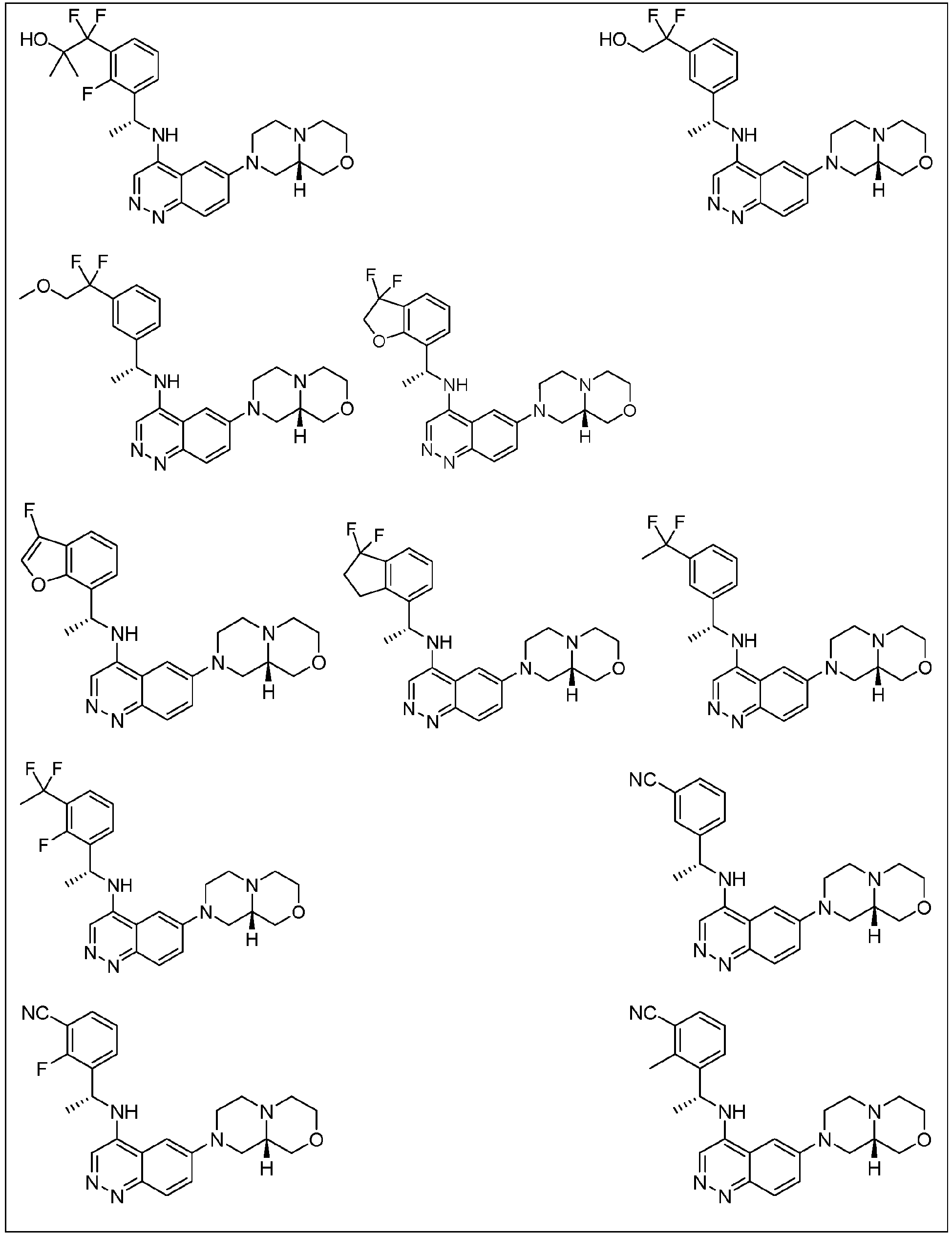

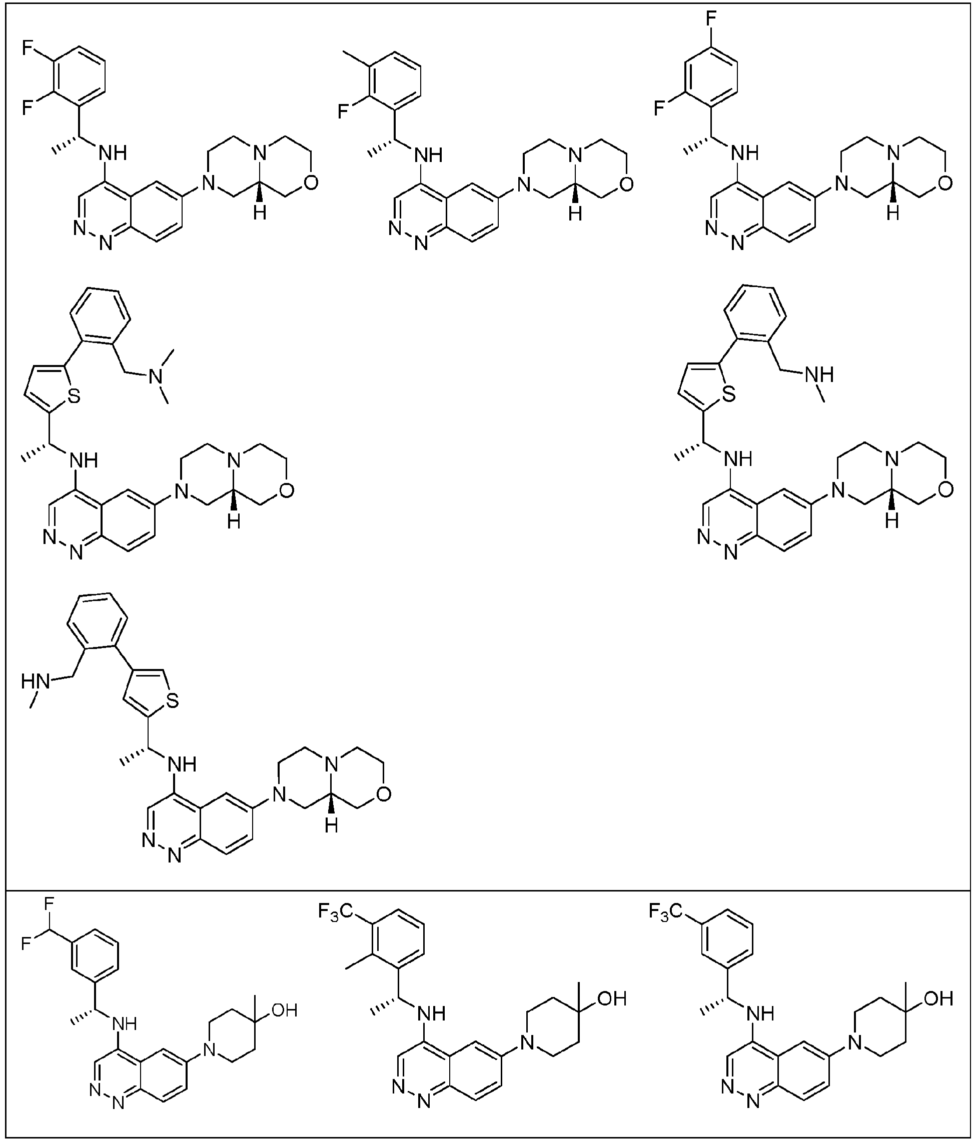

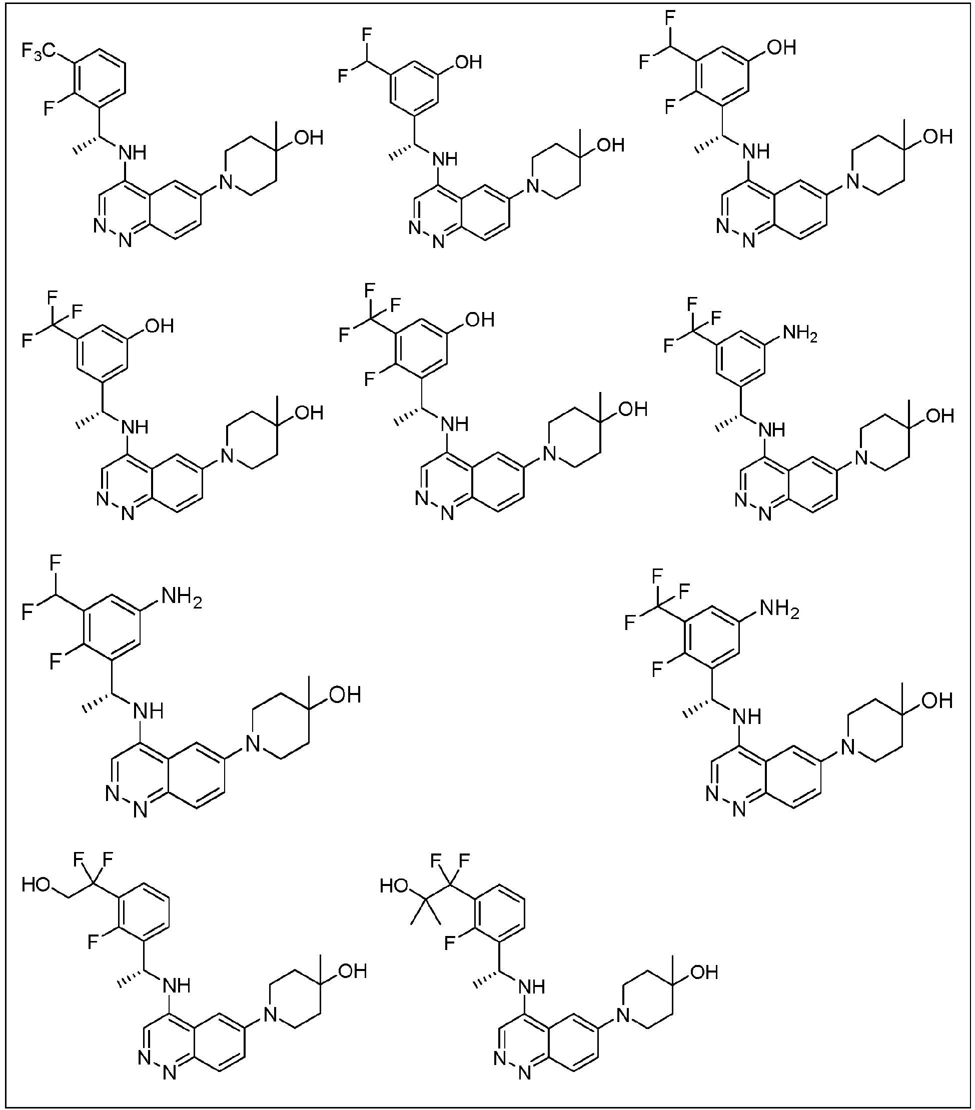

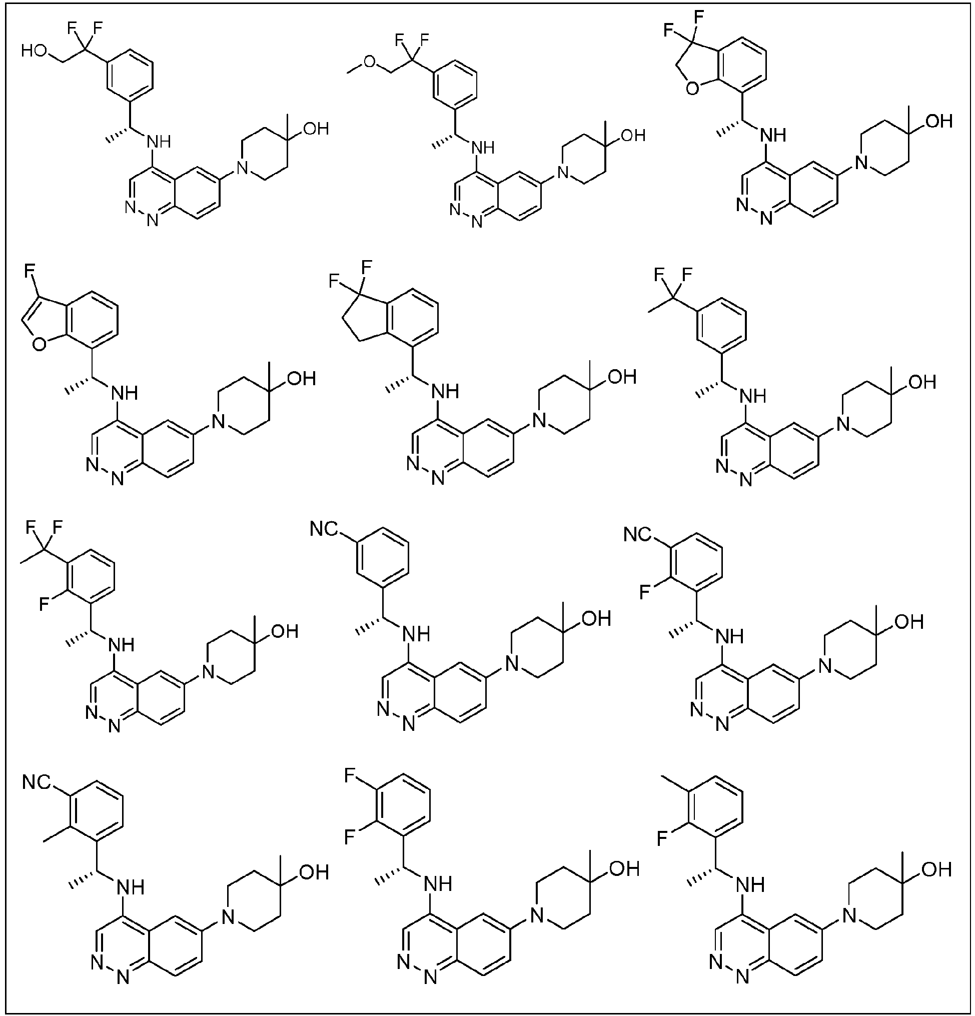

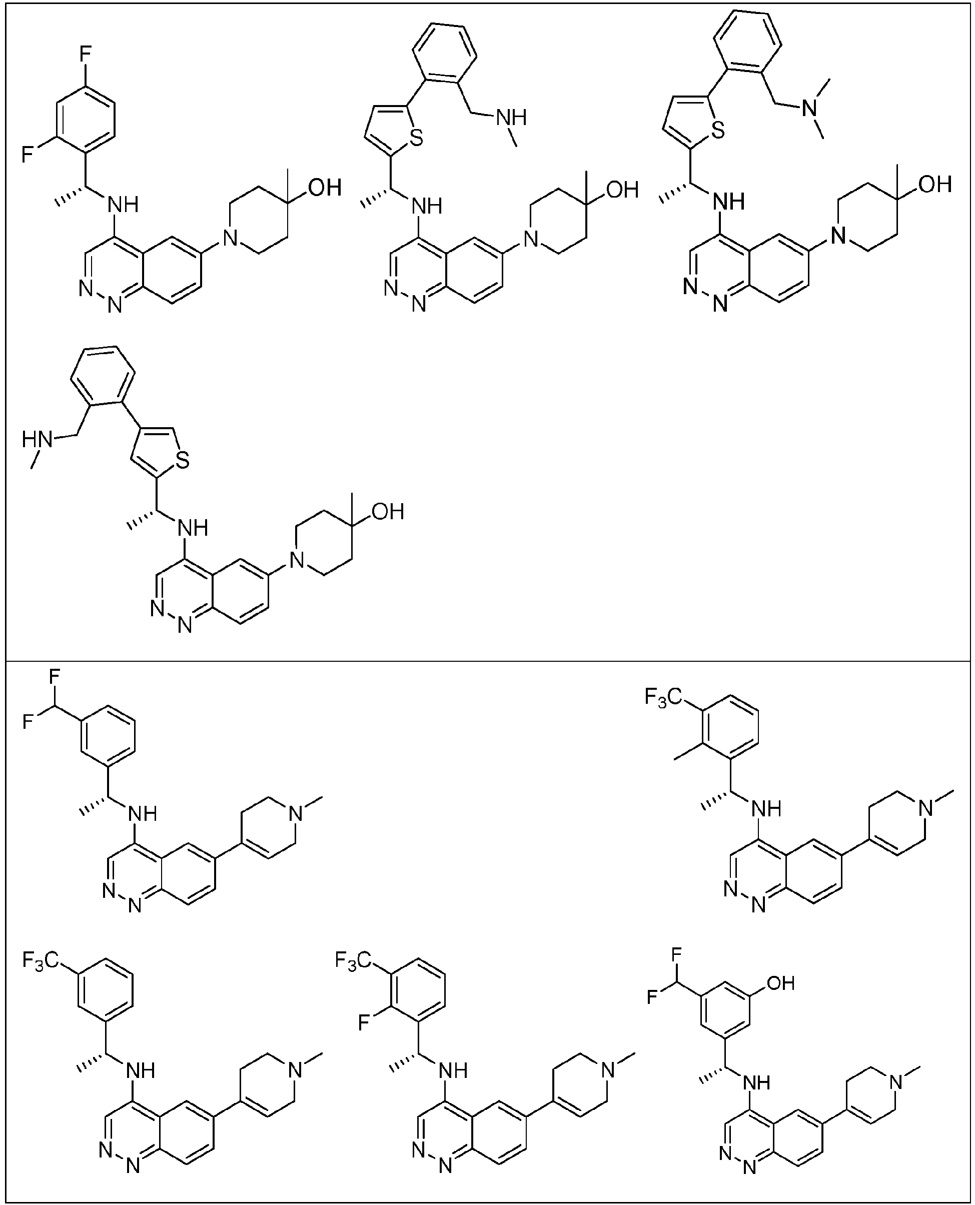

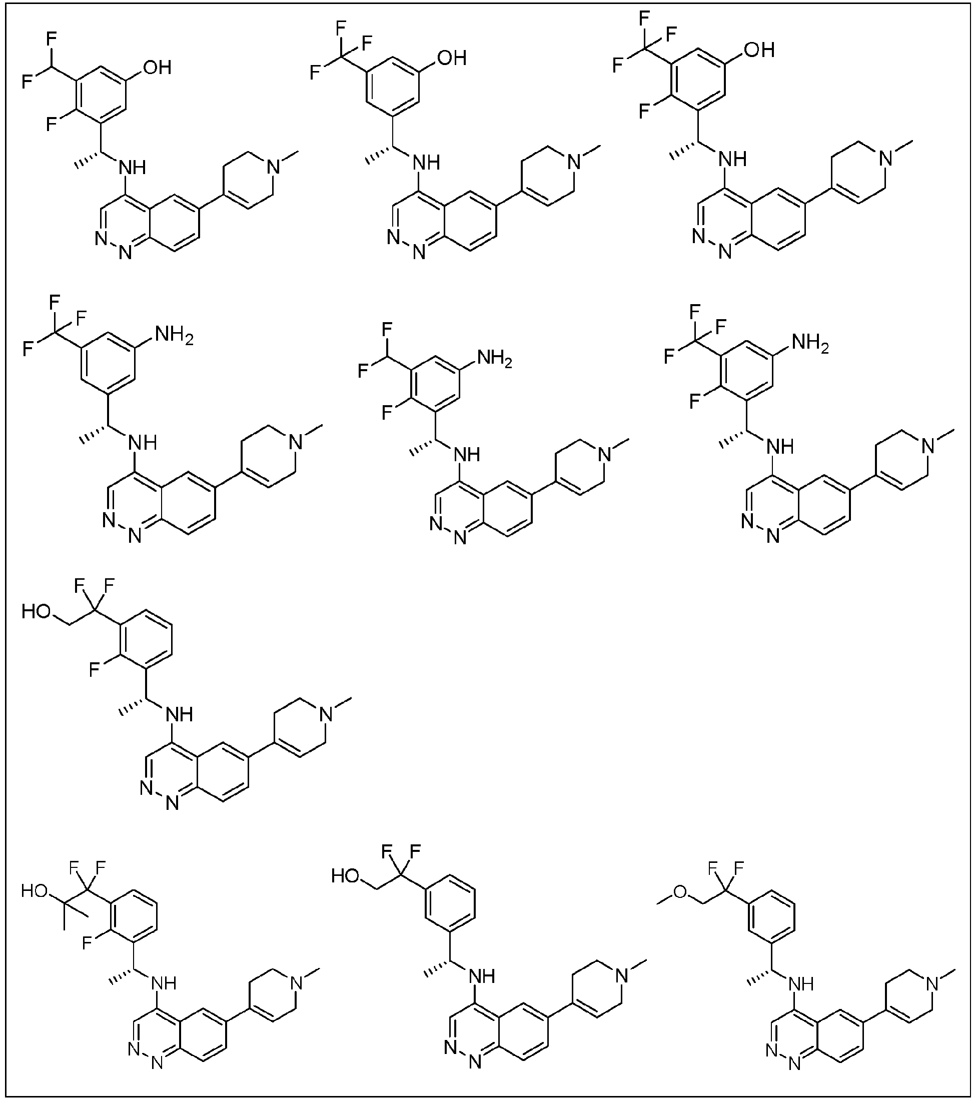

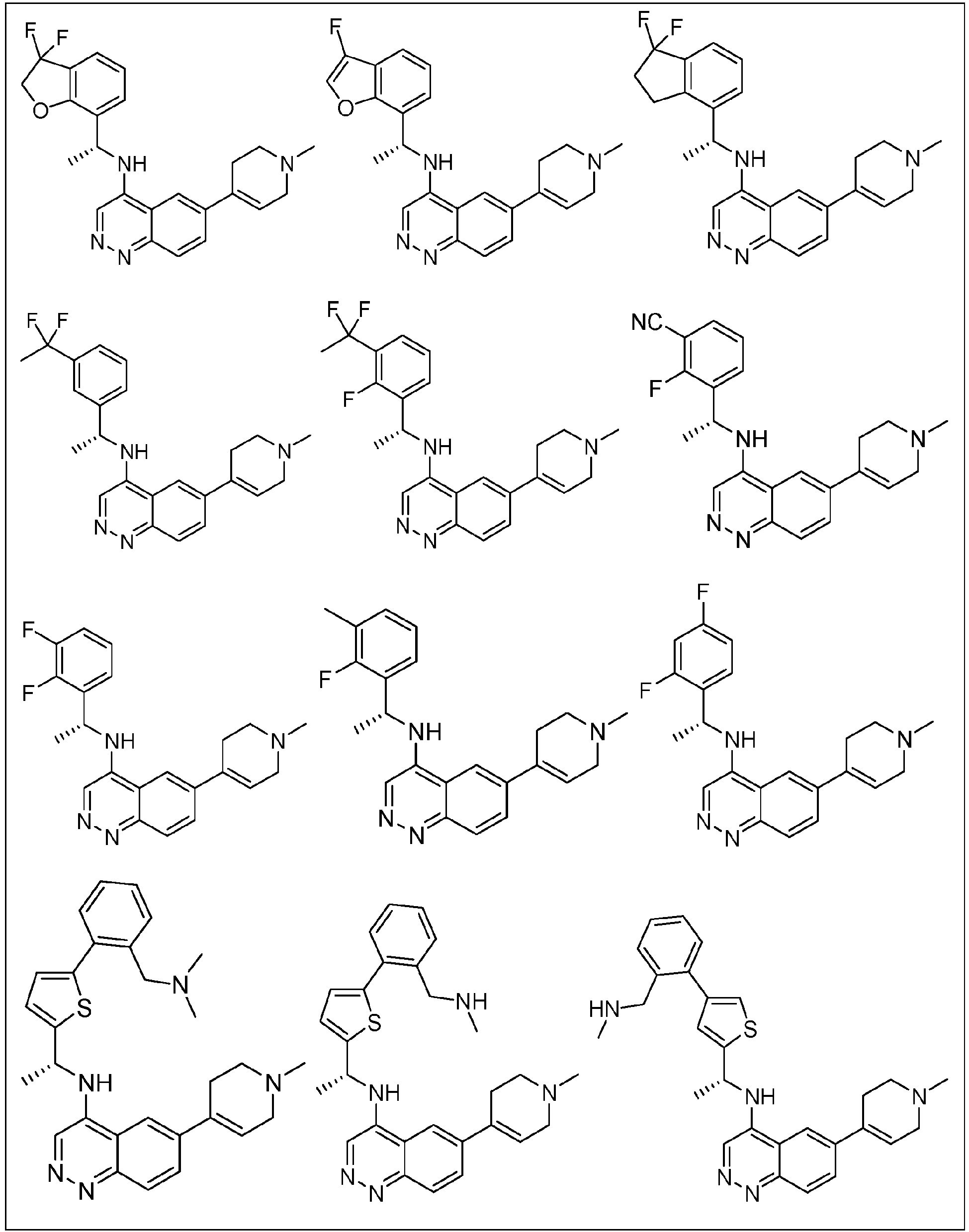

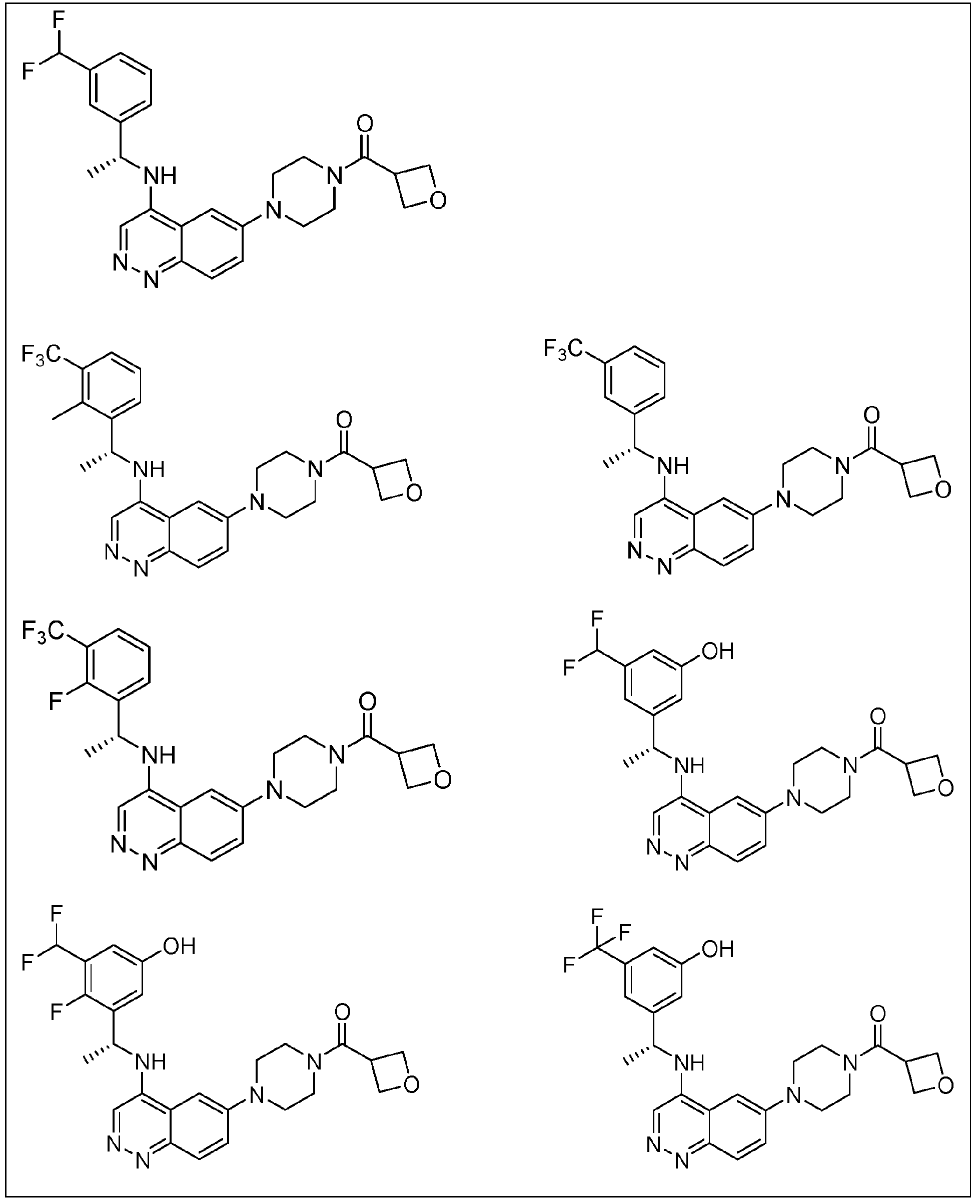

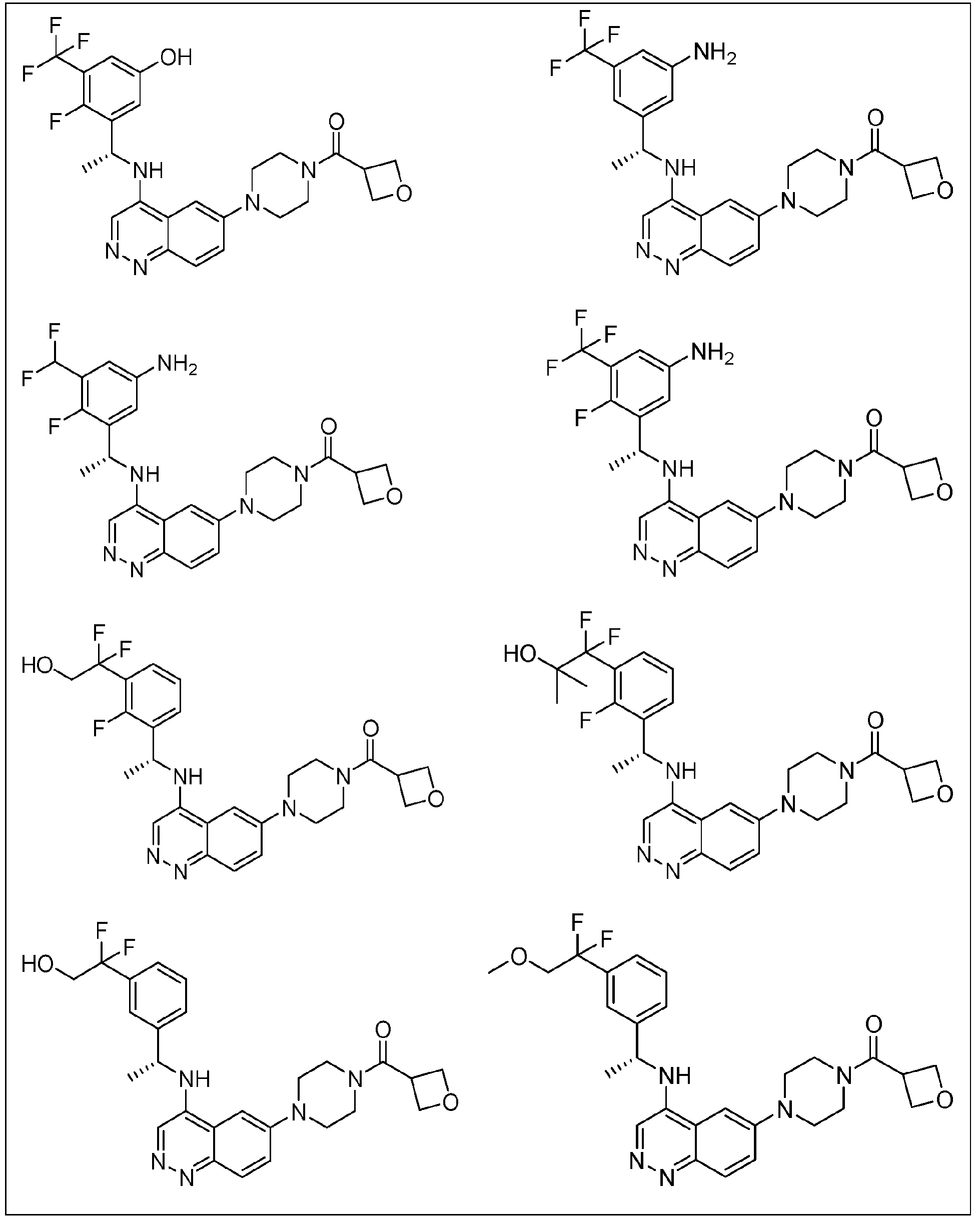

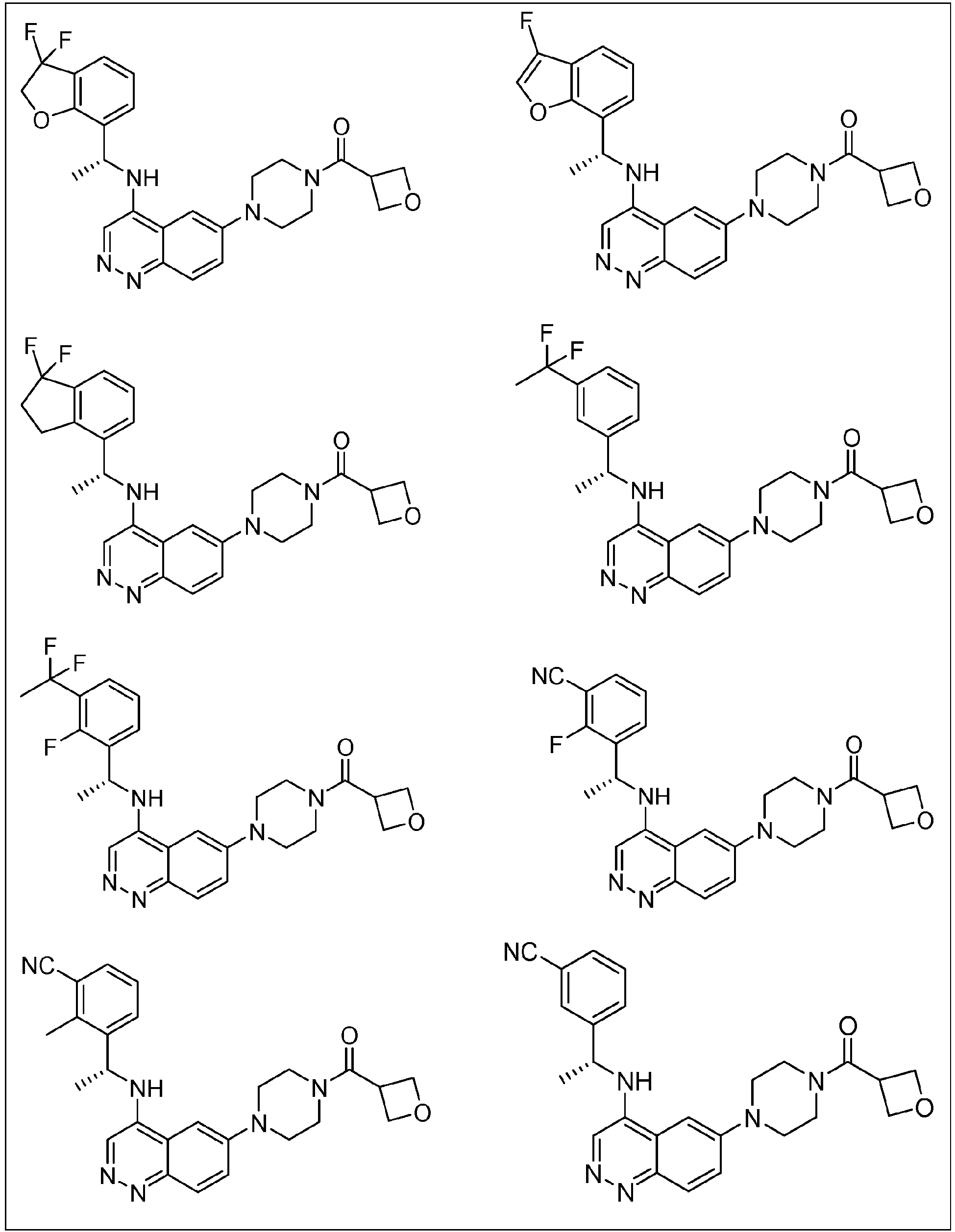

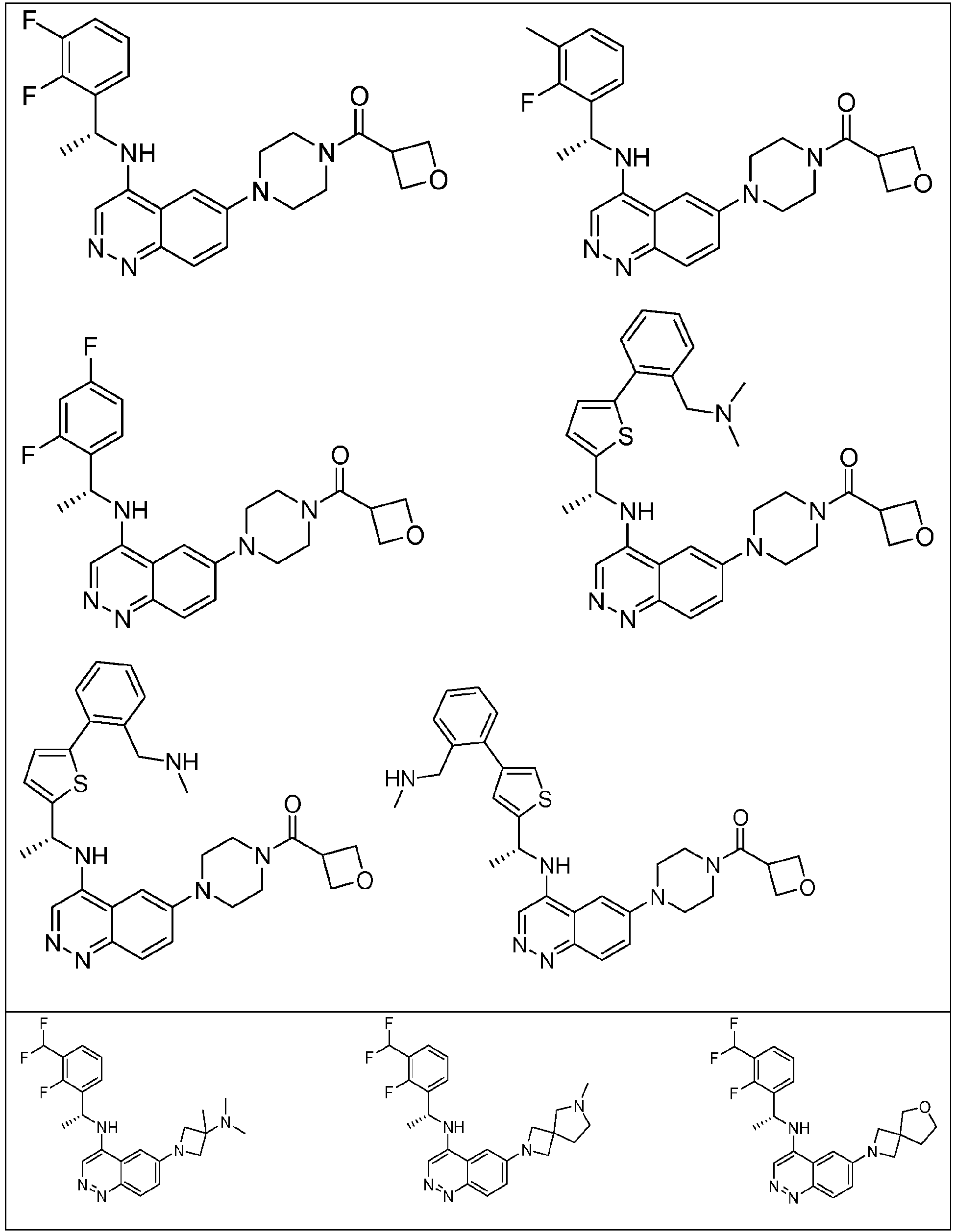

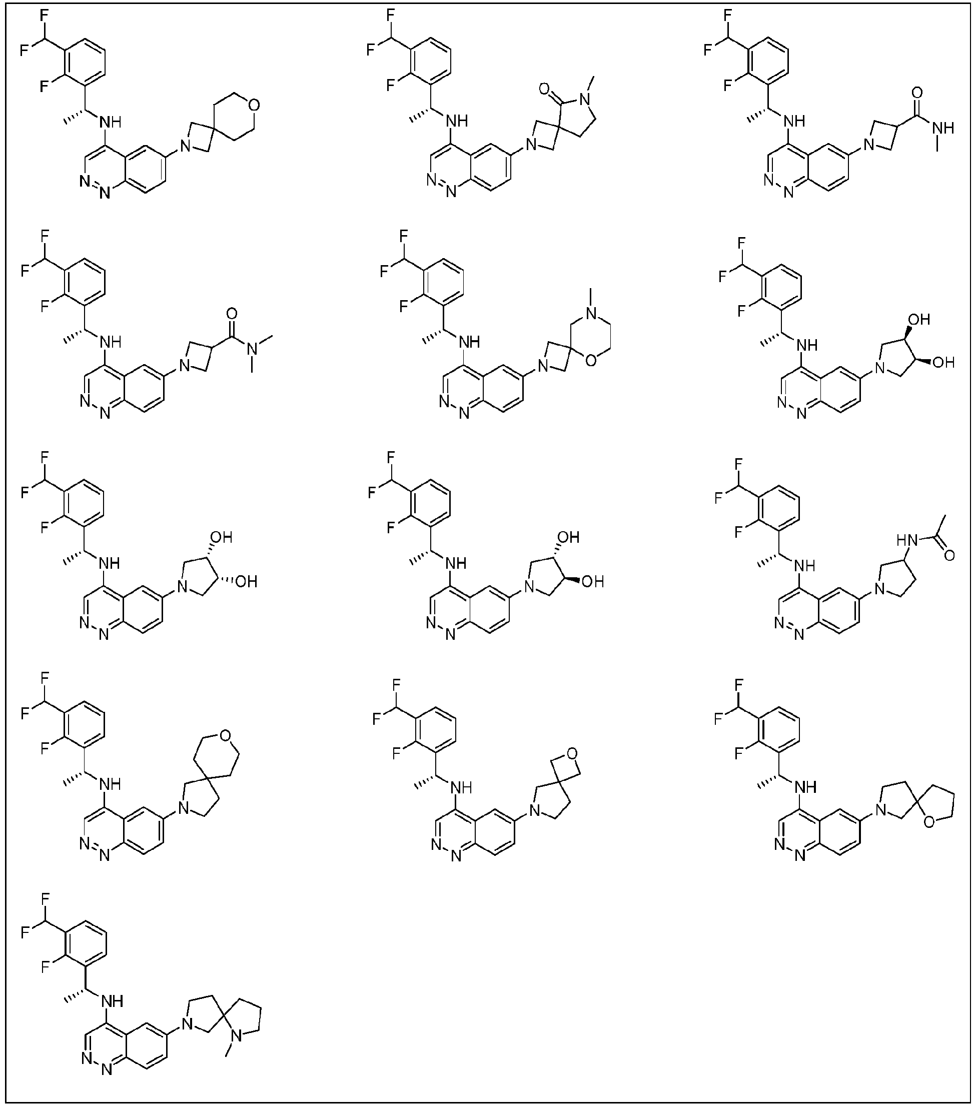

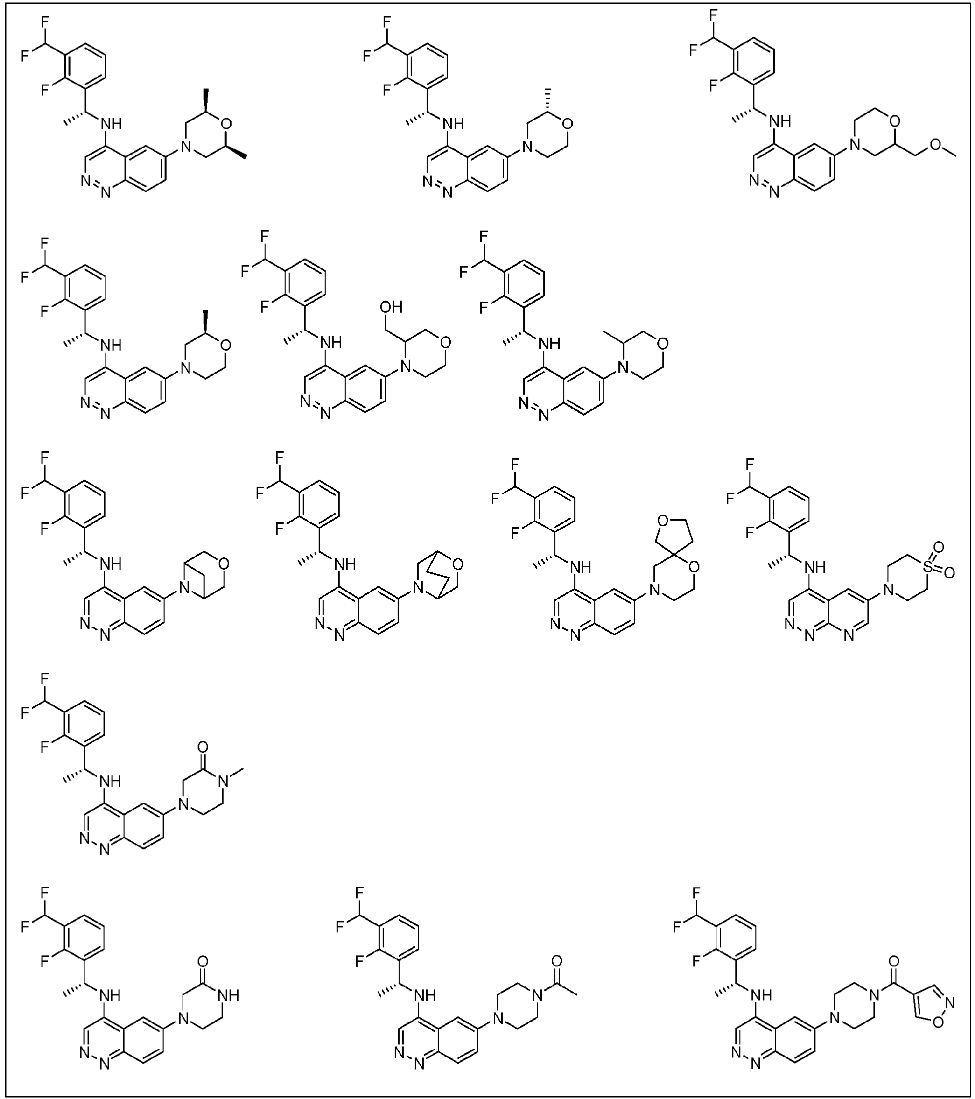

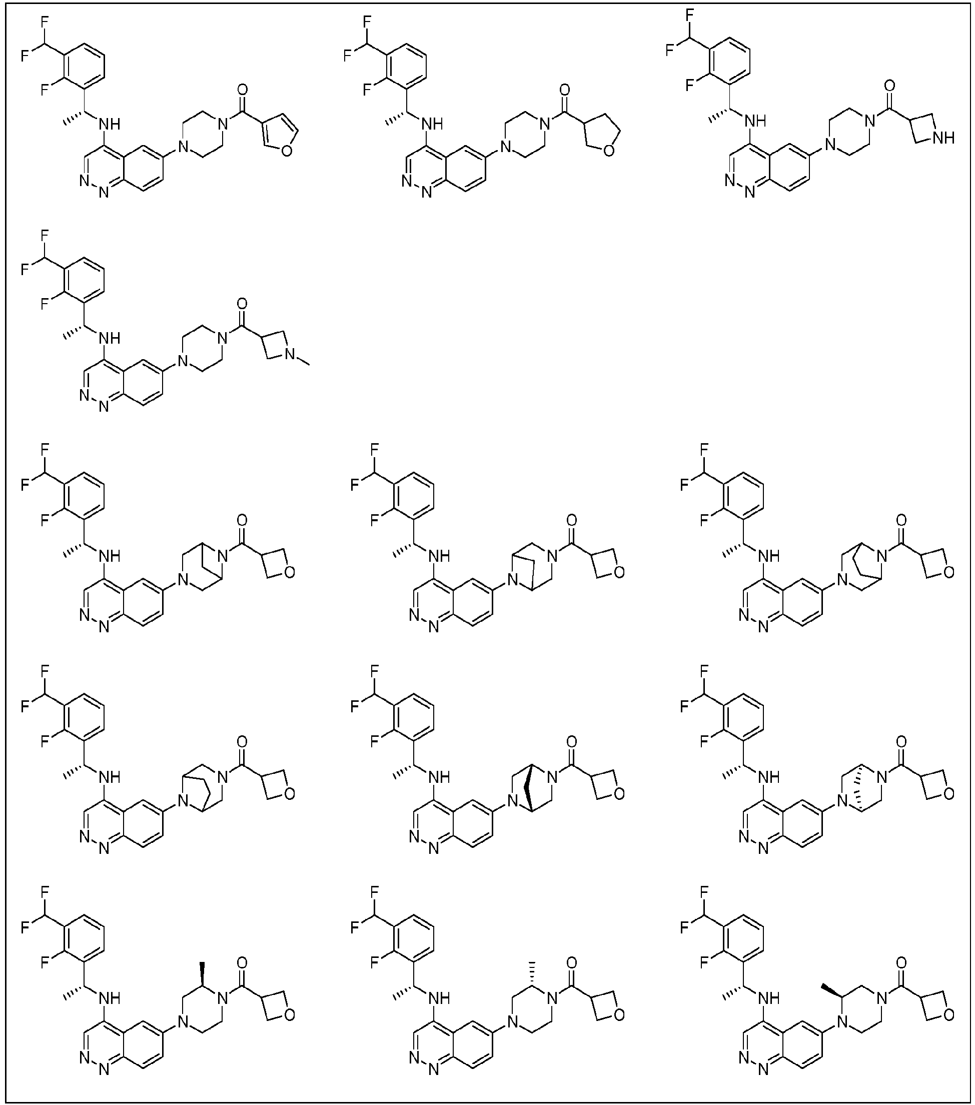

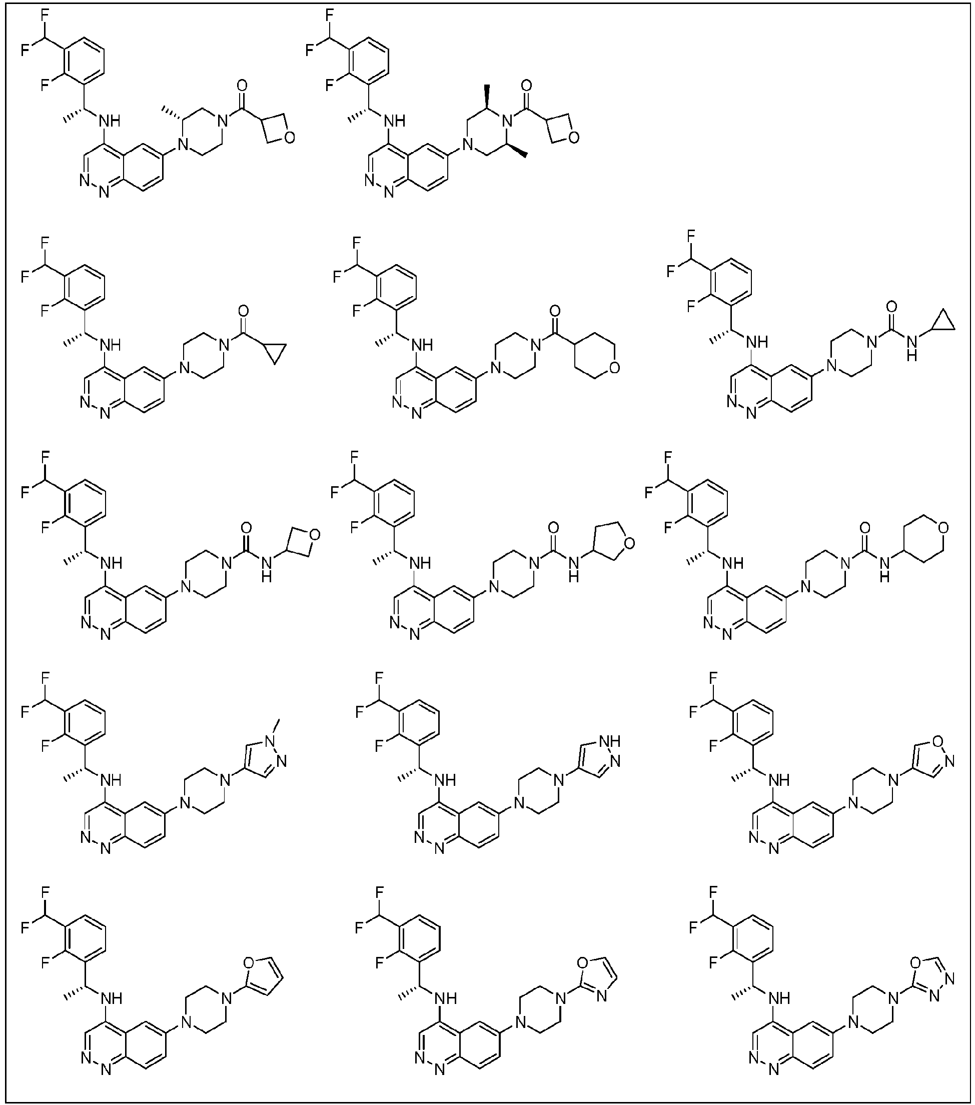

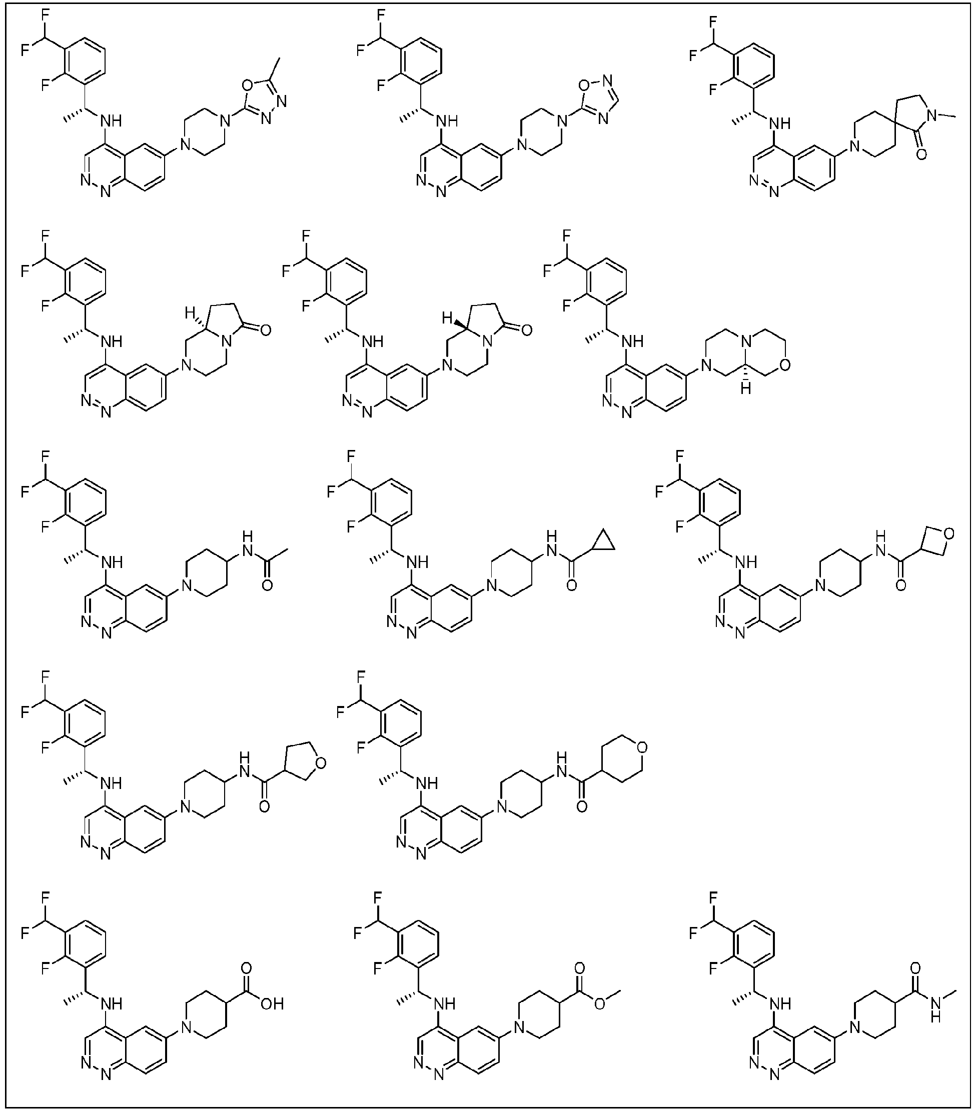

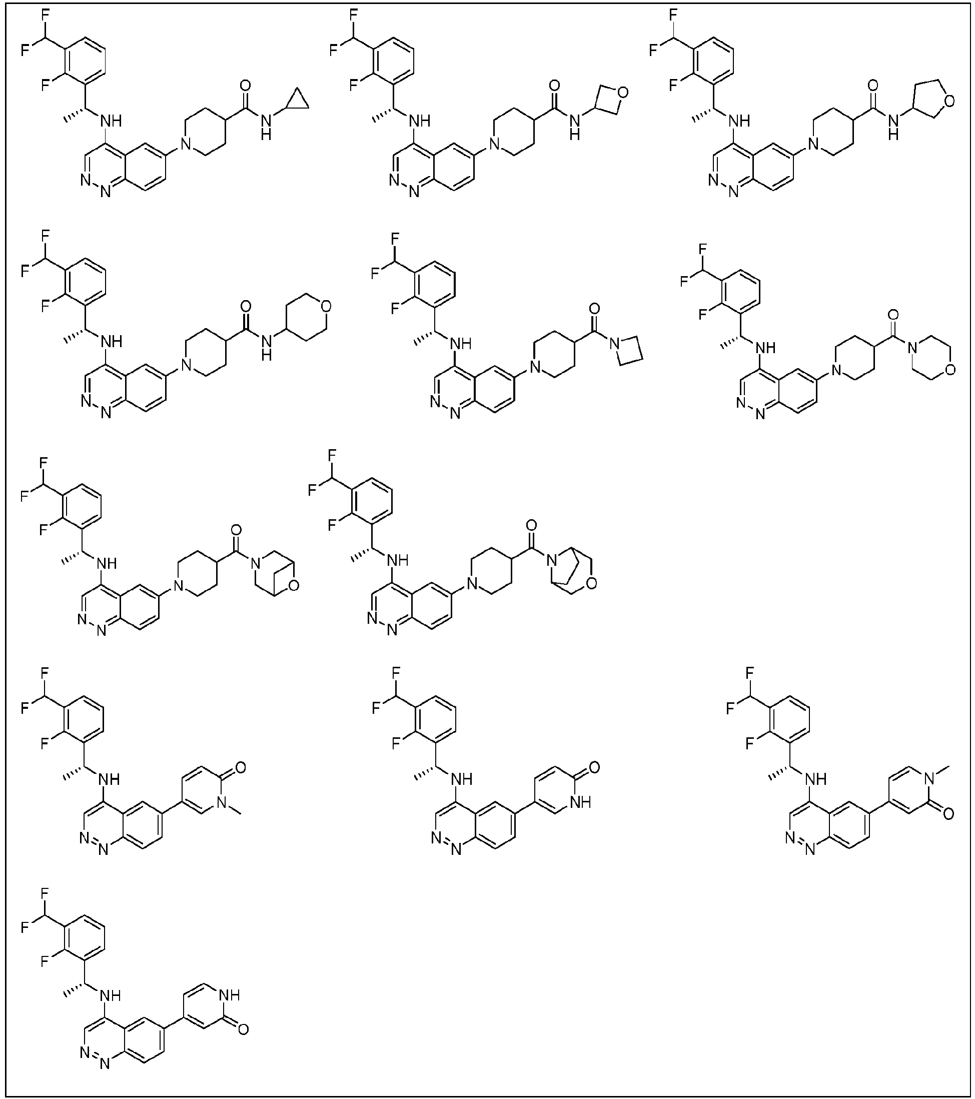

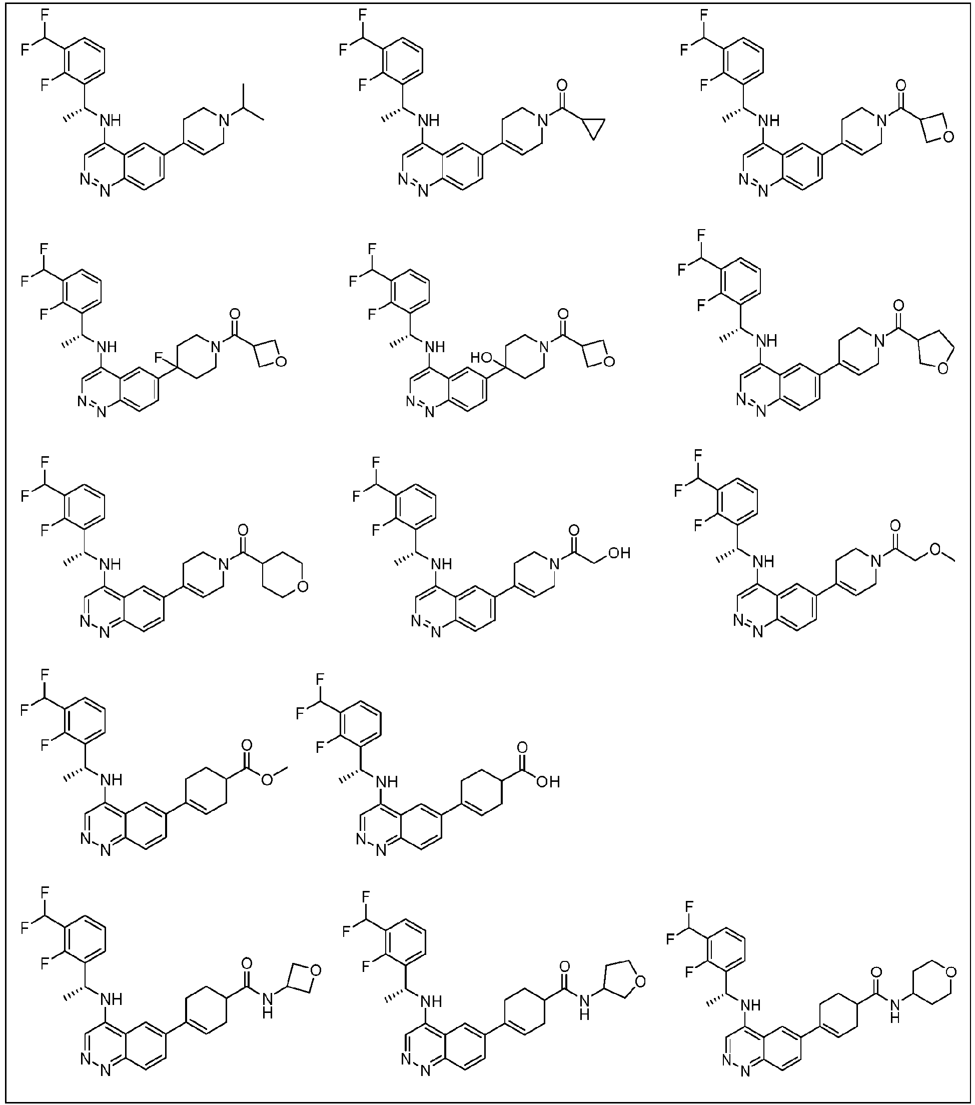

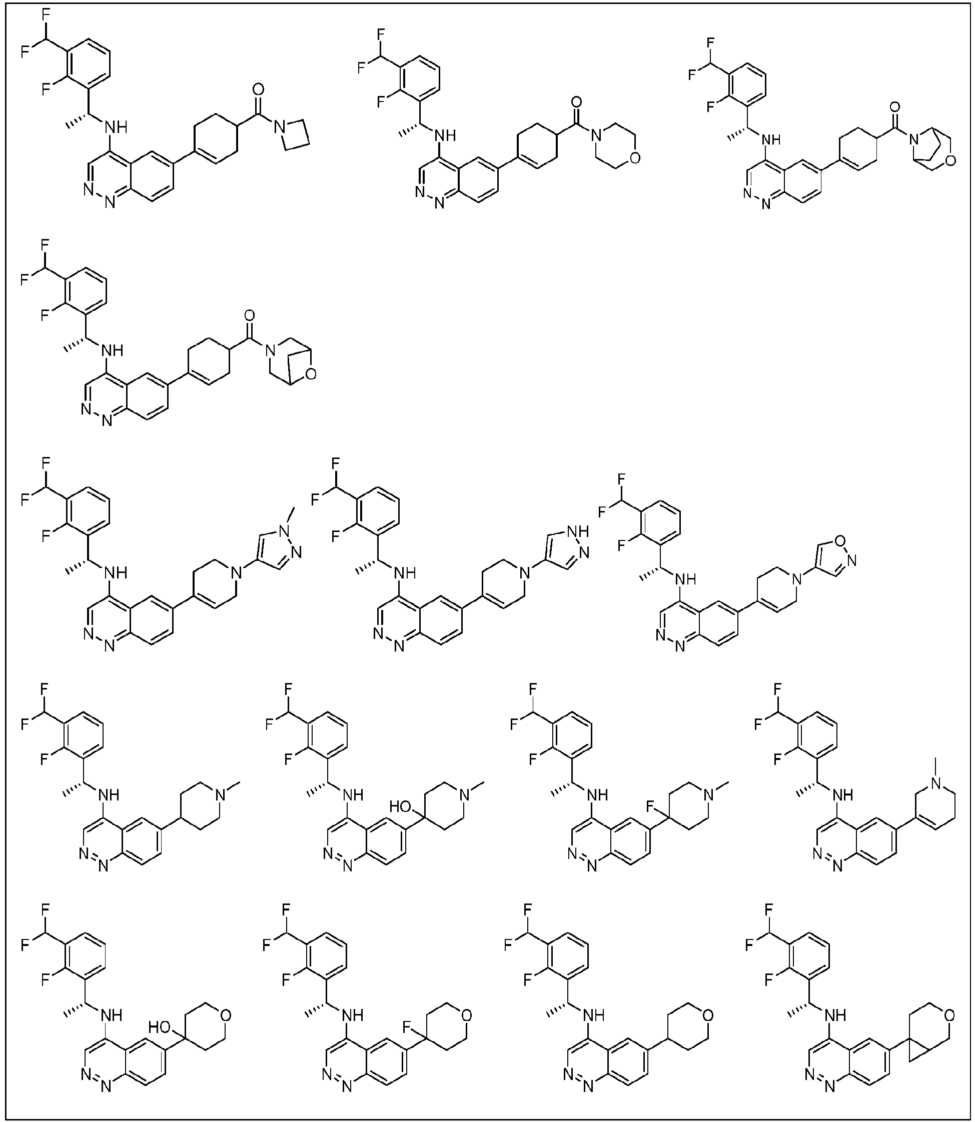

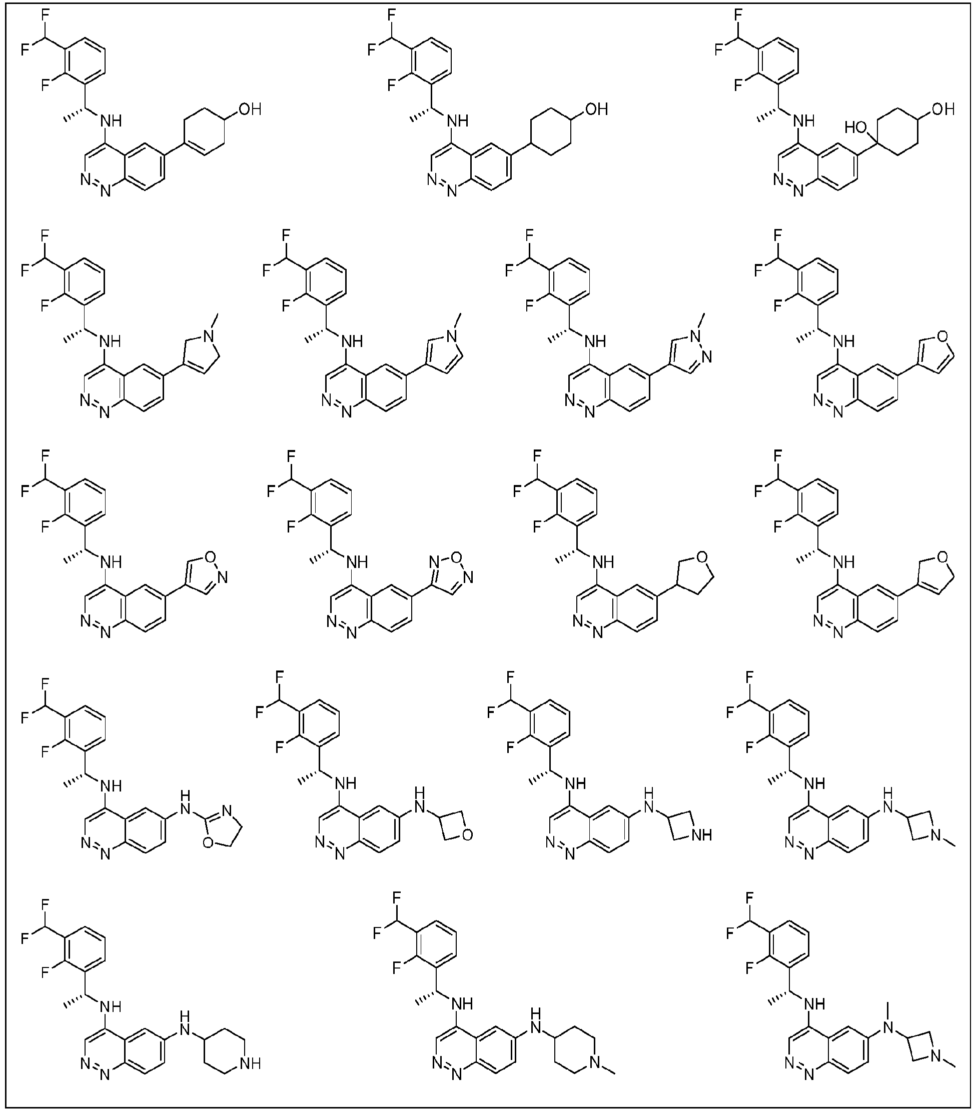

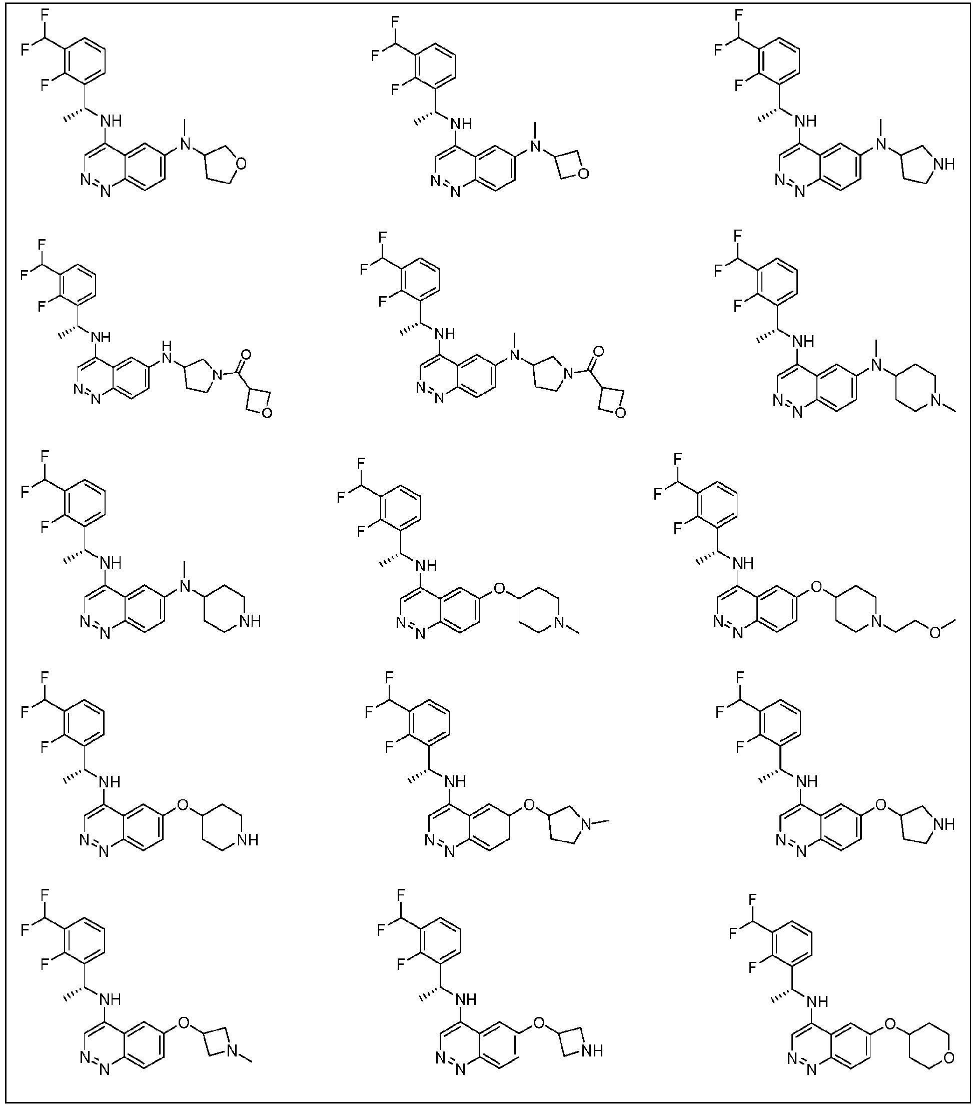

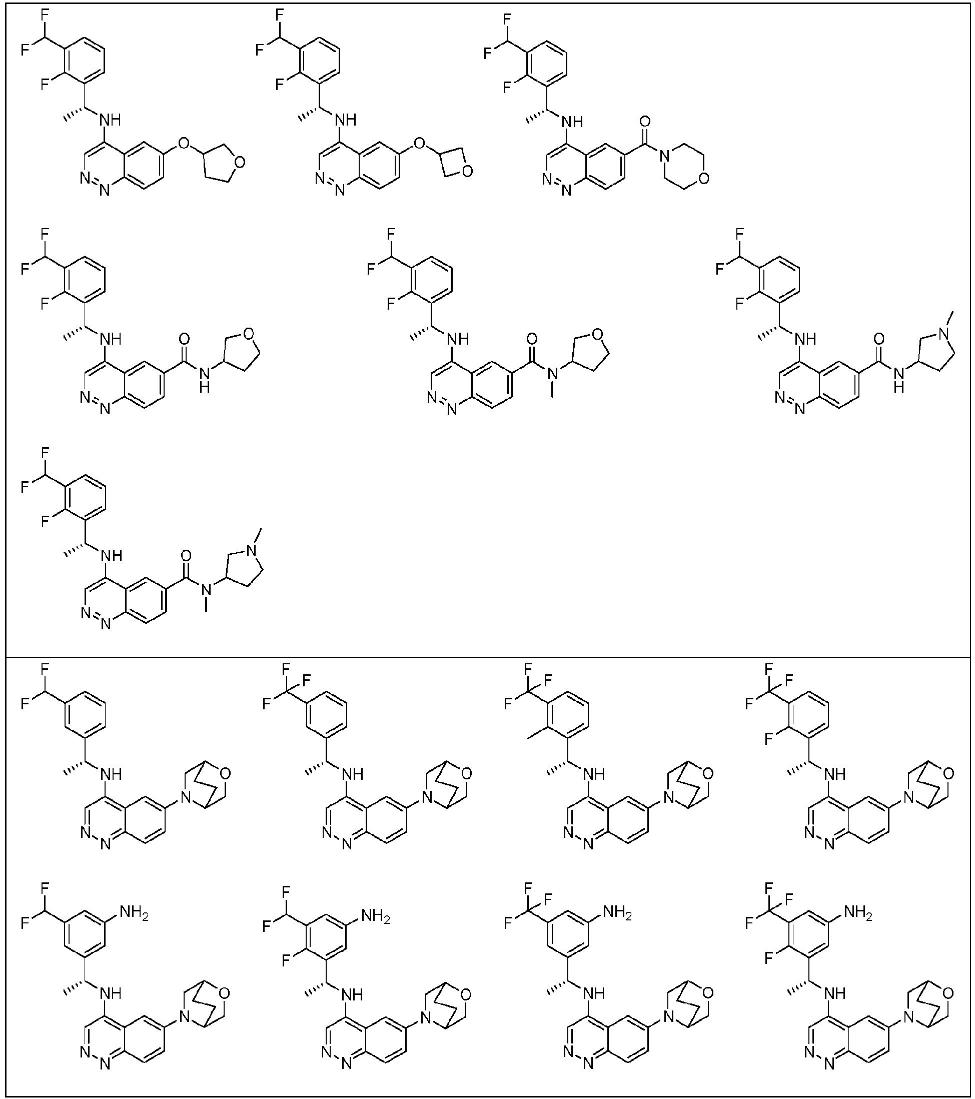

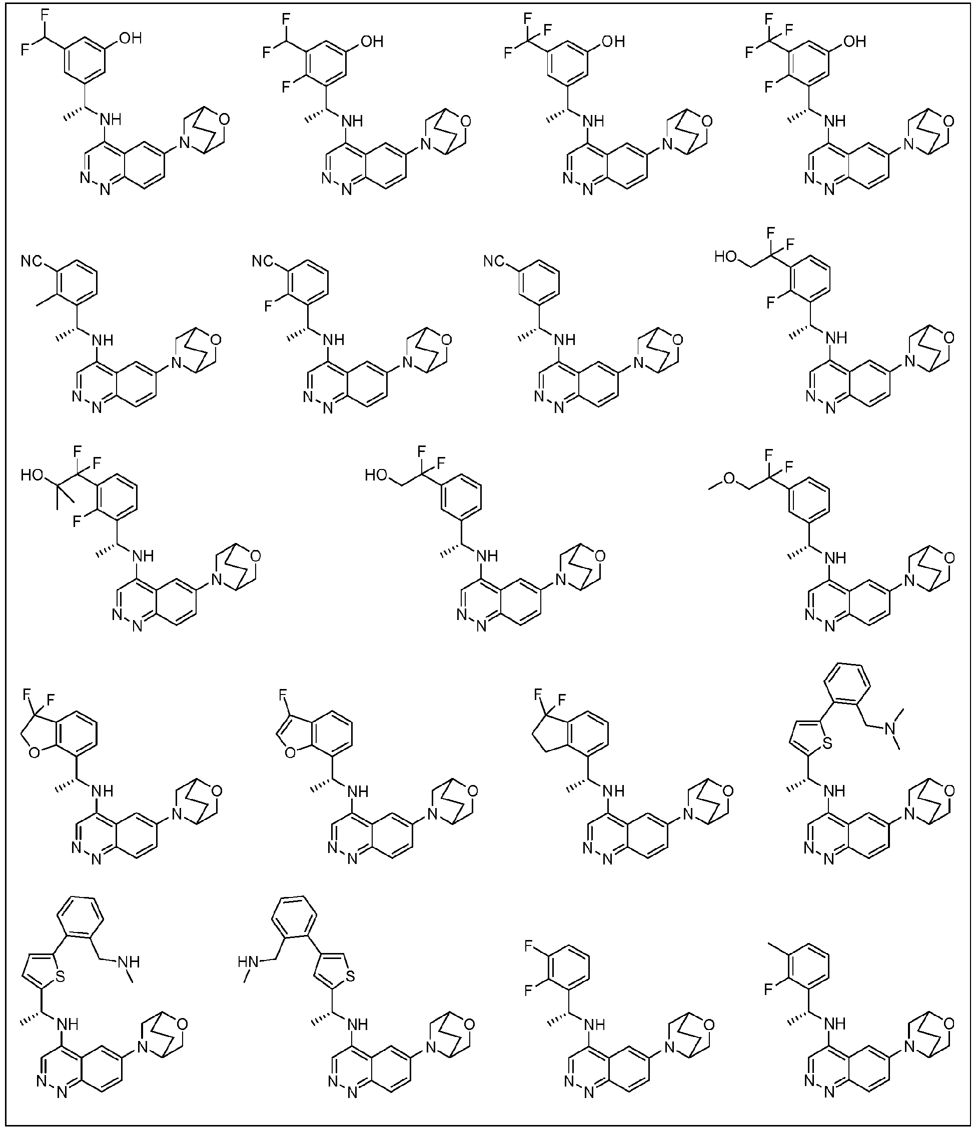

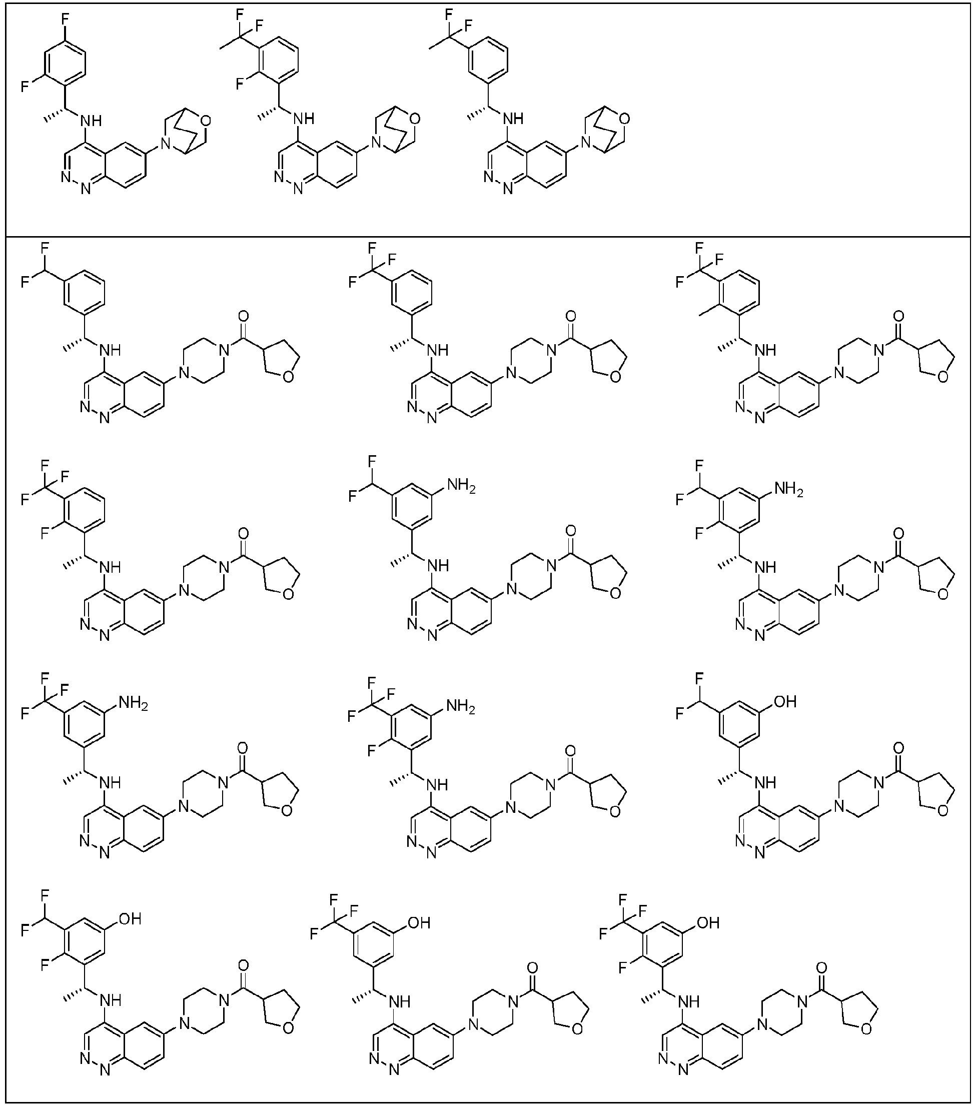

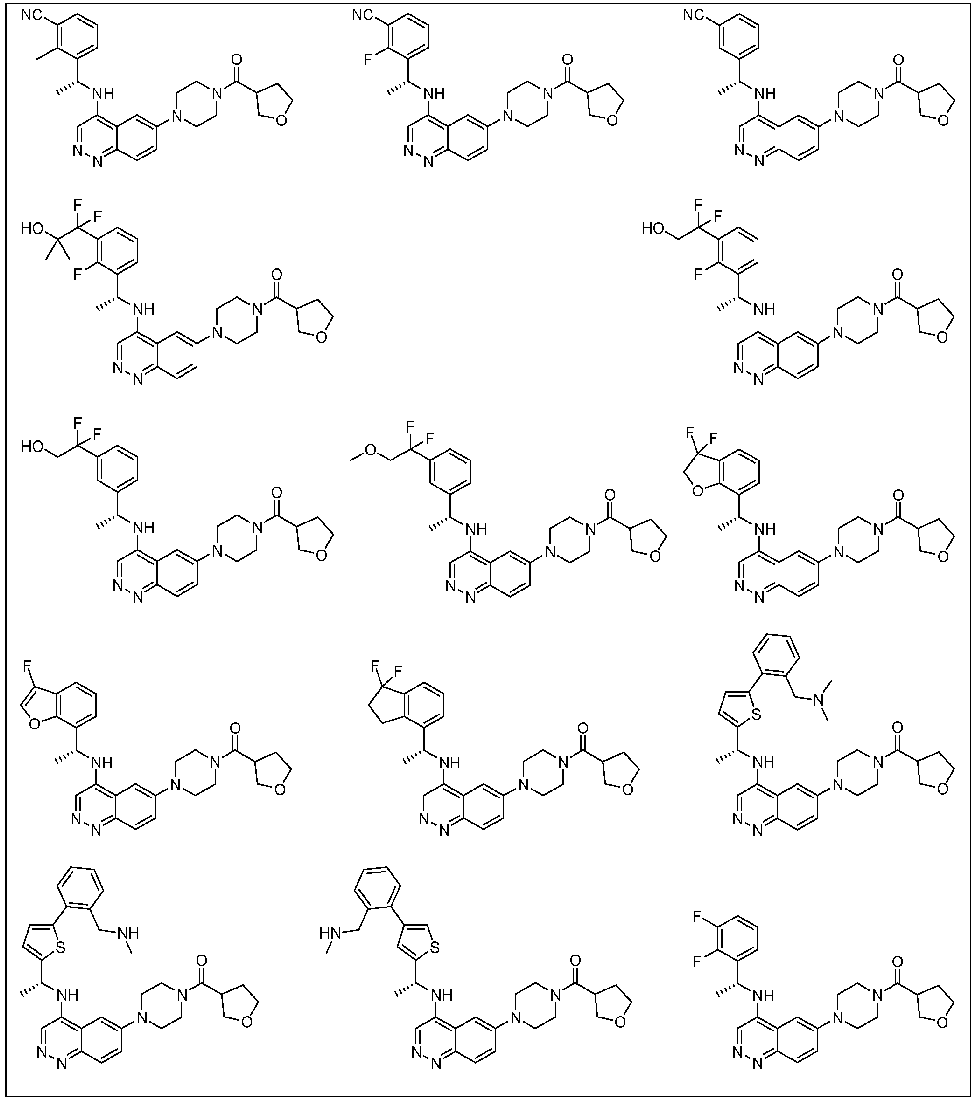

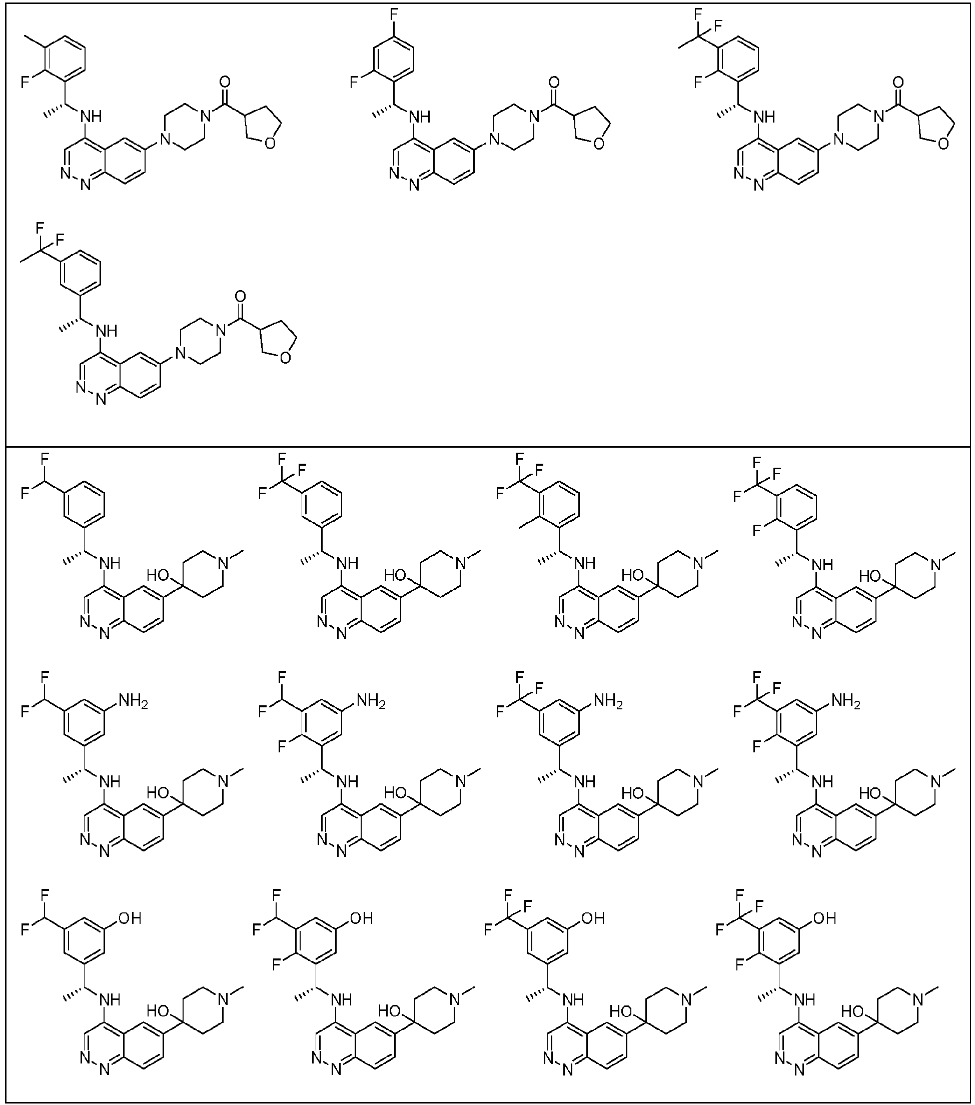

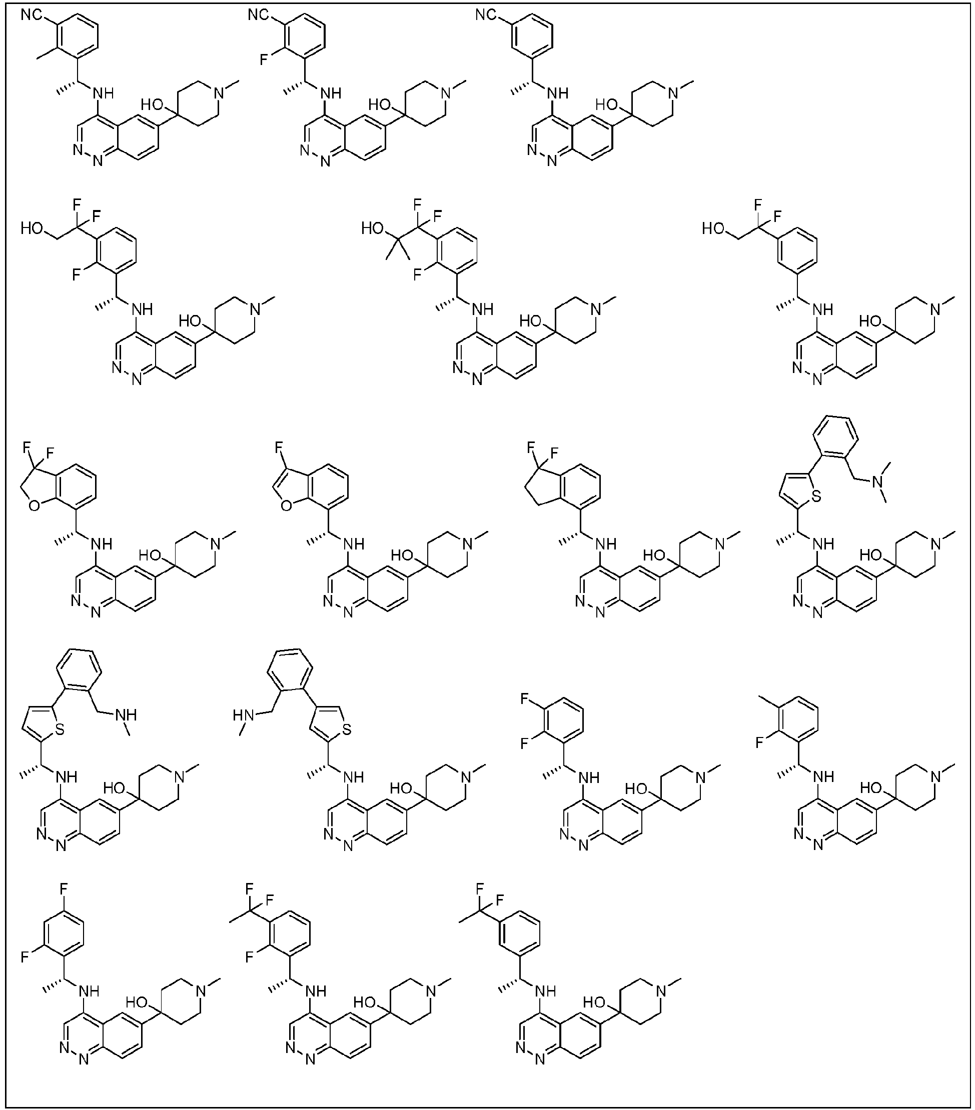

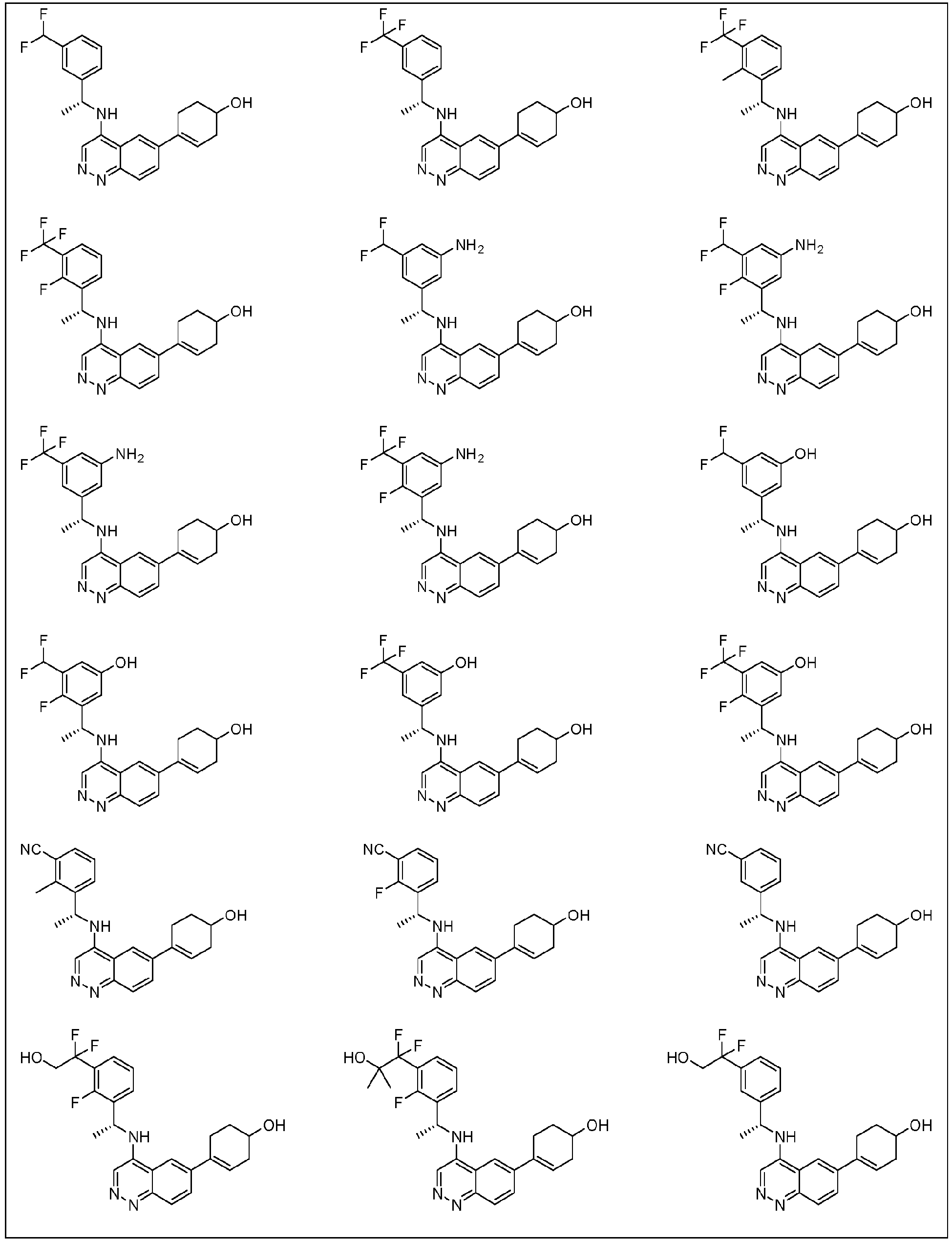

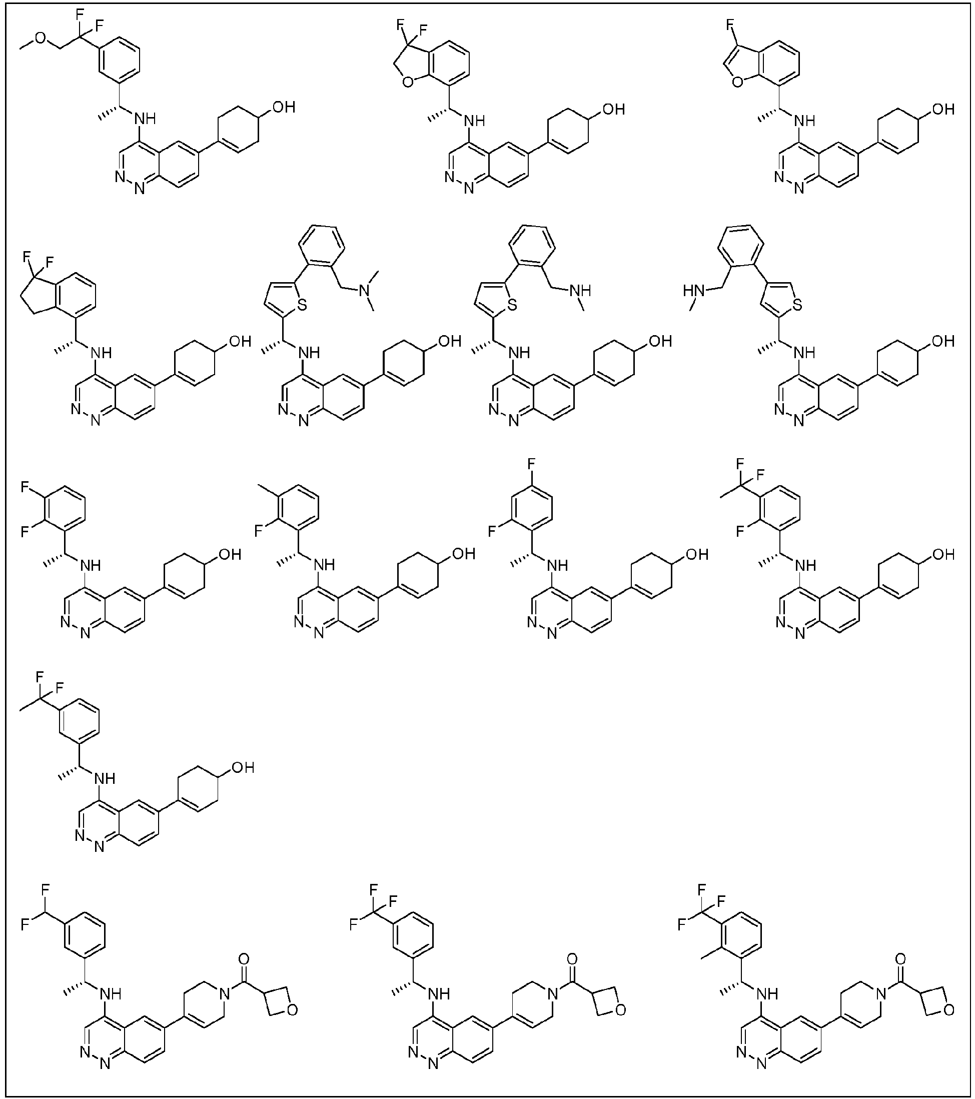

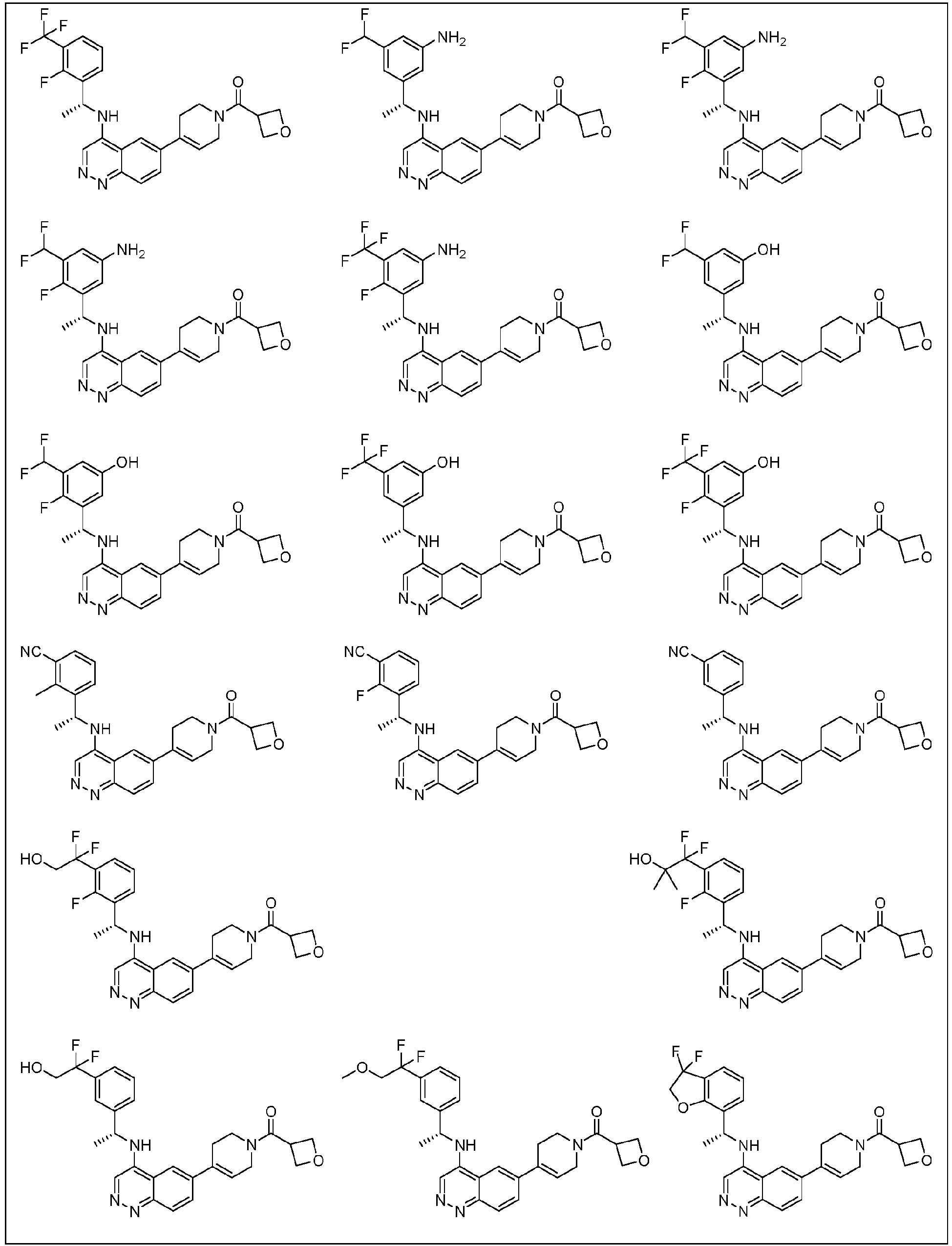

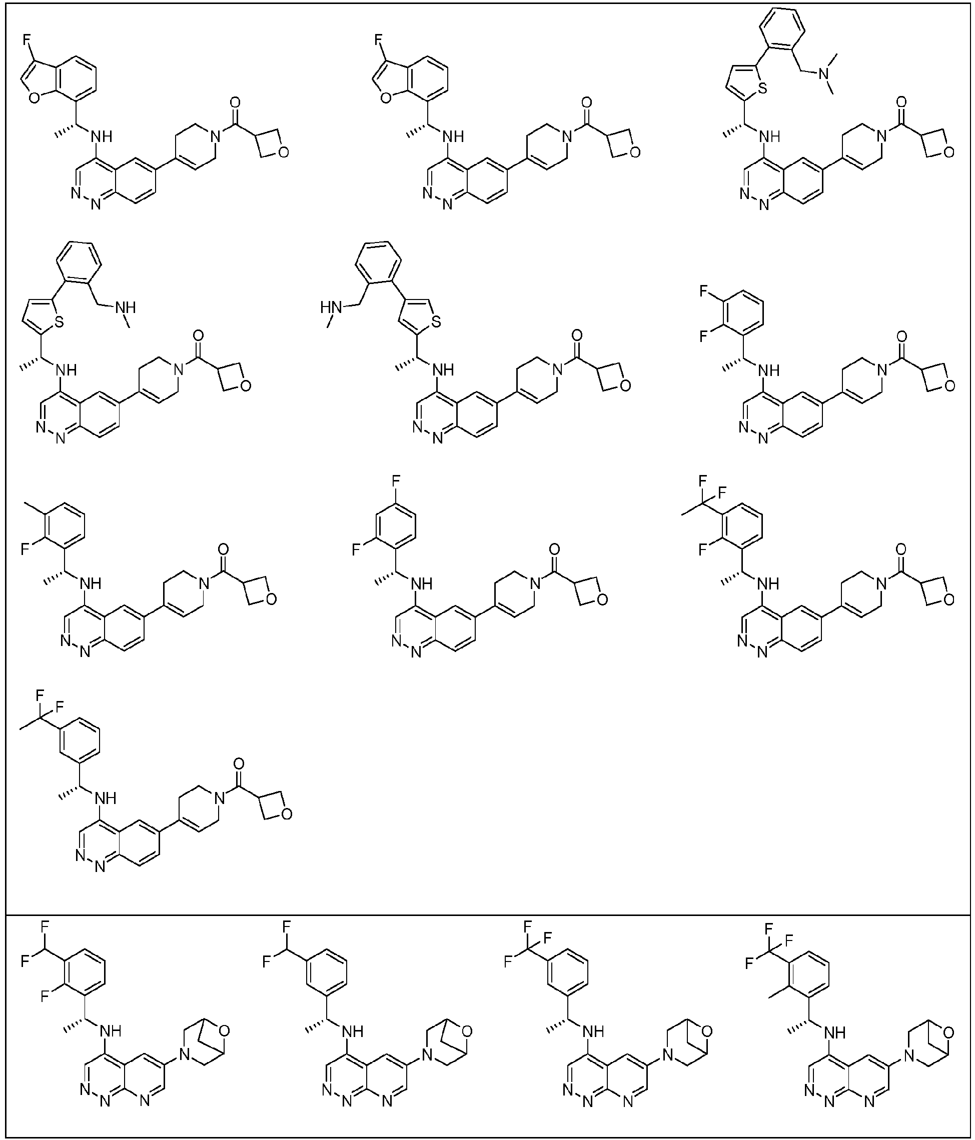

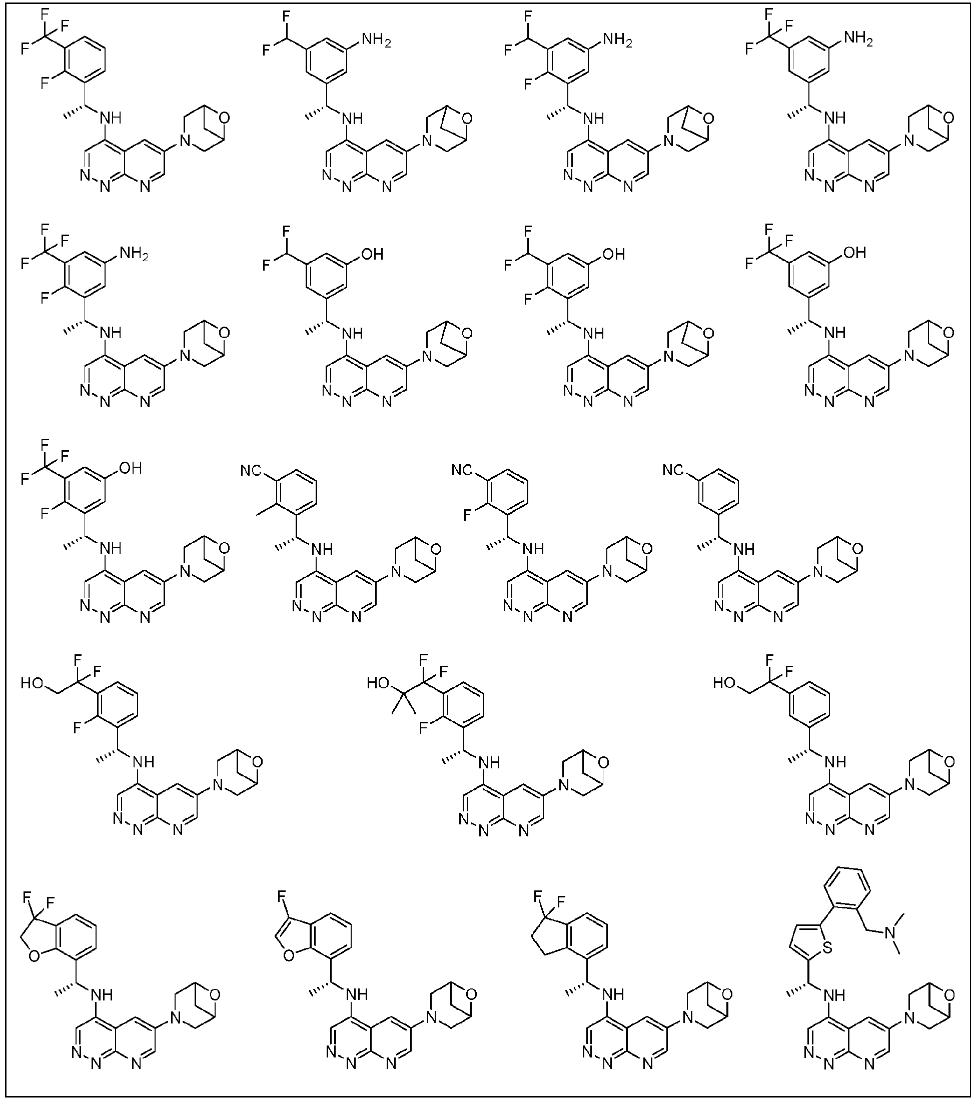

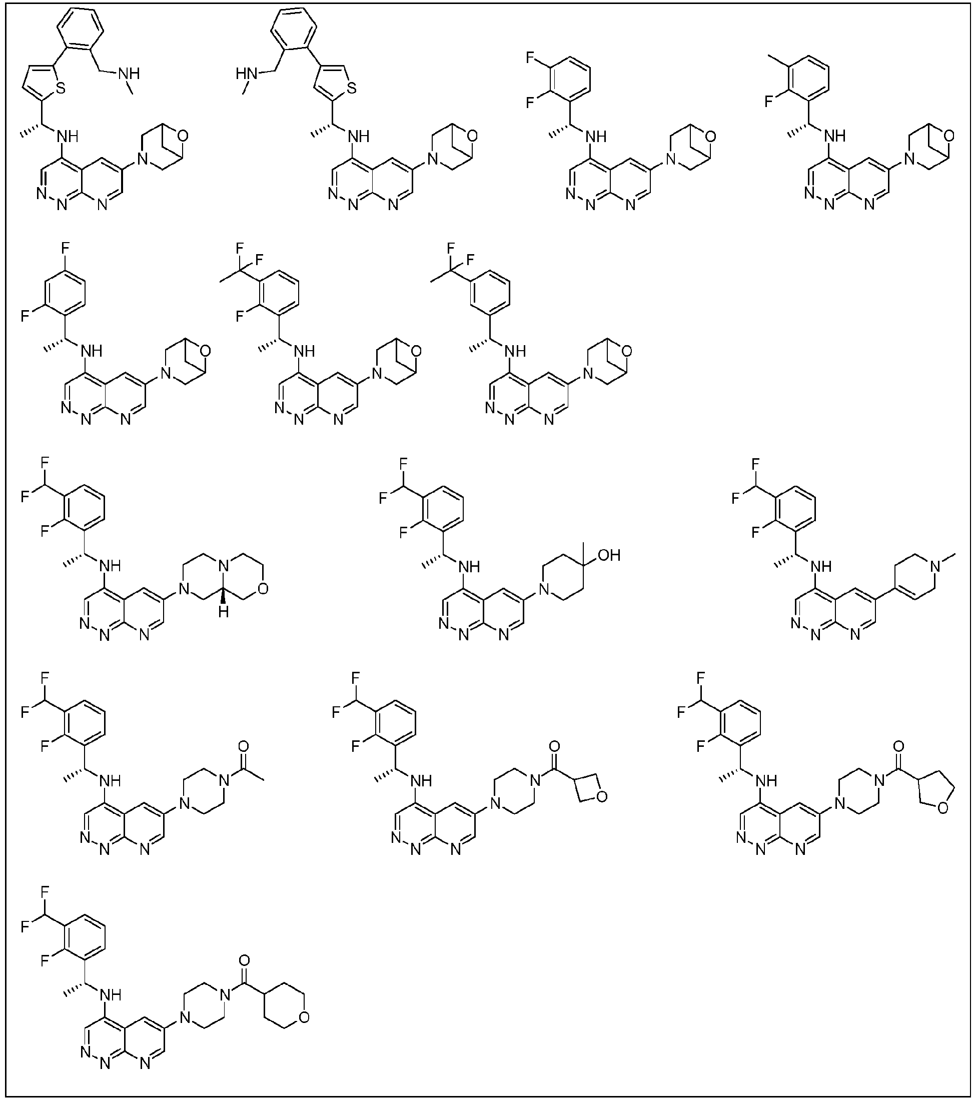

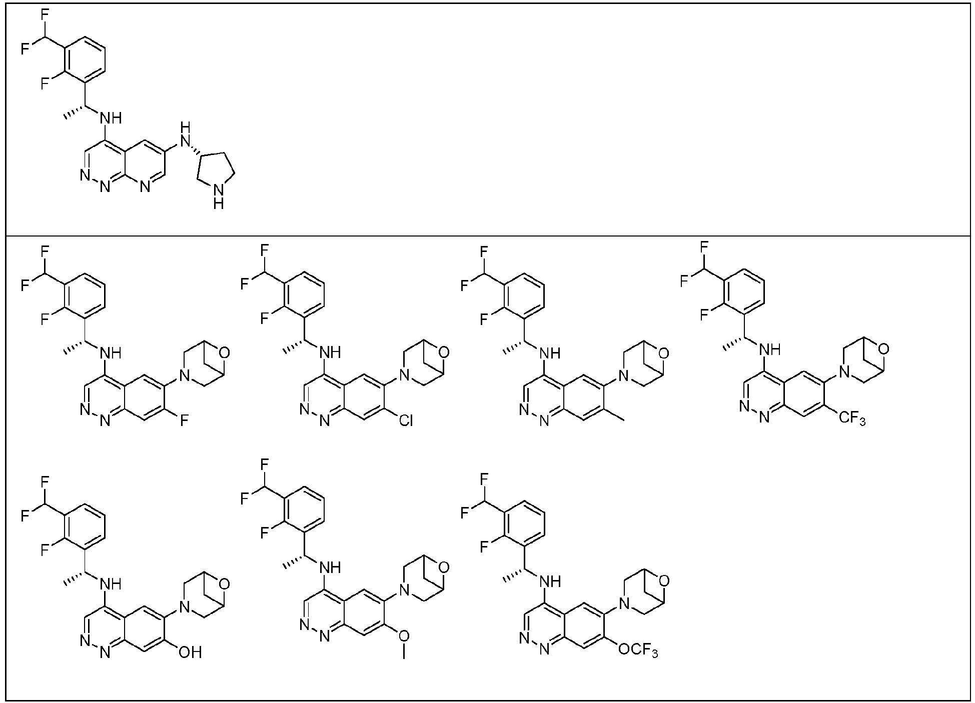

In the present invention, "pharmaceutically acceptable salt" may mean salt conventionally used in a pharmaceutical industry, and may be prepared by a conventional method known to those skilled in the art.

In the present invention, the pharmaceutically acceptable salt may include, for example, inorganic ion salt prepared from calcium, potassium, sodium, magnesium or the like; inorganic acid salt prepared from hydrochloric acid, nitric acid, phosphoric acid, bromic acid, iodic acid, perchloric acid, sulfuric acid or the like; organic acid salt prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbric acid, carbonic acid, vanillic acid, hydroiodic acid, etc.; sulfonic acid salt prepared from methanesulfonic acid, ethanesulfonic acid, benzenesulfonic acid, p-toluenesulfonic acid, naphthalenesulfonic acid or the like; amino acid salt prepared from glycine, arginine, lysine, etc.; amine salts prepared from trimethylamine, triethylamine, ammonia, pyridine, picoline, etc.; or the like, but types of salt meant in the present invention are not limited to those listed salts. In one embodiment of the present invention, said salt may be hydrochloride.

In the present invention, "stereoisomer" may include a diastereomer and an optical isomer (enantiomer), in which the optical isomer may include not only an enantiomer, but also a mixture of the enantiomer and even a racemate. Such isomer may be separated by split according to the related art, for example, column chromatography, HPLC or the like. Alternatively, a stereoisomer of each of the compound represented by formula 1 may be stereospecifically synthesized by using a known array of optically pure starting materials and/or reagents.

In the present invention, "prevention" may refer to all the acts, which inhibit or delay the occurrence of a disease by administering the compound represented by formula 1 of the present invention, stereoisomers thereof, or pharmaceutically acceptable salts thereof.

In the present invention, "treatment" may refer to all the acts, by which a symptom of an individual likely to develop or suffering from a disease gets better or takes a favorable turn by administering the compound represented by formula 1 of the present invention, stereoisomers thereof, or pharmaceutically acceptable salts thereof.

In the present invention, "SOS1" may be meant to include nucleic acids, polynucleotides, oligonucleotides, sense and antisense polynucleotide strands, complementary sequences, peptides, polypeptides, proteins, homologous and/or orthologous SOS1 molecules, isotypes, precursors, mutants, variants, derivatives, splice variants, alleles, other species, and active fragments thereof.

The compound represented by formula 1 of the present invention, stereoisomers thereof, or pharmaceutically acceptable salts thereof may be advantageously used for preventing, ameliorating or treating various diseases related to SOS1 activity caused by the SOS1 activity.

The compound represented by formula 1 of the present invention, stereoisomer thereof, or pharmaceutically acceptable salt thereof may inhibit/suppress SOS1 and may neutralize an RAS pathway to inhibit the growth and proliferation of cells in various diseases caused by the SOS1 activity, particularly cancer (tumor). Thus, they may show an excellent effect of preventing, ameliorating or treating various diseases caused by the SOS1 activity, particularly cancer (tumor).

The compound represented by formula 1 of the present invention, stereoisomer thereof, or pharmaceutically acceptable salt thereof may show an effect of preventing, ameliorating or treating the diseases related to SOS1 activity at a level similar to, substantially the same as, or higher than that of a conventionally known drug for preventing, alleviating or treating the diseases related to SOS1 activity.

### Composition including compound represented by formula 1

The present invention provides a pharmaceutical composition including the compound represented by above formula 1, optical isomer thereof, stereoisomer thereof, solvate thereof, isotopic variant thereof, tautomer thereof, or pharmaceutically acceptable salt thereof as an active ingredient.

In addition, the present invention provides a pharmaceutical composition including the compound represented by above formula 1, optical isomer thereof, stereoisomer thereof, solvate thereof, isotopic variant thereof, tautomer thereof, or pharmaceutically acceptable salt thereof as an active ingredient for preventing, alleviating or treating the diseases related to SOS1 activity.

In other words, the pharmaceutical composition including the compound represented by formula 1 of the present invention, optical isomer thereof, stereoisomer thereof, solvate thereof, isotopic variant thereof, tautomer thereof, or pharmaceutically acceptable salt thereof as an active ingredient may be advantageously used for preventing, alleviating or treating the diseases related to SOS1 activity.

The diseases related to SOS1 activity may include various diseases caused by SOS1 mutation, SOS1 overexpression, and SOS1 activity, and may include, for example, cancer (tumor).

Cancer may include lung cancer, pancreatic cancer, gastric cancer, myelodysplastic syndrome, blood cancer, leukemia including acute lymphocytic leukemia (ALL) and acute myeloid leukemia (AML), adrenal cancer, anal cancer, basosquamous cell skin cancer, bile duct cancer, bladder cancer, bone cancer, cerebrospinal tumor, breast cancer, cervical cancer, chronic lymphocytic leukemia (CLL), chronic myeloid leukemia (CML), chronic myelomonocytic leukemia (CMML), colorectal cancer, endometrial cancer, esophageal cancer, Ewing family of tumors, eye cancer, gallbladder cancer, gastrointestinal carcinoid tumor, gastrointestinal stromal tumor (GIST), gestational choriocarcinoma, glioma, Hodgkin's lymphoma, Kaposi's sarcoma, renal cancer, hypopharyngeal cancer, liver cancer, lung carcinoma, lymphoma comprising cutaneous T-cell lymphoma, malignant mesothelioma, melanoma skin cancer, Merkel cell skin cancer, multiple myeloma, nasal and paranasal cancer, nasopharyngeal cancer, neuroblastoma, non-Hodgkin's lymphoma, non-small cell lung cancer, oral and oropharyngeal cancer, osteosarcoma, ovarian cancer, penile cancer, pituitary tumor, prostate cancer, retinoblastoma, rhabdomyosarcoma, salivary gland cancer, skin cancer, small cell lung cancer, small intestine cancer, soft tissue sarcoma, gastric cancer, testicular cancer, thymic cancer, thyroid carcinoma comprising anaplastic thyroid carcinoma, uterine sarcoma, vaginal cancer, vulval cancer, Waldenstrom macroglobulinemia, Wilms tumor, embryo rhabdomyosarcoma, Sertoli cell testis tumor, skin granular cell tumor, lung adenocarcinoma, and the like.

In addition, the diseases related to SOS1 activity may include RAS diseases such as neurofibromatosis type 1 (NF1), noonan syndrome (NS), noonan syndrome with lentigines (NSML) (also referred to as LEOPARD syndrome), capillary malformation-arteriovenous malformation syndrome (CM-AVM), Costello syndrome (CS), cardio-facio-cutaneous (CFC) syndrome, Legius syndrome (also referred to as NF1-like syndrome), and hereditary gingival fibromatosis type 1.

The pharmaceutical composition of the present invention may further include at least one pharmaceutically acceptable carrier in addition to the compound represented by above formula 1, optical isomers thereof, stereoisomers thereof, solvates thereof, isotopic variants thereof, tautomers thereof, or pharmaceutically acceptable salts thereof. The pharmaceutically acceptable carrier may be one which is conventionally used in the art, specifically including, but not limited thereto, lactose, dextrose, sucrose, sorbitol, mannitol, starch, acacia rubber, calcium phosphate, alginate, gelatin, calcium silicate, microcrystalline cellulose, polyvinyl pyrrolidine, cellulose, water, syrup, methyl cellulose, methyl hydroxybenzoate, propyl hydroxybenzoate, talc, magnesium stearate, mineral, or oil. The pharmaceutical composition of the present invention may further include lubricants, humectants, sweetening agents, flavoring agents, emulsifiers, suspending agents, preservatives, dispersing agents, stabilizing agents, etc., in addition to the above ingredients. In addition, the pharmaceutical composition of the present invention may be formulated into an oral dosage form such as tablet, powder, granule, pill, capsule, suspension, emulsion, liquid for internal use, emulsion, syrup, etc., as well as a form of external application, suppository and sterile solution for injection by using a pharmaceutically acceptable carrier and excipient, and thus may be prepared in a unit dose form or prepared by being inserted into a multidose container. The preparations may be prepared according to a conventional method used for formulation in the art or a method disclosed in Remington's Pharmaceutical Science (19th ed., 1995), and may be formulated into various preparations depending on each disease or ingredient.

A non-limiting example of preparations for oral administration using the pharmaceutical composition of the present invention may include a tablet, troches, lozenge, water-soluble suspension, oil suspension, prepared powder, granule, emulsion, hard capsule, soft capsule, syrup, elixirs or the like. To formulate the pharmaceutical composition of the present invention into a preparation for oral administration, the followings may be used: binders such as lactose, saccharose, sorbitol, mannitol, starch, amylopectin, cellulose, gelatin or the like; excipients such as dicalcium phosphate, etc.; disintegrants such as maize starch, sweet potato starch or the like; lubricants such as magnesium stearate, calcium stearate, sodium stearyl fumarate, polyethylene glycol wax, or the like; etc., in which sweetening agents, flavoring agents, syrups, etc. may also be used. Furthermore, in case of the capsule, liquid carriers such as fatty oil, etc. may be further used in addition to the above-mentioned materials.

A non-limiting example of parenteral preparations using the pharmaceutical composition of the present invention may include an injectable solution, suppository, powder for respiratory inhalation, aerosol for spray, ointment, powder for application, oil, cream, etc. To formulate the pharmaceutical composition of the present invention into a preparation for parenteral administration, the followings may be used: a sterilized aqueous solution, non-aqueous solvent, suspension, emulsion, freeze-dried preparation, external preparation, etc. As said non-aqueous solvent and suspension, the followings may be used, but without limitation thereto: propylene glycol, polyethylene glycol, vegetable oil such as olive oil, injectable ester such as ethyl oleate, etc. The present invention provides a method for preventing, ameliorating or treating the diseases related to SOS1 activity including administering the compound represented by above formula 1, optical isomer thereof, stereoisomer thereof, solvate thereof, isotopic variant thereof, tautomer thereof, or pharmaceutically acceptable salt thereof, or the composition including the same into an individual (in need thereof).

In the present invention, "administration" refers to introducing a predetermined substance into an individual by an appropriate method.

In the present invention, "individual" refers to all the animals such as rats, mice, livestock, etc., including humans, who have already developed or are likely to develop the diseases related to SOS1 activity, and specifically may refer to mammals including humans, but is not limited thereto.

The method for preventing, ameliorating or treating the diseases related to SOS1 activity of the present invention may include administering a therapeutically effective amount of the compound represented by above formula 1, optical isomer thereof, stereoisomer thereof, solvate thereof, isotopic variant thereof, tautomer thereof, or pharmaceutically acceptable salt thereof, or the pharmaceutical composition including the same.

In the present invention, the term "therapeutically effective amount" refers to an amount enough to treat a disease at a reasonable risk/benefit ratio applicable to medical treatment and not to cause a side effect, and may be determined by those skilled in the art according to factors including a patient's gender, age, weight and health condition, a type of disease, severity, activity of a drug, sensitivity to a drug, an administration method, an administration time, an administration route, an excretion rate, a treatment period, a drug combined or concurrently used, as well as other factors well known in a pharmaceutical field. It is preferable to differently apply a specific therapeutically effective amount for a certain patient depending on various factors including a type and degree of reaction to be achieved therefrom, a specific composition including a presence of other preparations used in some cases, a patient's age, weight, general health condition, gender and diet, an administration time, an administration route, a secretion rate of the composition, a treatment period and a drug used together with the specific composition or simultaneously therewith, as well as other similar factors well known in a pharmaceutical field.

The present invention provides a use of the compound represented by above formula 1, optical isomer thereof, stereoisomer thereof, solvate thereof, isotopic variant thereof, tautomer thereof, or pharmaceutically acceptable salt thereof, or the composition including the same for preventing, ameliorating or treating the diseases related to SOS1 activity.

The present invention provides a use of the compound represented by above formula 1, optical isomer thereof, stereoisomer thereof, solvate thereof, isotopic variant thereof, tautomer thereof, or pharmaceutically acceptable salt thereof, or the composition including the same in the preparation of a medicament for preventing, ameliorating or treating the diseases related to SOS 1 activity.

In the preparation of a medicament for preventing, ameliorating or treating the diseases related to SOS1 activity, the compound represented by above formula 1, optical isomer thereof, stereoisomer thereof, solvate thereof, isotopic variant thereof, tautomer thereof, or pharmaceutically acceptable salt thereof may be mixed with pharmaceutically acceptable adjuvants, diluents, carriers, etc., and may be prepared into a complex preparation together with other active agents, thus providing a synergy action.

Matters mentioned in the compound, pharmaceutical composition, treatment method and use of the present invention may be applied the same, if not contradictory to each other.

### Advantageous Effects

A compound represented by formula 1 of the present invention, optical isomer thereof, stereoisomer thereof, solvate thereof, isotopic variant thereof, tautomer thereof, or pharmaceutically acceptable salt thereof; and a pharmaceutical composition including the same as an active ingredient may be advantageously used for preventing, ameliorating or treating diseases related to SOS1 activity.

### Mode for Invention

Hereinafter, the present invention will be described in more detail through exemplary embodiments. These exemplary embodiments are provided only for the purpose of illustrating the present invention, and thus it will be apparent to those skilled in the art that the scope of the present invention is not limited thereto.

### Preparation of compound represented by formula 1

The compound represented by formula 1 of the present invention may be prepared through the method described below. A starting material may be commercially available or may be prepared by known methods, unless otherwise described. The use of all examples provided herein or exemplary languages is merely intended to better illustrate the invention and does not limit the scope of the claimed invention.

### <Intermediate>

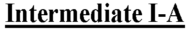

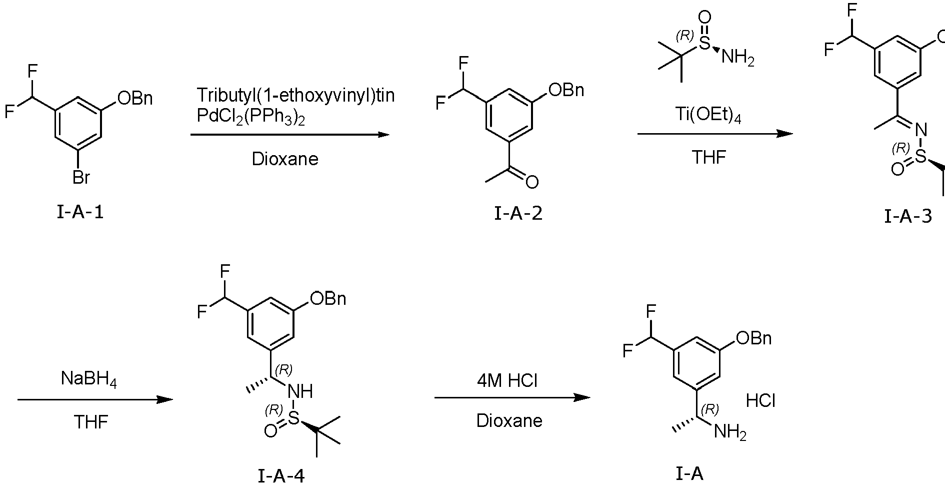

### Step 1

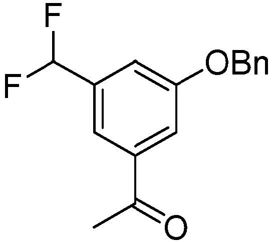

### 1-(3-(benzyloxy)-5-(difluoromethyl)phenyl)ethanone

To a pressure flask containing a solution of 1-(benzyloxy)-3-bromo-5-(difluoromethyl)benzene (1.17 mg, 3.74 mmol) in dioxane (11 ml), tributyl(1-ethoxyvinyl)tin (1.3 ml, 3.9 mmol) and PdCl₂(PPh₃)₂ (262 mg, 0.37 mmol) were added at room temperature and stirred for 16 hours while heating to 80°C. After cooling to room temperature, an aqueous 1 N HCl solution was added and stirred for one hour, and then extracted with ethyl acetate. The combined organic extracts were dried over sodium sulfate and concentrated. The residue was purified by column chromatography to afford 1-(3-(benzyloxy)-5-(difluoromethyl)phenyl)ethanone (1.03 g, 100%) as a yellow solid.

### Step 2

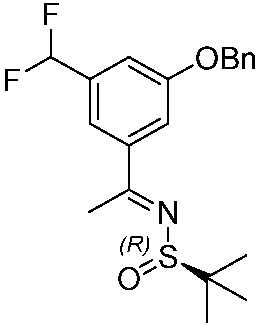

### (R,E)-N-(1-(3-(benzyloxy)-5-(difluoromethyl)phenyl)ethylidene)-2-methylpropan-2-sulfinamide

To a solution in which 1-(3-(benzyloxy)-5-(difluoromethyl)phenyl)ethanone (1 g, 3.62 mmol) was dissolved in tetrahydrofuran (10 ml), (R)-(+)-2-methyl-2-propanesulfinamide (658 mg, 5.43 mmol) and Ti(OEt)₄ (1.5 ml, 7.24 mmol) were added at room temperature and stirred for 16 hours while heating to 80°C. After cooling to room temperature, the solid precipitated when cold water was added was filtered out, dissolved in ethyl acetate, and then filtered through a celite pad. The filtrate was concentrated under vacuum without column purification to afford (R,E)-N-(1-(3-(benzyloxy)-5-(difluoromethyl)phenyl)ethylidene)-2-methylpropan-2-sulfinamide (1 g, 98%) as a yellow liquid.

MS (ESI+) m/z 380 (M+H)⁺

### Step 3

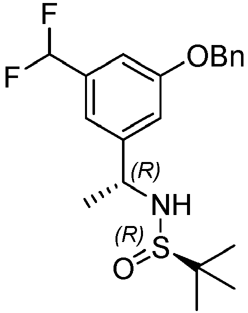

### N-((R)-1-(3-(benzyloxy)-5-(difluoromethyl)phenyl)ethyl)-2-methylpropan-2-sulfinamide

To a solution in which (R,E)-N-(1-(3-(benzyloxy)-5-(difluoromethyl)phenyl)ethylidene)-2-methylpropan-2-sulfinamide (1 g, 3.60 mmol) was dissolved in tetrahydrofuran (10 ml), NaBH₄ (164 mg, 4.34 mmol) was added at 0°C and stirred at room temperature for five hours. After confirming the completion of the reaction, H₂O was added and extracted with ethyl acetate. The combined organic extracts were dried over sodium sulfate and concentrated. The residue was purified by column chromatography to afford (R)-N-((R)-1-(3-(benzyloxy)-5-(difluoromethyl)phenyl)ethyl)-2-methylpropan-2-sulfinamide (830 mg, 58%) as a white solid.

MS (ESI+) m/z 382 (M+H)⁺

### Step 4

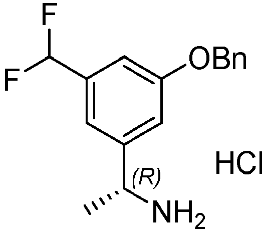

### (R)-1-(3-(benzyloxy)-5-(difluoromethyl)phenyl)ethanamine hydrochloride

To a solution in which N-((R)-1-(3-(benzyloxy)-5-(difluoromethyl)phenyl)ethyl)-2-methylpropan-2-sulfinamide (873 mg, 2.28 mmol) was dissolved in dioxane (7 ml), a solution of 4 M HCl (1.71 ml, 6.84 mmol) in dioxane was added and stirred at room temperature for one hour. After completion of the reaction, the resulting mixture was concentrated to afford (R)-1-(3-(benzyloxy)-5-(difluoromethyl)phenyl)ethanamine hydrochloride (682 mg, 100%) as a white solid.

MS (ESI+) m/z 278 (M+H)⁺

### <Synthesis methods and Examples>

### Synthesis method A

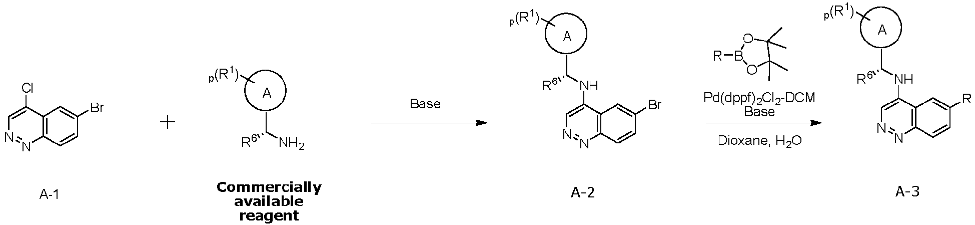

A general synthesis of compound A-3 is illustrated in synthesis method A. With regard to A-1, diisopropylethylamine is used to synthesize A-2 having amine introduced thereinto, and a Suzuki coupling reaction is performed to obtain final compound A-3.

### Example 1

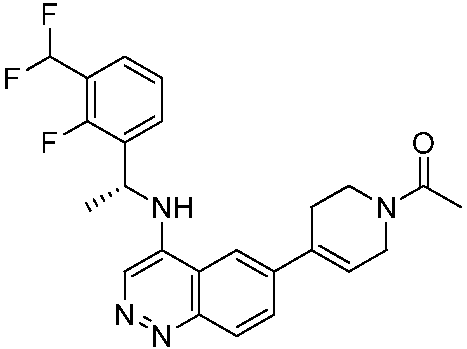

### (R)-1-(4-(4-(1-(3-(difluoromethyl)-2-fluorophenyl)ethylamino)cinnolin-6-yl)-5,6-dihydropyridin-1(2H)-yl)ethanone

### Step 1

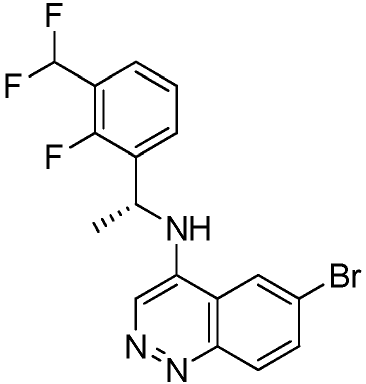

### (R)-6-bromo-N-(1-(3-(difluoromethyl)-2-fluorophenyl)ethyl)cinnolin-4-amine

To 6-bromo-4-chlorocinnoline (intermediate A-1) (150 mg, 0.62 mmol), (R)-1-(3-(difluoromethyl)-2-fluorophenyl)ethanamine (0.15 ml, 0.92 mmol) and diisopropylethylamine (0.15 ml, 1.83 mmol) were added dropwise and the resulting mixture was stirred at 130°C for four hours. Upon completion of the reaction, the reaction mixture was concentrated, and then purified by combi flash to afford (R)-6-bromo-N-(1-(3-(difluoromethyl)-2-fluorophenyl)ethyl)cinnolin-4-amine (220 mg, 90%) as a brown solid.

MS (ESI+) m/z 396, 398 (M+H)⁺

### Step 2

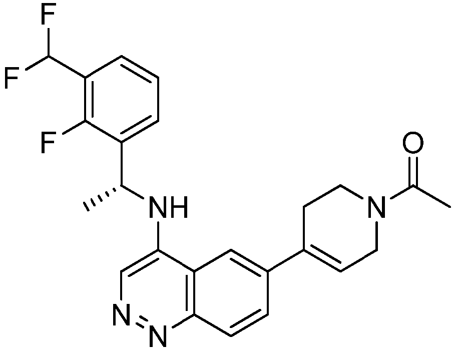

### (R)-1-(4-(4-(1-(3-(difluoromethyl)-2-fluorophenyl)ethylamino)cinnolin-6-yl)-5,6-dihydropyridin-1(2H)-yl)ethanone

To a solution in which (R)-6-bromo-N-(1-(3-(difluoromethyl)-2-fluorophenyl)ethyl)cinnolin-4-amine (80 mg, 0.20 mmol) was dissolved in dioxane (4 ml), 1-acetyl-5,6-dihydro-2H-pyridin-4-boronic acid, pinacol ester (120 mg, 0.30 mmol), Pd(dppf)₂Cl₂·DCM (40 mg, 0.02 mmol), K₂CO₃ (160 mg, 0.60 mmol), and H₂O (4 ml) were added and stirred at 90°C for six hours, and then stirred at room temperature for 12 hours. The reaction mixture was cooled to room temperature, diluted with distilled water, and extracted with DCM. The combined organic extracts were dried over sodium sulfate and then concentrated. The concentrated residue was purified by combi flash to afford (R)-1-(4-(4-(1-(3-(difluoromethyl)-2-fluorophenyl)ethylamino)cinnolin-6-yl)-5,6-dihydropyridin-1(2H)-yl)ethanone (18 mg, 14%) as a yellow solid.
¹H NMR (400 MHz, DMSO-d6) δ 8.43 (s, 1H), 8.34 (s, 1H), 8.08 (d, *J* = 8.8 Hz, 1H), 7.96 (t, *J =* 6.8 Hz, 1H), 7.80-7.76 (m, 1H), 7.64 (t, *J =* 7.2 Hz, 1H), 7.55 (t, *J =* 7.2 Hz, 1H), 7.32-7.27 (m, 2H), 6.49 (d, *J =* 3.6 Hz, 1H), 5.29 (t, *J =* 6.8 Hz, 1H), 4.21 (d, *J =* 2.0 Hz, 2H), 3.75-3.71 (m, 2H), 2.10 (d, *J* = 16.8 Hz, 3H), 1.27 (d, *J* = 6.8 Hz, 3H)
MS (ESI+) m/z 441 (M+H)⁺

### Example 2

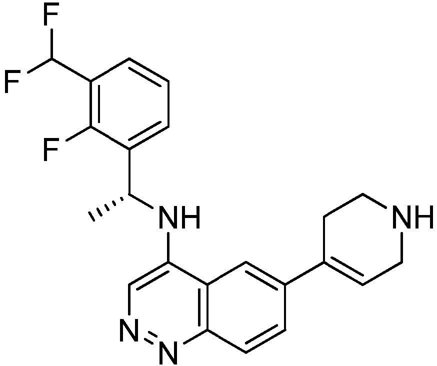

### (R)-N-(1-(3-(difluoromethyl)-2-fluorophenyl)ethyl)-6-(1,2,3,6-tetrahydropyridin-4-yl)cinnolin-4-amine

Intermediate A-1 and (R)-1-(3-(difluoromethyl)-2-fluorophenyl)ethanamine were used as described in Example 1 to synthesize compound A-2, and then 3,6-dihydro-4-(4,4,5,5-tetramethyl-1,3,2-dioxaborolan-2-yl)-1-1-dimethylethyl ester-1-(2H)-pyridine carboxylic acid was used in substantially the same manner as in the synthesis method of Example 1 to afford (R)-tert-butyl-4-(4-(1-(3-(difluoromethyl)-2-fluorophenyl)ethylamino)cinnolin-6-yl)-5,6-dihydropyridin-1(2H)-carboxylate (50 mg, 67%).

To a solution in which (R)-tert-butyl-4-(4-(1-(3-(difluoromethyl)-2-fluorophenyl)ethylamino)cinnolin-6-yl)-5,6-dihydropyridin-1(2H)-carboxylate (50 mg, 0.10 mmol) was dissolved in dichloromethane (1 ml), a solution of 4 N hydrochloric acid (0.40 ml, 1.0 mmol) in 1,4-dioxane was added at 0°C, and then stirred at room temperature for two hours. Upon completion of the reaction, the resulting mixture was concentrated and extracted using an aqueous sodium hydrogen carbonate solution and ethyl acetate. The combined organic extracts were dried over sodium sulfate and then concentrated to afford (R)-N-(1-(3-(difluoromethyl)-2-fluorophenyl)ethyl)-6-(1,2,3,6-tetrahydropyridin-4-yl)cinnolin-4-amine (6 mg, 15%) as a yellow solid.
¹H NMR (400 MHz, DMSO-d6) δ 8.40 (s, 1H), 8.33 (s, 1H), 8.06 (d, *J* = 8.8 Hz, 1H), 7.95 (dd, *J =* 2.0 Hz, 7.2 Hz, 1H), 7.81 (d, *J =* 6.8 Hz, 1H), 7.64 (t, *J =* 7.2 Hz, 1H), 7.55 (t, *J =* 7.2 Hz, 1H), 7.41(s, 0.5H), 7.32-7.34 (m, 2H), 7.14 (s, 0.5H), 6.53 (s, 1H), 5.28 (t, *J =* 6.8 Hz, 1H), 3.56 (d, H = 2.4 Hz, 2H), 3.10 (t, *J =* 5.6 Hz, 2H), 2.64 (s, 2H), 1.70 (d, *J =* 6.8 Hz, 3H)
MS (ESI+) m/z 399 (M+H)⁺

### Example 3

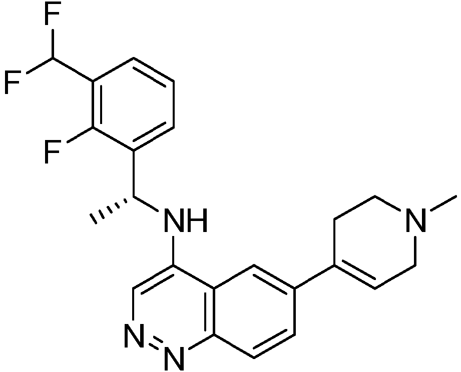

### (R)-N-(1-(3-(difluoromethyl)-2-fluorophenyl)ethyl)-6-(1-methyl-1,2,3,6-tetrahydropyridin-4-yl)cinnolin-4-amine

Intermediate A-1 and (R)-1-(3-(difluoromethyl)-2-fluorophenyl)ethanamine were used as described in Example 1 to synthesize compound A-2, and then 1-methyl-1,2,3,6-tetrahydropyridin-4-boronic acid pinacol ester was used in substantially the same manner as in the synthesis method of Example 1 to afford the title compound (26 mg, 31%).
¹H NMR (400 MHz, DMSO-d6) δ 8.40 (s, 1H), 8.31 (s, 1H), 8.06 (d, *J* = 8.8 Hz, 1H), 7.95 (dd, *J* = 2.0 Hz, 8.8 Hz, 1H), 7.82 (d, *J =* 7.2 Hz, 1H), 7.64 (t, *J =* 7.2 Hz, 1H), 7.55 (t, *J =* 7.2 Hz, 1H), 7.42-7.12 (m, 2H), 6.53 (s, 1H), 5.28 (quint, *J* = 6.8 Hz, 1H), 3.23-3.11 (m, 2H), 2.76-2.63 (m, 4H), 2.38 (s, 3H), 1.70 (d, *J =* 6.8 Hz, 3H)
MS (ESI+) m/z 413 (M+H)⁺

### Example 4

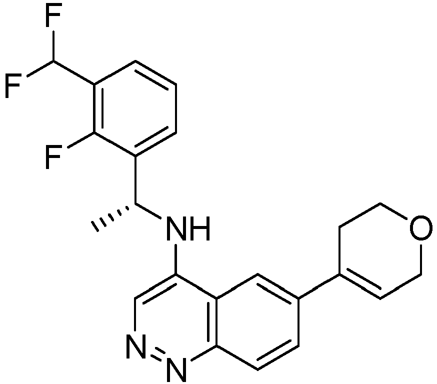

### (R)-N-(1-(3-(difluoromethyl)-2-fluorophenyl)ethyl)-6-(3,6-dihydro-2H-pyran-4-yl)cinnolin-4-amine

Intermediate A-1 and (R)-1-(3-(difluoromethyl)-2-fluorophenyl)ethanamine were used as described in Example 1 to synthesize compound A-2, and then 1,2,3,6-tetrahydropyran-4-boronic acid pinacol ester was used in substantially the same manner as in the synthesis method of Example 1 to afford the title compound (28 mg, 35%).
¹H NMR (400 MHz, DMSO-d6) δ 8.42 (s, 1H), 8.33 (s, 1H), 8.06 (d, *J* = 8.8 Hz, 1H), 7.98 (dd, *J* = 2.0 Hz, 8.8 Hz, 1H), 7.83 (d, *J =* 7.2 Hz, 1H), 7.64 (t, *J =* 7.2 Hz, 1H), 7.55 (t, *J =* 7.2 Hz, 1H), 7.42-7.14 (m, 2H), 6.59 (s, 1H), 5.29 (quint, *J =* 6.8 Hz, 1H), 4.36-4.32 (m, 2H), 3.92 (t, *J =* 5.6 Hz, 2H), 2.72-2.63 (m, 2H), 1.70 (d, *J =* 6.8 Hz, 3H)
MS (ESI+) m/z 400 (M+H)⁺

### Synthesis method B

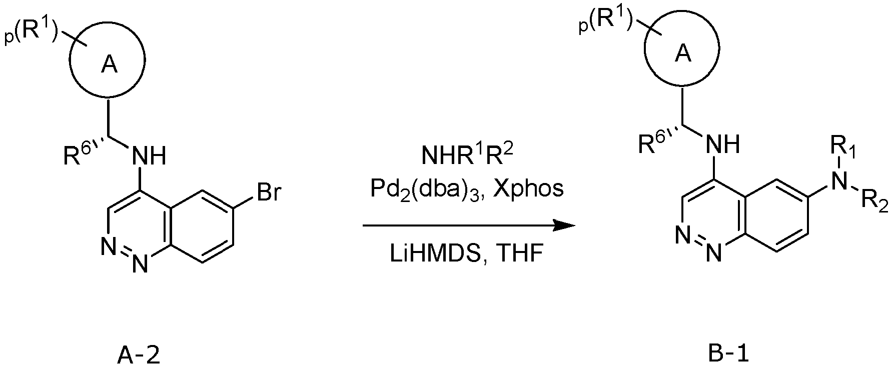

A general synthesis of compound B-1 is illustrated in synthesis method B. The bromine of intermediate A-2 is substituted with amine by using the Buchwald reaction to afford final compound B-1.

### Example 5

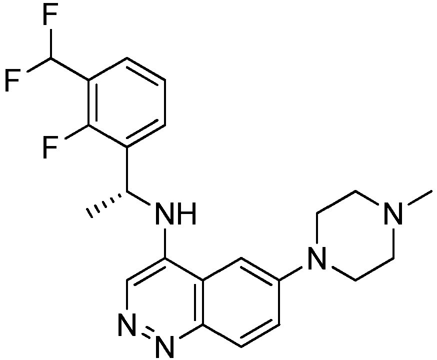

### (R)-N-(1-(3-(difluoromethyl)-2-fluorophenyl)ethyl)-6-(4-methylpiperazin-1-yl)cinnolin-4-amine

To a solution in which (R)-6-bromo-N-(1-(3-(difluoromethyl)-2-fluorophenyl)ethyl)cinnolin-4-amine (intermediate A-2) (230 mg, 0.58 mmol) was dissolved in tetrahydrofuran (4 ml), N-methylpiperazine (0.23 ml, 1.16 mmol), Pd₂(dba)₃ (160 mg, 0.058 mmol), and Xphos (160 mg, 0.116 mmol) were dissolved and 1 N LiHMDS THF solution (2 ml, 2.9 mmol) was added to the resulting solution and stirred for one hour while heating to 100°C. Upon completion of the reaction, the resulting mixture was cooled to room temperature, diluted with distilled water, and extracted with DCM. The combined organic extracts were dried over sodium sulfate and concentrated. The residue was purified by combi flash to afford (R)-N-(1-(3-(difluoromethyl)-2-fluorophenyl)ethyl)-6-(4-methylpiperazin-1-yl)cinnolin-4-amine (100 mg, 42%) as a yellow solid.
¹H NMR (400 MHz, DMSO-d6) δ 8.10 (s, 1H), 7.95 (d, *J =* 9.6 Hz, 1H), 7.64 (dd, *J =* 2.4 Hz, 6.8 Hz, 1H), 7.59 (t, *J =* 7.2 Hz, 1H), 7.54 (t, *J* = 7.2 Hz, 1H), 7.47(d, *J* = 2.4 Hz, 1H), 7.41 (s, 0.5H), 7.32-7.27 (m, 3H), 7.14 (s, 0.5H), 3.44 (s, 4H), 2.53 (s, 4H), 2.27 (s, 3H), 1.68 (d, *J =* 6.8 Hz, 3H)
MS (ESI+) m/z 416 (M+H)⁺

### Example 6

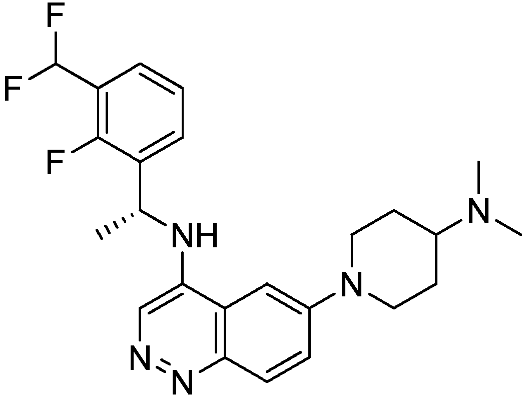

### (R)-N-(1-(3-(difluoromethyl)-2-fluorophenyl)ethyl)-6-(4-(dimethylamino)piperidin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (8 mg, 16%) was obtained in substantially the same manner as described in the synthesis method of Example 5, except for using N,N-dimethylpiperidin-4-amine.
1H NMR (400 MHz, DMSO-d6) δ 8.01 (s, 1H), 7.93(d, *J =* 9.6 Hz, 1H), 7.64-7.59 (m, 2H), 7.48 (s, 1H), 7.32 (t, *J =* 6.8 Hz, 1H), 7.28 (d, *J =* 4.0 Hz, 1H), 5.25-5.18 (m, 1H), 4.07 (d, *J* = 10.0 Hz, 2H), 2.91-2.90 (m, 2H), 2.50-2.50 (m, 2H), 2.23 (s, 3H), 1.91 (s, 3H), 1.68 (d, *J =* 6.8 Hz, 3H), 1.55-1.52 (m, 2H)
MS (ESI+) m/z 444 (M+H)⁺

### Example 7

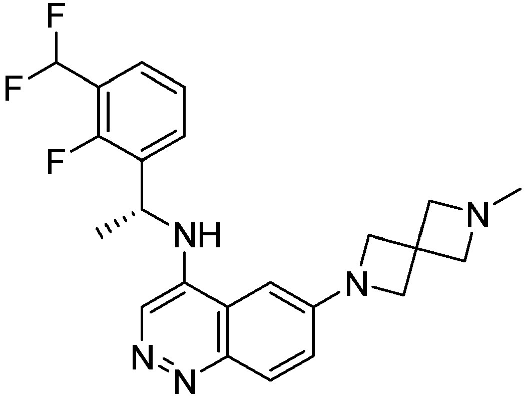

### (R)-N-(1-(3-(difluoromethyl)-2-fluorophenyl)ethyl)-6-(6-methyl-2,6-diazaspiro[3.3]heptan-2-yl)cinnolin-4-amine

Intermediate A-1 and (R)-1-(3-(difluoromethyl)-2-fluorophenyl)ethanamine were used as described in Example 1 to synthesize compound A-2, and then the title compound (33 mg, 38%) was obtained in substantially the same manner as described in the synthesis method of Example 5, except for using 2-methyl-2,6-diazaspiro[3.3]heptane.
¹H NMR (400 MHz, DMSO-d6) δ 8.05 (s, 1H), 7.93 (d, *J =* 9.6 Hz, 1H), 7.60-7.52 (m, 2H), 7.41-7.14 (m, 3H), 7.07-7.04 (m, 2H), 5.19 (q, *J =* 7.2 Hz, 1H), 4.13-4.08 (m, 4H), 3.40-3.30 (m, 4H), 2.21 (s, 3H), 1.68 (d, *J =* 6.8 Hz, 3H)
MS (ESI+) m/z 428 (M+H)⁺

### Example 8

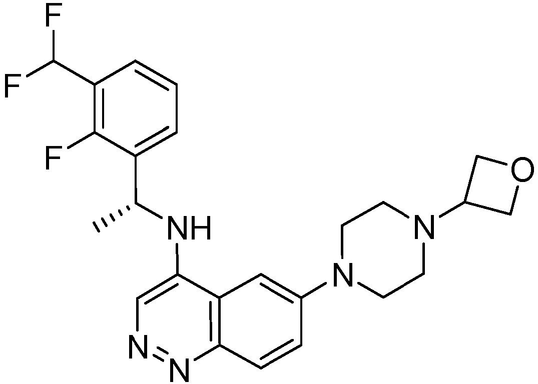

### (R)-N-(1-(3-(difluoromethyl)-2-fluorophenyl)ethyl)-6-(4-(oxetan-3-yl)piperazin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (33 mg, 36%) was obtained in substantially the same manner as described in the synthesis method of Example 5, except for using 1-(oxetan-3-yl)piperazine.
¹H NMR (400 MHz, DMSO-d6) δ 8.10 (s, 1H), 7.96 (d, *J =* 9.6Hz, 1H), 7.66 (dd, *J =* 9.6, 2.4 Hz, 1H), 7.62-7.49 (m, 2H), 7.49 (d, *J =* 2.4 Hz, 1H), 7.41-7.14 (m, 3H), 5.21 (q, *J =* 7.2 Hz, 1H), 4.61 (t, *J =* 6.4 Hz, 2H), 4.52 (t, *J =* 6.0 Hz, 2H), 3.55-3.46 (m, 5H), 3.40-3.30 (m, 4H), 1.69 (d, *J* = 6.8 Hz, 3H)
MS (ESI+) m/z 458 (M+H)⁺

### Example 9

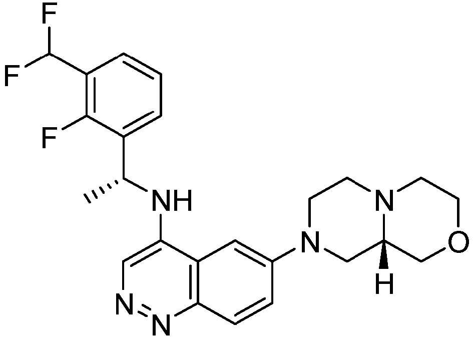

### N-((R)-1-(3-(difluoromethyl)-2-fluorophenyl)ethyl)-6-((S)-hexahydropyrazino[2,1-c][1,4]oxazin-8(1H)-yl)cinnolin-4-amine

Intermediate A-1 and (R)-1-(3-(difluoromethyl)-2-fluorophenyl)ethanamine were used as described in Example 1 to synthesize compound A-2, and then the title compound (17 mg, 25%) was obtained in substantially the same manner as described in the synthesis method of Example 5, except for using (S)-octahydropyrazino[2,1-c][1,4]oxazine.
1H NMR (400 MHz, DMSO-d6) δ 8.10 (s, 1H), 7.96 (d, *J =* 9.2 Hz, 1H), 7.72 (dd, *J =* 2.4 Hz, 6.8 Hz, 1H), 7.59 (t, *J =* 7.2 Hz, 1H), 7.54 (t, *J =* 7.2 Hz, 1H), 7.46 (d, *J =* 2.4 Hz, 1H), 7.42 (s, 0.25H), 7.34 (s, 0.25H), 7.31 (d, *J =* 3.2 Hz, 1H), 7.28 (d, *J =* 4.0 Hz, 1H), 7.27 (s, 0.25H), 7.14 (s, 0.25 H), 5.25-5.18 (m, 1H), 4.02 (d, *J =* 11.6 Hz, 1H), 3.85-3.79 (m, 3H), 3.61-3.55 (m, 1H), 3.25 (t, *J =* 10.4 Hz, 2H), 3.00-2.92 (m, 2H), 2.74 (d, *J =* 11.2 Hz, 1H), 2.41-2.24 (m, 3H), 1.70 (d, *J= 6.8* Hz, 3H)
MS (ESI+) m/z 458 (M+H)⁺

### Example 10

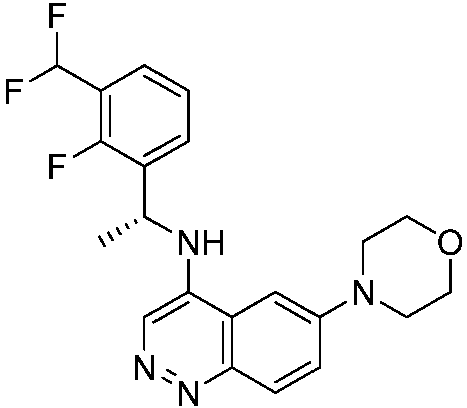

### (R)-N-(1-(3-(difluoromethyl)-2-fluorophenyl)ethyl)-6-morpholinocinnolin-4-amine

Intermediate A-1 and (R)-1-(3-(difluoromethyl)-2-fluorophenyl)ethanamine were used as described in Example 1 to synthesize compound A-2, and then the title compound (50 mg, 62%) was obtained in substantially the same manner as described in the synthesis method of Example 5, except for using morpholine.
¹H NMR (400 MHz, DMSO-d6) δ 8.12 (s, 1H), 7.98 (d, *J =* 9.2 Hz, 1H), 7.65 (dd, *J =* 2.4 Hz, 7.2 Hz, 1H), 7.60 (t, *J =* 7.2 Hz, 1H), 7.54 (t, *J =* 6.8 Hz, 1H), 7.50 (d, *J =* 2.4 Hz, 1H), 7.41 (s, 0.5H), 7.35-7.19 (m, 3H), 7.14 (s, 0.5H), 5.22 (t, *J =* 6.8 Hz, 1H), 3.82 (s, 4H), 3.41 (s, 4H), 1.69 (d, *J =* 6.8 Hz, 3H)
MS (ESI+) m/z 403 (M+H)⁺

### Example 11

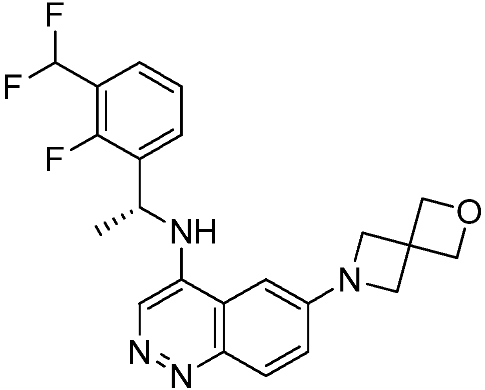

### (R)-N-(1-(3-(difluoromethyl)-2-fluorophenyl)ethyl)-6-(2-oxa-6-azaspiro[3.3]heptan-6-yl)cinnolin-4-amine

Intermediate A-1 and (R)-1-(3-(difluoromethyl)-2-fluorophenyl)ethanamine were used as described in Example 1 to synthesize compound A-2, and then the title compound (16 mg, 20%) was obtained in substantially the same manner as described in the synthesis method of Example 5, except for using 2-oxa-6-azaspiro[3.3]heptane.
¹H NMR (400 MHz, DMSO-d6) δ8.06 (s, 1H), 7.94 (d, *J =* 8.8 Hz, 1H), 7.59-7.52 (m, 2H), 7.41 (s, 0.5H), 7.30-7.26 (m, 1H), 7.21 (d, *J =* 6.8 Hz, 1H), 7.14 (s, 0.5H), 7.09-7.06 (m, 2H), 5.19 (t, *J =* 6.8 Hz, 1H), 4.79 (s, 4H), 4.22 (s, 4H), 1.68 (d, *J =* 6.8 Hz, 3H)
MS (ESI+) m/z 415 (M+H)⁺

### Example 12

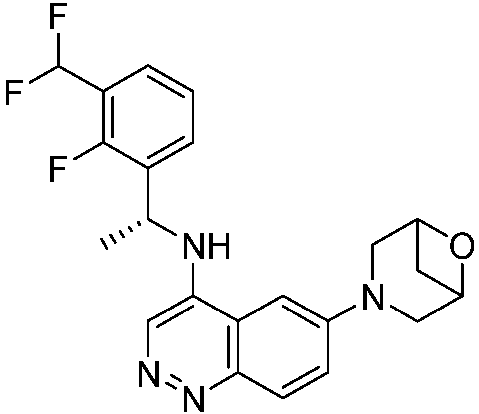

### 6-(6-oxa-3-azabicyclo[3.1.1]heptan-3-yl)-N-((R)-1-(3-(difluoromethyl)-2-fluorophenyl)ethyl)cinnolin-4-amine

Intermediate A-1 and (R)-1-(3-(difluoromethyl)-2-fluorophenyl)ethanamine were used as described in Example 1 to synthesize compound A-2, and then the title compound (913 mg, 50%) was obtained in substantially the same manner as described in the synthesis method of Example 5, except for using 6-oxa-3-azabicyclo[3.1.1]heptane hydrochloride.
¹H NMR (400 MHz, DMSO-d6) δ 8.07 (s, 1H), 8.03 (d, *J =* 9.6 Hz, 1H), 7.60 (t, *J =* 7.4 Hz, 1H), 7.54 (t, *J* = 7.8 Hz, 1H), 7.51 (d, *J =* 9.6 Hz, 1H), 7.42-7.15 (m, 4H), 5.23 (t, *J =* 6.8 Hz, 1H), 4.83 (d, *J =* 6.8 Hz, 2H), 3.80 (t, *J =* 12.8 Hz, 2H), 3.65 (t, *J =* 13.4 Hz, 2H), 3.23-3.18 (m, 1H), 1.99 (d, *J =* 8.8 Hz, 1H), 1.70 (d, *J =* 6.4 Hz, 3H)
MS (ESI+) m/z 415 (M+H)⁺

### Example 13

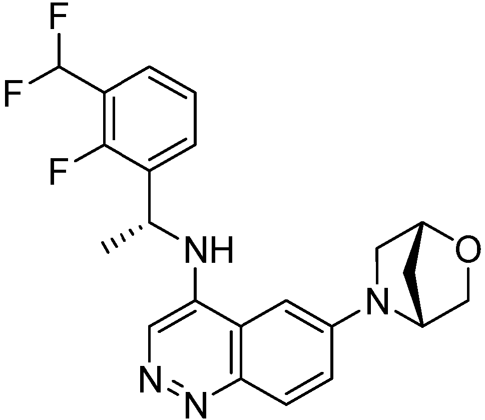

### 6-((1R,4R)-2-oxa-5-azabicyclo[2.2.1]heptan-5-yl)-N-((R)-1-(3-(difluoromethyl)-2-fluorophenyl)ethyl)cinnolin-4-amine

Intermediate A-1 and (R)-1-(3-(difluoromethyl)-2-fluorophenyl)ethanamine were used as described in Example 1 to synthesize compound A-2, and then the title compound (32 mg, 39%) was obtained in substantially the same manner as described in the synthesis method of Example 5, except for using (1R,4R)-2-oxa-5-azabicyclo[2.2.1]heptane hydrochloride.
¹H NMR (400 MHz, DMSO-d6) δ 8.03 (s, 1H), 7.95 (d, *J* = 9.2 Hz, 1H), 7.56 (dd, *J* = 8.0 Hz, 15.9 Hz, 2H), 7.41-7.10 (m, 5H), 5.19 (t, *J =* 6.8 Hz, 1H), 4.90 (s, 1H), 4.75 (s, 1H), 3.88 (d, *J= 6.0* Hz, 1H), 3.74 (d, *J* = 7.2 Hz, 1H), 3.65 (d, *J* = 8.4 Hz, 1H), 3.32-3.25 (m, 1H), 1.99 (q, *J=* 10.8 Hz, 2H), 1.68 (d, *J =* 6.8 Hz, 3H)
MS (ESI+) m/z 415 (M+H)⁺

### Example 14

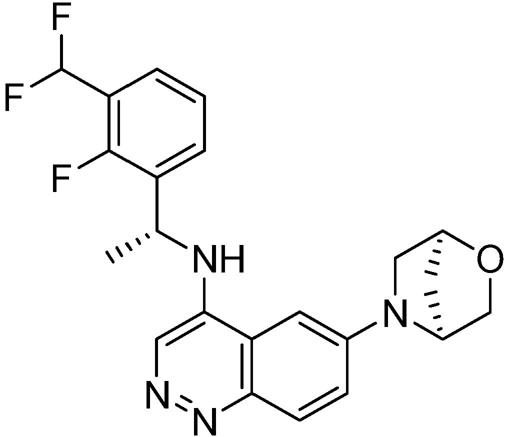

### 6-((1S,4S)-2-oxa-5-azabicyclo[2.2.1]heptan-5-yl)-N-((R)-1-(3-(difluoromethyl)-2-fluorophenyl)ethyl)cinnolin-4-amine

Intermediate A-1 and (R)-1-(3-(difluoromethyl)-2-fluorophenyl)ethanamine were used as described in Example 1 to synthesize compound A-2, and then the title compound (30 mg, 36%) was obtained in substantially the same manner as described in the synthesis method of Example 5, except for using (1S,4S)-2-oxa-5-azabicyclo[2.2.1]heptane hydrochloride.
¹H NMR (400 MHz, DMSO-d6) δ 8.03 (s, 1H), 7.95 (d, *J =* 9.2 Hz, 1H), 7.59 (t, *J =* 7.4 Hz, 1H), 7.54 (t, *J =* 6.2 Hz, 1H), 7.41-7.27 (m, 3H), 7.16-7.12 (m, 2H), 5.20 (t, *J =* 6.8 Hz, 1H), 4.92 (s, 1H), 4.76 (s, 1H), 3.87 (d, *J =* 6.4 Hz, 1H), 3.71 (d, *J =* 7.2 Hz, 1H), 3.64 (d, *J =* 9.6 Hz, 1H), 3.32-3.25 (m, 1H), 1.97 (q, *J =* 11.6 Hz, 2H), 1.68 (d, *J =* 6.8 Hz, 3H)
MS (ESI+) m/z 415 (M+H)⁺

### Example 15

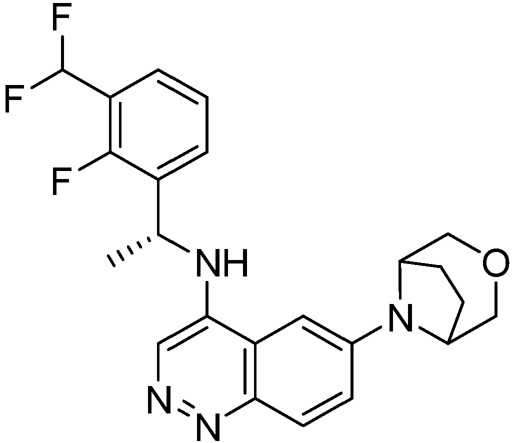

### 6-(3-oxa-8-azabicyclo[3.2.1]octan-8-yl)-N-((R)-1-(3-(difluoromethyl)-2-fluorophenyl)ethyl)cinnolin-4-amine

Intermediate A-1 and (R)-1-(3-(difluoromethyl)-2-fluorophenyl)ethanamine were used as described in Example 1 to synthesize compound A-2, and then the title compound (45 mg, 45%) was obtained in substantially the same manner as described in the synthesis method of Example 5, except for using 3-oxa-8-azabicyclo[3.2.1]octane hydrochloride.
¹H NMR (400 MHz, DMSO-d6) δ 8.06 (s, 1H), 7.96 (d, *J =* 9.2 Hz, 1H), 7.55-7.52 (m, 3H), 7.42-7.14 (m, 4H), 5.20 (t, *J =* 6.8 Hz, 1H), 4.52 (s, 2H), 3.77 (d, *J =* 7.6 Hz, 1H), 3.76 (d, *J* = 7.6 Hz, 1H), 3.58 (d, *J =* 10.8 Hz, 2H), 2.09-2.01 (m, 4H), 1.68 (d, *J =* 6.8 Hz, 3H)
MS (ESI+) m/z 429 (M+H)⁺

### Example 16

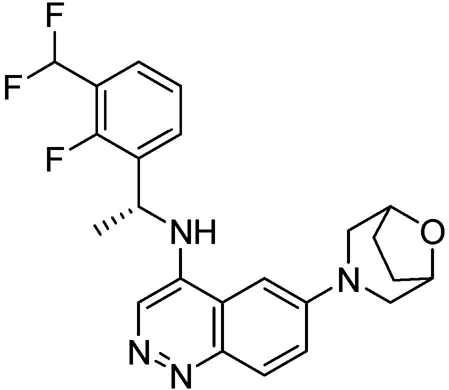

### 6-(8-oxa-3-azabicyclo[3.2.1]octan-3-yl)-N-((R)-1-(3-(difluoromethyl)-2-fluorophenyl)ethyl)cinnolin-4-amine

Intermediate A-1 and (R)-1-(3-(difluoromethyl)-2-fluorophenyl)ethanamine were used as described in Example 1 to synthesize compound A-2, and then the title compound (27 mg, 25%) was obtained in substantially the same manner as described in the synthesis method of Example 5, except for using 8-oxa-3-azabicyclo[3.2.1]octane hydrochloride.
¹H NMR (400 MHz, DMSO-d6) δ 8.09 (s, 1H), 7.96 (d, *J =* 9.6 Hz, 1H), 7.61-7.52 (m, 3H), 7.42-7.14 (m, 4H), 5.21 (t, *J =* 6.8 Hz, 1H), 4.54 (s, 2H), 3.73 (d, *J =* 10.4 Hz, 2H), 3.05-3.00 (m, 2H), 1.89 (s, 4H), 1.68 (d, *J =* 6.8 Hz, 3H)
MS (ESI+) m/z 429 (M+H)⁺

### Example 17

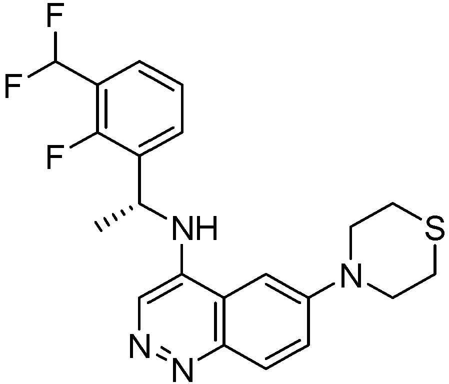

### (R)-N-(1-(3-(difluoromethyl)-2-fluorophenyl)ethyl)-6-thiomorpholinocinnolin-4-amine

Intermediate A-1 and (R)-1-(3-(difluoromethyl)-2-fluorophenyl)ethanamine were used as described in Example 1 to synthesize compound A-2, and then the title compound (24 mg, 40%) was obtained in substantially the same manner as described in the synthesis method of Example 5, except for using thiomorpholine.
1H NMR (400 MHz, DMSO-d6) δ 8.19 (s, 1H), 7.96 (d, *J* = 9.2 Hz, 1H), 7.62 (d, *J* = 2.4 Hz, 1H), 7.60-7.54 (m, 2H), 7.47 (d, *J=* 2.4 Hz, 1H), 7.42 (s, 0.25H), 7.34 (s, 0.25H), 7.32 (d, *J=* 2.8 Hz, 1H), 7.29 (d, *J =* 3.6 Hz, 1H), 7.27 (s, 0.25H), 7.15 (s, 0.25 H), 5.23-5.19 (m, 1H), 3.86-3.83 (m, 4H), 2.77-2.75 (m, 4H), 1.69 (d, *J =* 6.8 Hz, 3H)
MS (ESI+) m/z 419 (M+H)⁺

### Example 18

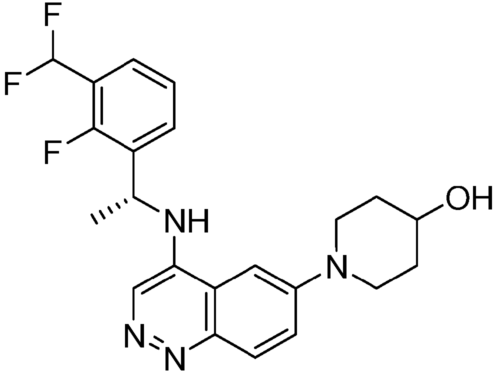

### (R)-1-(4-((1-(3-(difluoromethyl)-2-fluorophenyl)ethyl)amino)cinnolin-6-yl)piperidin-4-ol

Intermediate A-1 and (R)-1-(3-(difluoromethyl)-2-fluorophenyl)ethanamine were used as described in Example 1 to synthesize compound A-2, and then the title compound (30 mg, 36%) was obtained in substantially the same manner as described in the synthesis method of Example 5, except for using 4-hydroxypiperidine.
¹H NMR (400 MHz, DMSO-d6) δ 8.08 (s, 1H), 7.93 (d, *J =* 9.6 Hz, 1H), 7.64-7.52 (m, 3H), 7.47 (d, *J= 2.4* Hz, 1H), 7.41-7.14 (m, 3H), 5.21 (q, *J =* 7.2 Hz, 1H), 4.76 (d, *J* = 4.0 Hz, 1H), 3.91-3.82 (m, 2H), 3.78-3.71 (m, 1H), 3.16-3.09 (m, 2H), 1.93-1.89 (m, 2H), 1.70 (d, *J =* 6.8 Hz, 3H), 1.58-1.50 (m, 2H)
MS (ESI+) m/z 417 (M+H)⁺

### Example 19

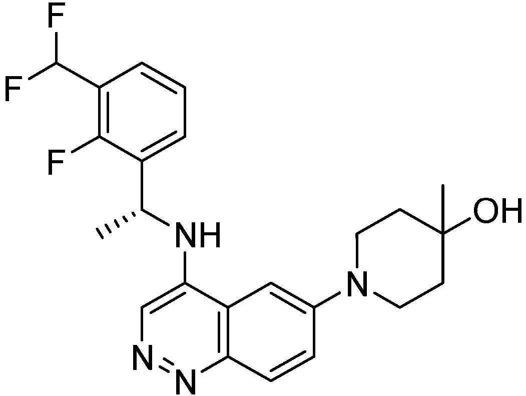

### (R)-1-(4-((1-(3-(difluoromethyl)-2-fluorophenyl)ethyl)amino)cinnolin-6-yl)-4-methylpiperidin-4-ol

Intermediate A-1 and (R)-1-(3-(difluoromethyl)-2-fluorophenyl)ethanamine were used as described in Example 1 to synthesize compound A-2, and then the title compound (29 mg, 33%) was obtained in substantially the same manner as described in the synthesis method of Example 5, except for using 4-methyl-4-piperidinol.
¹H NMR (400 MHz, DMSO-d6) δ 8.07 (s, 1H), 7.92 (d, *J =* 9.2 Hz, 1H), 7.65-7.52 (m, 3H), 7.46 (d, *J =* 2.4 Hz, 1H), 7.41-7.14 (m, 3H), 5.21 (q, *J* = 7.2 Hz, 1H), 4.41 (s, 1H), 3.69-3.65 (m, 2H), 3.40-3.35 (m, 2H), 1.70 (d, *J =* 6.8 Hz, 3H), 1.65-1.63 (m, 4H), 1.20 (s, 3H)
MS (ESI+) m/z 431 (M+H)⁺

### Example 20

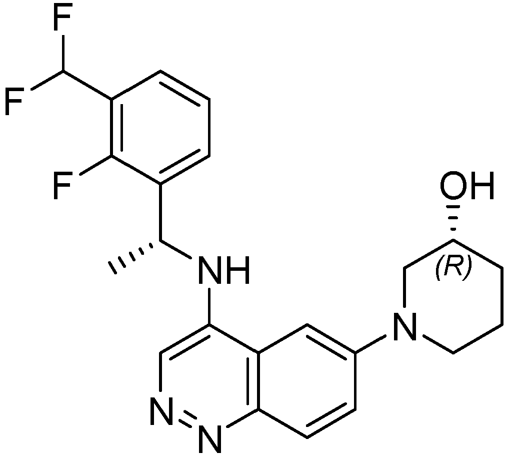

### (R)-1-(4-(((R)-1-(3-(difluoromethyl)-2-fluorophenyl)ethyl)amino)cinnolin-6-yl)piperidin-3-ol

Intermediate A-1 and (R)-1-(3-(difluoromethyl)-2-fluorophenyl)ethanamine were used as described in Example 1 to synthesize compound A-2, and then the title compound (40 mg, 48%) was obtained in substantially the same manner as described in the synthesis method of Example 5, except for using (R)-3-hydroxypiperidine.
¹H NMR (400 MHz, DMSO-d6) δ 8.07 (s, 1H), 7.92 (d, *J =* 9.2 Hz, 1H), 7.62-7.52 (m, 3H), 7.46 (d, *J =* 2.0 Hz, 1H), 7.43-7.14 (m, 3H), 5.21 (q, *J =* 7.2 Hz, 1H), 4.90 (d, *J* = 4.8 Hz, 1H), 3.95-3.91 (m, 1H), 3.81-3.78 (m, 1H), 3.70-3.63 (m, 1H), 2.99-2.92 (m, 1H), 2.83-2.78 (m, 1H), 1.97-1.94 (m, 1H), 1.88-1.83 (m, 1H), 1.70 (d, *J* = 6.8 Hz, 3H), 1.64-1.54 (m, 1H), 1.44-1.36 (m, 1H)
MS (ESI+) m/z 417 (M+H)⁺

### Example 21

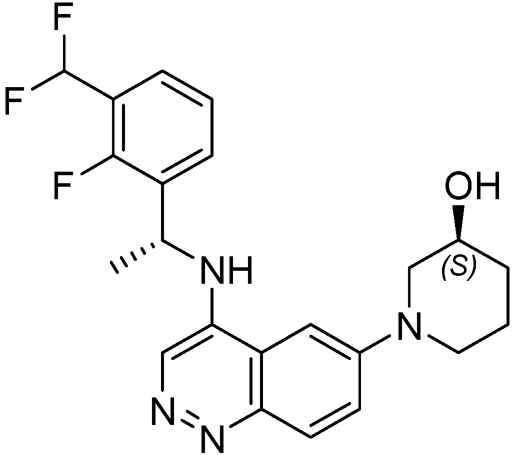

### (S)-1-(4-(((R)-1-(3-(difluoromethyl)-2-fluorophenyl)ethyl)amino)cinnolin-6-yl)piperidin-3-ol

Intermediate A-1 and (R)-1-(3-(difluoromethyl)-2-fluorophenyl)ethanamine were used as described in Example 1 to synthesize compound A-2, and then the title compound (13 mg, 15%) was obtained in substantially the same manner as described in the synthesis method of Example 5, except for using (S)-3-hydroxypiperidine.
¹H NMR (400 MHz, DMSO-d6) δ 8.07 (s, 1H), 7.92 (d, *J =* 9.6 Hz, 1H), 7.62-7.52 (m, 3H), 7.47-7.43 (m, 2H), 7.43-7.14 (m, 2H), 5.21 (q, *J =* 7.2 Hz, 1H), 5.00 (d, *J =* 4.8 Hz, 1H), 3.94-3.90 (m, 1H), 3.83-3.80 (m, 1H), 3.68-3.63 (m, 1H), 2.98-2.91 (m, 1H), 2.84-2.79 (m, 1H), 1.98-1.94 (m, 1H), 1.88-1.83 (m, 1H), 1.70 (d, *J =* 6.8 Hz, 3H), 1.64-1.55 (m, 1H), 1.45-1.36 (m, 1H)
MS (ESI+) m/z 417 (M+H)⁺

### Example 22

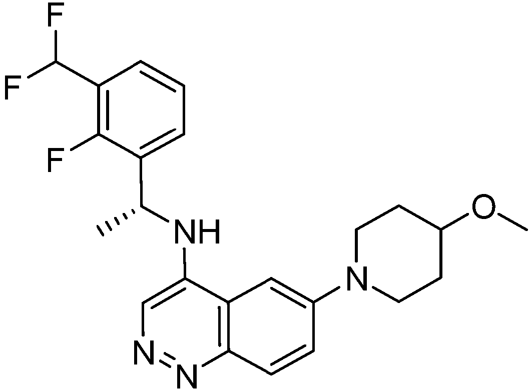

### (R)-N-(1-(3-(difluoromethyl)-2-fluorophenyl)ethyl)-6-(4-methoxypiperidin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (13 mg, 15%) was obtained in substantially the same manner as described in the synthesis method of Example 5, except for using 4-methoxypiperidine.
¹H NMR (400 MHz, DMSO-d6) δ 8.08 (s, 1H), 7.94 (d, *J =* 9.6 Hz, 1H), 7.65-7.52 (m, 3H), 7.48 (d, *J =* 2.4 Hz, 1H), 7.41-7.14 (m, 3H), 5.22 (q, *J =* 7.2 Hz, 1H), 3.81-3.76 (m, 2H), 3.49-3.43 (m, 1H), 3.33 (s, 3H), 3.22-3.14 (m, 2H), 2.03-1.99 (m, 2H), 1.69 (d, *J =* 6.8 Hz, 3H), 1.65-1.56 (m, 2H)
MS (ESI+) m/z 431 (M+H)⁺

### Example 23

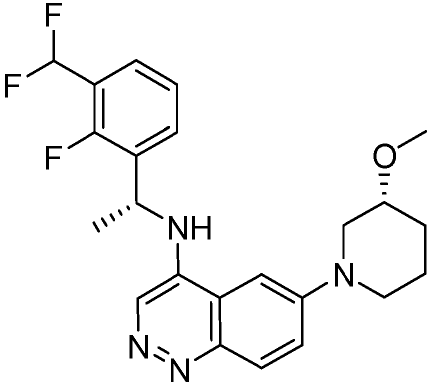

### N-((R)-1-(3-(difluoromethyl)-2-fluorophenyl)ethyl)-6-((R)-3-methoxypiperidin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (22 mg, 25%) was obtained in substantially the same manner as described in the synthesis method of Example 5, except for using (R)-3-methoxypiperidine.
¹H NMR (400 MHz, DMSO-d6) δ 8.08 (s, 1H), 7.93 (d, *J =* 9.6 Hz, 1H), 7.65-7.52 (m, 3H), 7.46 (d, *J= 2.4* Hz, 1H), 7.41-7.14 (m, 3H), 5.21 (q, *J=* 7.2 Hz, 1H), 3.92-3.88 (m, 1H), 3.71-3.67 (m, 1H), 3.43-3.37 (m, 1H), 3.33 (s, 3H), 3.17-3.02 (m, 2H), 2.10-1.99 (m, 1H), 1.91-1.85 (m, 1H), 1.69 (d, *J =* 6.8 Hz, 3H), 1.65-1.43 (m, 2H)
MS (ESI+) m/z 431 (M+H)⁺

### Example 24

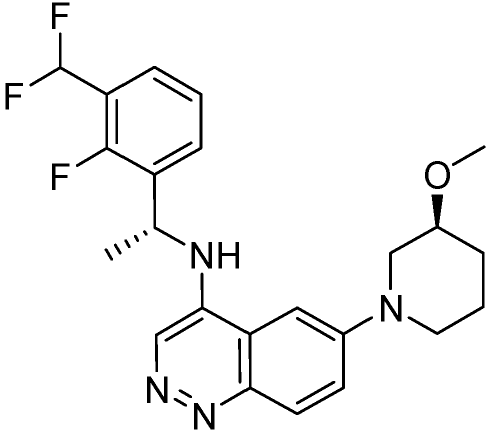

### N-((R)-1-(3-(difluoromethyl)-2-fluorophenyl)ethyl)-6-((S)-3-methoxypiperidin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (6 mg, 7%) was obtained in substantially the same manner as described in the synthesis method of Example 5, except for using (S)-3-methoxypiperidine.
¹H NMR (400 MHz, DMSO-d6) δ 8.08 (s, 1H), 7.93 (d, *J =* 9.6 Hz, 1H), 7.65-7.52 (m, 3H), 7.46 (d, *J= 2.4* Hz, 1H), 7.41-7.14 (m, 3H), 5.20 (q, *J=* 7.2 Hz, 1H), 3.91-3.88 (m, 1H), 3.73-3.70 (m, 1H), 3.43-3.37 (m, 1H), 3.33 (s, 3H), 3.14-3.02 (m, 2H), 2.10-1.99 (m, 1H), 1.91-1.85 (m, 1H), 1.69 (d, *J =* 6.8 Hz, 3H), 1.65-1.45 (m, 2H)
MS (ESI+) m/z 431 (M+H)⁺

### Example 25

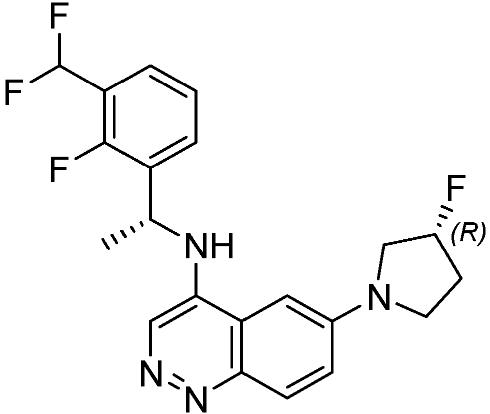

### N-((R)-1-(3-(difluoromethyl)-2-fluorophenyl)ethyl)-6-((R)-3-fluoropyrrolidin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (11 mg, 14%) was obtained in substantially the same manner as described in the synthesis method of Example 5, except for using (R)-3-fluoropyrrolidine hydrochloride.
¹H NMR (400 MHz, DMSO-d6) δ 8.04 (s, 1H), 7.97 (d, *J =* 9.2 Hz, 1H), 7.60-7.52 (m, 2H), 7.41-7.10 (m, 5H), 5.63-5.50 (m, 1H), 5.21 (q, *J =* 7.2 Hz, 1H), 3.79-3.54 (m, 4H), 2.39-2.23 (m, 2H), 1.69 (d, *J =* 6.8 Hz, 3H)
MS (ESI+) m/z 405 (M+H)⁺

### Example 26

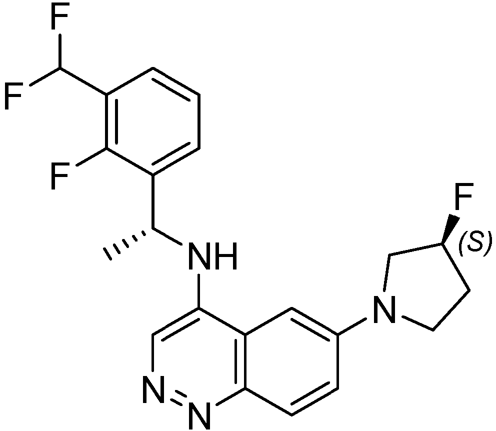

### N-((R)-1-(3-(difluoromethyl)-2-fluorophenyl)ethyl)-6-((S)-3-fluoropyrrolidin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (21 mg, 27%) was obtained in substantially the same manner as described in the synthesis method of Example 5, except for using (S)-3-fluoropyrrolidine hydrochloride.
¹H NMR (400 MHz, DMSO-d6) δ 8.04 (s, 1H), 7.97 (d, *J =* 9.2 Hz, 1H), 7.60-7.52 (m, 2H), 7.41-7.10 (m, 5H), 5.64-5.50 (m, 1H), 5.21 (q, *J =* 7.2 Hz, 1H), 3.79-3.67 (m, 3H), 3.57-3.50 (m, 1H), 2.38-2.19 (m, 2H), 1.69 (d, *J =* 6.8 Hz, 3H)
MS (ESI+) m/z 405 (M+H)⁺

### Example 27

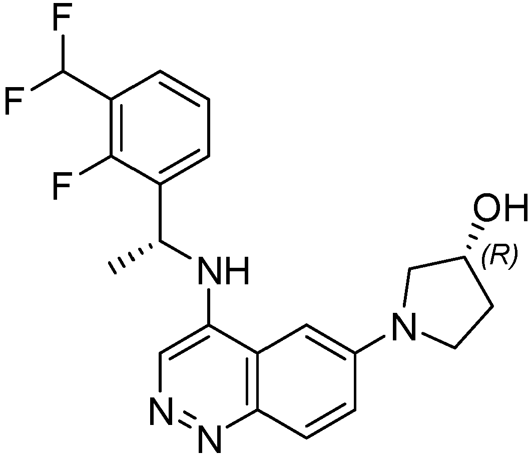

### (R)-1-(4-(((R)-1-(3-(difluoromethyl)-2-fluorophenyl)ethyl)amino)cinnolin-6-yl)pyrrolidin-3-ol

Intermediate A-1 and (R)-1-(3-(difluoromethyl)-2-fluorophenyl)ethanamine were used as described in Example 1 to synthesize compound A-2, and then the title compound (5 mg, 6%) was obtained in substantially the same manner as described in the synthesis method of Example 5, except for using (R)-pyrrolidin-3-ol.
¹H NMR (400 MHz, DMSO-d6) δ 8.03 (s, 1H), 7.93 (d, *J =* 9.2 Hz, 1H), 7.59 (t, *J =* 7.6 Hz, 1H), 7.54 (t, *J =* 7.2 Hz, 1H), 7.41-7.05 (m, 6H), 5.24 (q, *J =* 7.2 Hz, 1H), 5.11 (d, *J =* 3.6 Hz, 1H), 4.49 (s, 1H), 3.64-3.42 (m, 4H), 2.17-2.08 (m, 1H), 2.02-1.95 (m, 1H), 1.70 (d, *J =* 6.8 Hz, 3H)
MS (ESI+) m/z 403 (M+H)⁺

### Example 28

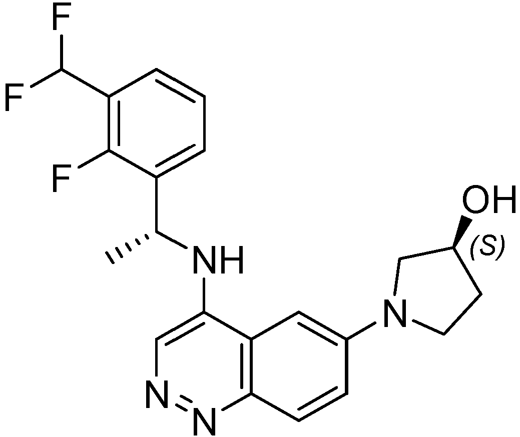

### (S)-1-(4-(((R)-1-(3-(difluoromethyl)-2-fluorophenyl)ethyl)amino)cinnolin-6-yl)pyrrolidin-3-ol

Intermediate A-1 and (R)-1-(3-(difluoromethyl)-2-fluorophenyl)ethanamine were used as described in Example 1 to synthesize compound A-2, and then the title compound (9 mg, 11%) was obtained in substantially the same manner as described in the synthesis method of Example 5, except for using (S)-pyrrolidin-3-ol.
¹H NMR (400 MHz, DMSO-d6) δ 8.03 (s, 1H), 7.94 (d, *J =* 9.6 Hz, 1H), 7.59 (t, *J =* 7.6 Hz, 1H), 7.54 (t, *J =* 7.2 Hz, 1H), 7.41-7.14 (m, 5H), 7.04 (d, *J* = 2.0 Hz, 1H), 5.23 (q, *J =* 7.2 Hz, 1H), 5.09 (d, *J =* 3.6 Hz, 1H), 4.50 (s, 1H), 3.61-3.53 (m, 3H), 3.39-3.36 (m, 1H), 2.16-2.10 (m, 1H), 2.01-1.95 (m, 1H), 1.70 (d, *J =* 6.8 Hz, 3H)
MS (ESI+) m/z 403 (M+H)⁺

### Example 29

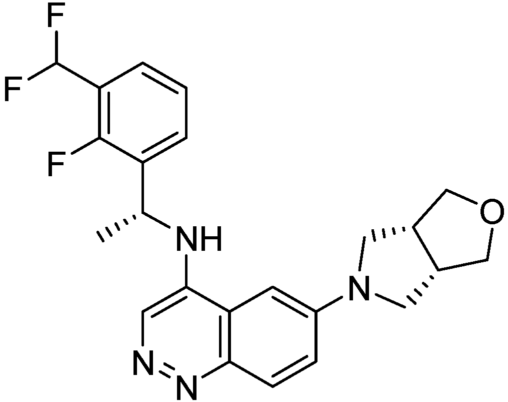

### N-((R)-1-(3-(difluoromethyl)-2-fluorophenyl)ethyl)-6-((3 aR,6aS)-tetrahydro-1H-furo[3,4-c]pyrrol-5(3H)-yl)cinnolin-4-amine

Intermediate A-1 and (R)-1-(3-(difluoromethyl)-2-fluorophenyl)ethanamine were used as described in Example 1 to synthesize compound A-2, and then the title compound (70 mg, 32%) was obtained in substantially the same manner as described in the synthesis method of Example 5, except for using (3aS,6aS)-hexahydro-1H-furo[3,4.-c]pyrrole hydrochloride.
¹H NMR (400 MHz, DMSO-d6) δ 7.91 (s, 1H), 7.82 (d, *J =* 9.2 Hz, 1H), 7.47-7.39 (m, 2H), 7.28-7.00 (m, 5H), 5.08 (q, *J =* 7.2 Hz, 1H), 3.79-3.76 (m, 2H), 3.53-3.46 (m, 4H), 3.35-3.25 (m, 2H), 3.02-2.95 (m, 2H), 1.55 (d, *J =* 6.8 Hz, 3H)
MS (ESI+) m/z 429 (M+H)⁺

### Example 30

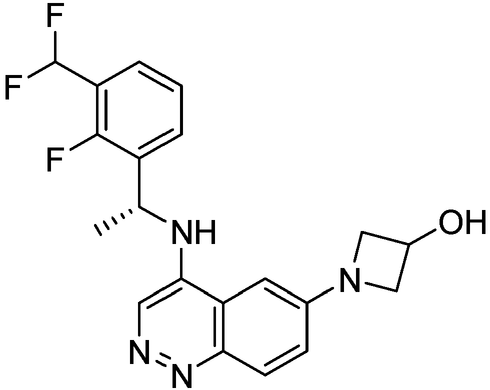

### (R)-1-(4-((1-(3-(difluoromethyl)-2-fluorophenyl)ethyl)amino)cinnolin-6-yl)azetidin-3-ol

Intermediate A-1 and (R)-1-(3-(difluoromethyl)-2-fluorophenyl)ethanamine were used as described in Example 1 to synthesize compound A-2, and then the title compound (3 mg, 3%) was obtained in substantially the same manner as described in the synthesis method of Example 5, except for using azetidin-3-ol hydrochloride.
¹H NMR (400 MHz, MeOD) δ 8.03 (s, 1H), 7.97 (d, *J =* 9.2 Hz, 1H), 7.56 (q, *J =* 8.7 Hz, 2H), 7.26 (t, *J* = 7.8 Hz, 1H), 7.21-6.93 (m, 3H), 5.27 (q, *J =* 6.9 Hz, 1H), 4.83-4.79 (m, 1H), 4.42-4.38 (m, 2H), 3.92 (x, *J* = 4.1 Hz, 8.3 Hz, 2H), 1.78 (d, *J =* 6.8 Hz, 3H)
MS (ESI+) m/z 389 (M+H)⁺

### Example 31

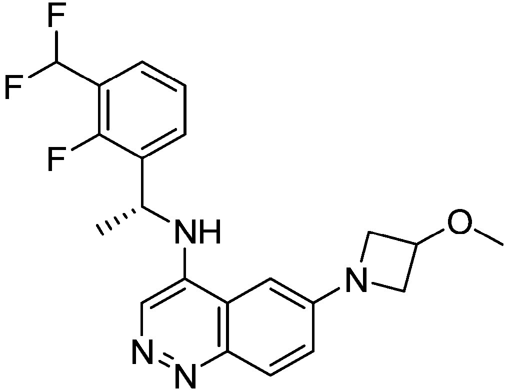

### (R)-N-(1-(3-(difluoromethyl)-2-fluorophenyl)ethyl)-6-(3-methoxyazetidin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (3 mg, 3%) was obtained in substantially the same manner as described in the synthesis method of Example 5, except for using 3-methoxyazetidine hydrochloride.
¹H NMR (400 MHz, MeOD) δ 8.03 (s, 1H), 7.97 (d, *J =* 9.2 Hz, 1H), 7.57-7.52 (m, 2H), 7.26 (t, *J =* 7.8 Hz, 1H), 7.21-6.93 (m, 3H), 5.27 (q, *J =* 7.6 Hz, 1H), 4.50-4.47 (m, 1H), 4.38-4.34 (m, 2H), 3.96 (x, *J =* 4.1 Hz, 8.3 Hz, 2H), 3.42 (s, 3H), 1.78 (d, *J =* 6.8 Hz, 3H)
MS (ESI+) m/z 403 (M+H)⁺

### Example 32

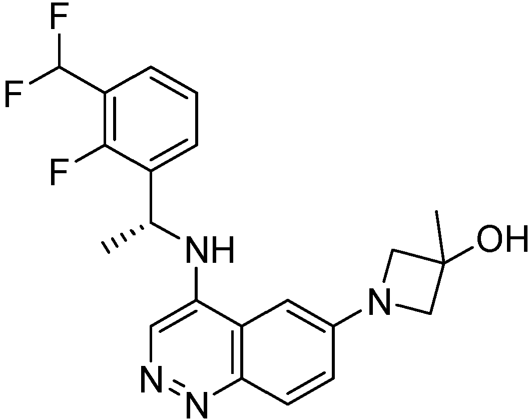

### (R)-1-(4-((1-(3-(difluoromethyl)-2-fluorophenyl)ethyl)amino)cinnolin-6-yl)-3-methylazetidin-3-ol

Intermediate A-1 and (R)-1-(3-(difluoromethyl)-2-fluorophenyl)ethanamine were used as described in Example 1 to synthesize compound A-2, and then the title compound (40 mg, 40%) was obtained in substantially the same manner as described in the synthesis method of Example 5, except for using 3-methylazetidin-3-ol hydrochloride.
¹H NMR (400 MHz, DMSO-d6) δ 8.05 (s, 1H), 7.94 (d, *J* = 9.2 Hz, 1H), 7.56 (dd, *J =* 9.6 Hz, 16.1 Hz, 2H), 7.41-7.07 (m, 5H), 5.68 (s, 1H), 5.19 (t, *J =* 6.8 Hz, 1H), 3.97 (t, *J =* 6.8 Hz, 2H), 3.87 (t, *J =* 8.6 Hz, 2H), 1.68 (d, *J =* 6.8 Hz, 3H), 1.51 (s, 3H)
MS (ESI+) m/z 403 (M+H)⁺

### Example 33

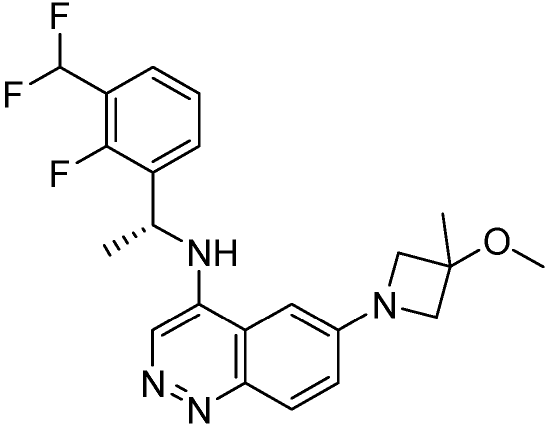

### (R)-N-(1-(3-(difluoromethyl)-2-fluorophenyl)ethyl)-6-(3-methoxy-3-methylazetidin-1-yl)cinnolin-4-amine

Intermediate A-1 and (R)-1-(3-(difluoromethyl)-2-fluorophenyl)ethanamine were used as described in Example 1 to synthesize compound A-2, and then the title compound (24 mg, 29%) was obtained in substantially the same manner as described in the synthesis method of Example 5, except for using 3-methoxy-3-methylazetidine hydrochloride.
¹H NMR (400 MHz, DMSO-d6) δ 8.05 (s, 1H), 7.95 (d, *J =* 9.6 Hz, 1H), 7.59 (t, *J =* 7.0 Hz, 1H), 7.53 (t, *J =* 6.4 Hz, 1H), 7.41-7.09 (m, 5H), 5.19 (t, *J =* 6.8 Hz, 1H), 3.98 (t, *J =* 7.8 Hz, 2H), 3.92-3.88 (m, 2H), 3.25 (s, 3H), 1.68 (d, *J =* 6.8 Hz, 3H), 1.54 (s, 3H)
MS (ESI+) m/z 417 (M+H)⁺

### Example 34

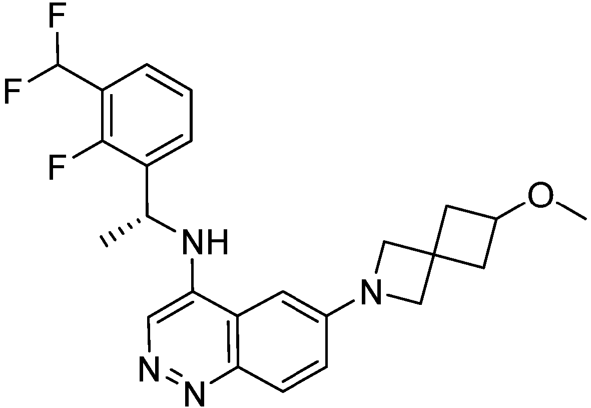

### (R)-N-(1-(3-(difluoromethyl)-2-fluorophenyl)ethyl)-6-(6-methoxy-2-azaspiro[3.3]heptan-2-yl)cinnolin-4-amine

Intermediate A-1 and (R)-1-(3-(difluoromethyl)-2-fluorophenyl)ethanamine were used as described in Example 1 to synthesize compound A-2, and then the title compound (32 mg, 36%) was obtained in substantially the same manner as described in the synthesis method of Example 5, except for using 6-methoxy-2-azaspiro[3.3]heptane hydrochloride.
¹H NMR (400 MHz, DMSO-d6) δ 8.06 (s, 1H), 7.93 (d, *J =* 9.6 Hz, 1H), 7.60-7.52 (m, 2H), 7.41-7.14 (m, 3H), 7.07-7.04 (m, 2H), 5.21 (q, *J =* 7.2 Hz, 1H), 4.11-3.95 (m, 4H), 3.83 (q, *J* = 6.8 Hz, 1H), 3.16 (s, 3H), 2.57-2.52 (m, 2H), 2.15-2.10 (m, 2H), 1.67 (d, *J =* 6.8 Hz, 3H)
MS (ESI+) m/z 443 (M+H)⁺

### Example 35

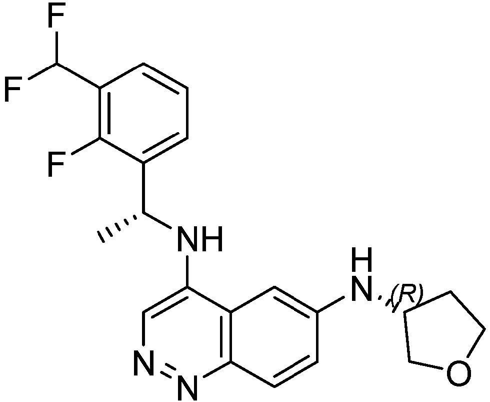

### N4-((R)-1-(3-(difluoromethyl)-2-fluorophenyl)ethyl)-N6-((R)-tetrahydrofuran-3-yl)cinnolin-4,6-diamine

Intermediate A-1 and (R)-1-(3-(difluoromethyl)-2-fluorophenyl)ethanamine were used as described in Example 1 to synthesize compound A-2, and then the title compound (10 mg, 17%) was obtained in substantially the same manner as described in the synthesis method of Example 5, except for using (3R)-tetrahydro-3-furanamine.
1H NMR (400 MHz, DMSO-d6) δ 8.01 (s, 1H), 7.82 (d, *J =* 9.2 Hz, 1H), 7.59 (t, *J =* 7.2 Hz, 1H), 7.54 (t, *J =* 6.8 Hz, 1H), 7.42 (s, 0.25H), 7.31 (s, 0.25H), 7.29 (d, *J =* 5.6 Hz, 1H), 7.22 (dd, *J= 2.4* Hz,6.8 Hz, 1H), 7.14 (s, 0.25H), 7.06 (s, 0.25H), 7.03 (d, *J=* 2.0 Hz, 1H), 6.80 (d, *J =* 7.2 Hz, 1H), 5.20-5.17 (m, 1H), 4.32-4.29 (m, 1H), 4.13-4.09 (m, 1H), 3.92-3.80 (m, 2H), 3.60-3.57 (m, 1H), 2.83-2.33 (m, 1H), 1.88-1.81 (m, 1H), 1.54 (d, *J =* 6.8 Hz, 3H)
MS (ESI+) m/z 403 (M+H)⁺

### Example 36

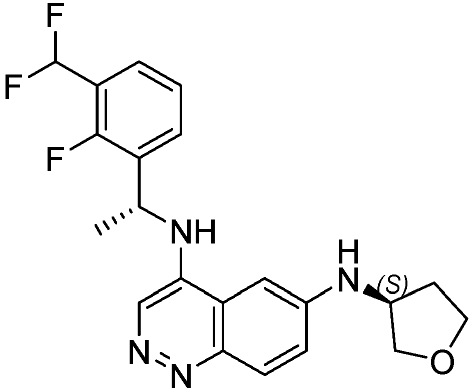

### N4-((R)-1-(3-(difluoromethyl)-2-fluorophenyl)ethyl)-N6-((S)-tetrahydrofuran-3-yl)cinnolin-4,6-diamine

Intermediate A-1 and (R)-1-(3-(difluoromethyl)-2-fluorophenyl)ethanamine were used as described in Example 1 to synthesize compound A-2, and then the title compound (24 mg, 40%) was obtained in substantially the same manner as described in the synthesis method of Example 5, except for using (3S)-tetrahydro-3-furanamine.
1H NMR (400 MHz, DMSO-d6) δ 8.01 (s, 1H), 7.82 (d, *J* = 9.2 Hz, 1H), 7.59 (t, *J* = 7.2 Hz, 1H), 7.54 (t, *J* = 6.8 Hz, 1H), 7.41 (s, 0.25H), 7.31 (s, 0.25H), 7.29 (d, *J* = 6.4 Hz, 1H), 7.22 (dd, *J* = 2.4 Hz,6.8 Hz, 1H), 7.14 (s, 0.25H), 7.06 (s, 0.25H), 7.03 (d, *J =* 2.0 Hz, 1H), 6.80 (d, *J* = 7.2 Hz, 1H), 5.20-5.17 (m, 1H), 4.32-4.29 (m, 1H), 4.11-4.07 (m, 1H), 3.92-3.82 (m, 2H), 3.57-3.54 (m, 1H), 2.83-2.33 (m, 1H), 1.88-1.81 (m, 1H), 1.69 (d, *J* = 6.8 Hz, 3H)
MS (ESI+) m/z 403 (M+H)⁺

### Example 37

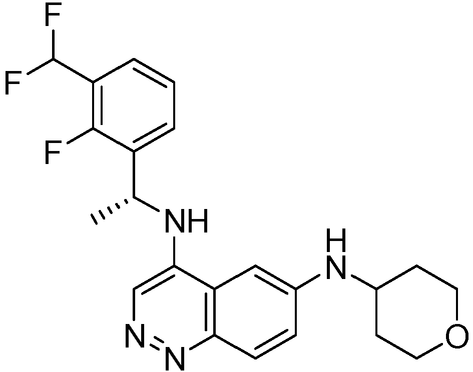

### (R)-N4-(1-(3-(difluoromethyl)-2-fluorophenyl)ethyl)-N6-(tetrahydro-2H-pyran-4-yl)cinnolin-4,6-diamine

Intermediate A-1 and (R)-1-(3-(difluoromethyl)-2-fluorophenyl)ethanamine were used as described in Example 1 to synthesize compound A-2, and then the title compound (27 mg, 43%) was obtained in substantially the same manner as described in the synthesis method of Example 5, except for using 4-aminotetrahydropyran.
1H NMR (400 MHz, DMSO-d6) δ 7.98 (s, 1H), 7.83 (d, *J* = 9.2 Hz, 1H), 7.60-7.52 (m, 2 H), 7.41 (s, 0.25H), 7.30-7.26 (m, 2.50H), 7.23 (dd, *J* = 2.0 Hz, 7.2 Hz, 1H), 7.14 (s, 0.25 H), 7.03 (d, *J* = 2.0 Hz, 1H), 6.95 (d, *J* = 7.2 Hz, 1H), 6.49 (d, *J* = 8.0 Hz, 1H), 5.19-5.16 (m, 1H), 3.98-3.91 (m, 2H), 3.83-3.80 (m, 1H), 3.87-3.49 (m, 2H), 2.03-1.99 (m, 2H), 1.68 (d, *J* = 6.8 Hz, 3H), 1.51-1.48 (m, 1H)
MS (ESI+) m/z 417 (M+H)⁺

### Example 38

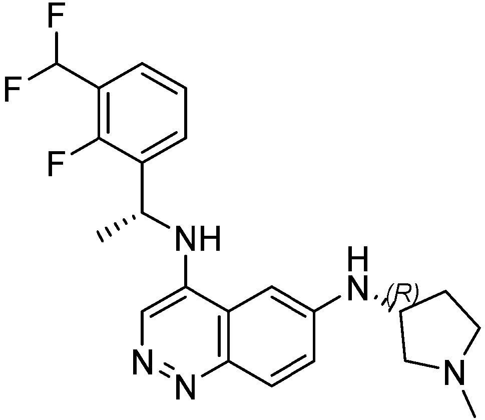

### N4-((R)-1-(3-(difluoromethyl)-2-fluorophenyl)ethyl)-N6-((R)-1-methylpyrrolidin-3-yl)cinnolin-4,6-diamine

Intermediate A-1 and (R)-1-(3-(difluoromethyl)-2-fluorophenyl)ethanamine were used as described in Example 1 to synthesize compound A-2, and then the title compound (6 mg, 10%) was obtained in substantially the same manner as described in the synthesis method of Example 5, except for using (3R)-1-methyl-3-pyrrolidinamine.
1H NMR (400 MHz, DMSO-d6) δ 7.98 (s, 1H), 7.80 (d, *J* = 9.2 Hz, 1H), 7.60-7.52 (m, 2H), 7.42 (s, 0.25H), 7.31 (s, 0.25H), 7.29-7.28 (m, 2.50H), 7.21 (dd, *J* = 2.4 Hz,6.8 Hz, 1H), 7.15 (s, 0.25H), 7.06 (d, *J* = 7.2 Hz, 1H), 6.98 (d, *J* = 2.0 Hz, 1H), 6.74 (d, *J* = 7.6 Hz, 1H), 5.20-5.15 (m, 1H), 4.23-4.22 (m, 1H), 3.00-2.97 (m, 1H), 2.63-2.31 (m, 4H), 2.31 (s, 1H), 1.68 (d, *J* = 6.8 Hz, 3H), 1.58-1.49 (m, 1H)
MS (ESI+) m/z 416 (M+H)⁺

### Example 39

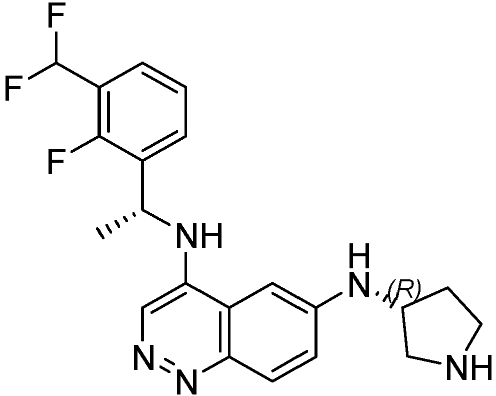

### N4-((R)-1-(3-(difluoromethyl)-2-fluorophenyl)ethyl)-N6-((R)-pyrrolidin-3-yl)cinnolin-4,6-diamine

Intermediate A-1 and (R)-1-(3-(difluoromethyl)-2-fluorophenyl)ethanamine were used as described in Example 1 to synthesize compound A-2, and then (R)-tert-butyl 3-(4-((R)-1-(3-(difluoromethyl)-2-fluorophenyl)ethylamino)cinnolin-6-ylamino)pyrrolidin-1-carboxylate (57 mg, 57%) was obtained in substantially the same manner as described in the synthesis method of Example 5, except for using (R)-tert-butyl 3-aminopyrrolidin-1-carboxylate.

To a solution in which (R)-tert-butyl 3-(4-((R)-1-(3-(difluoromethyl)-2-fluorophenyl)ethylamino)cinnolin-6-ylamino)pyrrolidin-1-carboxylate (57 mg, 0.11 mmol) was dissolved in dichloromethane (3 ml), a solution of 4 N hydrochloric acid (0.40 ml, 1.0 mmol) in 1,4-dioxane was added at 0°C, and then stirred at room temperature for two hours. Upon completion of the reaction, the resulting mixture was concentrated and extracted using an aqueous sodium hydrogen carbonate solution and ethyl acetate. The combined organic extracts were dried over sodium sulfate and then concentrated to afford N4-((R)-1-(3-(difluoromethyl)-2-fluorophenyl)ethyl)-N6-((R)-pyrrolidin-3-yl)cinnolin-4,6-diamine (6 mg, 13%) as a yellow solid.
1H NMR (400 MHz, DMSO-d6) δ 7.98 (s, 1H), 7.80 (d, *J* = 9.2 Hz, 1H), 7.60-7.52 (m, 2H), 7.42 (s, 0.25H), 7.31 (s, 0.25H), 7.29-7.28 (m, 1.25H), 7.54-7.02 (m, 3.25H), 6.78-6.62 (m, 1H), 5.20-5.15 (m, 1H), 4.23-4.08 (m, 1H), 3.78-2.70 (m, 1H), 2.99-2.83 (m, 1H), 2.22-2.13 (m, 1H), 1.89-1.82 (m, 1H), 1.68 (d, *J* = 6.8 Hz, 3H)
MS (ESI+) m/z 402 (M+H)⁺

### Example 40

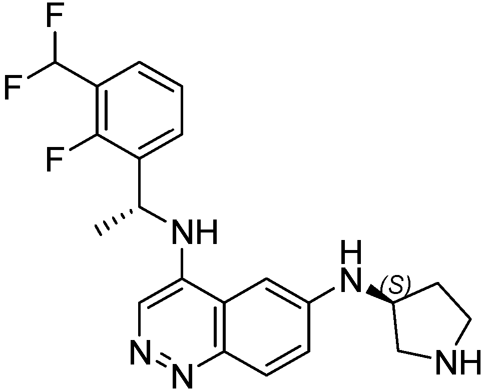

### N4-((R)-1-(3-(difluoromethyl)-2-fluorophenyl)ethyl)-N6-((S)-pyrrolidin-3-yl)cinnolin-4,6-diamine

Intermediate A-1 and (R)-1-(3-(difluoromethyl)-2-fluorophenyl)ethanamine were used as described in Example 1 to synthesize compound A-2, and then (S)-tert-butyl 3-(4-((R)-1-(3-(difluoromethyl)-2-fluorophenyl)ethylamino)cinnolin-6-ylamino)pyrrolidin-1-carboxylate (40 mg, 40%) was obtained in substantially the same manner as described in the synthesis method of Example 5, except for using (S)-tert-butyl 3-aminopyrrolidin-1-carboxylate.

To a solution in which (S)-tert-butyl 3-(4-((R)-1-(3-(difluoromethyl)-2-fluorophenyl)ethylamino)cinnolin-6-ylamino)pyrrolidin-1-carboxylate (40 mg, 0.11 mmol) was dissolved in dichloromethane (3 ml), a solution of 4 N hydrochloric acid (0.40 ml, 1.0 mmol) in 1,4-dioxane was added at 0°C, and then stirred at room temperature for two hours. Upon completion of the reaction, the resulting mixture was concentrated and extracted using an aqueous sodium hydrogen carbonate solution and ethyl acetate. The combined organic extracts were dried over sodium sulfate and then concentrated to afford N4-((R)-1-(3-(difluoromethyl)-2-fluorophenyl)ethyl)-N6-((S)-pyrrolidin-3-yl)cinnolin-4,6-diamine (17 mg, 60%) as a yellow solid.
1H NMR (400 MHz, DMSO-d6) δ 7.99 (s, 1H), 7.80 (d, *J* = 9.2 Hz, 1H), 7.59 (t, *J* = 7.6 Hz, 1H), 7.53 (t, *J* = 7..2 Hz, 1H), 7.42 (s, 0.25H), 7.31-7.27 (m, 1.50H), 7.20-7.02 (m, 3.25H), 6.78-6.62 (m, 1H), 5.19-5.16 (m, 1H), 4.23-4.14 (m, 1H), 3.75-2.72 (m, 1H), 3.23-3.20 (m, 1H), 2.90-2.79 (m, 2H), 2.32-2.15 (m, 1H), 1.98-1.89(m, 1H), 1.68 (d, *J* = 6.8 Hz, 3H)
MS (ESI+) m/z 402 (M+H)⁺

### Example 41

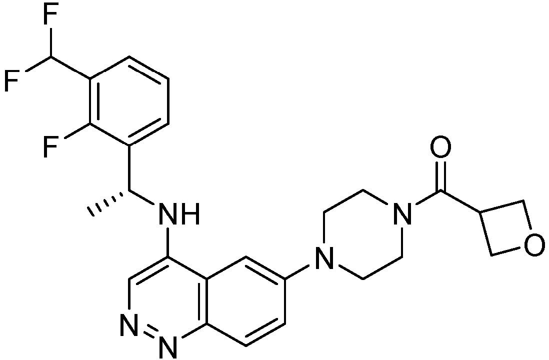

### (R)-(4-(4-(1-(3-(difluoromethyl)-2-fluorophenyl)ethylamino)cinnolin-6-yl)piperazin-1-yl)(oxetan-3-yl)methanone

### Step 1

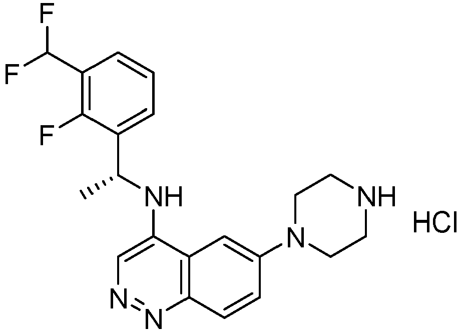

### (R)-N-(1-(3-(difluoromethyl)-2-fluorophenyl)ethyl)-6-(piperazin-1-yl)cinnolin-4-amine hydrochloride

Intermediate A-1 and (R)-1-(3-(difluoromethyl)-2-fluorophenyl)ethanamine were used as described in Example 1 to synthesize compound A-2, and then (R)-tert-butyl 4-(4-(1-(3-(difluoromethyl)-2-fluorophenyl)ethylamino)cinnolin-6-yl)piperazin-1-carboxylate (60 mg, 60%) was obtained in substantially the same manner as described in the synthesis method of Example 5, except for using tert-butoxycarbonylpiperazine.

To a solution in which (R)-tert-butyl 4-(4-(1-(3-(difluoromethyl)-2-fluorophenyl)ethylamino)cinnolin-6-yl)piperazin-1-carboxylate (60 mg, 0.11 mmol) was dissolved in dichloromethane (3 ml), a solution of 4 N hydrochloric acid (0.40 ml, 1.0 mmol) in 1,4-dioxane was added at 0°C, and then stirred at room temperature for two hours. After completion of the reaction, the resulting mixture was concentrated to afford (R)-N-(1-(3-(difluoromethyl)-2-fluorophenyl)ethyl)-6-(piperazin-1-yl)cinnolin-4-amine hydrochloride (40 mg, 90%) as a brown solid.

MS (ESI+) m/z 402 (M+H)⁺

### Step 2

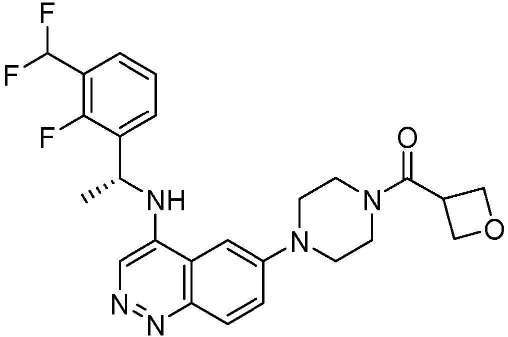

### (R)-(4-(4-(1-(3-(difluoromethyl)-2-fluorophenyl)ethylamino)cinnolin-6-yl)piperazin-1-yl)(oxetan-3-yl)methanone

After adding (R)-N-(1-(3-(difluoromethyl)-2-fluorophenyl)ethyl)-6-(piperazin-1-yl)cinnolin-4-amine hydrochloride (40 mg, 0.09 mmol), oxetan-3-carboxylic acid (11 mg, 0.11 mmol), HATU (60 mg, 0.27 mmol), and N,N-diisopropylethylamine (0.06 ml, 0.54 mmol) in dimethyl sulfoxide (1.5 ml), the resulting mixture was stirred at room temperature for one hour. After confirming the completion of the reaction, water was added and extracted with ethyl acetate. The combined organic extracts were dried over sodium sulfate and concentrated, and then the residue was purified by column chromatography to afford (R)-(4-(4-(1-(3-(difluoromethyl)-2-fluorophenyl)ethylamino)cinnolin-6-yl)piperazin-1-yl)(oxetan-3-yl)methanone (7 mg, 16%) as a yellow solid.
1H NMR (400 MHz, DMSO-d6) δ 8.27 (s, 1H), 8.19 (s, 1H), 7.94 (d, *J* = 9.2 Hz, 1H), 7.81 (d, *J* = 2.0 Hz, 1H), 7.69-7.55 (m, 3H), 7.42-7.12 (m, 2H), 5.45-5.42 (m, 1H), 4.76-4.68 (m, 4H), 4.26-4.18 (m, 1H), 3.71-3.65 (m, 2H), 3.50-3.49 (m, 2H), 1.73 (d, *J* = 6.8 Hz, 3H)
MS (ESI+) m/z 486 (M+H)⁺

### Example 42

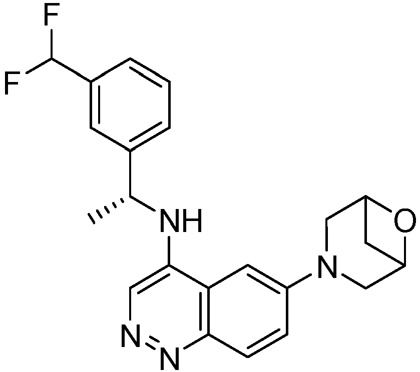

### 6-(6-oxa-3-azabicyclo[3.1.1]heptan-3-yl)-N-((R)-1-(3-(difluoromethyl)phenyl)ethyl)cinnolin-4-amine

Intermediate A-1 and (R)-1-(3-(difluoromethyl)phenyl)ethanamine were used as described in Example 1 to synthesize compound A-2a, and then the title compound (3 mg, 4%) was obtained in substantially the same manner as described in the synthesis method of Example 5, except for using 6-oxa-3-azabicyclo[3.1.1]heptane hydrochloride.
¹H NMR (400 MHz, DMSO-d6) δ 8.15 (s, 1H), 8.01 (d, *J* = 9.2 Hz, 1H), 7.68-7.65 (m, 2H), 7.50-7.43 (m, 3H), 7.26-7.25 (m, 2H), 7.02 (t, *J* = 55.8 Hz, 1H), 5.02 (t, *J =* 6.8 Hz, 1H), 4.83 (d, *J* = 6.4 Hz, 2H), 3.80 (t, *J* = 13.4 Hz, 2H), 3.64 (t, *J* = 14.0 Hz, 2H), 3.20 (q, *J* = 7.3 Hz, 1H), 2.00 (d, *J=* 8.8 Hz, 1H), 1.65 (d, *J=* 6.8 Hz, 3H)
MS (ESI+) m/z 397 (M+H)⁺

### Example 43

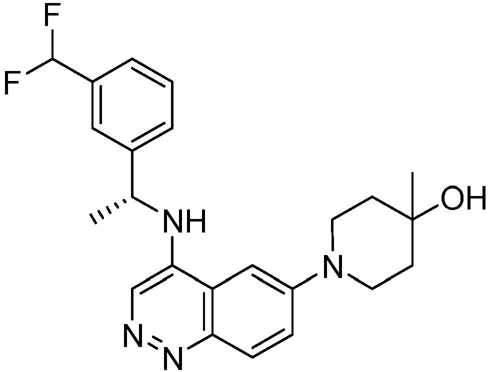

### (R)-1-(4-((1-(3-(difluoromethyl)phenyl)ethyl)amino)cinnolin-6-yl)-4-methylpiperidin-4-ol

Intermediate A-1 and (R)-1-(3-(difluoromethyl)phenyl)ethanamine were used as described in Example 1 to synthesize compound A-2a, and then the title compound (56 mg, 54%) was obtained in substantially the same manner as described in the synthesis method of Example 5, except for using 4-methyl-4-piperidinol.
¹H NMR (400 MHz, DMSO-d6) δ 8.15 (s, 1H), 7.91 (d, *J* = 9.2 Hz, 1H), 7.68-7.60 (m, 3H), 7.52-7.45 (m, 3H), 7.31 (d, *J =* 7.2 Hz, 1H), 7.02 (t, *J* = 55.8 Hz, 1H), 5.02 (t, *J* = 6.8 Hz, 1H), 4.43 (s, 1H), 4.68-3.64 (m, 2H), 3.35-3.24 (m, 2H), 1.64 (d, *J* = 6.8 Hz, 7H), 1.20 (s, 3H)
MS (ESI+) m/z 413 (M+H)⁺

### Example 44

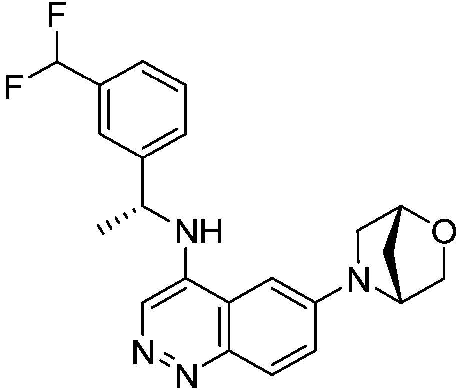

### 6-((1R,4R)-2-oxa-5-azabicyclo[2.2.1]heptan-5-yl)-N-((R)-1-(3-(difluoromethyl)phenyl)ethyl)cinnolin-4-amine

Intermediate A-1 and (R)-1-(3-(difluoromethyl)phenyl)ethanamine were used as described in Example 1 to synthesize compound A-2a, and then the title compound (60 mg, 58%) was obtained in substantially the same manner as described in the synthesis method of Example 5, except for using (1R,4R)-2-oxa-5-azabicyclo[2.2.1]heptane hydrochloride.
¹H NMR (400 MHz, DMSO-d6) δ 8.11 (s, 1H), 7.93 (d, *J* = 9.6 Hz, 1H), 7.67-7.63 (m, 2H), 7.51-7.43 (m, 2H), 7.34 (d, *J =* 9.2 Hz, 1H), 7.16-6.88 (m, 3H), 5.01 (t, *J =* 6.8 Hz, 1H), 4.90 (s, 1H), 4.75 (s, 1H), 3.88 (d, *J* = 6.0 Hz, 1H), 3.74 (d, *J* = 7.2 Hz, 1H), 3.65 (d, *J* = 8.4 Hz, 1H), 3.25 (d, *J =* 10.0 Hz, 1H), 1.99 (q, *J =* 11.3 Hz, 2H), 1.64 (d, *J=* 6.8 Hz, 3H)
MS (ESI+) m/z 397 (M+H)⁺

### Example 45

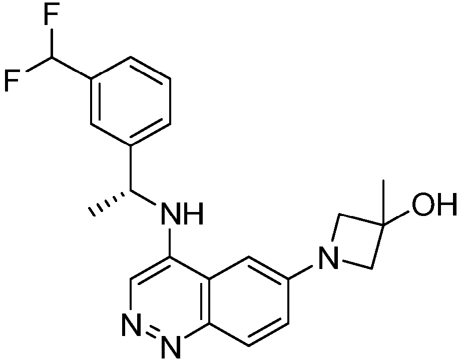

### (R)-1-(4-((1-(3-(difluoromethyl)phenyl)ethyl)amino)cinnolin-6-yl)-3-methylazetidin-3-ol

Intermediate A-1 and (R)-1-(3-(difluoromethyl)phenyl)ethanamine were used as described in Example 1 to synthesize compound A-2a, and then the title compound (45 mg, 45%) was obtained in substantially the same manner as described in the synthesis method of Example 5, except for using 3-methylazetidin-3-ol.
¹H NMR (400 MHz, DMSO-d6) δ 8.13 (s, 1H), 7.92 (d, *J* = 10.0 Hz, 1H), 7.67-7.63 (m, 2H), 7.51-7.43 (m, 2H), 7.18-6.88 (m, 4H), 5.68 (s, 1H), 5.00 (t, *J =* 7.0 Hz, 1H), 3.97 (t, *J* = 7.0 Hz, 2H), 3.87 (d, *J* = 9.2 Hz, 2H), 1.63 (d, *J =* 6.8 Hz, 3H), 1.51 (s, 3H)
MS (ESI+) m/z 385 (M+H)⁺

### Example 46

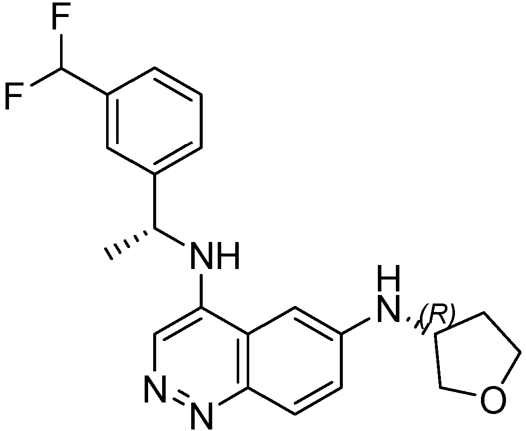

### N4-((R)-1-(3-(difluoromethyl)phenyl)ethyl)-N6-((R)-tetrahydrofuran-3-yl)cinnolin-4,6-diamine

Intermediate A-1 and (R)-1-(3-(difluoromethyl)phenyl)ethanamine were used as described in Example 1 to synthesize compound A-2a, and then the title compound (25 mg, 33%) was obtained in substantially the same manner as described in the synthesis method of Example 5, except for using (3R)-tetrahydro-3-furanamine.
¹H NMR (400 MHz, DMSO-d6) δ 8.09 (s, 1H), 7.80 (d, *J* = 9.2 Hz, 1H), 7.67-7.63 (m, 2H), 7.51-7.43 (m, 2H), 7.20 (dd, *J* = 2.4 Hz, 9.2 Hz, 1H), 7.19-6.88 (m, 3H), 6.78 (d, *J=* 7.2 Hz, 1H), 5.00 (t, *J* = 7.0 Hz, 1H), 4.31-4.29 (m, 1H), 4.11 (q, *J* = 4.9 Hz, 1H), 3.92-3.80 (m, 2H), 3.58 (q, *J* = 4.4 Hz, 1H), 2.36-2.34 (m, 1H), 1.88-1.82 (m, 1H), 1.64 (d, *J =* 6.8 Hz, 3H)
MS (ESI+) m/z 385 (M+H)⁺

### Example 47

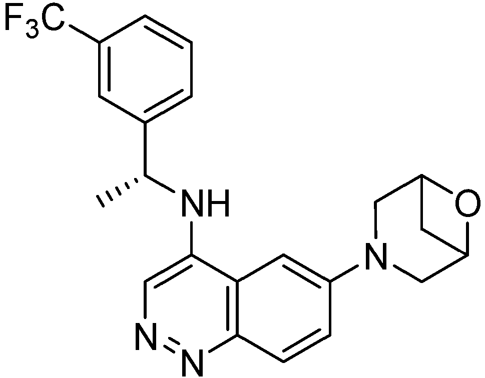

### 6-(6-oxa-3-azabicyclo[3.1.1]heptan-3-yl)-N-((R)-1-(3-(trifluoromethyl)phenyl)ethyl)cinnolin-4-amine

Intermediate A-1 and (R)-1-(3-(trifluoromethyl)phenyl)ethanamine were used as described in Example 1 to synthesize compound A-2b, and then the title compound (278 mg, 40%) was obtained in substantially the same manner as described in the synthesis method of Example 5, except for using 6-oxa-3-azabicyclo[3.1.1]heptane hydrochloride.
¹H NMR (400 MHz, DMSO-d6) δ 8.18 (s, 1H), 8.02 (d, *J =* 9.2 Hz, 1H), 7.87 (s, 1H), 7.80 (d, *J* = 6.8 Hz, 1H), 7.63-7.51 (m, 2H), 7.49 (dd, *J* = 2.8 Hz, 9.2 Hz, 1H), 7.33-7.21 (m, 2H), 5.10 (quint, *J* = 6.8 Hz, 1H), 4.82 (d, *J* = 6.4 Hz, 2H), 3.80 (t, *J* = 12.4 Hz, 2H), 3.66 (t, *J* = 12.4 Hz, 2H), 3.26-3.18 (m, 1H), 2.07-1.98 (m, 1H), 1.66 (d, *J* = 6.8 Hz, 3H)
MS (ESI+) m/z 415 (M+H)⁺

### Example 48

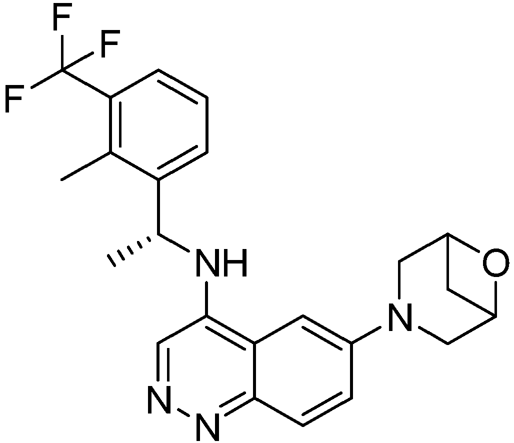

### 6-(6-oxa-3-azabicyclo[3.1.1]heptan-3-yl)-N-((R)-1-(2-methyl-3-(trifluoromethyl)phenyl)ethyl)cinnolin-4-amine

Intermediate A-1 and (R)-1-(2-methyl-3-(trifluoromethyl)phenyl)ethanamine were used as described in Example 1 to synthesize compound A-2c, and then the title compound (45 mg, 54%) was obtained in substantially the same manner as described in the synthesis method of Example 5, except for using 6-oxa-3-azabicyclo[3.1.1]heptane hydrochloride.
¹H NMR (400 MHz, DMSO-d6) δ 8.02 (d, *J* = 9.6 Hz, 1H), 7.89 (s, 1H), 7.64 (d, *J* = 8 Hz, 1H), 7.58 (d, *J* = 7.6 Hz, 1H), 7.51 (d, *J=* 2.4 Hz, 1H), 7.48 (d, *J=* 2.4 Hz, 1H), 7.34-7.29 (m, 3H), 5.20 (q, *J=* 7.2 Hz, 1H), 4.83 (d, *J=* 6.4 Hz, 2H), 3.82 (d, *J=* 15.6 Hz, 1H), 3.79 (d, *J =* 15.6 Hz, 1H), 3.66 (d, *J* = 16.4 Hz, 1H), 3.63 (d, *J* = 16.4 Hz, 1H), 3.23-3.18 (m, 1H), 2.61 (s, 3H), 1.99 (d, *J* = 8.8 Hz, 1H), 1.63 (d, *J* = 6.8 Hz, 3H)
MS (ESI+) m/z 429 (M+H)⁺

### Example 49

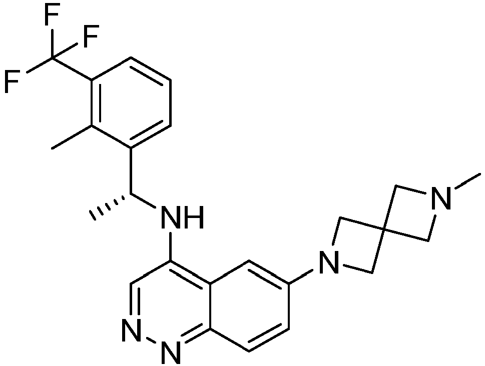

### (R)-N-(1-(2-methyl-3-(trifluoromethyl)phenyl)ethyl)-6-(6-methyl-2,6-diazaspiro[3.3]heptan-2-yl)cinnolin-4-amine

Intermediate A-1 and (R)-1-(2-methyl-3-(trifluoromethyl)phenyl)ethanamine were used as described in Example 1 to synthesize compound A-2c, and then the title compound (46 mg, 54%) was obtained in substantially the same manner as described in the synthesis method of Example 5, except for using 2-methyl-2,6-diazaspiro[3.3]heptane.
¹H NMR (400 MHz, DMSO-d6) δ 7.97 (d, *J* = 9.2 Hz, 1H), 7.91 (s, 1H), 7.67 (d, *J* = 8 Hz, 1H), 7.63 (d, *J* = 7.6 Hz, 1H), 7.36 (t, *J* = 7.6 Hz, 1H), 7.25 (d, *J* = 6.8 Hz, 1H), 7.15 (d, *J* = 2.4 Hz, 1H), 7.12-7.09 (m, 1H), 5.21 (q, *J* = 7.2 Hz, 1H), 4.18-4.13 (m, 4H), 3.40-3.30 (m, 4H), 2.65 (s, 3H), 2.27 (s, 3H), 1.66 (d, *J* = 6.8 Hz, 3H)
MS (ESI+) m/z 442 (M+H)⁺

### Example 50

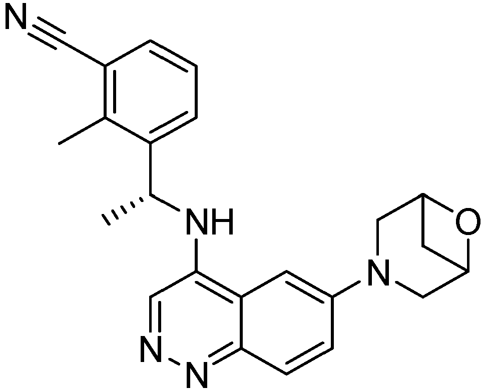

### 3-((1R)-1-((6-(6-oxa-3-azabicyclo[3.1.1]heptan-3-yl)cinnolin-4-yl)amino)ethyl)-2-methylbenzonitrile

Intermediate A-1 and 3-[(1R)-1-aminoethyl]-2-methylbenzonitrile were used as described in Example 1 to synthesize compound A-2d, and then the title compound (13 mg, 21%) was obtained in substantially the same manner as described in the synthesis method of Example 5, except for using 6-oxa-3-azabicyclo[3.1.1]heptane hydrochloride.
1H NMR (400 MHz, DMSO-d6) δ 8.02 (d, *J* = 9.2 Hz, 1H), 7.90 (s, 1H), 7.69-7.63 (m, 2H), 7.50 (dd, *J* = 2.8 Hz, 6.8 Hz, 1H), 7.34-7.26 (m, 3H), 5.15-5.12 (m, 1H), 4.83 (d, *J* = 6.4 Hz, 2H), 3.84-3.77 (m, 2H), 3.68-3.61 (m, 2H), 3.23-3.18 (m, 1H), 2.71 (s, 3H), 1.99 (d, *J* = 8.8 Hz, 1H), 1.62 (d, *J* = 6.8 Hz, 3H)
MS (ESI+) m/z 386 (M+H)⁺

### Example 51

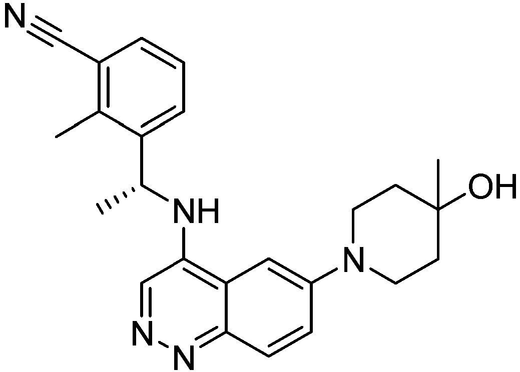

### (R)-3-(1-((6-(4-hydroxy-4-methylpiperidin-1-yl)cinnolin-4-yl)amino)ethyl)-2-methylbenzonitrile

Intermediate A-1 and 3-[(1R)-1-aminoethyl]-2-methylbenzonitrile were used as described in Example 1 to synthesize compound A-2d, and then the title compound (17 mg, 27%) was obtained in substantially the same manner as described in the synthesis method of Example 5, except for using 4-methyl-4-piperidinol.
1H NMR (400 MHz, DMSO-d6) δ 7.92-7.89 (m, 2H), 7.69 (d, *J* = 7.6 Hz, 1H), 7.65-7.62 (m, 2H), 7.49-7.48 (m, 1H), 7.37 (d, *J* = 6.4 Hz, 1H), 7.33 (t, *J* = 7.6 Hz, 1H), 5.14-5.11 (m, 1H), 4.44 (s, 1H), 3.70-3.65 (m, 2H), 2.70 (s, 3H), 1.64-1.59 (m, 5H), 1.20-1.19 (m, 5H)
MS (ESI+) m/z 402 (M+H)⁺

### Example 52

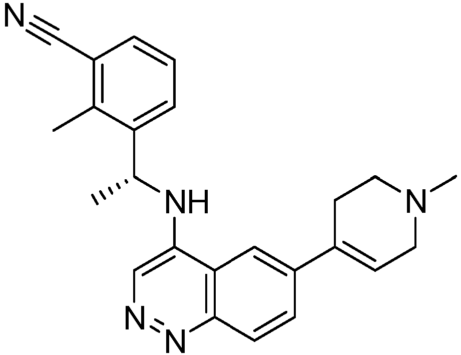

### (R)-2-methyl-3-(1-(6-(1-methyl-1,2,3,6-tetrahydropyridin-4-yl)cinnolin-4-ylamino)ethyl)benzonitrile

Intermediate A-1 and 3-[(1R)-1-aminoethyl]-2-methylbenzonitrile were used as described in Example 1 to synthesize compound A-2d, and then 1-methyl-1,2,3,6-tetrahydropyridin-4-boronic acid pinacol ester was used to afford the title compound (16 mg, 17%).
1H NMR (400 MHz, DMSO-d6) δ 8.43 (s, 1H), 8.12 (s, 1H), 8.05-7.95 (m, 2H), 7.83 (d, *J=* 6.4 Hz, 1H), 7.71-7.64 (m, 2H), 7.33 (t, *J* = 8.0 Hz, 1H), 6.49-6.48 (m, 1H), 5.19-5.17 (m, 1H), 3.14-3.12 (m, 2H), 2.73-2.65 (m, 7H), 2.33 (s, 3H), 1.62 (d, *J* = 6.8 Hz, 3H)
MS (ESI+) m/z 384 (M+H)⁺

### Example 53

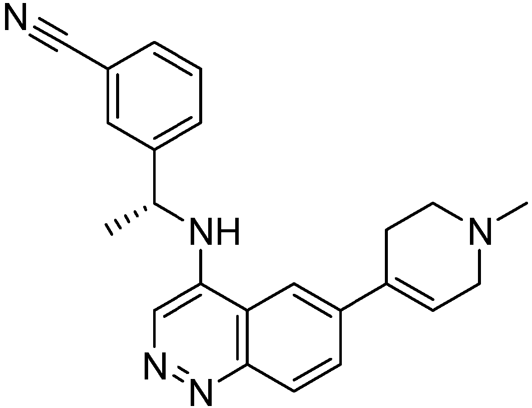

### (R)-3-(1-(6-(1-methyl-1,2,3,6-tetrahydropyridin-4-yl)cinnolin-4-ylamino)ethyl)benzonitrile

Intermediate A-1 and 3-[(1R)-1-aminoethyl]benzonitrile were used as described in Example 1 to synthesize compound A-2e, and then 1-methyl-1,2,3,6-tetrahydropyridin-4-boronic acid pinacol ester was used to afford the title compound (16 mg, 17%).
1H NMR (400 MHz, DMSO-d6) δ 8.41 (s, 1H), 8.38 (s, 1H), 8.05-7.94 (m, 3H), 7.83 (d, *J =* 8.0 Hz, 1H), 7.79 (d, *J* = 7.2 Hz, 1H), 7.75-7.72 (m, 1H), 7.57 (t, *J* = 7.6 Hz, 1H), 6.50-6.48 (m, 1H), 5.12-5.09 (m, 1H), 3.14-3.12 (m, 2H), 2.73-2.66 (m, 4H), 2.33 (s, 3H), 1.65 (d, *J* = 6.8 Hz, 3H)
MS (ESI+) m/z 370 (M+H)⁺

### Example 54

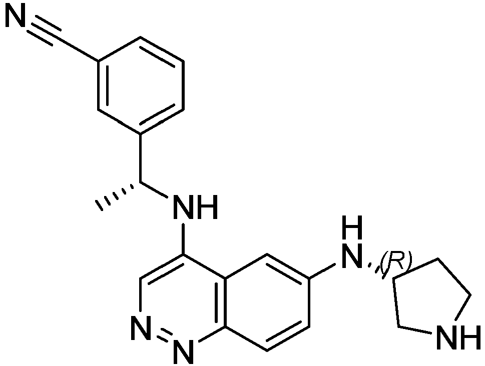

### 3-((R)-1-(6-((R)-pyrrolidin-3-ylamino)cinnolin-4-ylamino)ethyl)benzonitrile

Intermediate A-1 and 3-[(1R)-1-aminoethyl]benzonitrile were used as described in Example 1 to synthesize compound A-2e, and then (R)-tert-butyl 3-(4-((R)-1-(3-cyanophenyl)ethylamino)cinnolin-6-ylamino)pyrrolidin-1-carboxylate (50 mg, 64%) was obtained in substantially the same manner as described in the synthesis method of Example 5, except for using (R)-tert-butyl 3-aminopyrrolidin-1-carboxylate.

To a solution in which (R)-tert-butyl 3-(4-((R)-1-(3-cyanophenyl)ethylamino)cinnolin-6-ylamino)pyrrolidin-1-carboxylate (50 mg, 0.11 mmol) was dissolved in dichloromethane (3 ml), a solution of 4 N hydrochloric acid (0.40 ml, 1.0 mmol) in 1,4-dioxane was added at 0°C, and then stirred at room temperature for two hours. Upon completion of the reaction, the resulting mixture was concentrated and extracted using an aqueous sodium hydrogen carbonate solution and ethyl acetate. The combined organic extracts were dried over sodium sulfate and then concentrated to afford 3-((R)-1-(6-((R)-pyrrolidin-3-ylamino)cinnolin-4-ylamino)ethyl)benzonitrile (13 mg, 33%) as a yellow solid.
1H NMR (400 MHz, DMSO-d6) δ 8.08 (s, 1H), 7.95 (s, 1H), 7.81-7.78 (m, 2H), 7.72 (d, *J= 8.0* Hz, 1H), 7.56 (t, *J= 8.0* Hz, 1H), 7.19-7.16 (m, 1H), 7.04 (d, *J* = 7.2 Hz, 1H), 6.98 (s, 1H), 6.64 (d, *J* = 7.2 Hz, 1H), 5.02-4.98 (m, 1H), 4.32-4.11(m, 2H), 3.24-3.18(m, 1H), 2.98-2.81 (m, 2H), 2.71-2.65 (m, 2H), 2.19-2.12 (m, 1H), 1.63 (d, *J* = 6.8 Hz, 3H)
MS (ESI+) m/z 359 (M+H)⁺

### Example 55

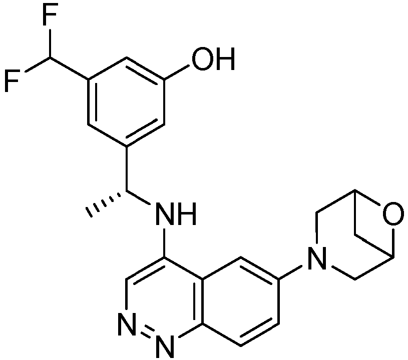

### 3-((1R)-1-((6-(6-oxa-3-azabicyclo[3.1.1]heptan-3-yl)cinnolin-4-yl)amino)ethyl)-5-(difluoromethyl)phenol

### Step 1

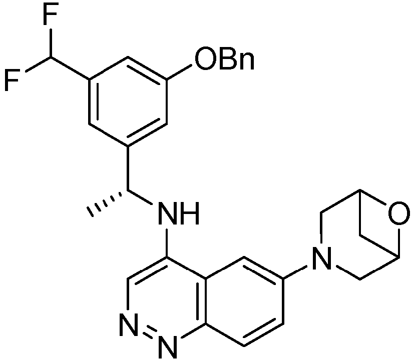

### N-((R)-1-(3-(benzyloxy)-5-(difluoromethyl)phenyl)ethyl)-6-(6-oxa-3-azabicyclo[3.1.1]heptan-3-yl)cinnolin-4-amine

Intermediate A-1 and intermediate I-A were used as described in Example 1 to synthesize compound A-2f, and then N-((R)-1-(3-(benzyloxy)-5-(difluoromethyl)phenyl)ethyl)-6-(6-oxa-3-azabicyclo[3.1.1]heptan-3-yl)cinnolin-4-amine (40 mg, 32%) was obtained in substantially the same manner as described in the synthesis method of Example 5, except for using 6-oxa-3-azabicyclo[3.1.1]heptane hydrochloride.

MS (ESI+) m/z 503 (M+H)⁺

### Step 2

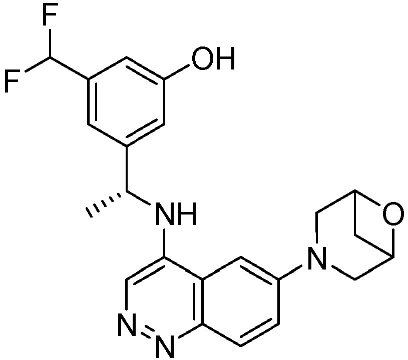

### 3-((1R)-1-((6-(6-oxa-3-azabicyclo[3.1.1]heptan-3-yl)cinnolin-4-yl)amino)ethyl)-5-(difluoromethyl)phenol

To a solution in which N-((R)-1-(3-(benzyloxy)-5-(difluoromethyl)phenyl)ethyl)-6-(6-oxa-3-azabicyclo[3.1.1]heptan-3-yl)cinnolin-4-amine (20 mg, 0.04 mmol) was dissolved in methanol (1 ml), 10% Pd/C (4 mg, 20% wt) was added, and then the reaction vessel was charged with hydrogen gas and stirred at 1 atmosphere. The reaction mixture was filtered through a celite pad, and then concentrated to afford 3-((1R)-1-((6-(6-oxa-3-azabicyclo[3.1.1]heptan-3-yl)cinnolin-4-yl)amino)ethyl)-5-(difluoromethyl)phenol (8 mg, 51%) as a yellow solid.
¹H NMR (400 MHz, DMSO-d6) δ 9.83 (s, 1H), 8.12 (s, 1H), 8.01 (d, *J =* 9.6 Hz, 1H), 7.49 (dd, *J =* 9.6, 2.4 Hz, 1H), 7.26-7.23 (m, 2H), 7.10 (s, 1H), 7.06-6.78 (m, 3H), 4.92 (q, *J* = 7.2 Hz, 1H), 4.82 (d, *J* = 6.4 Hz, 2H), 3.83-3.76 (m, 2H), 3.67-3.60 (m, 2H), 3.22-3.18 (m, 1H), 1.98 (d, *J* = 8.8 Hz, 1H), 1.69 (d, *J* = 6.8 Hz, 3H)
MS (ESI+) m/z 413 (M+H)⁺

### Example 56

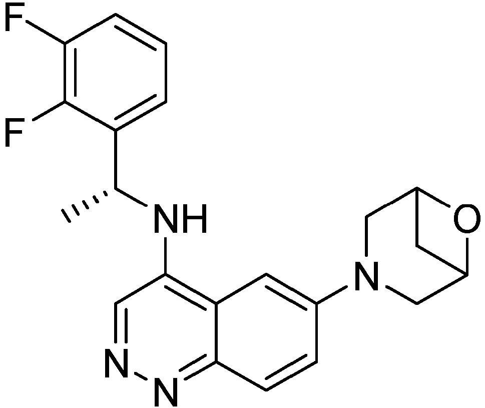

### 6-(6-oxa-3-azabicyclo[3.1.1]heptan-3-yl)-N-((R)-1-(2,3-difluorophenyl)ethyl)cinnolin-4-amine

Intermediate A-1 and (aR)-2,3-difluoro-a-methylbenzenemethanamine were used as described in Example 1 to synthesize compound A-2g, and then the title compound (15 mg, 20%) was obtained in substantially the same manner as described in the synthesis method of Example 5, except for using 6-oxa-3-azabicyclo[3.1.1]heptane hydrochloride.
¹H NMR (400 MHz, DMSO-d6) δ 8.08 (s, 1H), 8.02 (d, *J* = 9.2 Hz, 1H), 7.51-7.49 (m, 1H), 7.36-7.12 (m, 5H), 5.21 (q, *J* = 7.2 Hz, 1H), 4.82 (d, *J* = 6.4 Hz, 2H), 3.82-3.76 (m, 2H), 3.67-3.60 (m, 2H), 3.22-3.17 (m, 1H), 1.97 (d, *J* = 8.8 Hz, 1H), 1.69 (d, *J* = 6.8 Hz, 3H)
MS (ESI+) m/z 383 (M+H)⁺

### Example 57

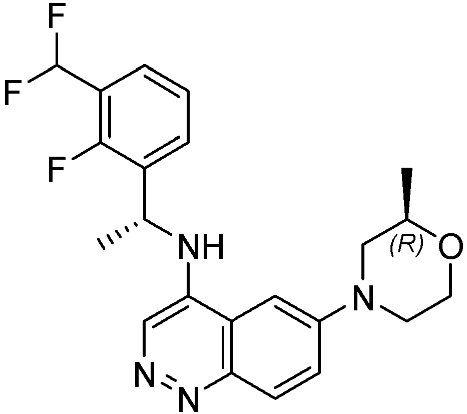

### N-((R)-1-(3-(difluoromethyl)-2-fluorophenyl)ethyl)-6-((R)-2-methylmorpholino)cinnolin-4-amine

Intermediate A-1 and (R)-1-(3-(difluoromethyl)-2-fluorophenyl)ethanamine were used as described in Example 1 to synthesize compound A-2, and then the title compound (20 mg, 24%) was obtained in substantially the same manner as described in the synthesis method of Example 5, except for using (R)-2-methylmorpholine.
¹H NMR (400 MHz, DMSO-d6) δ 8.01 (s, 1H), 7.98 (d, *J=* 9.2 Hz, 1H), 7.67 (dd, *J =* 2.4 Hz, 9.6 Hz, 1H), 7.62-7.7.53 (m, 2H), 7.48 (s, 1H), 7.42-7.15 (m, 3H), 5.22 (t, *J* = 6.6 Hz, 1H), 4.03 (d, *J* = 11.6 Hz, 1H), 3.90 (q, *J* = 11.7 Hz, 2H), 3.74-3.69 (m, 2H), 2.87 (t, *J* = 12.0 Hz, 1H), 2.59-2.56 (m, 1H), 1.70 (d, *J* = 6.8 Hz, 3H), 1.24 (d, *J* = 6.4 Hz, 3H)
MS (ESI+) m/z 417 (M+H)⁺

### Example 58

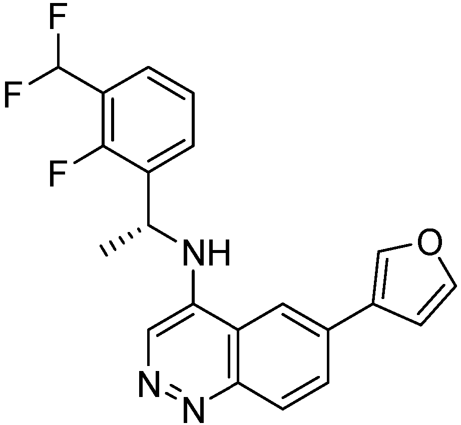

### (R)-N-(1-(3-(difluoromethyl)-2-fluorophenyl)ethyl)-6-(furan-3-yl)cinnolin-4-amine

Intermediate A-1 and (R)-1-(3-(difluoromethyl)-2-fluorophenyl)ethanamine were used as described in Example 1 to synthesize compound A-2, and then 3-furanboronic acid pinacol ester was used to afford the title compound (40 mg, 42%).
¹H NMR (400 MHz, DMSO-d6) δ 8.67 (s, 1H), 8.44 (s, 1H), 8.34 (s, 1H), 8.11-8.10 (m, 2H), 7.89 (s, 1H), 7.72 (d, *J =* 7.2 Hz, 1H), 7.66 (t, *J =* 7.8 Hz, 1H), 7.56 (t, *J =* 7.8 Hz, 1H), 7.42-7.15 (m, 3H), 5.30 (t, *J* = 6.8 Hz, 1H), 1.73 (d, *J* = 6.8 Hz, 3H)
MS (ESI+) m/z 484 (M+H)⁺

### Example 59

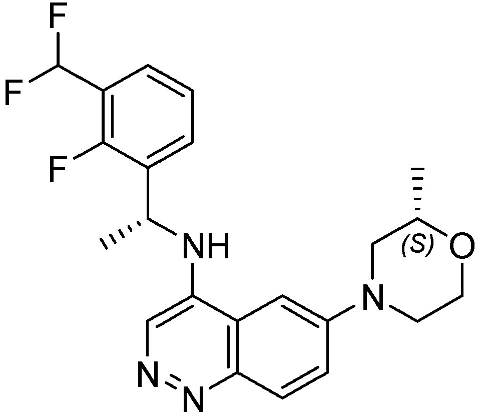

### N-((R)-1-(3-(difluoromethyl)-2-fluorophenyl)ethyl)-6-((S)-2-methylmorpholino)cinnolin-4-amine

Intermediate A-1 and (R)-1-(3-(difluoromethyl)-2-fluorophenyl)ethanamine were used as described in Example 1 to synthesize compound A-2, and then the title compound (41 mg, 49%) was obtained in substantially the same manner as described in the synthesis method of Example 5, except for using (S)-2-methylmorpholine.
¹H NMR (400 MHz, DMSO-d6) δ 8.11 (s, 1H), 7.98 (d, *J =* 9.2 Hz, 1H), 7.67 (dd, *J =* 2.4 Hz, 9.2 Hz, 1H), 7.57 (dd, *J* = 9.4 Hz, 15.8 Hz, 2H), 7.48 (s, 1H), 7.42-7.14 (m, 3H), 5.22 (t, *J* = 6.6 Hz, 1H), 4.02 (dd, *J* = 2.2 Hz, 11.8 Hz, 1H), 3.94 (d, *J* = 11.6 Hz, 1H), 3.84 (d, *J* = 12.0 Hz, 1H), 3.74-3.67 (m, 2H), 2.91-2.85 (m, 1H), 2.58-2.56 (m, 1H), 1.69 (d, *J =* 6.8 Hz, 3H), 1.23 (d, *J* = 6.4 Hz, 3H)
MS (ESI+) m/z 417 (M+H)⁺

### Example 60

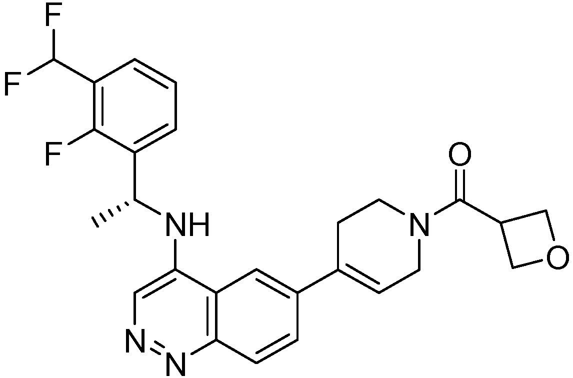

### (R)-(4-(4-(1-(3-(difluoromethyl)-2-fluorophenyl)ethylamino)cinnolin-6-yl)-5,6-dihydropyridin-1(2H)-yl)(oxetan-3-yl)methanone

### Step 1

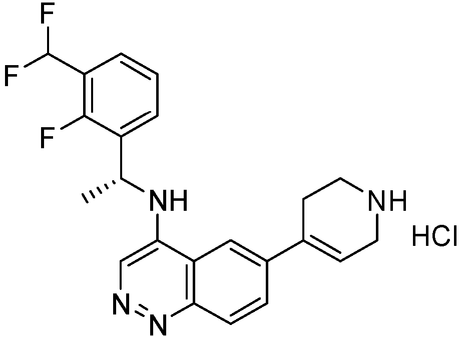

### (R)-N-(1-(3-(difluoromethyl)-2-fluorophenyl)ethyl)-6-(1,2,3,6-tetrahydropyridin-4-yl)cinnolin-4-amine hydrochloride

Intermediate A-1 and (R)-1-(3-(difluoromethyl)-2-fluorophenyl)ethanamine were used as described in Example 1 to synthesize compound A-2, and then 3,6-dihydro-4-(4,4,5,5-tetramethyl-1,3,2-dioxaborolan-2-yl)-1-1-dimethylethyl ester-1-(2H)-pyridine carboxylic acid was used to afford (R)-tert-butyl-4-(4-(1-(3-(difluoromethyl)-2-fluorophenyl)ethylamino)cinnolin-6-yl)-5,6-dihydropyridin-1(2H)-carboxylate (1.33 g, 67%).

To a solution in which (R)-tert-butyl-4-(4-(1-(3-(difluoromethyl)-2-fluorophenyl)ethylamino)cinnolin-6-yl)-5,6-dihydropyridin-1(2H)-carboxylate (1.33 g, 2.67 mmol) was dissolved in dichloromethane (10 ml), a solution of 4 N hydrochloric acid (10 ml, 40 mmol) in 1,4-dioxane was added at 0°C, and then stirred at room temperature for two hours. After completion of the reaction, the resulting mixture was concentrated to afford (R)-N-(1-(3-(difluoromethyl)-2-fluorophenyl)ethyl)-6-(1,2,3,6-tetrahydropyridin-4-yl)cinnolin-4-amine hydrochloride (1.15 g, 99%) as a brown solid.

MS (ESI+) m/z 399 (M+H)⁺

### Step 2

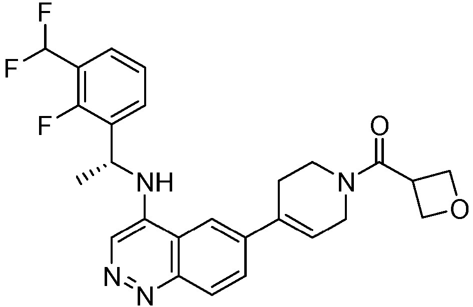

### (R)-(4-(4-(1-(3-(difluoromethyl)-2-fluorophenyl)ethylamino)cinnolin-6-yl)-5,6-dihydropyridin-1(2H)-yl)(oxetan-3-yl)methanone

After adding (R)-N-(1-(3-(difluoromethyl)-2-fluorophenyl)ethyl)-6-(1,2,3,6-tetrahydropyridin-4-yl)cinnolin-4-amine hydrochloride (50 mg, 0.12 mmol), oxetan-3-carboxylic acid (23 mg, 0.23 mmol), HATU (153 mg, 0.40 mmol), and N,N-diisopropylethylamine (0.1 ml, 0.58 mmol) in dimethyl sulfoxide (1 ml), the resulting mixture was stirred at room temperature for one hour. After confirming the completion of the reaction, water was added and extracted with ethyl acetate. The combined organic extracts were dried over sodium sulfate and concentrated, and then the residue was purified by column chromatography to afford (R)-(4-(4-(1-(3-(difluoromethyl)-2-fluorophenyl)ethylamino)cinnolin-6-yl)-5,6-dihydropyridin-1(2H)-yl)(oxetan-3-yl)methanone (16 mg, 29%) as a yellow solid.
¹H NMR (400 MHz, DMSO-d6) δ 8.43 (s, 1H), 8.33 (s, 1H), 8.06 (d, *J* = 8.8 Hz, 1H), 7.99-7.92 (m, 1H), 7.88-7.81 (m, 1H), 7.66-7.59 (m, 1H), 7.58-7.53 (m, 1H), 7.43-7.12 (m, 2H), 6.54-6.42 (m, 1H), 5.31 (q, *J* = 6.8 Hz, 1H), 4.82-4.61 (m, 4H), 4.33-4.21 (m, 2H), 4.03-3.99 (m, 1H), 3.83-3.79 (m, 1H), 1.70 (d, *J* = 6.8 Hz, 3H)
MS (ESI+) m/z 483 (M+H)⁺

### Example 61

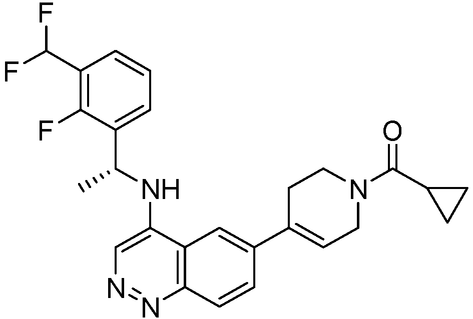

### (R)-cyclopropyl(4-(4-(1-(3-(difluoromethyl)-2-fluorophenyl)ethylamino)cinnolin-6-yl)-5,6-dihydropyridin-1(2H)-yl)methanone

Intermediate A-1 and (R)-1-(3-(difluoromethyl)-2-fluorophenyl)ethanamine were used as described in Example 60 to synthesize (R)-N-(1-(3-(difluoromethyl)-2-fluorophenyl)ethyl)-6-(1,2,3,6-tetrahydropyridin-4-yl)cinnolin-4-amine hydrochloride, and then cyclopropane carboxylic acid was used in substantially the same manner as in the synthesis method of Example 60 to afford the title compound (26 mg, 48%).
¹H NMR (400 MHz, DMSO-d6) δ 8.44 (s, 1H), 8.33 (s, 1H), 8.08 (d, *J* = 9.2 Hz, 1H), 8.04-7.94 (m, 1H), 7.89-7.81 (m, 1H), 7.63 (t, *J =* 7.2 Hz, 1H), 7.55 (t, *J =* 6.8 Hz, 1H), 7.43-7.13 (m, 2H), 6.54-6.48 (m, 1H), 5.29 (q, *J =* 6.8 Hz, 1H), 4.50 (s, 1H), 4.23 (s, 1H), 4.04-3.97 (m, 1H), 3.84-3.68 (m, 1H), 2.24-1.98 (m, 1H), 1.70 (d, *J* = 6.8 Hz, 3H), 0.83-0.71 (m, 4H)
MS (ESI+) m/z 467 (M+H)⁺

### Example 62

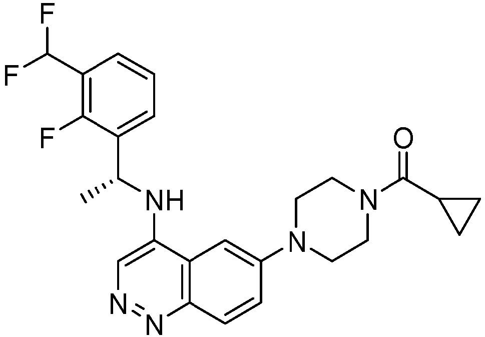

### (R)-cyclopropyl(4-(4-(1-(3-(difluoromethyl)-2-fluorophenyl)ethylamino)cinnolin-6-yl)piperazin-1-yl)methanone

Intermediate A-1 and (R)-1-(3-(difluoromethyl)-2-fluorophenyl)ethanamine were used as described in Example 41 to synthesize (R)-N-(1-(3-(difluoromethyl)-2-fluorophenyl)ethyl)-6-(piperazin-1-yl)cinnolin-4-amine hydrochloride, and then cyclopropane carboxylic acid was used in substantially the same manner as in the synthesis method of Example 41 to afford the title compound (26 mg, 23%).
¹H NMR (400 MHz, MeOD) δ 8.11 (s, 1H), 8.03 (d, *J=* 9.6 Hz, 1H), 7.74 (dd, *J =* 2.6 Hz, 9.4 Hz, 1H), 7.60-7.54 (m, 3H), 7.27 (t, *J* = 7.6 Hz, 1H), 7.08 (t, *J* = 54.8 Hz, 1H), 5.30 (q, *J* = 7.6 Hz, 1H), 4.04 (m, 2H), 3.87 (m, 2H), 3.63 (m, 2H), 3.54 (m, 2H), 2.10-2.06 (m, 1H), 1.80 (d, *J* = 6.8 Hz, 3H), 0.98-0.88 (m, 4H)
MS (ESI+) m/z 470 (M+H)⁺

### Example 63

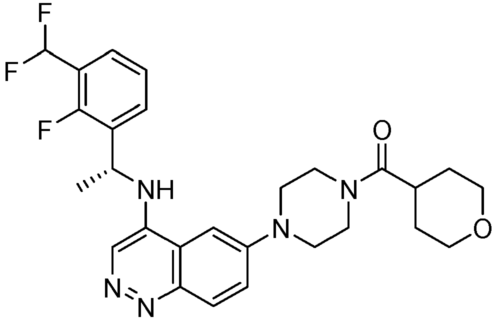

### (R)-(4-(4-(1-(3-(difluoromethyl)-2-fluorophenyl)ethylamino)cinnolin-6-yl)piperazin-1-yl)(tetrahydro-2H-pyran-4-yl)methanone

Intermediate A-1 and (R)-1-(3-(difluoromethyl)-2-fluorophenyl)ethanamine were used as described in Example 41 to synthesize (R)-N-(1-(3-(difluoromethyl)-2-fluorophenyl)ethyl)-6-(piperazin-1-yl)cinnolin-4-amine hydrochloride, and then tetrahydro-2H-pyran-4-carboxylic acid was used in substantially the same manner as in the synthesis method of Example 41 to afford the title compound (60 mg, 56%).
¹H NMR (400 MHz, MeOD) δ 8.11 (s, 1H), 8.03 (d, *J=* 9.2 Hz, 1H), 7.74 (dd, *J =* 2.6 Hz, 9.4 Hz, 1H), 7.60-7.54 (m, 3H), 7.27 (t, *J* = 7.8 Hz, 1H), 7.08 (t, *J* = 54.8 Hz, 1H), 5.30 (q, *J* = 7.6 Hz, 1H), 4.03-4.00 (m, 2H), 3.89-3.84 (m, 4H), 3.60-3.50 (m, 6H), 3.12-3.04 (m, 4H), 1.92-1.69 (m, 4H), 1.80 (d, *J* = 6.8 Hz, 3H)
MS (ESI+) m/z 514 (M+H)⁺

### Example 64

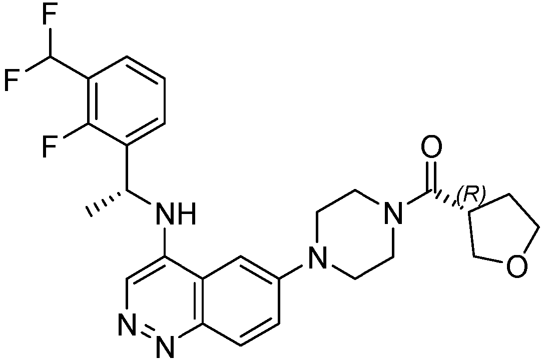

### (4-(4-((R)-1-(3-(difluoromethyl)-2-fluorophenyl)ethylamino)cinnolin-6-yl)piperazin-1-yl)((R)-tetrahydrofuran-3-yl)methanone

Intermediate A-1 and (R)-1-(3-(difluoromethyl)-2-fluorophenyl)ethanamine were used as described in Example 41 to synthesize (R)-N-(1-(3-(difluoromethyl)-2-fluorophenyl)ethyl)-6-(piperazin-1-yl)cinnolin-4-amine hydrochloride, and then (3R)-tetrahydro-3-furancarboxylic acid was used in substantially the same manner as in the synthesis method of Example 41 to afford the title compound (55 mg, 52%).
¹H NMR (400 MHz, MeOD) δ 8.18 (s, 1H), 7.99 (d, *J* = 9.6 Hz, 1H), 7.81 (dd, *J* = 2.6 Hz, 9.4 Hz, 1H), 7.63-7.56 (m, 3H), 7.30 (t, *J =* 7.6 Hz, 1H), 7.07 (t, *J =* 54.6 Hz, 1H), 5.40 (q, *J =* 6.7 Hz, 1H), 4.04 (t, *J* = 8.2 Hz, 1H), 3.96-3.93 (m, 1H), 3.91-3.83 (m, 6H), 3.62-3.55 (m, 5H), 2.24-2.15 (m, 2H), 1.82 (d, *J* = 6.8 Hz, 3H)
MS (ESI+) m/z 500 (M+H)⁺

### Example 65

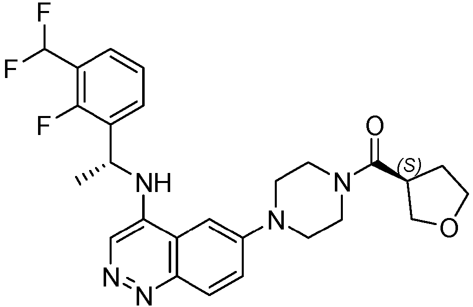

### (4-(4-((R)-1-(3-(difluoromethyl)-2-fluorophenyl)ethylamino)cinnolin-6-yl)piperazin-1-yl)((S)-tetrahydrofuran-3 -yl)methanone

Intermediate A-1 and (R)-1-(3-(difluoromethyl)-2-fluorophenyl)ethanamine were used as described in Example 41 to synthesize (R)-N-(1-(3-(difluoromethyl)-2-fluorophenyl)ethyl)-6-(piperazin-1-yl)cinnolin-4-amine hydrochloride, and then (3S)-tetrahydro-3-furancarboxylic acid was used in substantially the same manner as in the synthesis method of Example 41 to afford the title compound (15 mg, 14%).
¹H NMR (400 MHz, MeOD) δ 8.11 (s, 1H), 8.03 (d, *J=* 9.6 Hz, 1H), 7.74 (dd, *J =* 2.8 Hz, 9.6 Hz, 1H), 7.60-7.54 (m, 3H), 7.27 (t, *J* = 7.6 Hz, 1H), 7.08 (t, *J* = 54.8 Hz, 1H), 5.30 (q, *J* = 6.7 Hz, 1H), 4.04 (t, *J* = 8.0 Hz, 1H), 3.97-3.93 (m, 2H), 3.91-3.83 (m, 5H), 3.59-3.53 (m, 5H), 2.25-2.15 (m, 2H), 1.80 (d, *J* = 6.8 Hz, 3H)
MS (ESI+) m/z 500 (M+H)⁺

### Example 66

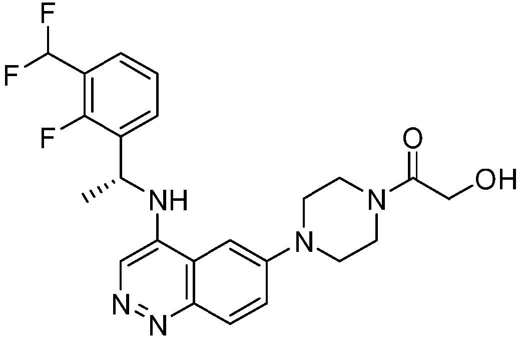

### (R)-1-(4-(4-(1-(3-(difluoromethyl)-2-fluorophenyl)ethylamino)cinnolin-6-yl)piperazin-1-yl)-2-hydroxyethanone

Intermediate A-1 and (R)-1-(3-(difluoromethyl)-2-fluorophenyl)ethanamine were used as described in Example 41 to synthesize (R)-N-(1-(3-(difluoromethyl)-2-fluorophenyl)ethyl)-6-(piperazin-1-yl)cinnolin-4-amine hydrochloride, and then glycolic acid was used in substantially the same manner as in the synthesis method of Example 41 to afford the title compound (20 mg, 21%).
¹H NMR (400 MHz, MeOD) δ 8.11 (s, 1H), 8.03 (d, *J=* 9.2 Hz, 1H), 7.73 (dd, *J =* 2.6 Hz, 9.4 Hz, 1H), 7.60-7.54 (m, 3H), 7.27 (t, *J* = 7.8 Hz, 1H), 7.08 (t, *J* = 54.8 Hz, 1H), 5.29 (q, *J* = 6.8 Hz, 1H), 4.35 (s, 2H), 3.86 (t, *J* = 5.2 Hz, 2H), 3.69 (t, *J* = 4.8 Hz, 2H), 3.57 (q, *J* = 5.6 Hz, 4H), 1.80 (d, *J* = 6.8 Hz, 3H)
MS (ESI+) m/z 460 (M+H)⁺

### Example 67

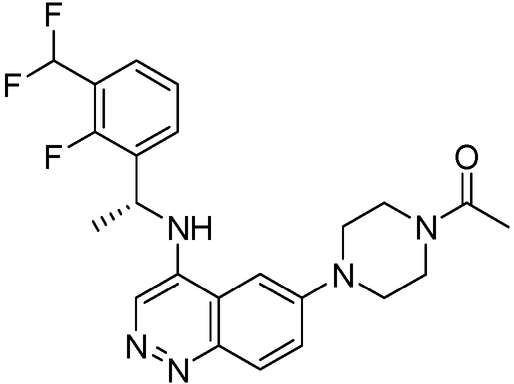

### (R)-1-(4-(4-(1-(3-(difluoromethyl)-2-fluorophenyl)ethylamino)cinnolin-6-yl)piperazin-1-yl)ethanone

Intermediate A-1 and (R)-1-(3-(difluoromethyl)-2-fluorophenyl)ethanamine were used as described in Example 1 to synthesize compound A-2, and then the title compound (24 mg, 22%) was obtained in substantially the same manner as in the synthesis method of Example 5, except for using 1-acetylpiperazine.
¹H NMR (400 MHz, MeOD) δ 8.11 (s, 1H), 8.03 (d, *J* = 9.6 Hz, 1H), 7.73 (dd, *J* = 2.8 Hz, 9.6 Hz, 1H), 7.60-7.54 (m, 3H), 7.27 (t, *J* = 7.6 Hz, 1H), 7.08 (t, *J* = 54.8 Hz, 1H), 5.29 (q, *J* = 6.8 Hz, 1H), 3.85 (t, *J* = 5.4 Hz, 2H), 3.80 (t, *J* = 5.2 Hz, 2H), 3.60 (t, *J =* 5.2 Hz, 2H), 3.54 (t, *J* = 5.4 Hz, 2H), 2.21 (s, 3H), 1.80 (d, *J* = 6.8 Hz, 3H)
MS (ESI+) m/z 444 (M+H)⁺

### Example 68

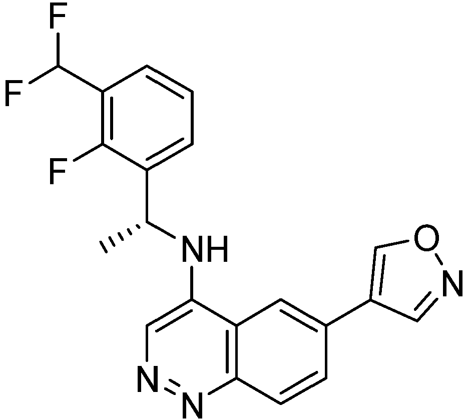

### (R)-N-(1-(3-(difluoromethyl)-2-fluorophenyl)ethyl)-6-(isoxazol-4-yl)cinnolin-4-amine

Intermediate A-1 and (R)-1-(3-(difluoromethyl)-2-fluorophenyl)ethanamine were used as described in Example 1 to synthesize compound A-2, and then 4-isoxazolboronic acid pinacol ester was used in substantially the same manner as in the synthesis method of Example 1 to afford the title compound (29 mg, 29%).
¹H NMR (400 MHz, DMSO-d6) δ 9.64 (s, 1H), 9.30 (s, 1H), 8.79 (s, 1H), 8.39 (s, 1H), 8.16 (q, *J* = 10.5 Hz, 2H), 7.68 (t, *J =* 8.0 Hz, 2H), 7.57 (t, *J* = 7.2 Hz, 1H), 7.31 (t, *J =* 7.8 Hz, 1H), 7.28 (t, *J* = 54.2 Hz, 1H), 5.32 (t, *J =* 6.8 Hz, 1H), 1.73 (d, *J* = 6.8 Hz, 3H)
MS (ESI+) m/z 385 (M+H)⁺

### Example 69

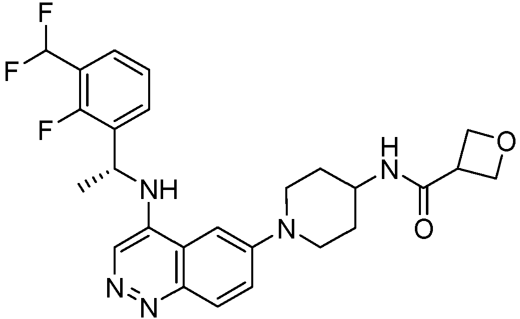

### (R)-N-(1-(4-(1-(3-(difluoromethyl)-2-fluorophenyl)ethylamino)cinnolin-6-yl)piperidin-4-yl)oxetan-3 -carboxamide

### Step 1

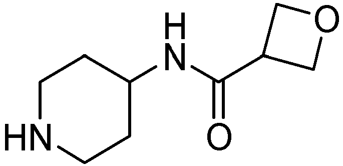

### N-(piperidin-4-yl)oxetan-3-carboxamide

After adding 1-benzyloxycarbonyl-4-aminopiperidine (1.9 g, 8.5 mmol), oxetan-3-carboxylic acid (790 mg, 7.7 mmol), HATU (3.8 g, 10 mmol), and N,N-diisopropylethylamine (2.7 ml, 15.4 mmol) in dimethyl sulfoxide (20 ml), the resulting mixture was stirred at room temperature for 16 hours. After confirming the completion of the reaction, water was added and extracted with ethyl acetate. The combined organic extracts were dried over sodium sulfate and concentrated, and then the residue was purified by column chromatography to afford benzyl 4-(oxetan-3-carboxamido)piperidin-1-carboxylate (120 mg, 6%) as a transparent liquid.

To a solution in which benzyl 4-(oxetan-3-carboxamido)piperidin-1-carboxylate (140 mg, 0.44 mmol) was dissolved in ethyl acetate/methanol (3:1, 4 ml), 10% Pd/C (30 mg, 20% wt) was added, and then the reaction vessel was charged with hydrogen gas and stirred at 1 atmosphere. The reaction mixture was filtered through a celite pad, and then concentrated to afford N-(piperidin-4-yl)oxetan-3-carboxamide (38 mg, 21%) as a yellow liquid.

### Step 2

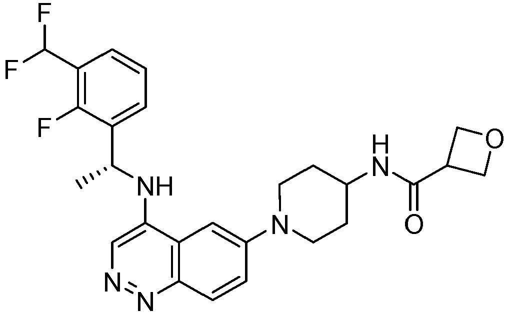

### (R)-N-(1-(4-(1-(3-(difluoromethyl)-2-fluorophenyl)ethylamino)cinnolin-6-yl)piperidin-4-yl)oxetan-3 -carboxamide

Intermediate A-1 and (R)-1-(3-(difluoromethyl)-2-fluorophenyl)ethanamine were used as described in Example 1 to synthesize compound A-2, and then the title compound (15 mg, 18%) was obtained in substantially the same manner as in the synthesis method of Example 5, except for using N-(piperidin-4-yl)oxetan-3-carboxamide.
¹H NMR (400 MHz, MeOD-d4) δ 7.95 (s, 1H), 7.85 (d, *J* = 9.6 Hz, 1H), 7.57 (dd, *J* = 9.6 Hz, 2.4 Hz, 1H), 7.47-7.40 (m, 3H), 7.14 (t, *J* = 8.0 Hz, 1H), 7.09 - 6.81 (t, *J* = 54.8 Hz, 1H), 5.15 (q, *J =* 6.8 Hz, 1H), 4.74-7.64 (m, 4H), 4.05-3.95 (m, 2H), 3.92-3.84 (m, 1H), 3.77-3.70 (m, 1H), 3.03-2.97 (m, 2H), 1.97-1.92 (m, 2H), 1.66 (d, *J* = 6.8 Hz, 3H), 1.60-1.49 (m, 2H)
MS (ESI+) m/z 500 (M+H)⁺

### Example 70

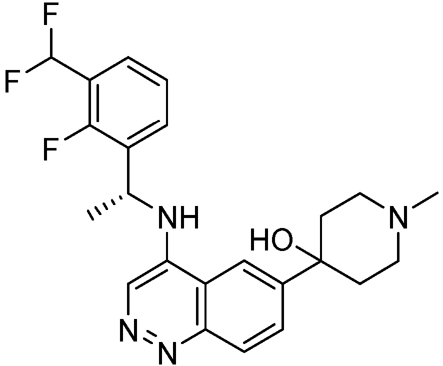

### (R)-4-(4-(1-(3-(difluoromethyl)-2-fluorophenyl)ethylamino)cinnolin-6-yl)-1-methylpiperidin-4-ol

A solution in which (R)-N-(1-(3-(difluoromethyl)-2-fluorophenyl)ethyl)-6-(1-methyl-1,2,3,6-tetrahydropyridin-4-yl)cinnolin-4-amine (100 mg, 0.24 mmol) and tris(2,2,6,6-tetramethyl-3,5-heptanedionato)manganese(III) (7 mg, 0.23 mmol) were dissolved in isopropyl alcohol and dichloromethane (4 ml/0.5 ml) was stirred under air bubbling for five minutes. Then, phenyl silane (50 mg, 0.47 mmol) was added and stirred at room temperature for two hours under air bubbling. After completion of the reaction, a saturated sodium thiosulfate solution was added and then stirred for one hour. 1 N hydrochloric acid was added to the reaction solution and then washed with dichloromethane. The obtained water layer was basified to pH 14 by adding an aqueous sodium hydrogen carbonate solution and extracted three times with dichloromethane. The combined organic layer was dried over sodium sulfate and concentrated, and then the residue was purified by column chromatography to afford (R)-4-(4-(1-(3-(difluoromethyl)-2-fluorophenyl)ethylamino)cinnolin-6-yl)-1-methylpiperidin-4-ol (11 mg, 11%) as a brown solid.
¹H NMR (400 MHz, DMSO-d6) δ 8.67 (s, 1H), 8.31 (s, 1H), 8.12-7.99 (m, 2H), 7.71 (s, 1H), 7.54-7.52 (m, 1H), 7.42-7.15 (m, 3H), 5.46 (s, 1H), 5.27 (s, 1H), 3.15-2.85 (m, 4H), 2.56-2.52 (m, 2H), 1.84-1.82 (m, 2H), 1.72 (s, 3H)
MS (ESI+) m/z 431 (M+H)⁺

### Example 71

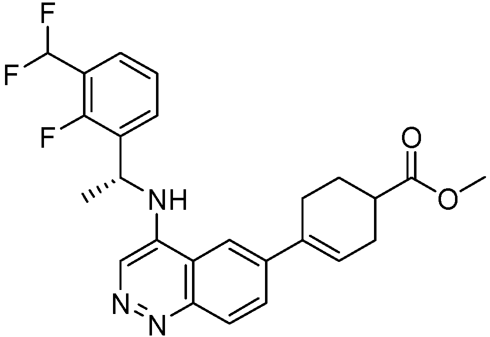

### Methyl 4-(4-((R)-1-(3-(difluoromethyl)-2-fluorophenyl)ethylamino)cinnolin-6-yl)cyclohex-3-enecarboxylate

Intermediate A-1 and (R)-1-(3-(difluoromethyl)-2-fluorophenyl)ethanamine were used as described in Example 1 to synthesize compound A-2, and then 1,3,2-dioxaborolane, 3-cyclohexen-1-carboxylic acid were used in substantially the same manner as in the synthesis method of Example 1 to afford the title compound (332 mg, 60%).
¹H NMR (400 MHz, DMSO-d6) δ 8.40 (s, 1H), 8.31 (d, *J* = 2.4 Hz, 1H), 8.05-8.03 (m, 1H), 7.96-7.92 (m, 1H), 7.85 (d, *J =* 6.0 Hz, 1H), 7.63 (t, *J =* 7.2 Hz, 1H), 7.55 (t, *J =* 6.8 Hz, 1H), 7.42 (s, 0.25H), 7.32-7.28 (m, 1.50H), 7.15 (s, 0.5H), 6.50-6.49 (m, 1H), 5.30-5.26 (m, 1H), 3.67 (s, 3H), 2.75-2.69 (m, 2H), 2.65-2.58 (m, 2H), 2.20-2.16 (m, 1H), 1.86-1.80 (m, 1H), 1.70 (d, *J* = 6.8 Hz, 3H)
MS (ESI+) m/z 456 (M+H)⁺

### Example 72

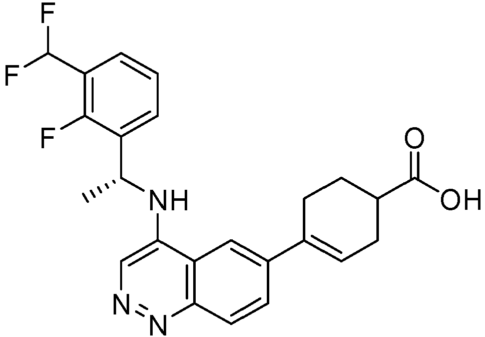

### 4-(4-((R)-1-(3-(difluoromethyl)-2-fluorophenyl)ethylamino)cinnolin-6-yl)cyclohex-3-enecarboxylic acid

To a solution in which methyl 4-(4-((R)-1-(3-(difluoromethyl)-2-fluorophenyl)ethylamino)cinnolin-6-yl)cyclohex-3-enecarboxylate (250 mg, 0.65 mmol) was dissolved in tetrahydrofuran and H₂O (3 ml/0.6 ml), LiOH (125 mg, 6.5 mmol) was added and stirred at room temperature for 16 hours. After confirming the completion of the reaction, 1 N HCl was added to reach pH 4 to 5, and then extracted with ethyl acetate. The combined organic extracts were dried over sodium sulfate and then concentrated to afford 4-(4-((R)-1-(3-(difluoromethyl)-2-fluorophenyl)ethylamino)cinnolin-6-yl)cyclohex-3-enecarboxylic acid (190 mg, 66%) as a yellow solid.
¹H NMR (400 MHz, DMSO-d6) δ 12.35 (s, 1H), 9.94 (s, 1H), 8.65 (d, *J* = 2.8 Hz, 2H), 8.27-8.24) (m, 1H), 7.96 (d, *J =* 9.2 Hz, 1H), 7.81 (t, *J* = 7.2 Hz, 1H), 7.64 (t, *J =* 6.8 Hz, 1H), 7.40 (t, *J=* 8.8 Hz, 1H), 7.26-7.12 (m, 1H), 6.57 (s, 1H), 5.80-5.77 (m, 1H), 2.67-2.60 (m, 4H), 2.16-2.13 (m, 1H), 1.80 (d, *J* = 6.8 Hz, 4H)
MS (ESI+) m/z 442 (M+H)⁺

### Example 73

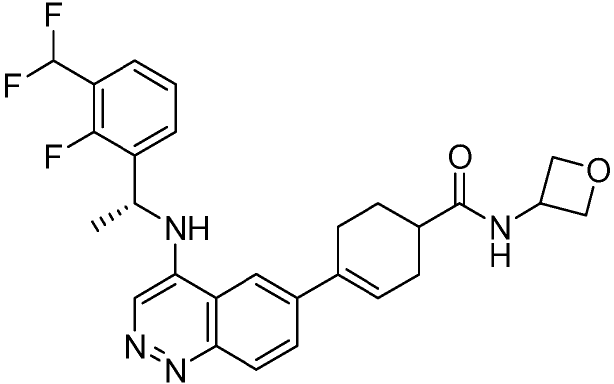

### 4-(4-((R)-1-(3-(difluoromethyl)-2-fluorophenyl)ethylamino)cinnolin-6-yl)-N-(oxetan-3-yl)cyclohex-3-enecarboxamide

After adding 4-(4-((R)-1-(3-(difluoromethyl)-2-fluorophenyl)ethylamino)cinnolin-6-yl)cyclohex-3-enecarboxylic acid (60 mg, 0.13 mmol), oxetan-3-carboxylic acid (21 mg, 0.15 mmol), HATU (100 mg, 0.40 mmol), and N,N-diisopropylethylamine (0.20 ml, 0.40 mmol) in dimethyl sulfoxide (1 ml), the resulting mixture was stirred at room temperature for one hour. After confirming the completion of the reaction, water was added and extracted with ethyl acetate. The combined organic extracts were dried over sodium sulfate and concentrated, and then the residue was purified by column chromatography to afford 4-(4-((R)-1-(3-(difluoromethyl)-2-fluorophenyl)ethylamino)cinnolin-6-yl)-N-(oxetan-3-yl)cyclohex-3-enecarboxamide (24 mg, 37%) as a yellow solid.
¹H NMR (400 MHz, DMSO-d6) δ 8.70 (d, *J* = 6.8 Hz, 1H), 8.40 (s, 1H), 8.31 (d, *J* = 2.0 Hz, 1H), 8.05-8.03 (m, 1H), 7.96-7.93 (m, 1H), 7.84 (t, *J* = 6.8 Hz, 1H), 7.63 (t, *J* = 7.2 Hz, 1H), 7.55 (t, *J =* 7.2 Hz, 1H), 7.42-7.15 (m, 2H), 6.51 (s, 1H), 5.30-5.26 (m, 1H), 4.85-4.81 (m, 1H), 4.74 (t, *J* = 6.8 Hz, 2H), 4.45 (t, *J =* 7.2 Hz, 2H), 2.80-2.67 (m, 1H), 2.49-2.42 (m, 3H), 2.08-2.04 (m, 1H),1.80-1.79 (m, 1H), 1.70 (d, *J* = 6.8 Hz, 3H)
MS (ESI+) m/z 497 (M+H)⁺

### Example 74

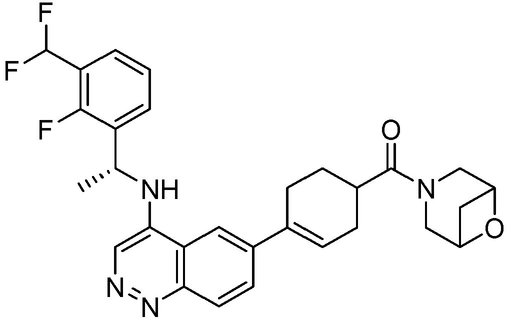

### 6-oxa-3-azabicyclo[3.1.1]heptan-3-yl(4-(4-((R)-1-(3-(difluoromethyl)-2-fluorophenyl)ethylamino)cinnolin-6-yl)cyclohex-3-enyl)methanone

6-oxa-3-azabicyclo[3.1.1]heptane hydrochloride was used in substantially the same manner as in the synthesis method of Example 73 to afford the title compound (45 mg, 66%).
¹H NMR (400 MHz, DMSO-d6) δ 8.43-8.42 (m, 1H), 8.33 (d, *J* = 4.8 Hz, 1H), 8.06-8.03 (m, 1H), 7.98-7.92 (m, 2H), 7.64 (d, *J =* 7.6 Hz, 1H), 7.56 (t, *J =* 6.8 Hz, 1H), 7.42-7.14 (m, 2H), 6.55 (s, 1H), 5.32-5.29 (m, 1H), 4.62 (d, *J* = 6.4 Hz, 2H), 3.88-3.85 (m, 2H), 3.71-3.67 (m, 1H), 3.47-3.43 (m, 2H), 3.12-3.06 (m, 1H), 2.91-2.67 (m, 3H), 2.10-2.07 (m, 1H), 1.82-1.74 (m, 2H), 1.70 (d, *J* = 6.8 Hz, 3H)
MS (ESI+) m/z 523 (M+H)⁺

### Example 75

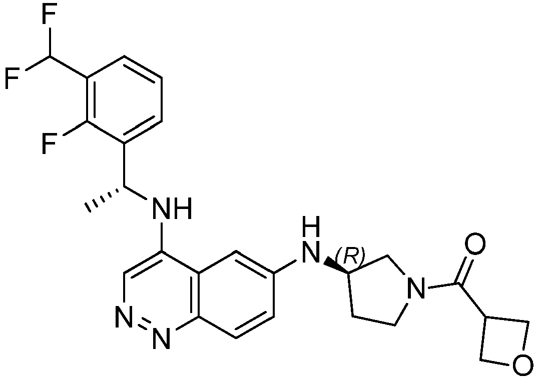

### ((R)-3-(4-((R)-1-(3-(difluoromethyl)-2-fluorophenyl)ethylamino)cinnolin-6-ylamino)pyrrolidin-1-yl)(oxetan-3-yl)methanone

After adding N4-((R)-1-(3-(difluoromethyl)-2-fluorophenyl)ethyl)-N6-((R)-pyrrolidin-3-yl)cinnolin-4,6-diamine (150 mg, 0.37 mmol), oxetan-3-carboxylic acid (50 mg, 0.44 mmol), HATU (200 mg, 0.44 mmol), and N,N-diisopropylethylamine (0.50 ml, 1.11 mmol) in dimethyl sulfoxide (1 ml), the resulting mixture was stirred at room temperature for one hour. After confirming the completion of the reaction, water was added and extracted with ethyl acetate. The combined organic extracts were dried over sodium sulfate and concentrated, and then the residue was purified by column chromatography to afford ((R)-3-(4-((R)-1-(3-(difluoromethyl)-2-fluorophenyl)ethylamino)cinnolin-6-ylamino)pyrrolidin-1-yl)(oxetan-3-yl)methanone (90 mg, 50%) as a yellow solid.
¹H NMR (400 MHz, DMSO-d6) δ 8.05 (d, *J =* 16.4 Hz, 1H), 7.83 (dd, *J =* 2.0 Hz, 7.2 Hz, 1H), 7.63-7.53 (m, 2H), 7.42-7.7.07 (m, 5H), 6.87 (d, *J =* 6.4 Hz, 1H), 5.25-5.20 (m, 1H), 4.75-4.63 (m, 4H), 4.34-4.26 (m, 1H), 4.11-4.04 (m, 1H), 3.81-3.73 (m, 1H), 3.53-3.36 (m, 4H), 2.33-2.23 (m, 1H), 1.70 (d, *J* = 6.8 Hz, 3H)
MS (ESI+) m/z 486 (M+H)⁺

### Example 76

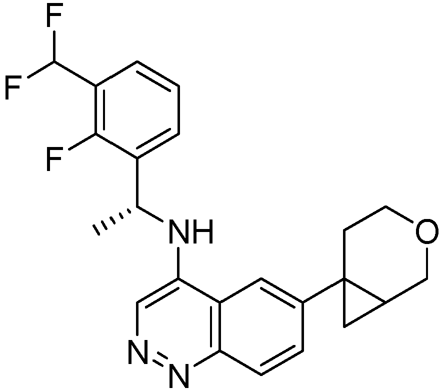

### 6-(3-oxabicyclo[4.1.0]heptan-6-yl)-N-((R)-1-(3-(difluoromethyl)-2-fluorophenyl)ethyl)cinnolin-4-amine

Intermediate A-1 and (R)-1-(3-(difluoromethyl)-2-fluorophenyl)ethanamine were used as described in Example 1 to synthesize compound A-2, and then 4,4,5,5-tetramethyl-2-[3-oxabicyclo[4.1.0]heptan-6-yl]1,3,2-dioxaborolane was used in substantially the same manner as in the synthesis method of Example 1 to afford the title compound (4 mg, 4%).
¹H NMR (400 MHz, MeOD) δ 8.24 (s, 1H), 8.15 (s, 1H), 7.97 (d, *J* = 8.8 Hz, 1H), 7.74 (dd, *J =* 2.0 Hz, 8.8 Hz, 1H), 7.57 (ddd, *J* = 7.4 Hz, 7.4 Hz, 15.2 Hz, 2H), 7.16 (t, *J* = 7.8 Hz, 1H), 6.96 (t, *J* = 54.8 Hz, 1H), 5.21 (q, *J* = 6.8 Hz, 1H), 4.06 (dd, *J* = 4.4 Hz, 11.2 Hz, 1H), 3.90 (d, *J* = 11.2 Hz, 1H), 3.59-3.54 (m, 2H), 3.49-3.42 (m, 1H), 2.22-2.14 (m, 1H), 2.10-2.04 (m, 1H), 1.69 (d, *J =* 6.8 Hz, 3H), 1.54-1.47 (m, 1H), 0.97 (t, *J* = 5.4 Hz, 1H)
MS (ESI+) m/z 414 (M+H)⁺

### <Experimental Example>

### Experimental Example 1. Biochemical test

### 1-1. KRAS::SOS1 HTRF binding analysis

An SOS1 inhibitory ability of the example compounds of the present invention was confirmed by identifying the effect of inhibiting a protein-protein interaction between SOS1 and KRAS G12C of the compounds according to examples of the present invention.

Specifically, a KRAS G12C/SOS1 binding kit (64KRASG12PEG, Cisbio, France) was purchased and used. 2 *µ*ℓ of Tag1-KRAS G12C and 2 *µ*ℓ of GTP were premixed and dispensed 4 µl each into a 384 well plate (6007290, PerkinElmer, USA). 2 *µ*ℓ of the compound and 4 *µ*ℓ of Tag2-SOS1 were placed and then 10 *µ*ℓ of premixed Anti-Tag1 XL665 antibody and Anti-Tag2 Tb cryptate antibody was dispensed to each well. The plates were sealed and allowed to react at room temperature for one hour and 40 minutes, and then 665 nm wavelength and 620 nm wavelength were measured using a HTRF^{®} compatible reader (Synergy H4, BioTeK, USA), and the ratio of receptor and donor emission signals for each well was calculated as 665 nm/620 nm×10⁴.

This method demonstrates a molecular mode of action of the compounds, and a high inhibition rate in the context of this assay indicates the high potency of the SOS1 inhibitor compound.

The calculation results are shown in Table 4 below.
(+++: more than 70 to 100% inhibition, ++: more than 40 to 70% inhibition, +: 0 to 40% inhibition)

**[Table 4]**

| **Compound** | **% inhibition at 0.1 uM** | **Compound** | **% inhibition at 0.1 uM** |
|---|---|---|---|
| Example 1 | + | Example 35 | + |
| Example 2 | ++ | Example 36 | + |
| Example 3 | +++ | Example 37 | + |
| Example 4 | + | Example 38 | ++ |
| Example 5 | ++ | Example 39 | +++ |
| Example 6 | ++ | Example 40 | ++ |
| Example 7 | +++ | Example 41 | +++ |
| Example 8 | + | Example 42 | + |
| Example 9 | ++ | Example 43 | + |
| Example 10 | + | Example 44 | + |
| Example 11 | + | Example 45 | + |
| Example 12 | ++ | Example 47 | + |
| Example 13 | ++ | Example 48 | + |
| Example 14 | + | Example 49 | + |
| Example 15 | + | Example 50 | + |
| Example 16 | + | Example 51 | + |
| Example 17 | + | Example 52 | + |
| Example 18 | +++ | Example 53 | + |
| Example 19 | ++ | Example 54 | + |
| Example 20 | + | Example 55 | + |
| Example 21 | ++ | Example 56 | + |
| Example 22 | + | Example 57 | + |
| Example 23 | + | Example 58 | + |
| Example 24 | + | Example 59 | + |
| Example 25 | + | Example 60 | +++ |
| Example 26 | + | Example 61 | + |
| Example 27 | ++ | Example 62 | ++ |
| Example 28 | + | Example 63 | ++ |
| Example 29 | ++ | Example 64 | +++ |
| Example 30 | + | Example 65 | ++ |
| Example 31 | + | Example 69 | ++ |
| Example 32 | + | | |
| Example 33 | + | | |
| Example 34 | + | | |

As shown in above Table 4, it can be confirmed that the compounds according to examples of the present invention may inhibit SOS1 at a concentration of 0.1 uM, and it can be confirmed that most of the compounds of the present invention exhibit a very high SOS1 inhibition rate.

In other words, it can be confirmed that the compounds according to the present invention may exhibit high SOS1 inhibitory efficacy.

### 1-2. EGFR kinase inhibitory analysis

An EGFR kinase inhibitory efficacy of the compounds according to examples of the present invention was confirmed.

Specifically, an ADP-Glo^{™} enzyme analysis system (V3831, Promega Corporation, USA) was purchased and used. 5 x kinase reaction buffer (phosphorylase reaction buffer) was diluted in 1.5 x and prepared, and then 6 ng of epidermal growth factor receptor (EGFR) and 25 M of ATP were prepared in EGFR kinase (phosphorylase) and ATP using 1.5 x kinase reaction buffer, and then 2 *µ*ℓ of EGFR kinase and 2 *µ*ℓ of substrate/2 *µ*ℓ of ATP mix were mixed with 1 *µ*ℓ of compound 1 in a 384 well plate (REF4513, Corning, USA) and reacted at room temperature for 60 minutes. The ADP-Glo^{™} reagent was added in an amount of 5 *µ*ℓ per well and allowed to react at room temperature for 40 minutes to stop the EGFR kinase reaction and to remove unused ATP. 10 *µ*ℓ of kinase detection reagent (phosphorylase detection reagent) was added and reacted at room temperature for 30 minutes to convert ADP to ATP and show ATP using luciferase and luciferin. Detection was performed using luminescence of an ELISA reader (Synergy H4, BioTek, USA).

This method demonstrates an EGFR kinase selective molecular mode of action of the compounds, with high IC₅₀ values in said analysis environment indicating no EGFR kinase inhibitory efficacy.

The analysis results are shown in Table 5 below.

**[Table 5]**

| **Compound** | **IC₅₀ (nM)** |
|---|---|
| Gefitinib | 13.90 |
| Example 2 | >10,000 |
| Example 5 | >10,000 |

As can be seen from above Table 5, the compounds of the present invention were found to have no EGFR kinase inhibitory effect compared to the control EGFR kinase inhibitor (Gefitinib), suggesting that the compounds of the present invention have high selectivity for SOS1.

In other words, the compounds of the present invention may exhibit high selectivity for SOS1 and high SOS1 inhibitory efficacy, and thus the compounds of the present invention may be advantageously used in the treatment of diseases related to SOS1 activity.

While the present invention have been described in detail above, it is apparent to those skilled in the art that such detailed descriptions are set forth to illustrate exemplary embodiments only, but are not construed to limit the scope of the present invention. Thus, it should be understood that the substantial scope of the present invention is defined by the accompanying claims and equivalents thereto.

## Claims

1. A compound represented by formula 1 below, optical isomer thereof, stereoisomer thereof, solvate thereof, isotopic variant thereof, tautomer thereof, or pharmaceutically acceptable salt thereof: 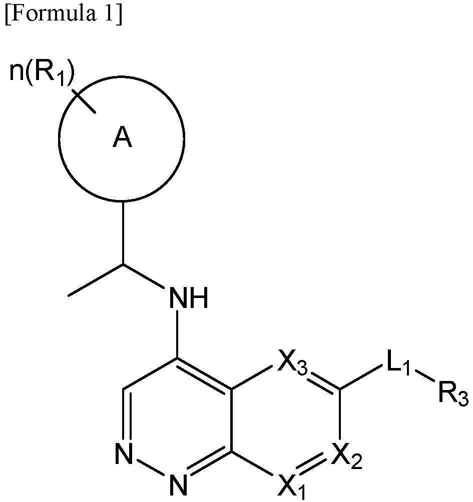
wherein in above formula 1,
A is C₃₋₈ cycloalkyl, C₃₋₈ cycloalkenyl, 3- to 8-membered heterocycloalkyl comprising 1 to 3 heteroatoms independently selected from the group consisting of N, O, and S in a ring, 3- to 8-membered heterocycloalkenyl comprising 1 to 3 heteroatoms independently selected from the group consisting of N, O, and S in a ring, C₆₋₁₂ aryl, 5- to 12-membered heteroaryl comprising 1 to 3 heteroatoms independently selected from the group consisting of N, O, and S in a ring, 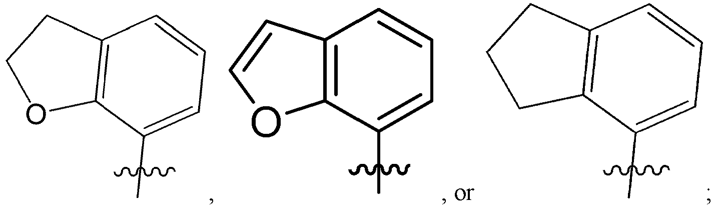
n is 0, 1, 2, 3, or 4;
R₁ is C₁₋₆ alkyl, C₆₋₁₂ aryl, -CF₂H, -CF₃, -CN, -OH, -NH₂, or halogen (in which when n is 2 or more, n R₁s are each independent of each other),
at least one H of C₁₋₆ alkyl, C₆₋₁₂ aryl, -CF₂H, and -OH of the R₁ is each independently substituted with C₁₋₆ alkyl (in which at least one H of C₁₋₆ alkyl is each independently substituted with -OH, -O-C₁₋₆ alkyl, or -NRₐR_{b}), or halogen;
X₁ is CH or N, X₂ is CR₂, and X₃ is CH;
R₂ is H, C₁₋₆ alkyl, -CF₃, -O-C₁₋₆ alkyl, C₃₋₆ cycloalkyl, -O-C₃₋₆ cycloalkyl, -OH, -OCF₃, - NR_{c}R_{d}, or halogen;
L₁ is a single bond, -(C=O)-, -(C=O)O-, -O-, -(C=O)NRₑ-, -NRₑ-, -NRₑ(C=O)-, -NRₑSO₂-, or -NRₑ(C=O)NR_{f}-;
R₃ is H, -OH, C₁₋₆ alkyl, C₃₋₈ cycloalkyl, C₆₋₁₂ aryl, 3- to 12-membered heterocycloalkyl comprising 1 to 3 heteroatoms independently selected from the group consisting of N, O, and S in a ring, C₃₋₈ cycloalkenyl, 3- to 8-membered heterocycloalkenyl comprising 1 to 3 heteroatoms independently selected from the group consisting of N, O, and S in a ring, 5- to 12-membered heteroaryl comprising 1 to 3 heteroatoms independently selected from the group consisting of N, O, and S in a ring, 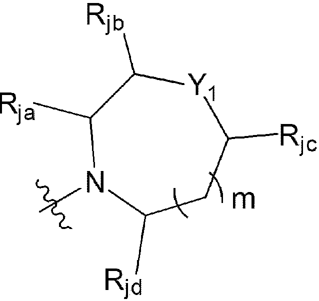 (in which m is 0 or 1, Y₁ is CH₂, NRⱼ, or O, and Rⱼₐ, R_{jb}, R_{jc}, and R_{jd} are each independently H or C₁₋₅ alkyl, provided that two selected from Rⱼₐ, R_{jb}, R_{jc}, and R_{jd} are linked to form CH₂ or CH₂-CH₂), or 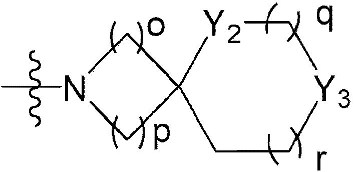 (in which o and p are each independently 1 or 2, q and r are each independently 0, 1, or 2, and Y₂ and Y₃ are each independently CH₂, NRₖ, or O),
in the R₃, at least one -CH₂- of 3- to 12-membered heterocycloalkyl comprising 1 to 3 heteroatoms independently selected from the group consisting of N, O, and S in a ring, or 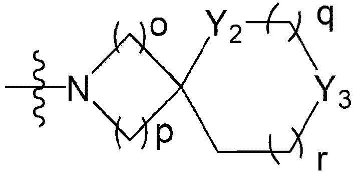 is substituted with -(C=O)-, -SO-, or -SO₂-,
at least one -CH₂- of C₃₋₈ cycloalkyl of the R₃ is substituted with -SO₂-,
at least one H of the R₃ is each independently substituted with C₁₋₆ alkyl, -OH, halogen, or -L₂-R₄;
L₂ is a single bond, C₁₋₆ alkylene, -O-, -(C=O)-, -SO₂-, -(C=O)NR_{g}-, or -NR_{g}(C=O)-;
R₄ is H, C₁₋₆ alkyl, -O-C₁₋₆ alkyl, C₃₋₈ cycloalkyl, 3- to 8-membered heterocycloalkyl comprising 1 to 3 heteroatoms independently selected from the group consisting of N, O, and S in a ring, C₆₋₁₂ aryl, 5- to 12-membered heteroaryl comprising 1 to 3 heteroatoms independently selected from the group consisting of N, O, and S in a ring, -NRₕRi, -CF₃, -CF₂H, -OH, or halogen,
at least one H of the R₄ is each independently substituted with C₁₋₆ alkyl, -OH, -O-C₁₋₆ alkyl, -NRₘRₙ, or halogen; and
Rₐ, R_{b}, R_{c}, R_{d}, Rₑ, R_{f}, R_{g}, Rₕ, Rᵢ, Rⱼ, Rₖ, Rₘ, and Rₙ are each independently H or C₁₋₆ alkyl.

2. The compound represented by formula 1, optical isomer thereof, stereoisomer thereof, solvate thereof, isotopic variant thereof, tautomer thereof, or pharmaceutically acceptable salt thereof of claim 1,
wherein in above formula 1,
Ais C₆₋₁₂ aryl, 5- to 12-membered heteroaryl comprising 1 to 3 heteroatoms independently selected from the group consisting of N, O, and S in a ring, 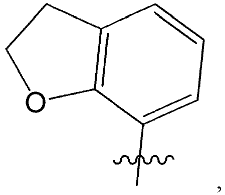 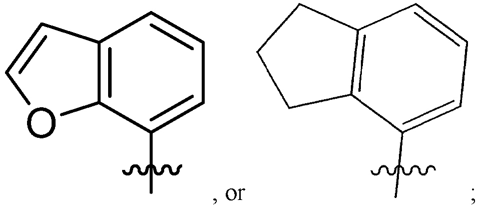
n is 1, 2, or 3;
R₁ is C₁₋₆ alkyl, C₆₋₁₂ aryl, -CF₂H, -CF₃, -CN, or halogen (in which when n is 2 or more, n R₁ s are each independent of each other),
at least one H of C₁₋₆ alkyl, C₆₋₁₂ aryl, and -CF₂H of the R₁ is each independently substituted with C₁₋₆ alkyl (in which at least one H of C₁₋₆ alkyl is each independently substituted with -OH, -O-C₁₋₆ alkyl, or -NRₐR_{b}), or halogen;
X₁ and X₃ are each CH;
X₂ is CR₂; and
R₂, R₃, R₄, L₁, L₂, Y₁, Y₂, Y₃, m, o, p, q, r, Rⱼₐ, R_{jb}, R_{jc}, R_{jd}, Rₐ, R_{b}, R_{c}, R_{d}, Rₑ, R_{f}, R_{g}, Rₕ, Rᵢ, Rⱼ, Rₑ, Rₘ, and Rₙ are each the same as defined in claim 1.

3. The compound represented by formula 1, optical isomer thereof, stereoisomer thereof, solvate thereof, isotopic variant thereof, tautomer thereof, or pharmaceutically acceptable salt thereof of claim 1,
wherein in above formula 1,
A is C₆₋₁₂ aryl;
n is 1 or 2;
R₁ is each independently C₁₋₆ alkyl, -CF₂H, -CF₃, -CN, or halogen;
X₁, X₂, and X₃ are each CH;
L₁ is a single bond or -NRₑ-;
R₃ is 3- to 12-membered heterocycloalkyl comprising 1 to 3 heteroatoms independently selected from the group consisting of N, O, and S in a ring, C₃₋₈ cycloalkenyl, 3- to 8-membered heterocycloalkenyl comprising 1 to 3 heteroatoms independently selected from the group consisting of N, O, and S in a ring, or 5- to 12-membered heteroaryl comprising 1 to 3 heteroatoms independently selected from the group consisting of N, O, and S in a ring, in which the heterocycloalkyl comprises 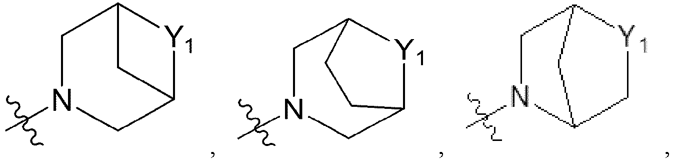 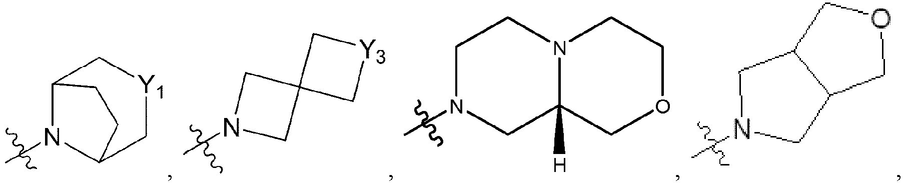 or 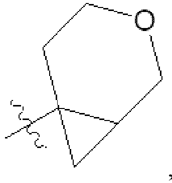 in which Y₁ is O, and Y₃ is each independently CH₂, NRₖ, or O,
at least one H of the R₃ is each independently substituted with C₁₋₆ alkyl, -OH, halogen, or -L₂-R₄;
L₂ is a single bond, C₁₋₆ alkylene, -O-, -(C=O)-, -(C=O)NR_{g}-, or -NR_{g}(C=O)-;
R₄ is H, -OH, C₁₋₆ alkyl, -O-C₁₋₆ alkyl, C₃₋₈ cycloalkyl, 3- to 8-membered heterocycloalkyl comprising 1 to 3 heteroatoms independently selected from the group consisting of N, O, and S in a ring, -NRₕRᵢ, or halogen,
at least one H of the R₄ is each independently substituted with -OH; and
Rₑ, R_{g}, Rₕ, Rᵢ, and Rₖ are each independently H or C₁₋₆ alkyl.

4. The compound represented by formula 1, optical isomer thereof, stereoisomer thereof, solvate thereof, isotopic variant thereof, tautomer thereof, or pharmaceutically acceptable salt thereof of claim 1,
wherein in above formula 1,
A is C₆₋₁₂ aryl;
n is 2;
R₁ is each independently C₁₋₆ alkyl, -CF₂H, -CF₃, -CN, or halogen;
X₁, X₂, and X₃ are each CH;
L₁ is a single bond or -NRₑ-;
R₃ is 3- to 12-membered heterocycloalkyl comprising 1 to 3 heteroatoms independently selected from the group consisting of N, O, and S in a ring, or 3- to 8-membered heterocycloalkenyl comprising 1 to 3 heteroatoms independently selected from the group consisting of N, O, and S in a ring, in which the heterocycloalkyl comprises 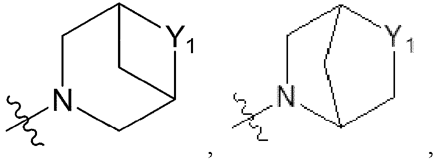 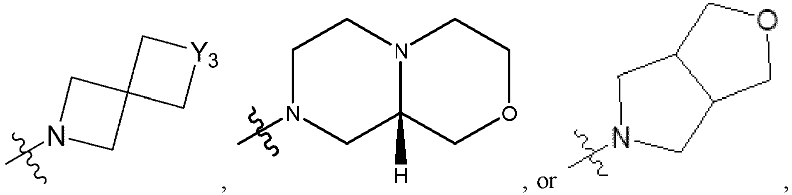 in which Y₁ is O, and Y₃ is each independently CH₂,
at least one H of the R₃ is each independently substituted with C₁₋₆ alkyl, -OH, or -L₂-R₄;
L₂ is a single bond, -(C=O)-, or -NR_{g}(C=O)-;
R₄ is H, -OH, C₁₋₆ alkyl, C₃₋₈ cycloalkyl, 3- to 8-membered heterocycloalkyl comprising 1 to 3 heteroatoms independently selected from the group consisting of N, O, and S in a ring, or - NRₕRᵢ,
at least one H of the R₄ is each independently substituted with -OH; and
Rₑ, R_{g}, Rₕ, and Rᵢ are each independently H or C₁₋₆ alkyl.

5. A compound described in a table below, optical isomer thereof, stereoisomer thereof, solvate thereof, isotopic variant thereof, tautomer thereof, or pharmaceutically acceptable salt thereof:
| No. | Compound Structure | No. | Compound Structure |
|---|---|---|---|
| 1 | 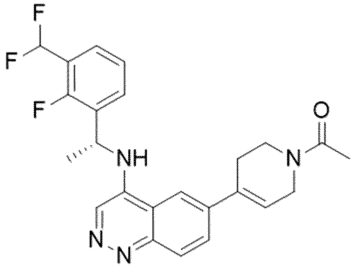 | 2 | 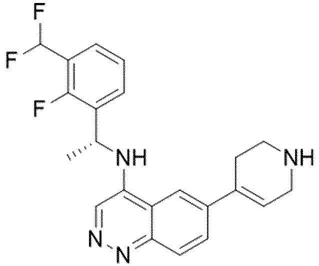 |
| 3 | 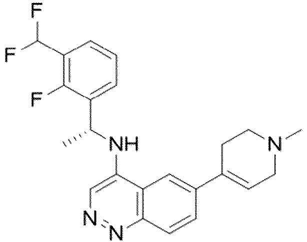 | 4 | 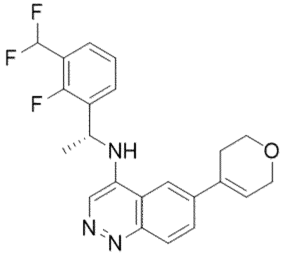 |
| 5 | 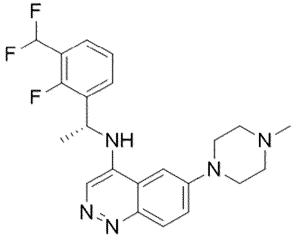 | 6 | 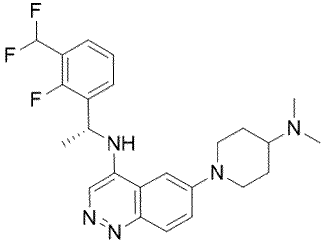 |
| 7 | 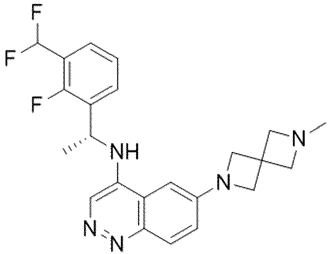 | 8 | 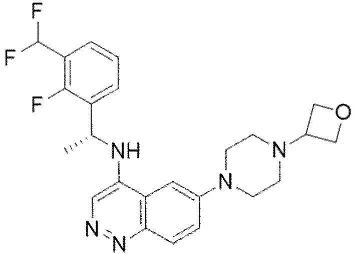 |
| 9 | 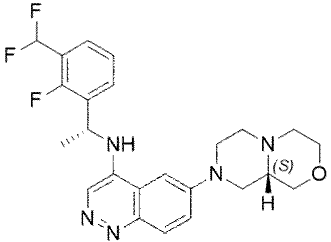 | 10 | 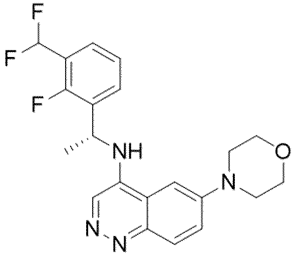 |
| 11 | 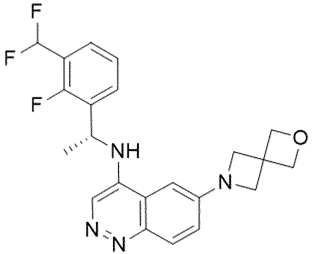 | 12 | 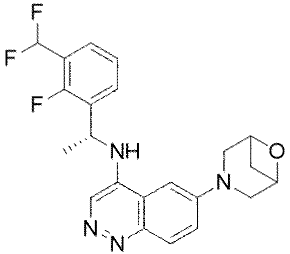 |
| 13 | 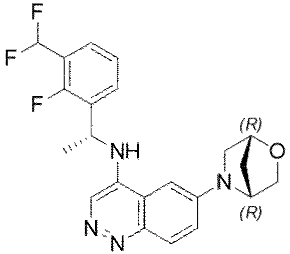 | 14 | 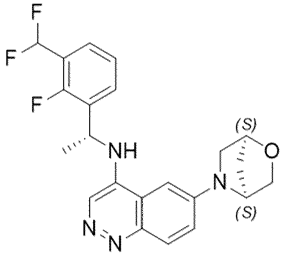 |
| 15 | 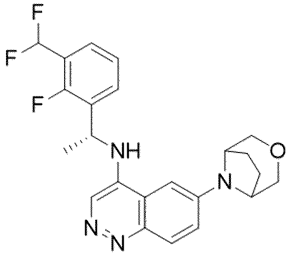 | 16 | 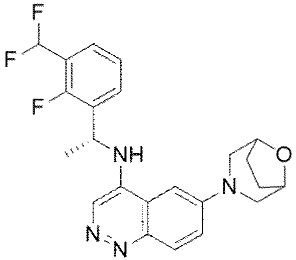 |
| 17 | 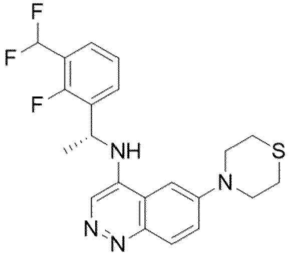 | 18 | 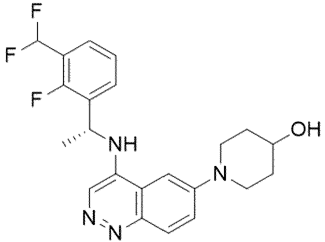 |
| 19 | 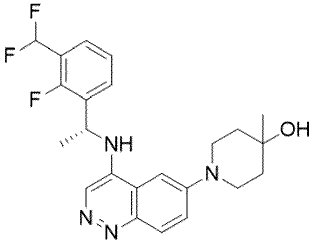 | 20 | 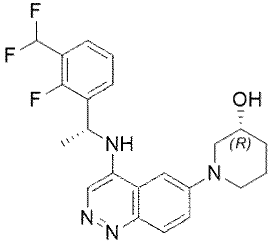 |
| 21 | 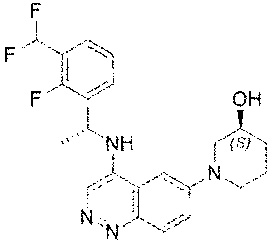 | 22 | 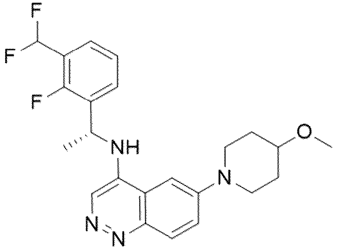 |
| 23 | 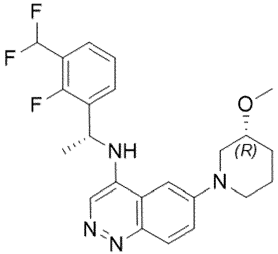 | 24 | 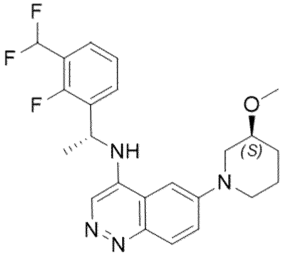 |
| 25 | 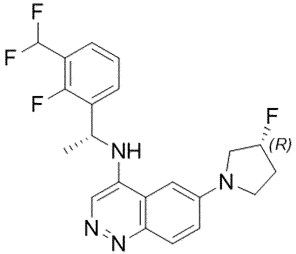 | 26 | 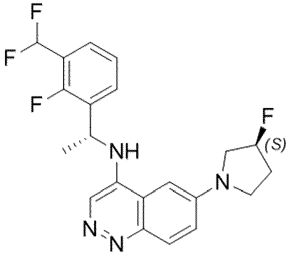 |
| 27 | 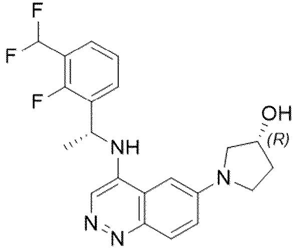 | 28 | 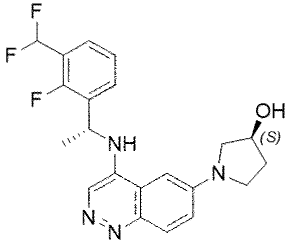 |
| 29 | 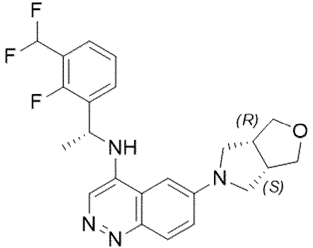 | 30 | 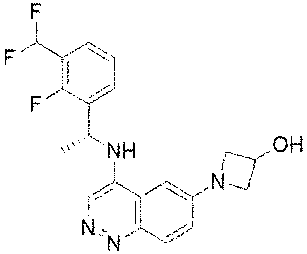 |
| 31 | 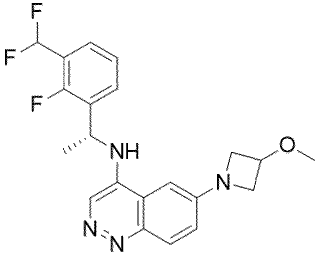 | 32 | 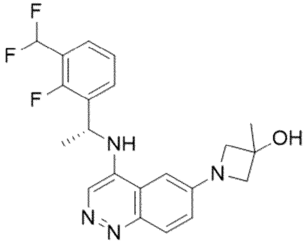 |
| 33 | 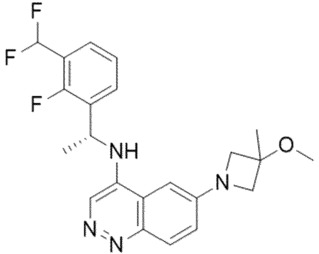 | 34 | 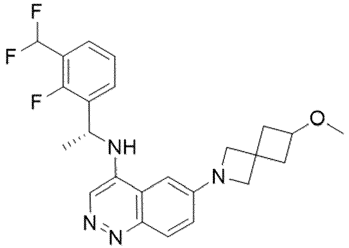 |
| 35 | 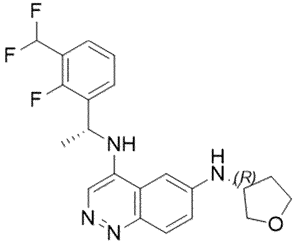 | 36 | 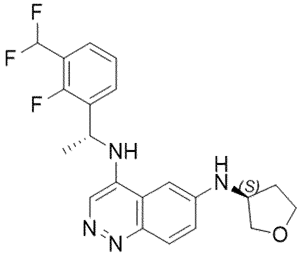 |
| 37 | 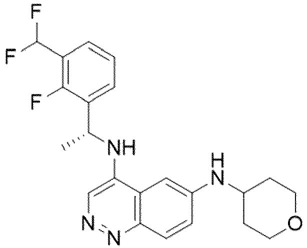 | 38 | 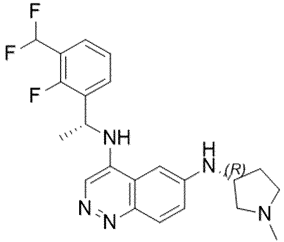 |
| 39 | 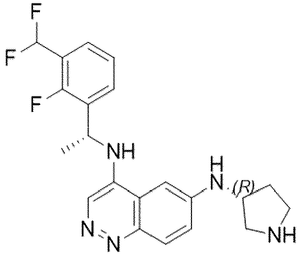 | 40 | 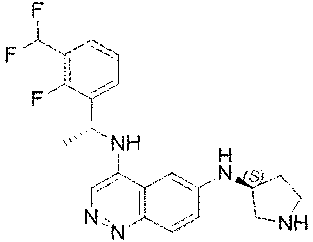 |
| 41 | 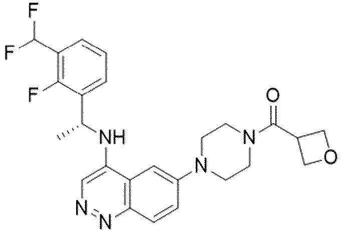 | 42 | 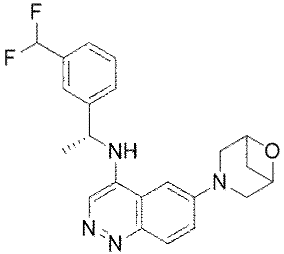 |
| 43 | 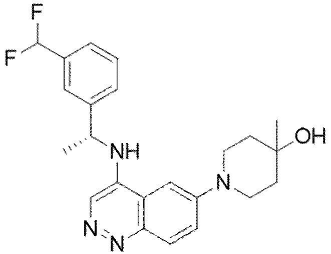 | 44 | 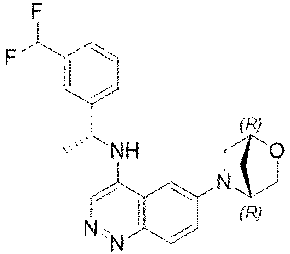 |
| 45 | 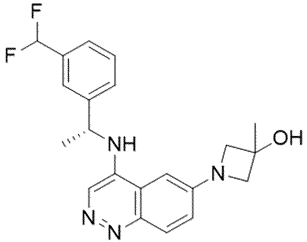 | 46 | 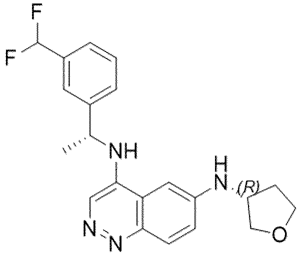 |
| 47 | 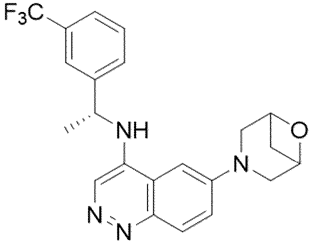 | 48 | 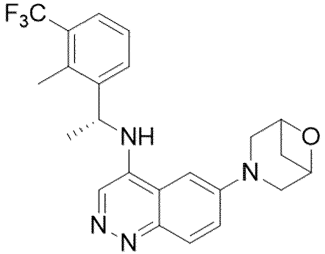 |
| 49 | 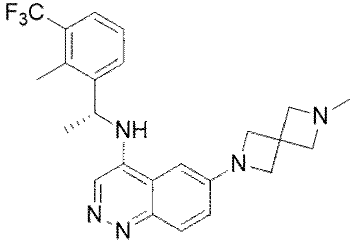 | 50 | 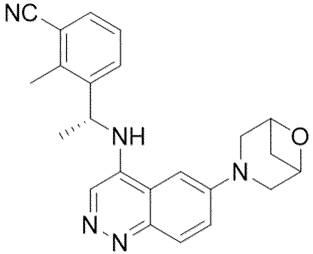 |
| 51 | 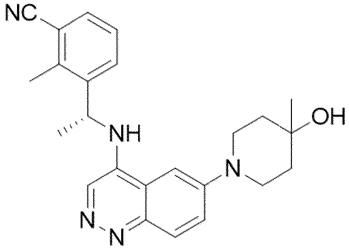 | 52 | 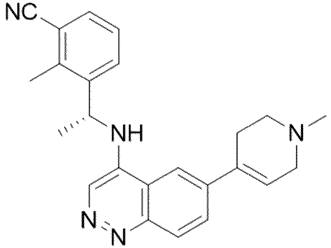 |
| 53 | 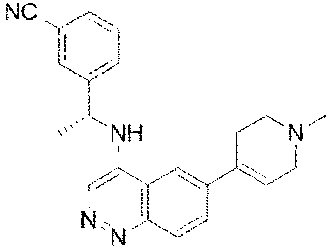 | 54 | 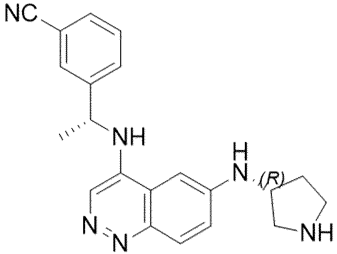 |
| 55 | 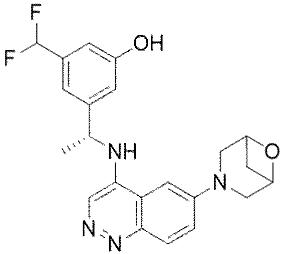 | 56 | 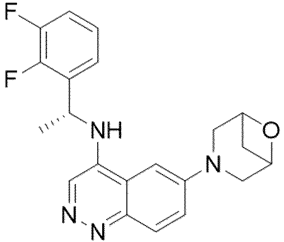 |
| 57 | 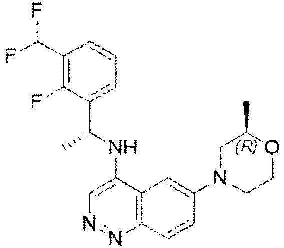 | 58 | 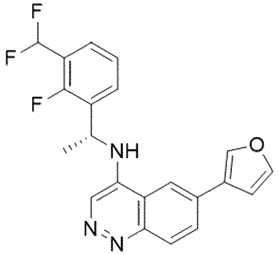 |
| 59 | 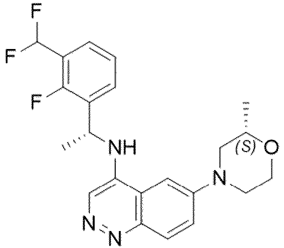 | 60 | 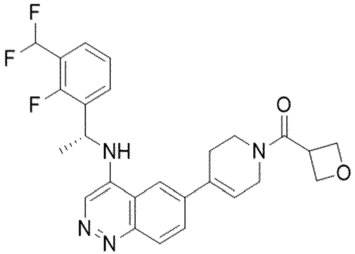 |
| 61 | 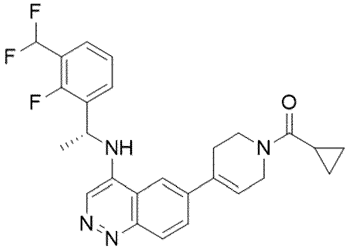 | 62 | 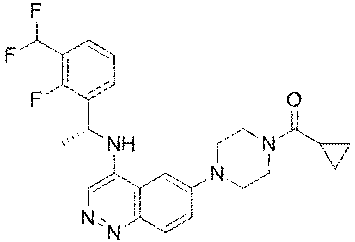 |
| 63 | 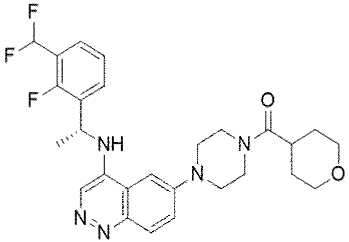 | 64 | 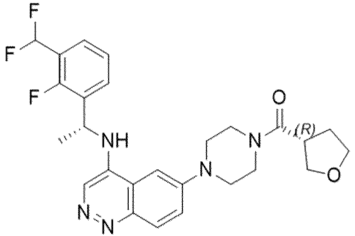 |
| 65 | 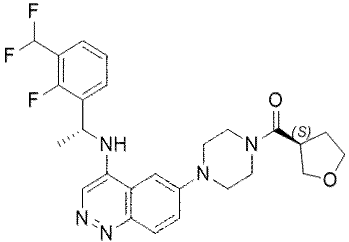 | 66 | 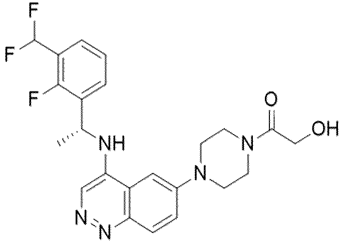 |
| 67 | 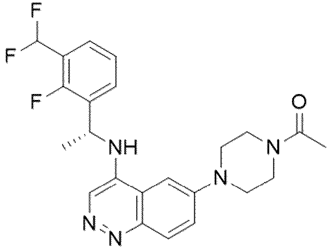 | 68 | 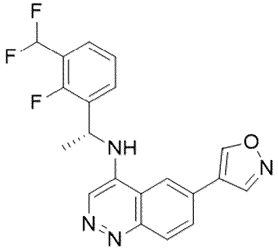 |
| 69 | 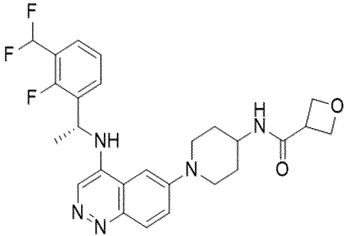 | 70 | 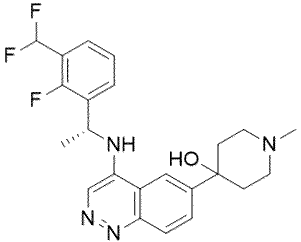 |
| 71 | 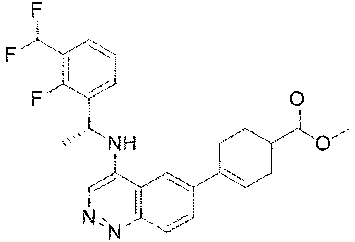 | 72 | 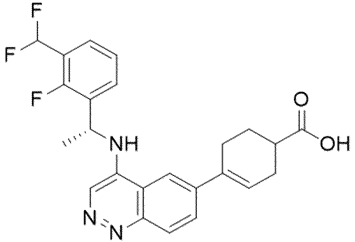 |
| 73 | 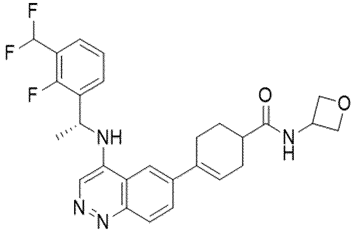 | 74 | 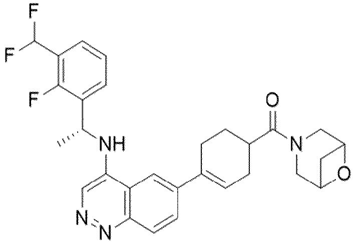 |
| 75 | 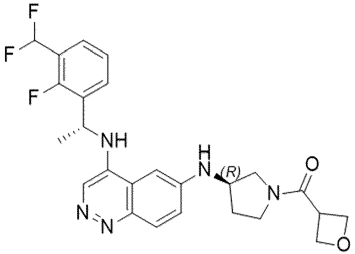 | 76 | 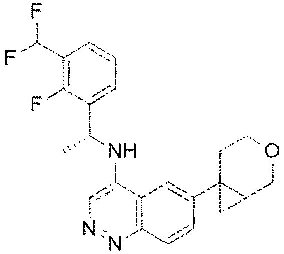 |

6. A pharmaceutical composition comprising a compound according to any one of claims 1 to 5, optical isomer thereof, stereoisomer thereof, solvate thereof, isotopic variant thereof, tautomer thereof, or pharmaceutically acceptable salt thereof for preventing, ameliorating, or treating disease related to SOS1 activity.

7. The pharmaceutical composition of claim 6, wherein the disease related to SOS1 activity comprise cancer.

8. The pharmaceutical composition of claim 7, wherein cancer comprises at least one disease selected from lung cancer, pancreatic cancer, gastric cancer, myelodysplastic syndrome, blood cancer, leukemia comprising acute lymphocytic leukemia (ALL) and acute myeloid leukemia (AML), adrenal cancer, anal cancer, basosquamous cell skin cancer, bile duct cancer, bladder cancer, bone cancer, cerebrospinal tumor, breast cancer, cervical cancer, chronic lymphocytic leukemia (CLL), chronic myeloid leukemia (CML), chronic myelomonocytic leukemia (CMML), colorectal cancer, endometrial cancer, esophageal cancer, Ewing family of tumors, eye cancer, gallbladder cancer, gastrointestinal carcinoid tumor, gastrointestinal stromal tumor (GIST), gestational choriocarcinoma, glioma, Hodgkin's lymphoma, Kaposi's sarcoma, renal cancer, hypopharyngeal cancer, liver cancer, lung carcinoma, lymphoma comprising cutaneous T-cell lymphoma, malignant mesothelioma, melanoma skin cancer, Merkel cell skin cancer, multiple myeloma, nasal and paranasal cancer, nasopharyngeal cancer, neuroblastoma, non-Hodgkin's lymphoma, non-small cell lung cancer, oral and oropharyngeal cancer, osteosarcoma, ovarian cancer, penile cancer, pituitary tumor, prostate cancer, retinoblastoma, rhabdomyosarcoma, salivary gland cancer, skin cancer, small cell lung cancer, small intestine cancer, soft tissue sarcoma, gastric cancer, testicular cancer, thymic cancer, thyroid carcinoma comprising anaplastic thyroid carcinoma, uterine sarcoma, vaginal cancer, vulval cancer, Waldenstrom macroglobulinemia, Wilms tumor, embryo rhabdomyosarcoma, Sertoli cell testis tumor, skin granular cell tumor, and lung adenocarcinoma.

9. A method for preventing, ameliorating, or treating disease related to SOS1 activity, the method comprising administering a compound according to any one of claims 1 to 5, optical isomer thereof, stereoisomer thereof, solvate thereof, isotopic variant thereof, tautomer thereof, or pharmaceutically acceptable salt thereof, or a pharmaceutical composition comprising same into an individual in need thereof.

10. The method of claim 9, wherein the disease related to SOS1 activity comprises cancer.

11. A use of a compound according to any one of claims 1 to 5, optical isomer thereof, stereoisomer thereof, solvate thereof, isotopic variant thereof, tautomer thereof, or pharmaceutically acceptable salt thereof, or a pharmaceutical composition comprising same for preventing, ameliorating, or treating disease related to SOS1 activity.

12. A use of a compound according to any one of claims 1 to 5, optical isomer thereof, stereoisomer thereof, solvate thereof, isotopic variant thereof, tautomer thereof, or pharmaceutically acceptable salt thereof, or a pharmaceutical composition comprising same in the preparation of a medicament for preventing, ameliorating, or treating disease related to SOS1 activity.

13. The use of claim 11 or 12, wherein the disease related to SOS1 activity comprises cancer.
